# EUROPEAN PATENT APPLICATION

(11) **EP 1 359 139 A1**
(43) Date of publication of application: **05.11.2003**
(21) Application number: 02715768.4
(22) Date of filing: 17.01.2002
(51) Int. Cl.: C07C 217/80, C07C 211/54, C07C 229/52, C07C 233/43, C07C 311/08, C07C 311/09, C07C 311/51, C07C 311/48, C07C 323/25, C07C 307/10, C07D 209/08, C07D 307/52, C07D 235/26, C07D 241/42, C07D 235/06, C07D 213/36, C07D 209/38, C07D 209/42, C07D 491/113

(54) **TERPHENYL COMPOUNDS BEARING SUBSTITUTED AMINO GROUPS**

(30) Priority: 18.01.2001 JP 2001010057
(71) Applicant: SHIONOGI & CO., LTD., Osaka-shi, Osaka 541-0045 (JP)
(72) Inventor: TSURI, Tatsuo, c/o Shionogi & Co., Ltd., Osaka-shi, Osaka 553-0002 (JP); TAKECHI, Shozo, c/o Shionogi & Co., Ltd., Osaka-shi, Osaka 553-0002 (JP); HORIBE, Isao, Hirakata-shi, Osaka 573-0124 (JP)
(74) Representative: VOSSIUS & PARTNER
(86) International application number: JP0200259
(87) International publication number: WO02057216

(57) **Abstract**

The present invention provides a compound of the formula (I): 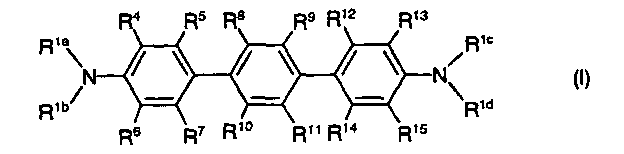 wherein R^{1a}, R^{1b}, R^{1c} and R^{1d} are each independently hydrogen, lower alkyl, lower alkenyl, lower alkynyl, lower alkylsulfonyl or cycloalkyl and the like, R⁴ to R¹⁵ are hydrogen, halogen, hydroxy, lower alkyl, lower alkoxy, carboxy, lower alkoxycarbonyl, amino, nitro, lower alkylsulfonyl, lower alkylsulfonyloxy and the like,
which has superior immunosuppressive and /or anti-allergic activity and is useful for a pharmaceutical composition.

## Description

### Technical Field

The present invention relates to a novel aminoterphenyl compound and an IgE production suppressive composition, a Th2 differentiation inhibitory composition, an anti-allergic composition and/or an immunosuppressive composition each comprising the same.

### Background Art

A serious problem of a transplantation of a tissue or an organ which is frequently performed in recent years is a rejection symptom for excluding a transplanted part after an operation. Prevention of the rejection symptom is very important for a success of the transplantation.

Various immunosuppressants such as azathioprine, corticoid, Cyclosporin A, Tacrolimus and the like are developed and come into practical use for prevention and a treatment of a rejection symptom against a transplantation of an organ or a tissue or a graft-versus-host reaction which is caused by a bone marrow transplantation. But they are not so satisfactory in view of their effects and side effects.

Allergic diseases such as atopic dermatitis, allergic rhinitis, bronchial asthma, allergic conjunctivitis and the like tend to globally increase in recent years and become serious problems. The conventional antiinflammatory agents are suppressants of releasing chemical mediators from mast cells, receptor inhibitors of the released chemical mediators, suppressants of allergic inflammation response or the like. All of these are agents for symptomatic therapy and are not fundamental therapeutic agents for allergic diseases.

Although the compounds having a similar structure to a compound of the present invention and exhibiting an immunosuppressive or anti-allergic effect are described in WO98/04508, WO99/38829 and the like, more efficacious and safety medicaments are desired.

### Disclosure of Invention

The object of the present invention is to provide a novel aminoterphenyl compound, an IgE production suppressive composition, a Th2 differentiation inhibitory composition, an anti-allergic composition and/or an immunosuppressive composition each containing the same.

The present invention provides
[1] A compound of the formula (I): 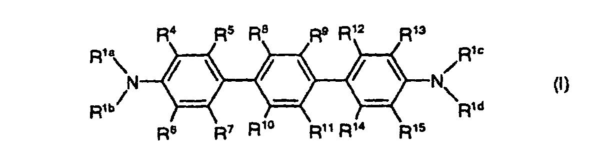 wherein R^{1a}, R^{1b}, R^{1c} and R^{1d} are each independently
   hydrogen;
   lower alkyl optionally substituted with at least one group selected from the group of (i)hydroxy; (ii)lower alkylthio; (iii)lower alkoxy; (iv)cycloalkyl; (v)lower alkoxycarbonyl; (vi)optionally substituted heterocyclyl wherein the substituents are lower alkyl; (vii)optionally substituted aryl wherein the substituents are lower alkoxycarbonyl and/or carboxy;
   (viii)carboxy; and
   (ix)amino;
   lower alkenyl;
   lower alkynyl;
   acyl optionally substituted with halogen;
   lower alkylsulfonyl optionally substituted with halogen;
   or cycloalkyl,
   R⁴ and R^{1a} taken together may form optionally substituted C2 or C3 alkenylene or optionally substituted C2 or C3 alkylene,
   R¹³ and R^{1c} taken together may form optionally substituted C2 or C3 alkenylene or optionally substituted C2 or C3 alkylene,
   R⁸ and R⁹ taken together may form optionally substituted C2 or C3 alkenylene diamino or optionally substituted C2 or C3 alkylene diamino,
   R¹⁰ and R¹¹ taken together may form optionally substituted C2 or C3 alkenylene diamino or optionally substituted C2 or C3 alkylene diamino, and
   R⁴, R⁵, R⁶, R⁷, R⁸, R⁹, R¹⁰, R¹¹, R¹², R¹³, R¹⁴ and R¹⁵ are each independently hydrogen, halogen, hydroxy, optionally substituted lower alkyl, optionally substituted lower alkoxy, optionally substituted lower alkenyl, optionally substituted lower alkenyloxy, optionally substituted cycloalkyloxy, optionally substituted acyl, optionally substituted acyloxy, carboxy, optionally substituted lower alkoxycarbonyl, optionally substituted lower alkenyloxycarbonyl, optionally substituted lower alkylthio, optionally substituted lower alkenylthio, optionally substituted amino, optionally substituted carbamoyl, optionally substituted sulfamoyl, guanidino, nitro, cyano, optionally substituted lower alkylsulfonyl, optionally substituted lower alkylsulfonyloxy, optionally substituted arylsulfonyl, optionally substituted lower alkylsulfinyl or optionally substituted arylsulfonyloxy,
   a prodrug, a pharmaceutically acceptable salt or solvate thereof (hereinafter referred to as Compound (I)).
[2] The compound as described in [1] wherein R^{1a} is lower alkylsulfonyl or 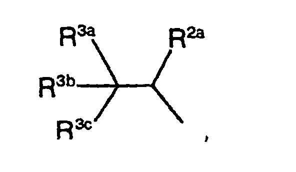 R^{1c} is lower alkylsulfonyl or 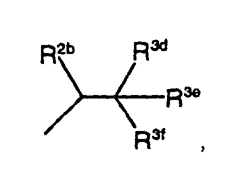 R^{1b} and R^{1d} are each independently hydrogen, lower alkyl or acyl,
   R^{2a} and R^{2b} are each independently hydrogen or lower alkyl,
   R^{3a} and R^{3d} are each independently hydrogen, lower alkyl or lower alkenyl,
   R^{3b}, R^{3e}, R^{3c} and R^{3f} are each independently hydrogen or lower alkyl,
   R^{3a} and R^{2a} or R^{3b} taken together may form optionally substituted alkylene, and
   R^{3d} and R^{2b} or R^{3e} taken together may form optionally substituted alkylene excluding N⁴,N⁴"-diisopropyl -2',3',5',6'-tetramethyl -[1,1';4',1"]-terphenyl -4,4"-diamine, N⁴,N⁴"-diisopropyl -2',6'-dimethoxy-3',5'-dimethyl -[1,1';4',1"]-terphenyl -4,4"-diamine, N⁴,N⁴"-diisopropyl -2'-methoxy-3',5',6'-trimethyl -[1,1';4',1"]-terphenyl -4,4"-diamine, N⁴,N⁴"-diisopropyl -2',5'-dimethyl [1,1';4',1"]-terphenyl "4,4"-diamine, N⁴,N⁴"-diisopropyl-2'-hydroxy-3',5',6'-trimethyl -[1,1';4',1"]-terphenyl -4,4"-diamine and N⁴,N⁴"-diisopropyl -2',3',5'-trimethyl -[1,1';4',1"]-terphenyl -4,4"-diamine, a prodrug, a pharmaceutically acceptable salt or solvate thereof.
[3] The compound as described in [1] or [2] wherein at least one of R⁸, R⁹, R¹⁰ and R¹¹ is hydroxy, optionally substituted lower alkyl, optionally substituted lower alkoxy, optionally substituted lower alkenyl, optionally substituted lower alkenyloxy, optionally substituted cycloalkyloxy, optionally substituted acyl, optionally substituted acyloxy, carboxy, optionally substituted lower alkoxycarbonyl, optionally substituted lower alkenyloxycarbonyl, optionally substituted lower alkylthio, optionally substituted lower alkenylthio, optionally substituted amino, optionally substituted carbamoyl, optionally substituted sulfamoyl, guanidino, nitro, cyano, optionally substituted lower alkylsulfonyl, optionally substituted lower alkylsulfonyloxy, optionally substituted arylsulfonyl, optionally substituted lower alkylsulfinyl or optionally substituted arylsulfonyloxy, a prodrug, a pharmaceutically acceptable salt or solvate thereof.
[4] The compound as described in any one of [1] to [3] wherein at least one of R^{1b} and R^{1d} is hydrogen, a prodrug, a pharmaceutically acceptable salt or solvate thereof.
[5] The compound as described in any one of claims [1] to [3] wherein both of R^{1b} and R^{1d} are hydrogen, a prodrug, a pharmaceutically acceptable salt or solvate thereof.
[6] The compound as described in any one of [2] to [5] wherein R^{2a} and R^{2b} are each independently hydrogen or C1 to C3 alkyl, a prodrug, a pharmaceutically acceptable salt or solvate thereof.
[7] The compound as described in any one of [2] to [6] wherein R^{3a} and R^{3d} are each independently hydrogen or C1 to C3 alkyl, a prodrug, a pharmaceutically acceptable salt or solvate thereof.
[8] The compound as described in any one of [2] to [6] wherein R^{3a} and R^{2a} or R^{3b} taken together may form unsubstituted alkylene and/or R^{3d} and R^{2b} or R^{3e} taken together may form unsubstituted alkylene, a prodrug, a pharmaceutically acceptable salt or solvate thereof.
[9] The compound as described in any one of claims [2] to [8] wherein R^{3b} and R^{3e} are each independently hydrogen or C1 to C3 alkyl, a prodrug, a pharmaceutically acceptable salt or solvate thereof.
[10] The compound as described in any one of [2] to [9] wherein R^{3c} and R^{3f} are each independently hydrogen or C1 to C3 alkyl, a prodrug, a pharmaceutically acceptable salt or solvate thereof.
[11] The compound as claimed in [2] or [3] wherein both of R^{1b} and R^{1d} are hydrogen, R^{2a} R^{2b}, R^{3a}, R^{3d}, R^{3b}, R^{3e}, R^{3c} and R^{3f} are each independently hydrogen or simultaneously C1 to C3 alkyl, and
   R^{3a} and R^{2a} or R^{3b} taken together may form unsubstituted alkylene and/or R^{3a} and R^{2b} or R^{3e} taken together may form unsubstituted alkylene, a prodrug, a pharmaceutically acceptable salt or solvate thereof.
[12] The compound as described in [2] or [3] wherein both of R^{1b} and R^{1d} are hydrogen, and R^{2a}, R^{2b}, R^{3a}, R^{3d}, R^{3b}, R^{3e}, R^{3c} and R^{3f} are each independently hydrogen or methyl, a prodrug, a pharmaceutically acceptable salt or solvate thereof.
[13] The compound as described in any one of [1] to [3] wherein both of R^{1a} and R^{1c} are isopropyl, and both R^{1b} and R^{1d} are hydrogen, a prodrug, a pharmaceutically acceptable salt or solvate thereof.
[14] The compound as described in any one of [1] to [12] wherein R⁴ and R⁵ are each independently hydrogen, halogen, lower alkyl or lower alkoxy, and both of R⁶ and R⁷ are hydrogen, a prodrug, a pharmaceutically acceptable salt or solvate thereof.
[15] The compound as described in any one of [1] and [3] to [14] wherein 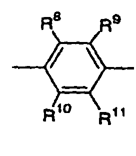 is 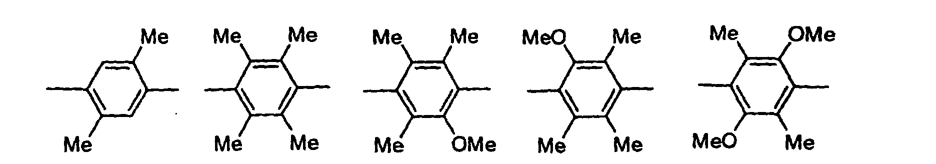 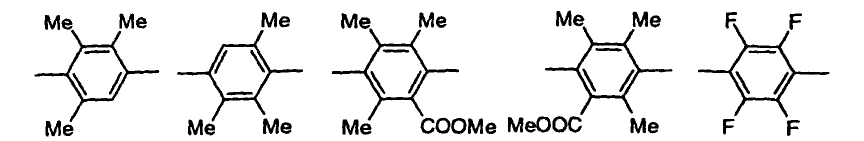 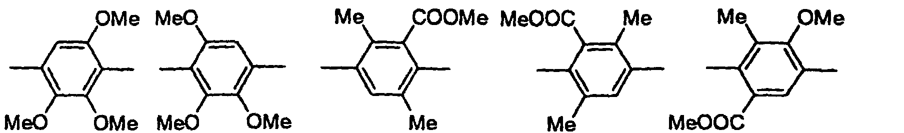 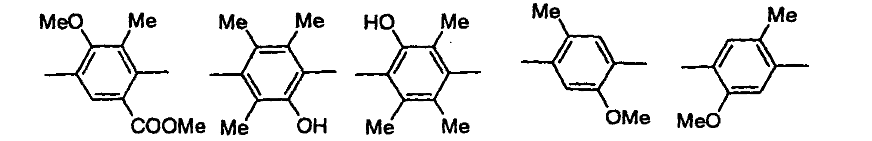 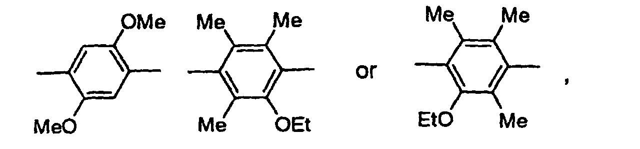 a prodrug, a pharmaceutically acceptable salt or solvate thereof.
[16] The compound as described in any one of [1] and [3] to [14] wherein 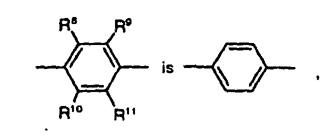 a prodrug, a pharmaceutically acceptable salt or solvate thereof.
[17] The compound as described in any one of [1] and [3] to [14] wherein 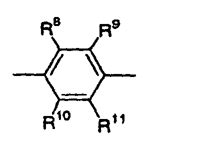 is 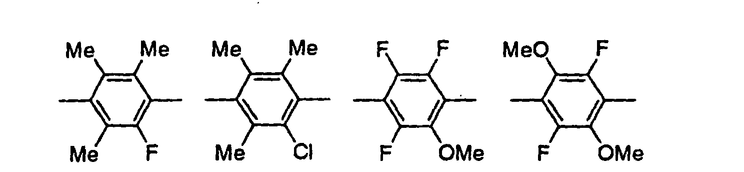 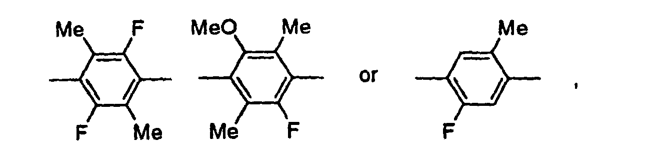 a prodrug, a pharmaceutically acceptable salt or solvate thereof.
[18] The compound as described in any one of [1] to [17] wherein both of R¹² and R¹³ are hydrogen, and R¹⁴ and R¹⁵ are each independently hydrogen, halogen, lower alkyl or lower alkoxy, a prodrug, a pharmaceutically acceptable salt or solvate thereof.
[19] The compound as described in [1] wherein R^{1a} and R^{1c} are each independently C1 to C3 alkyl optionally substituted with C3 to C6 cycloalkyl, R^{1b}, R^{1d}, R⁴ to R⁷ and R¹² to R¹⁵ are hydrogen, and 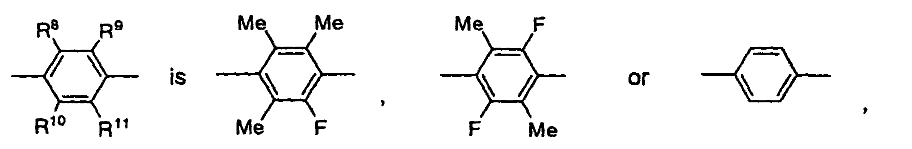 a prodrug, a pharmaceutically acceptable salt or solvate thereof.
[20] A pharmaceutical composition comprising a compound, a prodrug, a pharmaceutically acceptable salt or solvate thereof of any one of [1] to [19].
[21] An IgE production suppressive composition comprising a compound, a prodrug, a pharmaceutically acceptable salt or solvate thereof of any one of [1] to [19].
[22] A Th2 differentiation inhibitory composition comprising a compound, a prodrug, a pharmaceutically acceptable salt or solvate thereof of any one of [1] to [19].
[23] An anti-allergic composition comprising a compound, a prodrug, a pharmaceutically acceptable salt or solvate thereof of any one of [1] to [19].
[24] An immunosuppressive composition comprising a compound, a prodrug, a pharmaceutically acceptable salt or solvate thereof of any one of [1] to [19].
   As another embodiment, the present invention provides a method for suppressing IgE production, a method for inhibiting Th2 differentiation, a method for suppressing an immune reaction, or a method for treating and/or preventing an anti-allergic disease each comprising administering a compound (I). As other embodiments, the present invention provides use of compound (I) for preparing a medicine for suppressing IgE production, for inhibiting Th2 differentiation, for suppressing an immune reaction, and for treating and/or preventing an allergic disease.
   For example, the following inventions are included in the present invention.
[25] A method for suppressing IgE production comprising administering a compound, a prodrug, a pharmaceutically acceptable salt or solvate thereof of any one of [1] to [19].
[26] A method for inhibiting Th2 differentiation comprising administering a compound, a prodrug, a pharmaceutically acceptable salt or solvate thereof of any one of [1] to [19].
[27] A method for treating and/or preventing an allergic disease comprising administering a compound, a prodrug, a pharmaceutically acceptable salt or solvate thereof of any one of [1] to [19].
[28] A method for suppressing an immune reaction comprising administering a compound, a prodrug, a pharmaceutically acceptable salt or solvate thereof of any one of [1] to [19].
[29] Use of a compound, a prodrug, a pharmaceutically acceptable salt or solvate thereof of any one of [1] to [19] for preparing a medicine for suppressing IgE production.
[30] Use of a compound, a prodrug, a pharmaceutically acceptable salt or solvate thereof of any one of [1] to [19] for preparing a medicine for inhibiting Th2 differentiation.
[31] Use of a compound, a prodrug, a pharmaceutically acceptable salt or solvate thereof of any one of [1] to [19] for preparing an anti-allergic agent.
[32] Use of a compound, a prodrug, a pharmaceutically acceptable salt or solvate thereof of any one of [1] to [19] for preparing an immunosuppressive agent.

In the present specification, the term "halogen" includes fluorine, chlorine, bromine and iodine. Fluorine or chlorine is preferable.

The term "lower alkyl" includes straight or branched chain alkyl having 1 to 10 carbon atoms. For example, included are methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl, n-decyl and the like.

The term "lower alkyl" as R^{1b} and R^{1d} includes alkyl having 2 to 6 carbon atoms, preferably 2 to 3 carbon atoms. The term "lower alkyl" as the other symbols includes alkyl having 1 to 6 carbon atoms, preferably 1 to 3 carbon atoms and most preferably methyl.

As substituents of "optionally substituted lower alkyl", exemplified are halogen; hydroxy; lower alkoxy optionally substituted with lower alkoxy; acyl; acyloxy; carboxy; lower alkoxycarbonyl; mercapt; lower alkylthio; amino optionally substituted with hydroxy, lower alkyl or optionally substituted acyl; imino optionally substituted with hydroxy, lower alkoxy, carboxy(lower)alkoxy, aryl(lower)alkoxy or heterocycle; hydrazono optionally substituted with carbamoyl or lower alkoxycarbonyl; carbamoyl optionally substituted with lower alkyl or amino; thiocarbamoyl optionally substituted with lower alkyl; cycloalkyl optionally substituted with lower alkyl or lower alkoxy; cycloalkenyl optionally substituted with lower alkyl; cyano; phenyl optionally substituted with at least one substituent selected from the group of hydroxy, lower alkyl, carboxy, lower alkoxycarbonyl and lower alkoxy; 5- or 6-membered heterocycle which may be substituted with lower alkyl and may fuse with benzene ring; and the like. The lower alkyl may be substituted with one or more of these substituents at any possible positions. Unsubstituted lower alkyl is preferable.

The lower alkyl part of "lower alkoxy" is the same as the above "lower alkyl".

As substituents for "optionally substituted lower alkoxy", exemplified are halogen; hydroxy; lower alkoxy optionally substituted with acyloxy; acyl; acyloxy optionally substituted with hydroxy or carboxy; carboxy; lower alkoxycarbonyl; lower alkylthio; amino optionally substituted with lower alkyl; phenyl optionally substituted with lower alkyl or lower alkoxy; heterocycle; heterocyclylcarbonyloxy and the like. Unsubstituted lower alkoxy is preferable.

The lower alkyl parts of "lower alkoxycarbonyl", "lower alkylsulfonyl", "lower alkylsulfonyloxy", "lower alkylsulfinyl", "lower alkylthio", "carboxy(lower)alkoxy", "aryl(lower)alkoxy", "acyloxy(lower)alkoxy", "cycloalkyl(lower)alkyl", "heterocyclyl(lower)alkyl" and "lower alkylenedioxy" are the same as the above "lower alkyl". Substituents for "optionally substituted lower alkoxycarbonyl", "optionally substituted lower alkylsulfonyl", "optionally substituted lower alkylsulfonyloxy", "optionally substituted lower alkylsulfinyl" and "optionally substituted lower alkylthio" are the same as those for the above "optionally substituted lower alkoxy".

The term "lower alkenyl" includes straight or branched chain alkenyl of 2 to 10 carbon atoms, preferably 2 to 8 carbon atoms, more preferably 3 to 6 carbon atoms having at least one double bond at any possible positions. For example, included are vinyl, propenyl such as 2-propenyl and the like, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl and the like. Substituents for "optionally substituted lower alkenyl" are the same as those for the above "optionally substituted lower alkoxy". Unsubstituted alkenyl is preferable.

The lower alkenyl parts of "lower alkenyloxy", "lower alkenyloxythio" and "lower alkenyloxycarbonyl" are the same as the above "lower alkenyl". Substituents for "optionally substituted lower alkenyloxy", "optionally substituted lower alkenyloxycarbonyl" and "optionally substituted lower alkenylthio" are the same as those for the above "optionally substituted lower alkoxy".

The term "lower alkynyl" includes straight or branched chain alkynyl having 2 to 10 carbon atoms, preferably 2 to 8 carbon atoms, and more preferably 3 to 6 carbon atoms and is exemplified by ethynyl, propynyl such as 2-propynyl, butynyl such as 2-butynyl, pentynyl, hexynyl, heptynyl, octynyl, nonynyl, decynyl and the like. These have at least one triple bond and may have some double bonds at any possible positions.

The lower alkynyl parts in "lower alkynyloxy" and "lower alkynylthio" are the same as the above "lower alkynyl".

The term "acyl" includes straight or branched chain aliphatic acyl having 1 to 10 carbon atoms, preferably 1 to 6 carbon atoms, more preferably 1 to 4 carbon atoms, cyclic aliphatic acyl having 4 to 9 carbon atoms, preferably 4 to 7 carbon atoms and aroyl. For example, formyl, acetyl, propionyl, butyryl, isobutyryl, valeryl, pivaloyl, hexanoyl, acryloyl, propioloyl, methacryloyl, crotonoyl, cyclopropylcarbonyl, cyclohexylcarbonyl, cyclooctylcarbonyl, benzoyl and the like are included and acyl is preferable.

Substituents for "optionally substituted acyl" are the same as those for the above "optionally substituted lower alkoxy" and aroyl may further be substituted with lower alkyl. Unsubstituted acyl is preferable.

The acyl parts of "acyloxy", "acyloxy(lower)alkoxy", "hydroxy-substituted acyloxy" and "carboxy-substituted acyloxy" are the same as the above "acyl". Substituents for "optionally substituted acyloxy" are the same as those for the above "optionally substituted acyl".

The term "cycloalkyl" includes carbocycle having 3 to 6 carbon atoms and cyclopropyl, cyclobutyl, cyclopentyl, cyclohexyl and the like.

The cycloalkyl parts for "cycloalkyloxy" and "cycloalkyl(lower)alkyl" are the same as the above "cycloalkyl".

Examples of substituents for "optionally substituted cycloalkoxy" are lower alkyl, halogen, hydroxy, carboxy, lower alkoxycarbonyl, lower alkoxy, lower alkylenedioxy, imino optionally substituted with lower alkoxy, aryl, heterocyclyl and the like. Cycloalkoxy may be substituted with one or more of these substituents.

The term "cycloalkenyl" includes the group having at least one double bond at any possible positions in the above cycloalkyl and is exemplified by cyclopropenyl, cyclobutenyl, cyclopentenyl, cyclohexenyl, cyclohexadienyl and the like.

As substituents for "optionally substituted amino", exemplified are optionally substituted lower alkyl {wherein the substituents are lower alkoxy, cycloalkyl, optionally substituted amino (wherein the substituents are aroyl optionally substituted with acyloxy(lower)alkoxy), optionally substituted aryl (wherein the substituents are lower alkyl, lower alkoxy, carboxy or lower alkoxycarbonyl) or heterocycle}; lower alkenyl; lower alkynyl; cycloalkyl; aryl optionally substituted with lower alkyl, carboxy, acyl, lower alkoxycarbonyl; sulfamoyl optionally substituted with lower alkyl; optionally substituted lower alkoxycarbonyl (the substituents are halogen, acyloxy, acyloxy substituted with hydroxy, acyloxy substituted with carboxy or heterocyclylcarbonyloxy or the like); lower alkylsulfonyl and the like. Preferable substituents are lower alkyl, acyl or lower alkylsulfonyl.

The term "optionally substituted carbamoyl" includes carbamoyl optionally substituted with lower alkyl, lower alkenyl, lower alkynyl or the like.

The term "optionally substituted sulfamoyl" includes sulfamoyl optionally substituted with lower alkyl, lower alkenyl, lower alkynyl or the like.

The term "aryl" includes phenyl, naphthyl, anthryl, phenanthryl, indenyl and the like and phenyl is preferable.

The aryl parts for "arylsulfonyl", "arylsulfonyloxy" and "aryl(lower)alkoxy" are the same as the above "aryl", and phenyl is preferable.

Examples of the substituents for "optionally substituted arylsulfony" and "optionally substituted arylsulfonyloxy" are halogen; hydroxy; lower alkyl optionally substituted with halogen or carboxy; lower alkoxy optionally substituted with halogen, aryl, heterocyclyl or lower alkoxy; lower alkenyl; lower alkynyl; cycloalkyl; lower alkenyloxy; lower alkynyloxy; cycloalkoxy; acyl; acyloxy; carboxy; lower alkoxycarbonyl; lower alkenyloxycarbonyl; lower alkylthio; lower alkynylthio; amino optionally substituted with lower alkyl, cycloalkyl(lower)alkyl, heterocyclyl(lower)alkyl, lower alkenyl, cycloalkyl, acyl optionally substituted with halogen, lower alkoxycarbonyl, or lower alkylsulfonyl; guanidino; nitro; lower alkylsulfonyl; dihydroxyborane; lower alkylsulfonyloxy optionally substituted with halogen; arylsulfonyl; arylsulfonyloxy; aryl; heterocycle and the like. The aromatic carbocycle and aryl may be substituted with these substituents at one or more of any possible positions. Preferable examples are halogen; hydroxy; lower alkyl optionally substituted with halogen; lower alkoxy optionally substituted with aryl or lower alkoxy; lower alkenyloxy; acyloxy; lower alkylthio; amino optionally substituted with lower alkyl, lower alkenyl, acyl optionally substituted with halogen, or lower alkylsulfonyl; nitro; lower alkylsulfonyl; lower alkylsulfonyloxy optionally substituted with halogen; or arylsulfonyloxy.

The term "heterocyclyl" represents a 5- or 6-membered heterocyclic group which contains one or more of hetero atoms arbitrarily selected from the group of O, S and N. Examples are aromatic heterocyclyl such as pyrrole ring, imidazole ring, pyrazole ring, pyridine ring such as 4-pyridyl, pyridazine ring, pyrimidine ring, pyrazine ring, triazole ring, triazine ring, isoxazole ring, oxazole ring, isothiazole ring, thiazole ring, furan ring such as 2-furyl and 3-furyl, thiophene ring such as 3-thienyl or the like and aliphatic heterocycle such as tetrahydropyrane ring, dihydropyridine ring such as 1,2-dihydropyridyl, dihydropyridazine ring such as 2,3-dihydropyridazinyl, dihydropyrazine ring such as 1,2-dihydrlpyrazinyl, dioxane ring, oxathiorane ring, thiane ring, pyrolidine ring, pyrroline ring, imidazolidine ring, imidazoline ring, pyrazolidine ring, pyrazoline ring, piperidine ring, piperazine ring, morpholine ring or the like.

The heterocyclyl parts for "heterocyclylcarbonyloxy" and "heterocyclyl(lower)alkyl" are the same as the above "heterocyclyl".

The phrase "R^{3a} and R^{2a} or R^{3b} taken together may form optionally substituted alkylene" means that R3a, R2a and their adjacent carbon atoms, or R^{3a}, R^{3b} and their adjacent carbon atoms may be joined together to form optionally substituted cyclopropane, optionally substituted cyclobutane, optionally substituted cyclopentane, optionally substituted cyclohexane, optionally substituted cycloheptane and the like. The Examples of substituents are halogen, hydroxy, lower alkyl, lower alkoxy, acyl or the like.

The phrase "R^{3d} and R^{2b} or R^{3e} taken together may form optionally substituted alkylene" can be interpreted as the same manner.
The phrases "R⁴ and R^{1a} taken together may form optionally substituted C2 or C3 alkenylene" and "R¹³ and R^{1c} taken together may form optionally substituted C2 or C3 alkenylne" mean as follows, respectively: 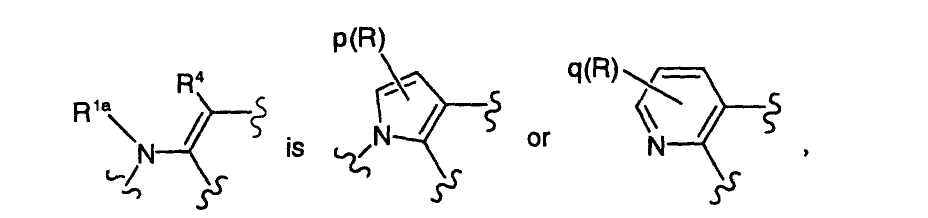 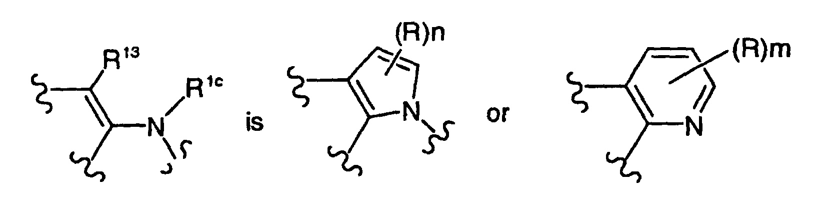 wherein R is halogen, hydroxy, lower alkyl, lower alkoxy, carboxy or lower alkoxycarbonyl, p and n are each independently an integer of 0 to 2, q and m are each independently an integer of 0 to 3.

The phrases "R⁴ and R^{1a} taken together may form optionally substituted C2 or C3 alkylene" and "R¹³ and R^{1c} taken together may form optionally substituted C2 or C3 alkylene" mean as follows, respectively: 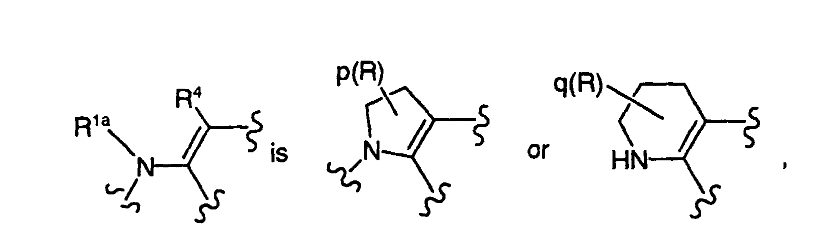 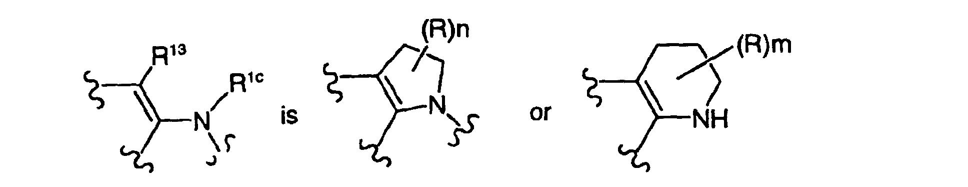 wherein R is alkylenedioxy (preferably methylenedioxy or ethylenedioxy), oxo, halogen, hydroxy, lower alkyl, lower alkoxy, carboxy or lower alkoxycarbonyl, p and n are each independently an integer of 0 to 2, q and m are each independently an integer of 0 to 3.

The phrases "R⁸ and R⁹ taken together may form optionally substituted C2 or C3 alkenylendiamino or optionally substituted C2 or C3 alkylnediamino" and "R¹⁰ and R¹¹ taken together may form optionally substituted C2 or C3 alkenylendiamino or optionally substituted C2 or C3 alkylnediamino" means as follows, respectively: 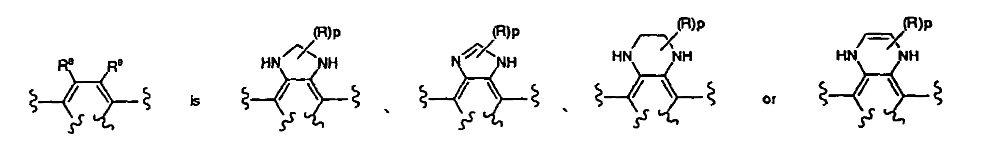 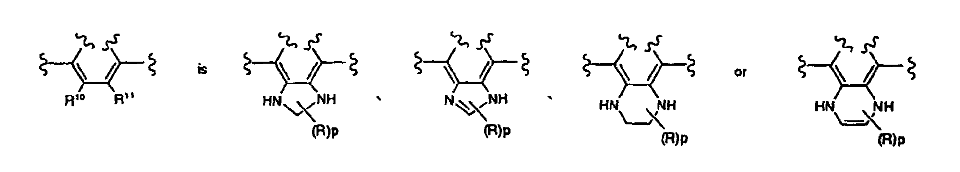 wherein R is oxo, lower alkyl, halogen, hydroxy, lower alkoxy, carboxy or lower alkoxycarbonyl, p and n are each independently an integer of 0 to 2, q and m are each independently an integer of 0 to 3, and R can be substituted on an N atom.

The term "compound (I)" includes formable and pharmaceutically acceptable salts of each compound. As "the pharmaceutically acceptable salt", exemplified are salts with mineral acids such as hydrochloric acid, sulfuric acid, nitric acid, phosphoric acid, hydrofluoric acid, hydrobromic acid and the like; salts with organic acids such as formic acid, acetic acid, tartaric acid, lactic acid, citric acid, fumaric acid, maleic acid, succinic acid and the like; salts with organic base such as ammonium, trimethylammonium, triethylammonium and the like; salts with alkaline metals such as sodium, potassium and the like and salts with alkaline earth metals such as calcium, magnesium and the like.

The compound of the present invention includes the solvate thereof and hydrate is preferable. An example of the solvate is a solvate with an organic solvent and/or water. The compound of the present invention may cooperate with arbitrary numbers of water molecules to give hydrate thereof.

The compound of the present invention includes all of stereoisomers, for example, atropisomers etc. thereof.

### Best Mode for Carrying Out the Invention

Compound (I) can be produced by the similar methods to WO98/04508, WO99138829 and the like. A process for producing the compound (I) is as follows.

### Process for Producing Compound (I)

Compound (I) can be produced by reacting a compound of the formula (IIa) (hereinafter referred to as "a compound (IIa)") with a bicyclic compound of the formula (IIIa) (hereinafter referred to as "a compound (IIIa)") or by reacting a compound of the formula (IIb) (hereinafter referred to as "a compound (IIb)") with a bicyclic compound of the formula (IIIb) (hereinafter referred to as "a compound (IIIb)"). 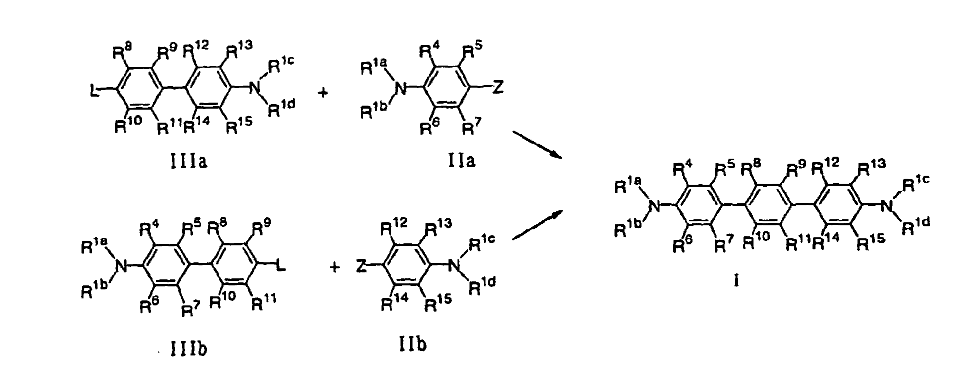 wherein either of L and Z is dihydroxyboryl, di(lower)alkyl boryl or di(lower) alkoxyboryl and the other is halogen or -OSO₂(C_{q}F_{2q+1}) (q is an integer of 0 to 4) and other symbols are the same as defined above.

The compound (I) can be produced by reacting the compound (IIa) with the compound (IIIa) or by reacting the compound (IIb) with the compound (IIIb) in a mixture of an appropriate solvent such as benzene, toluene, N, N-dimethylformamide, dimethoxyethane, tetrahydrofuran, dioxane, ethanol, methanol or the like and water or in an anhydrous solution in the presence of a palladium catalyst such as Pd(PPh₃)₄ PdCl₂(PPh₃)₂ PdCl₂(OAc)₂, PdCl₂(CH₃CN)₂ or the like, preferably Pd(PPh₃)₄, under a basic condition (for example, by K₃PO₄, NaHCO₃, NaOEt, Na₂CO₃, Ba(OH)₂, Cs₂CO₃, CsF, NaOH, Ag₂CO₃ or the like) at room temperature or heating for several tens minutes to several tens hours.

One of substituents L and Z of the compounds to be reacted may be any of the borane groups which are applicable in the Suzuki Reaction (Chemical Communication 1979, 866, Journal of Synthetic Organic Chemistry, Japan, 1993, Vol.51, No.11, 91-100) and dihydroxyborane is preferable. The other may be any of the leaving groups which are applicable in the Suzuki Reaction, for example, halogen, -OSO₂(C_{q}F_{2q+1}) wherein q is an integer of 0 to 4, or the like. Specifically, halogen, trifluoromethanesulfonyloxy (hereinafter referred to as OTf) or the like is preferable and bromine, iodine or OTf is more preferable.

R^{1a}, R^{1b}, R^{1c}, R^{1d}, R⁴ to R¹⁵ of the compounds (IIa), (IIIa), (IIb) and (IIIb) may be any of the groups which do not affect the Suzuki Reaction, for example, any groups other than halogen and -OSO₂(C_{q}F_{2q+1}) wherein q is an integer of 0 to 4.

Even if either of R⁴ to R¹⁵ is halogen, these reactions can be carried out without difficulty when the reactivity of the substituent L with the substituent Z is higher than that of halogen with either of substituents L and Z.

Even if either of R⁴ to R¹⁵ is hydroxy, the above reactions can be preferably carried out. Preferably the above reactions may be carried out after the protection of hydroxy group with a usual hydroxy-protecting group such as methoxymethyl, benzyl, tert-butyldimethylsilyl, methanesulfonyl, p-toluenesulfonyl or the like, followed by deprotection by the usual methods.

As processes for producing the compound (I), the above mentioned Suzuki Reaction is most preferable in view of the efficiency and easiness but silicon, zinc, tin or the like can be used in place of the boryl group in the above scheme.

For example, in the case that one of L and Z is -SiR₃₋ᵣ(Hal)ᵣ wherein R are independently lower alkyl, Hal is halogen and r is an integer of 1 to 3 and the other is halogen or -OSO₂(C_{q}F_{2q+1}) wherein q is an integer of 0 to 4, the coupling reaction may be carried out using a usual palladium catalyst (Synlett (1991) 845-853, J. Org. Chem. 1996, 61, 7232-7233). Examples of preferable palladium catalysts are (i-Pr₃P)₂PdCl₂, [(dcpe)PdCl₂] (dcpe=Cy₂PCH₂CH₂PCy₂), (η³-C₃H₅PdCl)₂ and the like.

Even in the case that one of L and Z is -SnR'₃ wherein R' are each independently lower alkyl and the other is halogen, acetyloxy or -OSO₂(C_{q}F_{2q+1}) wherein q is an integer of 0 to 4, an objective compound can be obtained using a usual palladium catalyst (preferably Pd(PPh₃)₄ or the like) (Angew. Chem. Int. Ed. Engl. 25 (1986) 508-524).

In the case that one of L and Z is -Zn(Hal) wherein Hal is halogen and the other is halogen, an objective compound can be obtained (Acc. Chem. Res. 1982, 15, 340-348). Any usual palladium catalyst is applicable and Pd(PPh₃)₄ PdCl₂(dppf), PdCl₂(PPh₃)₂, PdCl₂(P(o-Tolyl)₃)₂, Pd(OAc)₂ and the like are exemplified as preferable examples.

All of these reactions may be carried out in a suitable solvent such as N,N-dimethylformamide, tetrahydrofuran or the like at room temperature or heating for several tens minutes to several tens hours.

As compound (IIIa) and (IIIb) in the above reactions, may be used known compounds or compounds which are derived from a compound of the following formula (Va) (hereinafter referred to as "a compound (Va)") or the following formula (Vb) (hereinafter referred to as "a compound (Vb)") which can be produced by the known method or the following method. 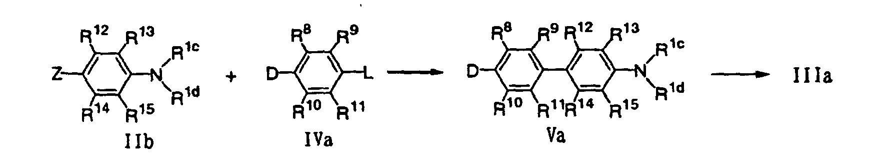 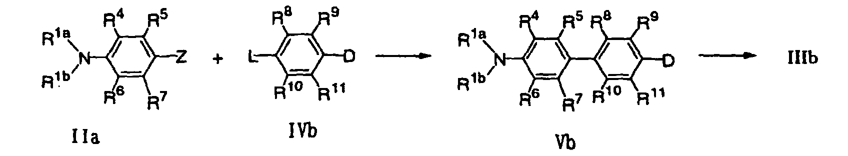 wherein D is any of the groups which do not affect the Suzuki Reaction of L with Z, and may be the same group as L when a compound of the formula (IVb) is a bisymmetric compound. The other symbols are the same as above.

The compound (IIb) is reacted with the compound (IVa) or the compound (IIa) is reacted with (IVb) to give the compound (Va) or (Vb). When the compound (IVa) or (IVb) is not a bisymmetric compound, D is preferably a group which does not affect the Suzuki Reaction of L with Z and can be easily converted to L. For example, hydroxy, hydrogen, formyl, nitro or the like is preferable. In the reaction of L with Z, silicon, zinc, tin or the like can be used in place of the borane group as mentioned above.

D is converted into a group L which is applicable to the Suzuki Reaction.

A compound wherein D is hydroxy may be reacted with a trifluoromethanesulfonating agent such as trifluoromethanesulfonic anhydride, trifluoromethanesulfonyl chloride, N-phenyltrifluoromethanesulfone imide or the like in a suitable solvent such as dichloromethane, chloroform, tetrahydrofuran or benzene in the presence of a base such as sodium hydride, pyridine, triethylamine, potassium carbonate or the like at - 20 °C or heating for several minutes to several tens hours to give an objective compound wherein L is OTf.

For example, a compound wherein D is hydrogen may be reacted with a halogenating agent such as bromine, chlorine, iodine, N-bromosuccinimide or the like in a suitable solvent such as acetic acid, dichloromethane, chloroform, carbon tetrachloride, benzene, water or the like at -20 °C or heating for several minutes to several tens hours to give an objective compound wherein L is halogen.

A compound wherein D is formyl may be oxidated by the Baeyer-Villiger reaction to give a compound wherein D is formyloxy, followed by hydrolysis to give a compound
wherein D is hydroxy. The compound wherein L is OTf can be obtained by the similar process as mentioned above.

A compound wherein D is nitro may be reduced to give a compound wherein D is amino, followed by the Sandmeyer Reaction to give a compound L is halogen.

As mentioned above, Compound (I) of the present invention can be synthesized from (IIa), (IIb), (IIIa) or (IIIb) wherein the objective substituents R^{1a} to R^{1d} are previously introduced. Alternatively, Compound (I) can be synthesized by constructing the terphenyl structure, followed by introducing the target substituents R^{1a} to R^{1d} therein.

For example, if a target compound is a compound wherein any one of R^{1a} to R^{1d} is alkylsulfonyl optionally substituted with halogen or acyl optionally substituted with halogen, a terphenyl compound having an amino group may be reacted with a compound having a substituent corresponding to the target substituent (for example, methanesulfonic anhydride, trifluoroacetic anhydride) in a suitable solvent such as dichloromethane, tetrahydrofuran and the like in the presence of a base such as pyridine, triethylamine, dimethylaminopyridine or the like at ice-cooling or heating.

If a target compound is a compound wherein any one of R^{1a} to R^{1d} is lower alkyl, lower alkenyl, lower alkynyl or cycloalkyl, a terphenyl compound having an amino group may be reacted with a halogen compound, sulfonate compound or alcohol compound having a substituent corresponding to a target substituent (for example, lower alkyl halide, lower alkylsulfonate, lower alkylalcohol or the like in the presence of a base such as an alkaline metal hydride, an alkaline metal hydrogencarbonate, an alkaline metal carbonate, an organic base and the like at ice-cooling or heating.

A terphenyl compound having an amino group can be reacted with an aldehyde compound or a ketone compound having an target substituent (for example, a lower alkyl aldehyde, a lower alkanone, a cycloalkanone and the like) in a suitable solvent such as dichloromethane, tetrahydrofuran or the like in the presence of an acidic catalyst such as acetic acid, pyridinium paratoluene sulfonate or the like at ice-cooling or heating to get Schiff's base. After isolation of the base, it may be reacted with a reductant to obtain an target compound. These reactions can be carried out in an one-pot system.

In the case that a compound has a substituent interfering of the above reaction, the substituent may be protected with a suitable protecting group in advance and the protecting group may be removed in a suitable step by the usual method. For example, if hydroxy interferes the reaction, it may be protected with methoxymethyl, methanesulfonyl, benzyl, trifluoromethanesulfonyl, tert-butyldimethylsilyl or the like, followed by deprotection in a suitable step.

For example, for a protection of hydroxy with methanesulfonyl, a compound which has hydroxy may be reacted with methanesulfonyl chloride in a solvent such as dichloromethane, chloroform, carbon tetrachloride or the like in the presence of a base such as triethylamine, pyridine or the like at ice-cooling or room temperature for several hours. The protected compound may be deprotected with 1-4 N sodium hydroxide, potassium hydroxide, aqueous solution thereof, sodium methoxide, ethyl magnesium bromide or the like in a solvent such as dimethylsulfoxide, dimethylformamide, tetrahydrofuran, dioxane, dimethoxyethane or the like at room temperature or heating for several tens minutes to several hours.

When methoxymethyl is used as a hydroxy-protecting group, a compound which has hydroxy may be reacted with chloromethylmethylether in a solvent such as tetrahydrofuran, dioxane, dimethoxyethane or the like in the presence of sodium hydride, diisopropylethylamine or the like to give a compound which has a protected hydroxy group. The compound may be subjected to a usual deprotection reaction with hydrochloric acid, sulfuric acid or the like in a solvent such as methanol, tetrahydrofuran, acetic acid or the like for a deprotection.

When tert-butyldimethylsilyl is used as a protecting group, a compound which has hydroxy may be reacted with tert-butyldimethylsilyl chloride, tert-butyldimethylsilyl triflate or the like in a solvent such as dimethylformamide, acetonitrile, tetrahydrofuran, dimethylformamide, dichloromethane or the like in the presence of imidazole, triethylamine, 2, 6-lutidine or the like. For a deprotection reaction the protected compound may be reacted with tetrabutylammonium fluoride or the like in a solvent such as tetrahydrofuran or the like.

Thus obtained compound of the present invention can be converted into a prodrug thereof. The term "prodrug" includes compounds which can easily be converted to the compound having the activity of the present invention in a living body. Any usual method for conversion into a prodrug may be used.

The methods for selecting and producing suitable prodrugs are described in Design of Prodrugs, Elsevier, Amsterdam 1985. According to this, a group generally used for prodrug may be introduced into carboxy, hydroxy, amino or the like at any position of the compound of the present invention.

For example, if a compound has a hydroxy group on any ring, -COCH₂CH₂COOH, -COCH=CHCOOH, -COCH₂SO₃H, -PO₃H₂, -COCH₂NMe₂, -CO-Py wherein Py means pyridyl can be introduced.

For example, if a compound has an amino group on any ring,

-COOCR^{a}R^{b}OCOCH₂R^{c}

wherein R^{a} and R^{b} are each independently hydrogen or lower alkyl, R^{c} is H, -OH,-CONHR^{d}, -OCONHR^{d}, -(NHCOCR^{e}R^{f})ᵤNHCOCH₃, -(NHCOCR^{e}R^{f})ᵤNHCOC₂H₅, -CSNH₂, -(OCH₂CH₂)ₜOH, -OCH₃, -(OCH₂CH₂)ₜOCH₃, -COCH₃, -COC₂H₅,-OCOCH₃, -OCOC₂H₅, -NHOH, -NHCONH₂, -NHCSNH₂, -NHSO₂CH₃,-N(SO₂CH₃)₂, -SO₂NH₂, -SOMe, -SO₂CH₃, -OCH₂CONH₂, -OCH₂CON(CH₃)₂,-SO₂N(CH₃)₂, -PO(OCH₃)₂, -NHCSNHC₂H₅Et, -CH=NNHCONH₂,-CH=NNHCSNH₂, -CH=NNHSO₂CH₃, triazolyl, tetrazolyl and the like, R^{d}, R^{e} and
R^{f} is hydrogen or lower alkyl, t is 1 or 2, u is an integer of 0 to 2, -COOCH(Me)OCOCMe₃, -COOCH₂OCO(CH₂)₁₄Me, -COOCH₂OCO-Pyr, -CH₂NHCO-C₆H₄-o-OCH₂OAc and the like wherein Pyr means pyridyl and Ac means acetyl can be introduced.

When a prodrug is produced by introducing a substituted acyloxycarbonyl (-COOCR^{a}R^{b}OCOCH₂R^{c}) into an amino group at any position of the compound of the present invention, the amino group may be α-haloalkoxycarbonated, followed by being reacted with a suitable carboxylic acid under a suitable condition.

The above-mentioned acyloxyalkylcarbamate can be produced according to known methods described in WO96/18605 and the like.

A compound of the present invention having an amino group is reacted with chloroformate α-haloalkyl ester in a non-active solvent such as diethyl ether, tetrahydrofuran, 1,4-dioxane, ethyl acetate, toluene and the like in the presence of a base such as pyridine, triethylamine, N-methylmorpholine and the like at 0 °C to room temperature to obtain a haloalkoxycarbamate. Thus-obtained compound may be reacted with a salt (for example, an alkaline metal salt, an alkaline earth metal salt, a silver salt, a mercury salt) of a substituted carboxylic acid compound at room temperature or heating for several hours to several days to obtain a prodrug compound.

Regarding compounds having a substituent interfering with processes for obtaining a prodrug compound, the group may be protected with a suitable protecting group in advance, and the protected group may be deprotected by the conventional method at a suitable stage.

Compounds of the present invention has not only IgE production suppressive effect but also Th2 differentiation inhibitory effect.

The term "Th2 differentiation inhibitory effect" means an inhibitory effect on the differentiation of Th0 cells to Th2 cells. The compounds of the present invention can be used for a pharmaceutical composition for treating and/or preventing diseases induced by Th2 cells or cytokines produced from Th2 cells.

A Th2 differentiation inhibitory composition of the present invention shows suppressive effects on activating of B cells and antibody production by decreasing Th2 cells and cytokines derived from Th2 cells. Additionally, the composition has the following features.

The contact of B cells with Th2 cells and the stimulation of B cells by cytokines derived from Th2 cells are considered to be necessary for the activation of B cells in the resting stage. Thus, a Th2 differentiation inhibitory composition of the present invention can suppress the direct activation of B cells by Th2 cells themselves. Therefore, the composition of the present invention can treat and prevent allergic or autoimmune diseases more effectively than the conventional anti-allergic agents.

Some kinds of allergic diseases such as asthma, respiratory inflammation and the like are known to be caused by cytokines produced by and released from Th2 cells. Thus, a Th2 differentiation inhibitory composition of the present invention is expected to show a more effective treating effect than a pharmaceutical composition showing only IgE production suppressive effect.

For example, the compound of the present invention is expected to be useful for preventing or treating the following diseases.

Rejection symptom against a transplantation of an organ or a tissue, transplantation immunology (acute or chronic GVHD), autoimmune diseases (especially organ non-specific autoimmune diseases), mixed connective tissue disease(MCTD), injury caused by ischemia-reperfusion, ulcerative colitis, systemic lupus erythematodes, myasthenia gravis, systemic progressive scleroderma, rheumatoid arthritis, interstitial cystitis, Hashimoto's diseases, Basedow's diseases, autoimmune hemolytic anemia, idiopathic thrombocytopenic purpura, Goodpasture's syndrome, atrophic gastritis, pernicious anemia, Addison diseases, pemphigus, pemphigoid, lenticular uveitis, sympathetic ophthalmia, primary biliary cirrhosis, active chronic hepatitis, Sjogren's syndrome, multiple myositis, dermatomyositis, polyarteritis nodosa, rheumatic fever, glomerular nephritis (lupus nephritis, IgA nephtopathy, membranous nephropathy and the like), allergic encephalitis, atopic allergic diseases (for example, bronchial asthma, allergic rhinitis, allergic dermatitis, allergic conjunctivitis, pollinosis, urticaria, food allergy and the like), psoriasis, Omenn's syndrome, vernal conjunctivitis and hypereosinophilic syndrome and the like.

Especially, the composition of the present invention is expected to be useful for organ non-specific autoimmune diseases such as chronic GVHD, ulcerative colitis, systemic lupus erythematodes, myasthenia gravis, systemic progressive scleroderma, rheumatoid arthritis, glomerular nephritis, interstitial cystitis and the like.

Further, because an immunosuppressant is expected to be a treating agent for chronic renal insufficiency induced by non-immune mechanism (Kidney International vol.54 (1998), pp. 1510-1519. Kidney International vol.55 (1999), pp. 945-955), the compound of the present invention can be a drug for non-immune chronic renal insufficiency.

The compound of the present invention has merits such as low toxity, high bioavailability etc. Many of them are negative in a heterochromosome test and little effective on other enzymes. Thus, they can be a superior pharmaceutical composition.

A compound of the present invention can be administered orally or parenterally as a suppressant on the IgE production, Th2 differentiation inhibitor, anti-allergic agent and/or immunosuppressant. In the case of oral administration, it may be in any usual form such as tablets, granules, powders, capsules, pills, solutions, syrups, buccal tablets, sublingual tablets and the like. When the compound is parenterally administered, any usual form is preferable, for example, injections (e.g., intravenous, intramuscular), suppositories, endermic agents, vapors and the like. Oral administration is particularly preferable.

A pharmaceutical composition may be manufactured by mixing an effective amount of a compound of the present invention with various pharmaceutical ingredients suitable for the administration form, such as excipients, binders, moistening agents, disintegrators, lubricants, diluents and the like. When the composition is of an injection, an active ingredient can be sterilized with a suitable carrier to give a pharmaceutical composition.

Specifically, examples of the excipients include lactose, saccharose, glucose, starch, calcium carbonate, crystalline cellulose and the like, examples of the binders include methylcellulose, carboxymethylcellulose, hydroxypropylcellulose, gelatin, polyvinylpyrrolidone and the like, examples of the disintegrators include carboxymethylcellulose, sodium carboxymethylcellulose, starch, sodium alginate, agar, sodium lauryl sulfate and the like, and examples of the lubricants include talc, magnesium stearate, macrogol and the like. Cacao oil, macrogol, methyl cellulose and the like may be used as base materials of suppositories. When the composition is manufactured as solutions, emulsified injections or suspended injections, dissolving accelerators, suspending agents, emulsifiers, stabilizers, preservatives, isotonic agents and the like may be added. For oral administration, sweetening agents, flavors and the like may be added.

Although the dosage of a compound of the present invention a suppressant on the IgE production, Th2 differentiation inhibitor, anti-allergic agent and/or immunosuppressant should be determined in consideration of the patient's age and body weight, the type and degree of diseases, the administration route or the like, a usual oral dosage for human adults is 0.05 - 100 mg/kg/day and preferable is 0.1 - 10 mg/kg/day. For parenteral administration, although the dosage highly varies with administration routes, a usual dosage is 0.005 - 10 mg/kg/day, preferably, 0.01-1 mg/kg/day. The dosage may be administered in one or several divisions per day.

The present invention is further explained by the following Examples and Experiments, which are not intended to limit the scope of the present invention.

### EXAMPLE

The present invention is further described in the following Examples, which are not intended to restrict the invention.

### Example 1 Synthesis of Compound 236

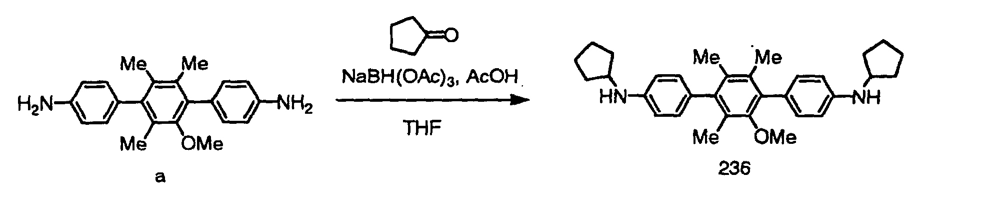

After 332 mg of Compound (a) (1.0 mmol) was dissolved in 14 ml of THF under nitrogen atmosphere, 0.88 ml of cyclopentanone (10.0 mmol) and 0.23 ml of acetic acid (4.0 mmol) were added and the mixture was stirred for 40 minutes at room temperature. To the reaction solution, 2.23 g of triacetoxy sodium borohydride (10.0 mmol) was added and the mixture was stirred for 5 hours at room temperature. The reaction mixture was poured into water and extracted with ethyl acetate. After the extract was neutralized with 3.5 % aqueous solution of sodium hydrogencarbonate, the extract was washed with saturated brine, dried and concentrated. The residue was purified by a silica gel chromatography (hexane-ethyl acetate 1:4) and crystallized from dichloromethane-hexane to obtain Compound 236 (0.276 g; 59 % yield).

### Example 2 Synthesis of Compound 420 and 367

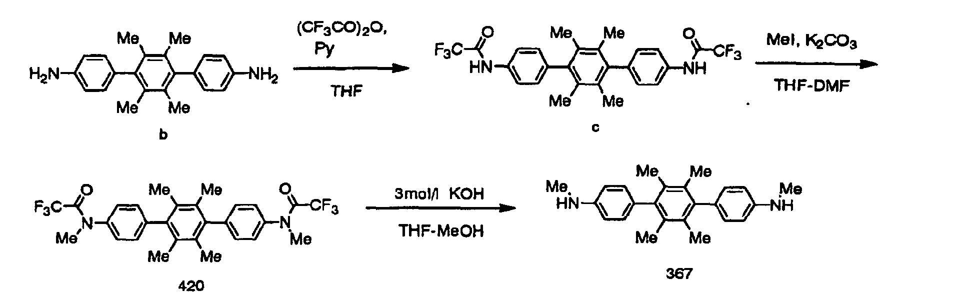

### (Step 1) Synthesis of Compound (c)

After 2.70 g of Compound (b) (8.51 mmol) was dissolved in 50 ml of THF under nitrogen atmosphere and 4.13 ml of pyridine (51.1 mmol) was added, 3.61 ml of trifluoroacetic anhydride (25.5 mmol) was added dropwise to the mixture and stirred for 3 hours. The reaction mixture was poured into 0.2 mol/L aqueous hydrochloric acid (120 ml) and extracted with ethyl acetate. The extract was washed with water, 3.5 % aqueous solution of sodium hydrogencarbonate and saturated brine. After the activated carbon was added to the extract, the mixture was stirred and filtered with celite. Crystalization from acetone-isopropyl ether gave Compound (c) (3.05 g; 71 % yield).

### (Step 2) Synthesis of Compound 420

After 2.00 g of Compound (c) (3.93 mmol) was dissolved in mixture of 40 ml of THF and 20 ml of DMF under nitrogen atmosphere, 2.22 g of potassium carbonate (15.7 mmol) was added. 1.98 ml of methyl iodide (31.4 mmol) was added dropwise to the mixture at room temperature and stirred for 80 minutes at 55 °C. The reaction mixture was cooled, poured into 5 % aqueous solution of citric acid (140 ml) to neutralize and extracted with ethyl acetate. The extract was washed with water, 3.5 % aqueous solution of sodium hydrogencarbonate and saturated brine and dried. Activated carbon was added to the extract, stirred and the mixture was filtered with celite. Crystallization from acetone-isopropyl ether gave Compound 420 (1.7.1g; 81 % yield).

### (Step 3) Synthesis of Compound 367

After 1.54 g of Compound 367 (2.86 mmol) was dissolved in mixture of 30 ml of THF and 30 ml of methanol under nitrogen atmosphere and 19 ml of 3 mol/l aqueous solution of potassium hydroxide (57.2 mmol) was added, the mixture was stirred for 80 minutes at 55 °C. The reaction mixture was cooled, poured into 5 % aqueous solution of citric acid (120 m) to neutralize and extracted with ethyl acetate. The extract was washed with water, 3.5 % aqueous solution of sodium hydrogencarbonate and saturated brine, successively, dried and concentrated. The residue was crystallized from THF-acetone-isopropyl ether, washed with isopropyl ether to obtain Compound 367 (924 mg; 94 % yield).

Other Compound (I) were synthesized by the similar methods. The structures and physical constants are as follows.

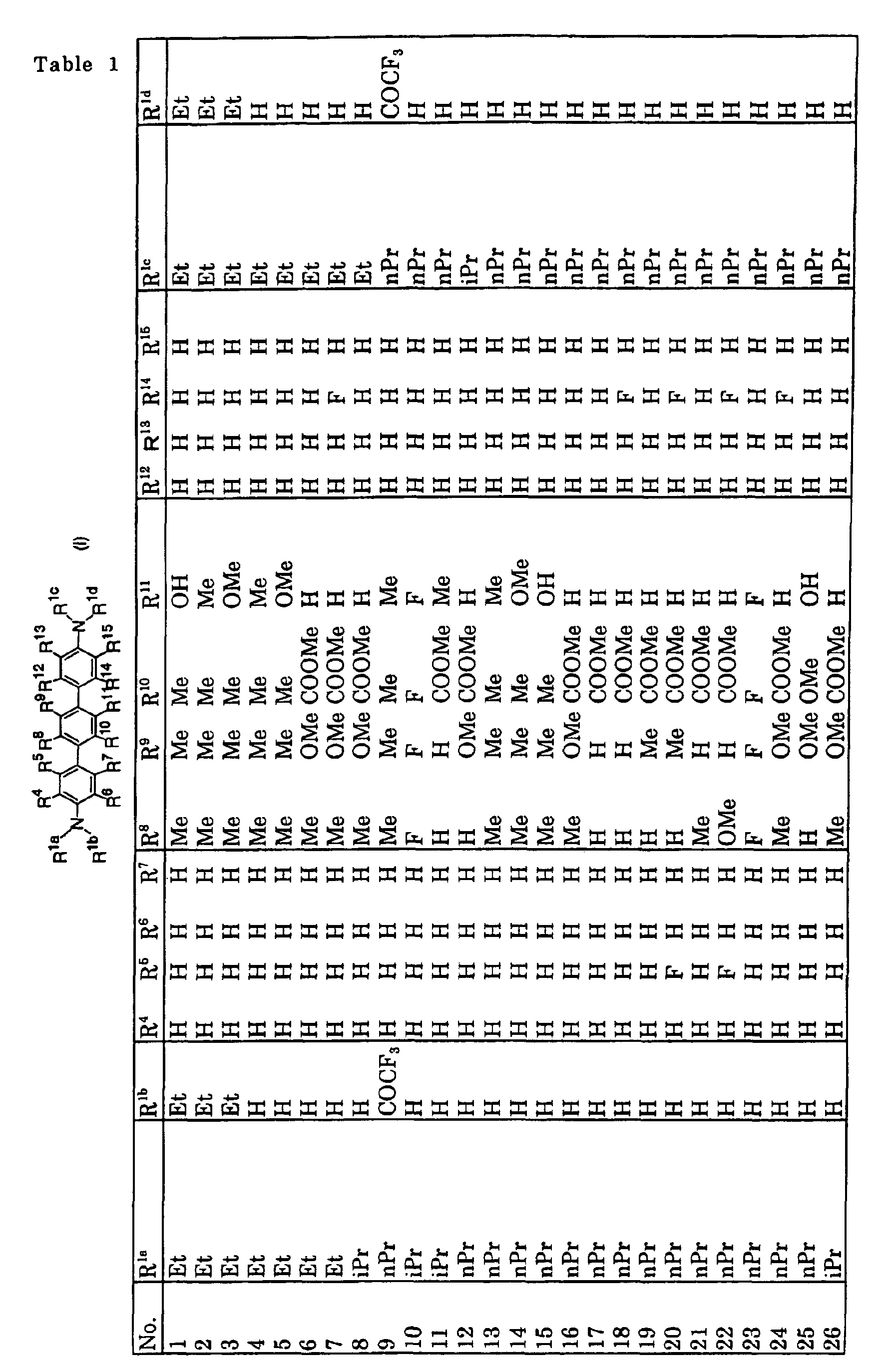

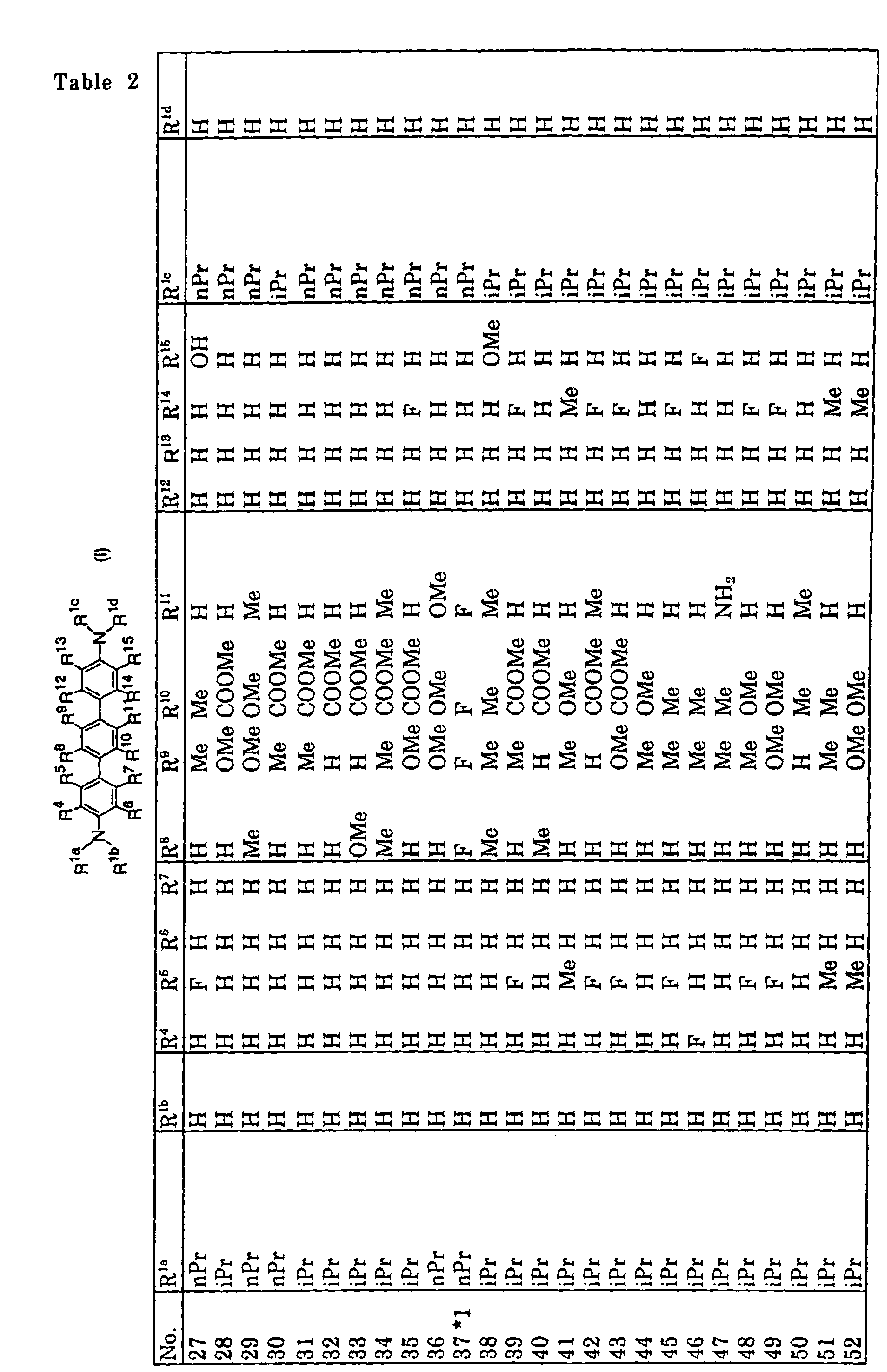

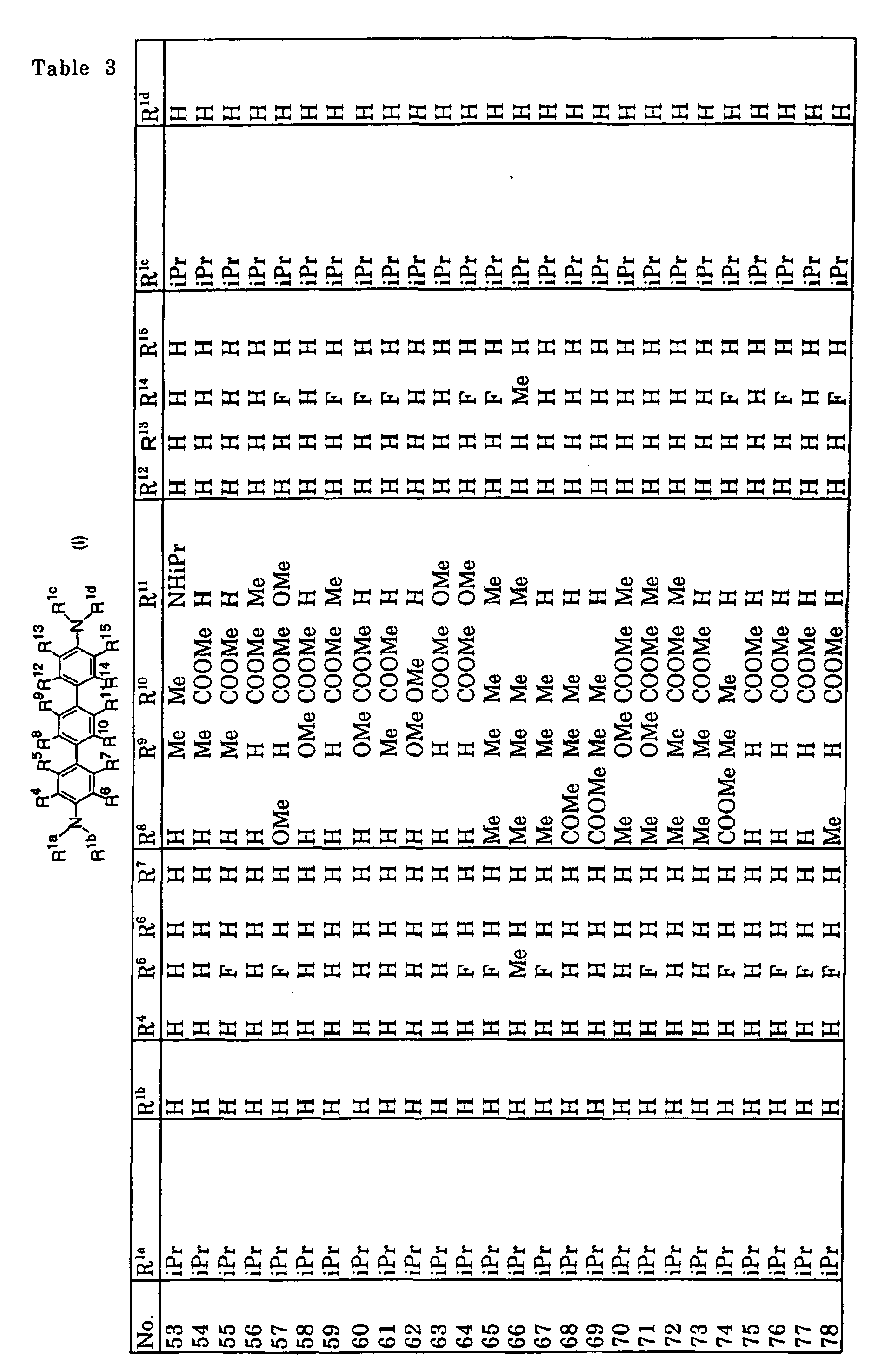

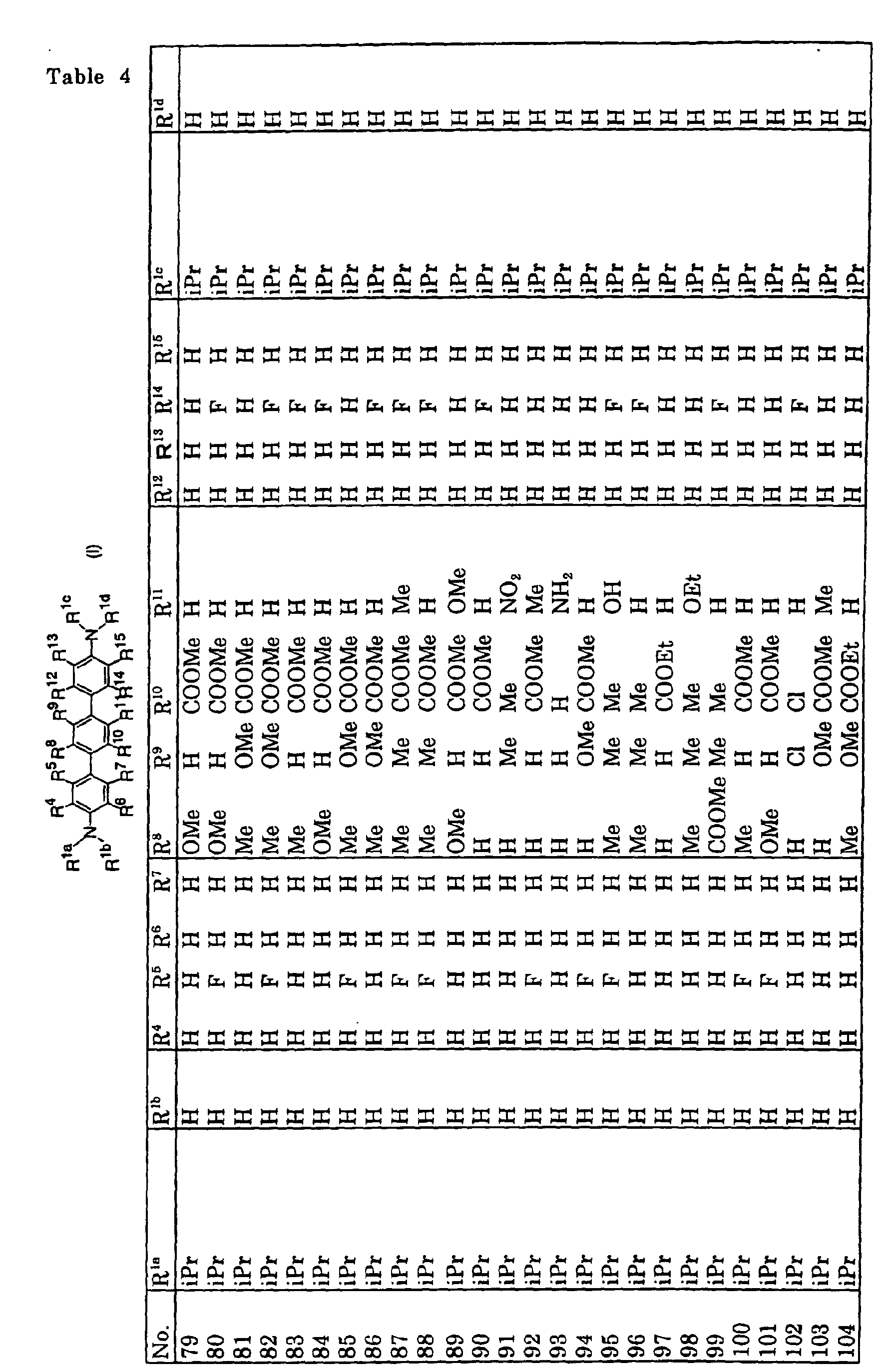

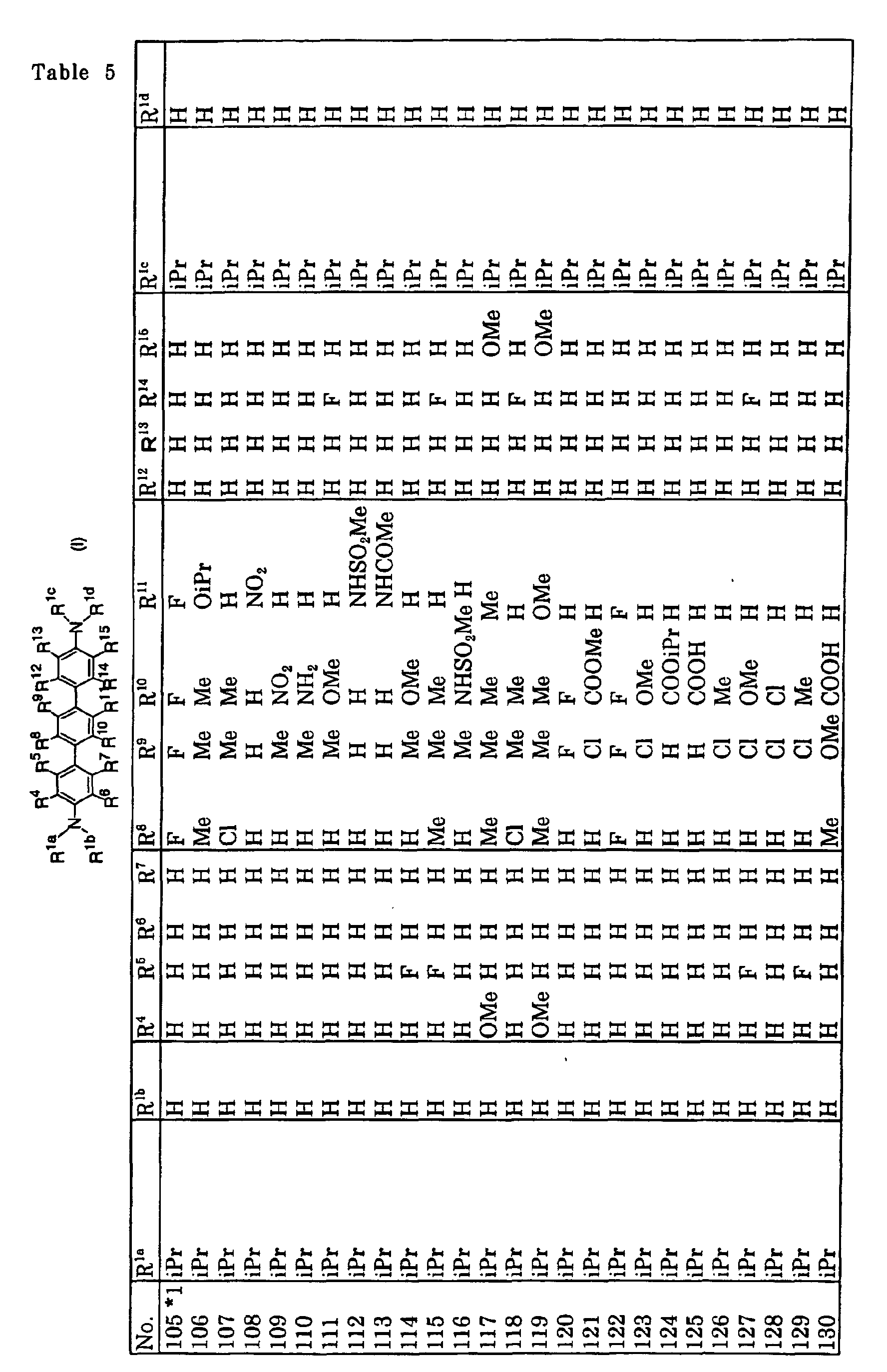

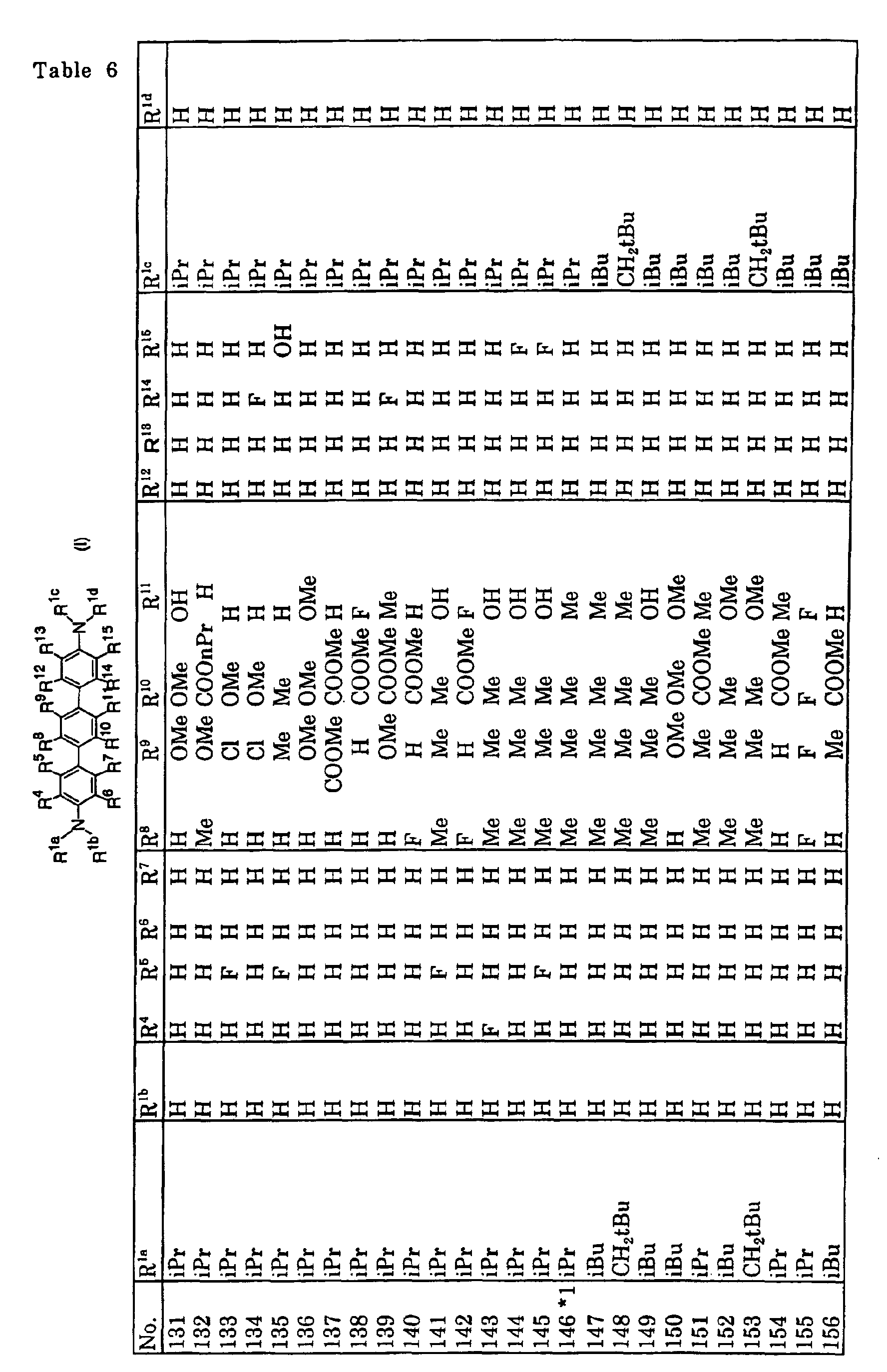

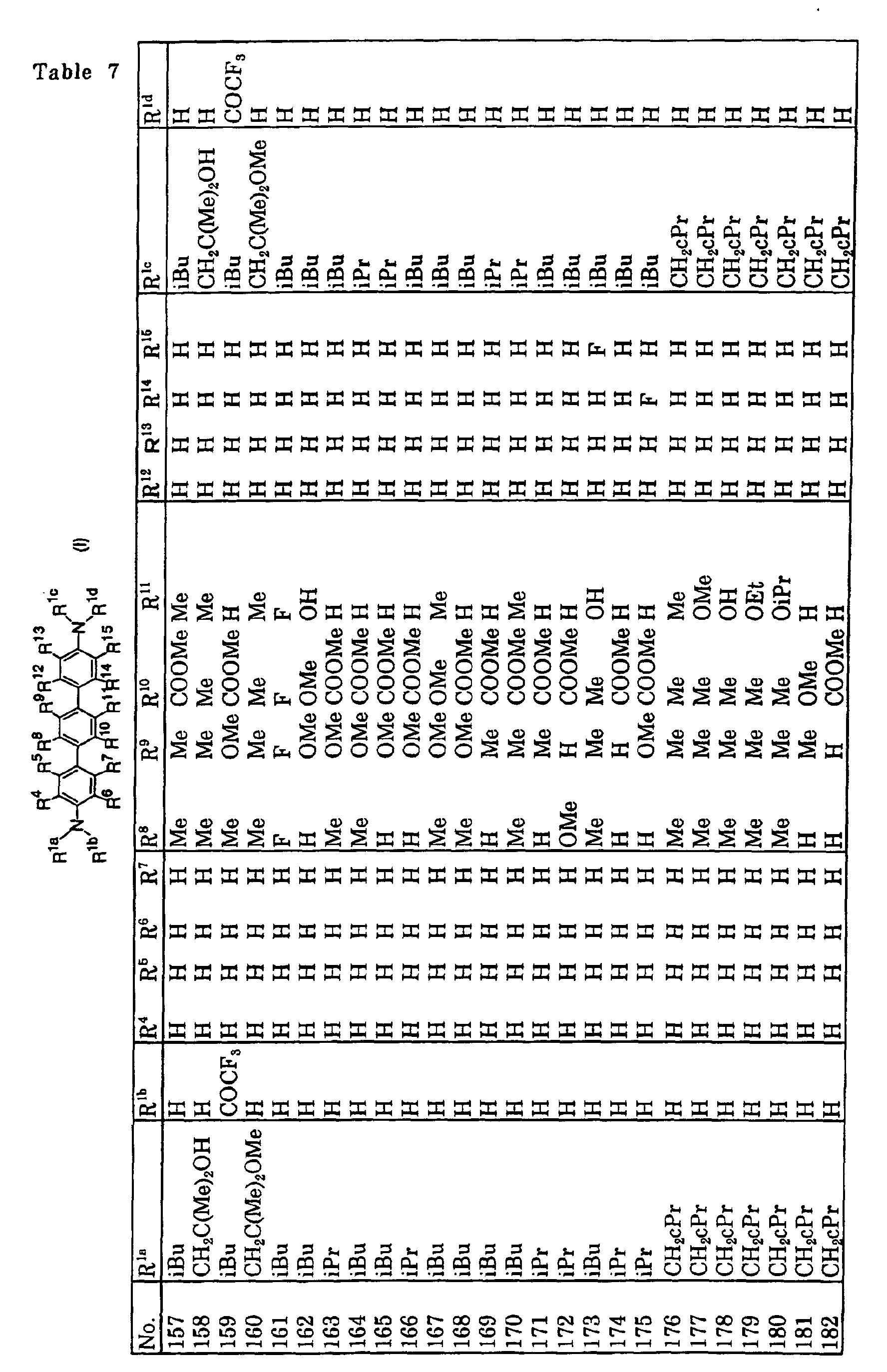

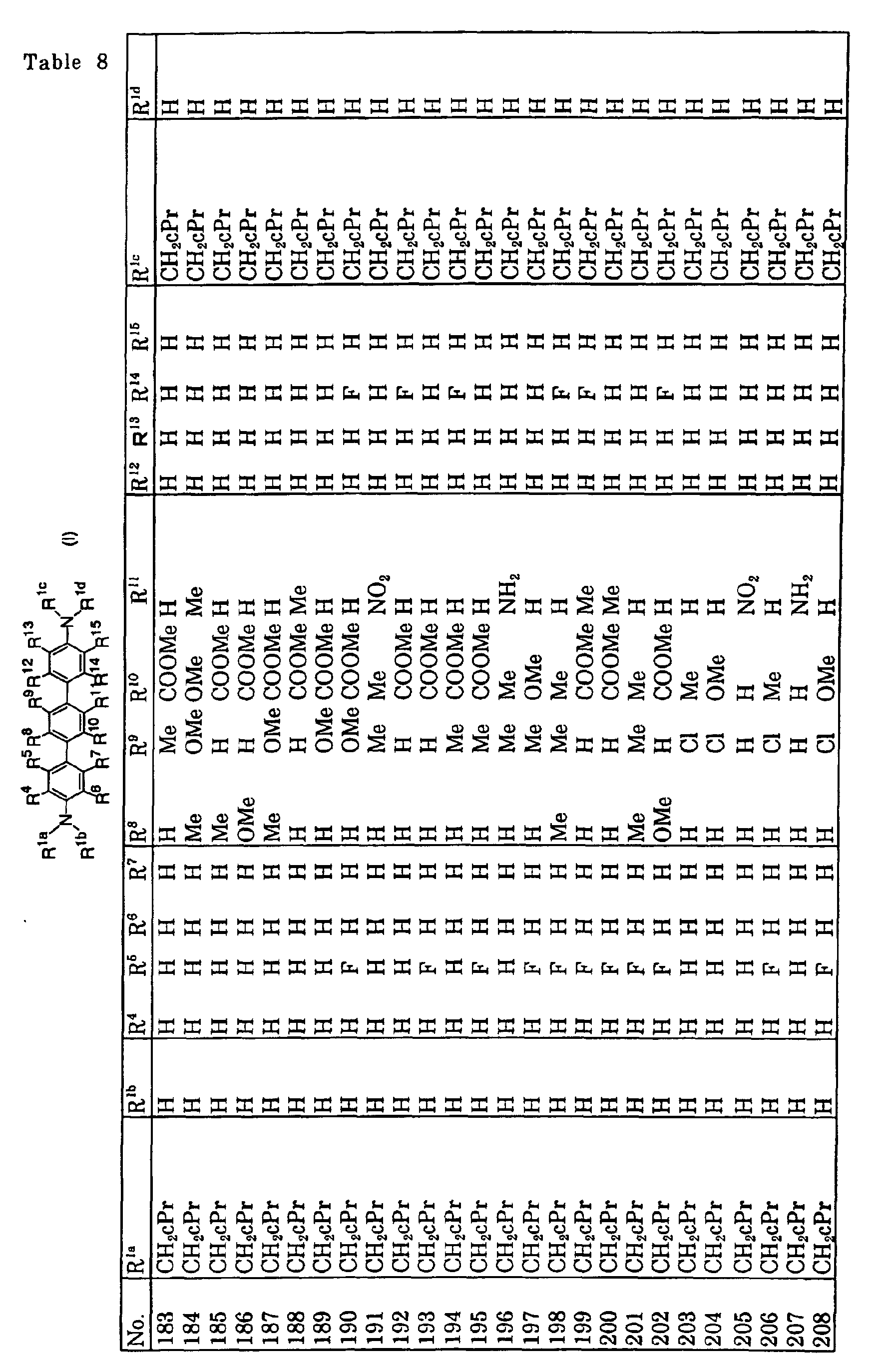

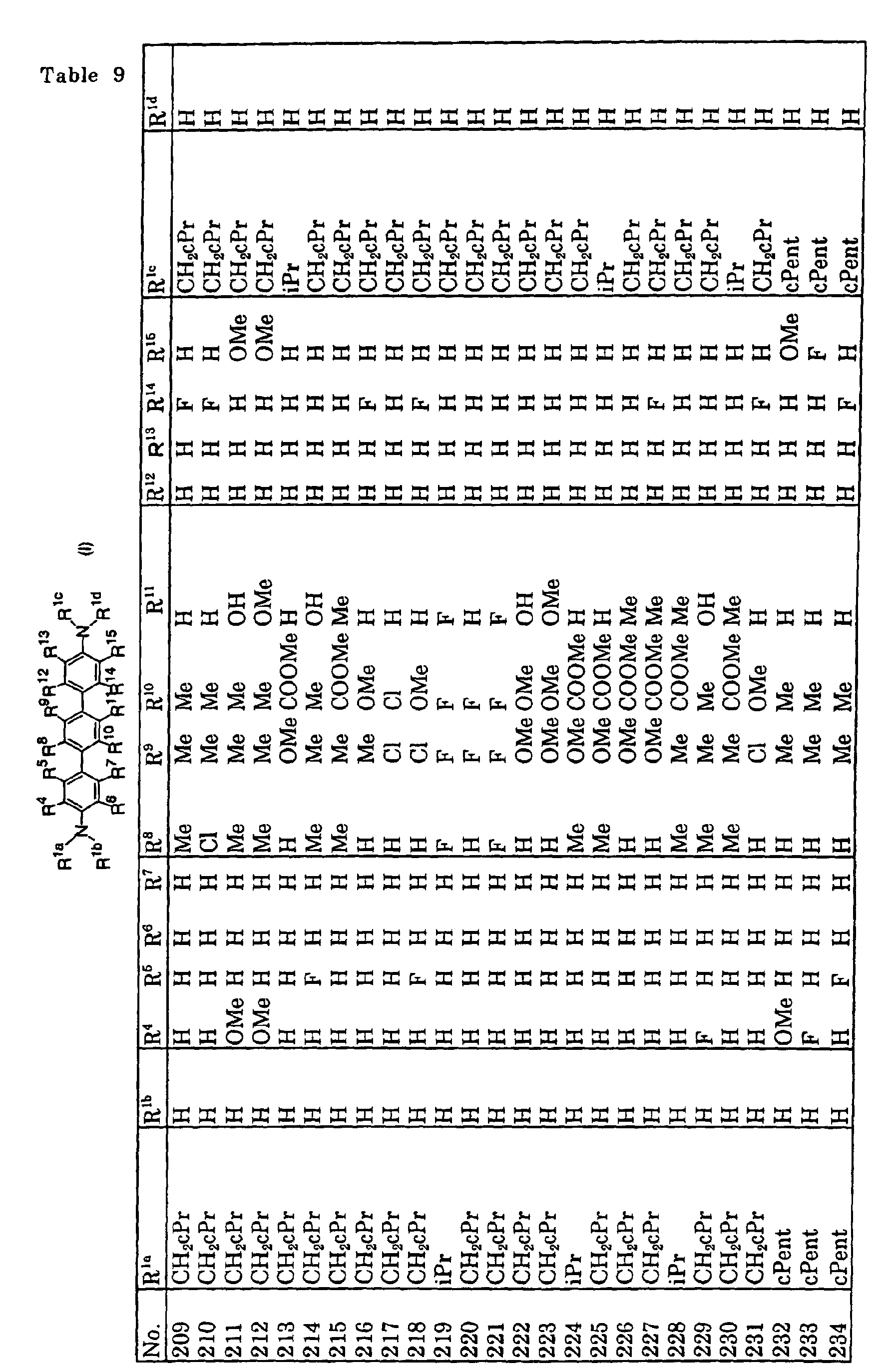

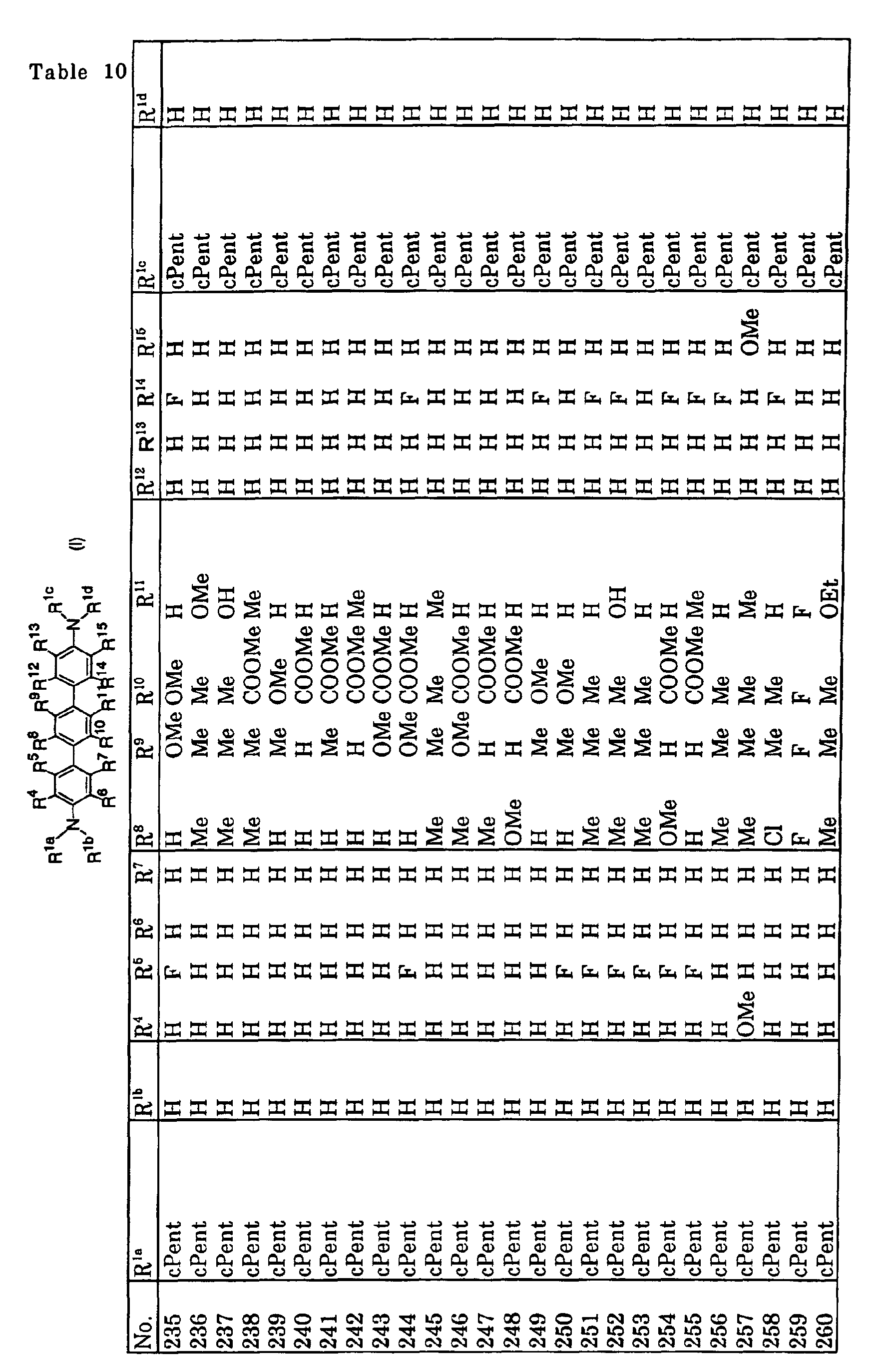

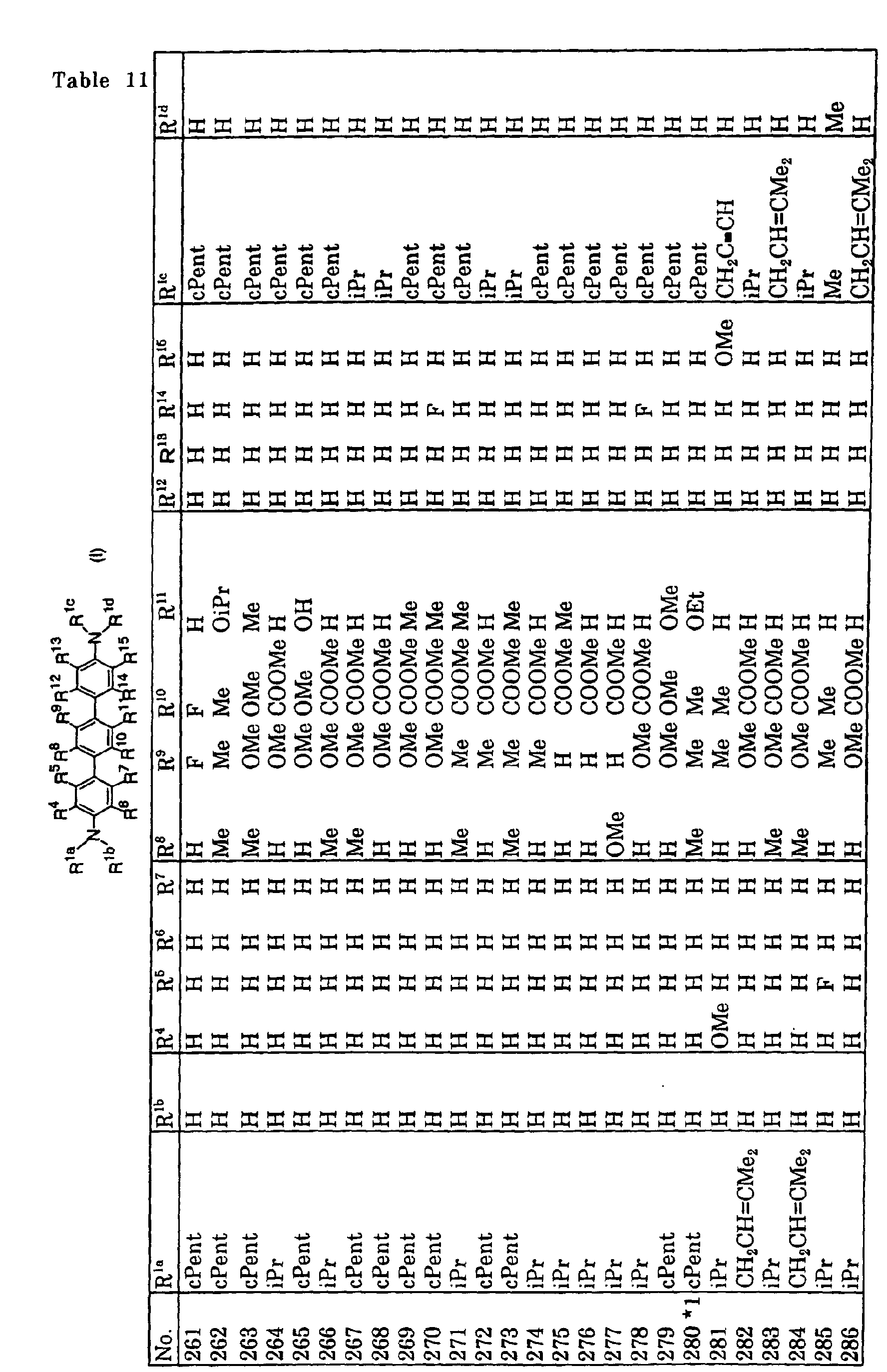

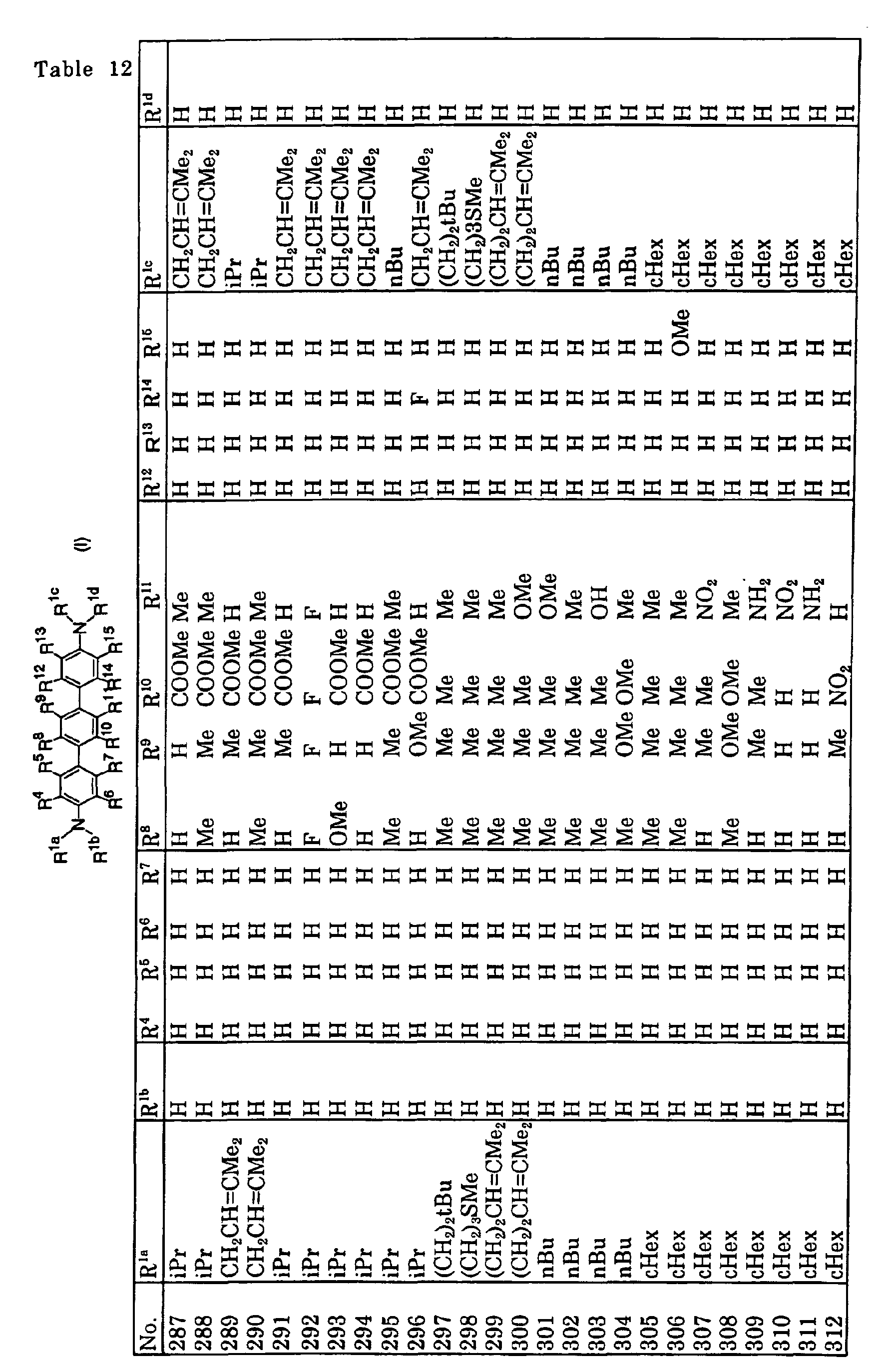

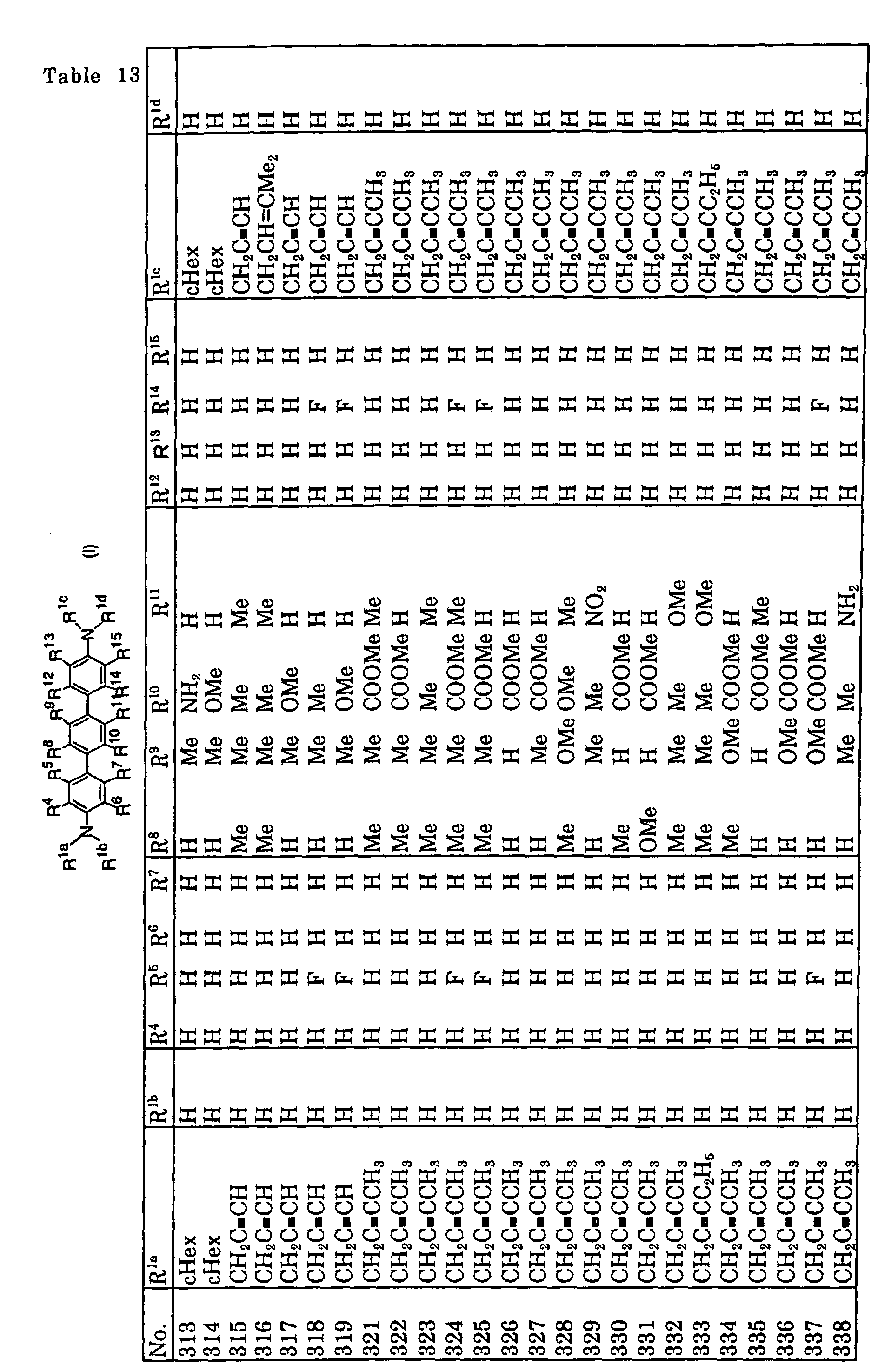

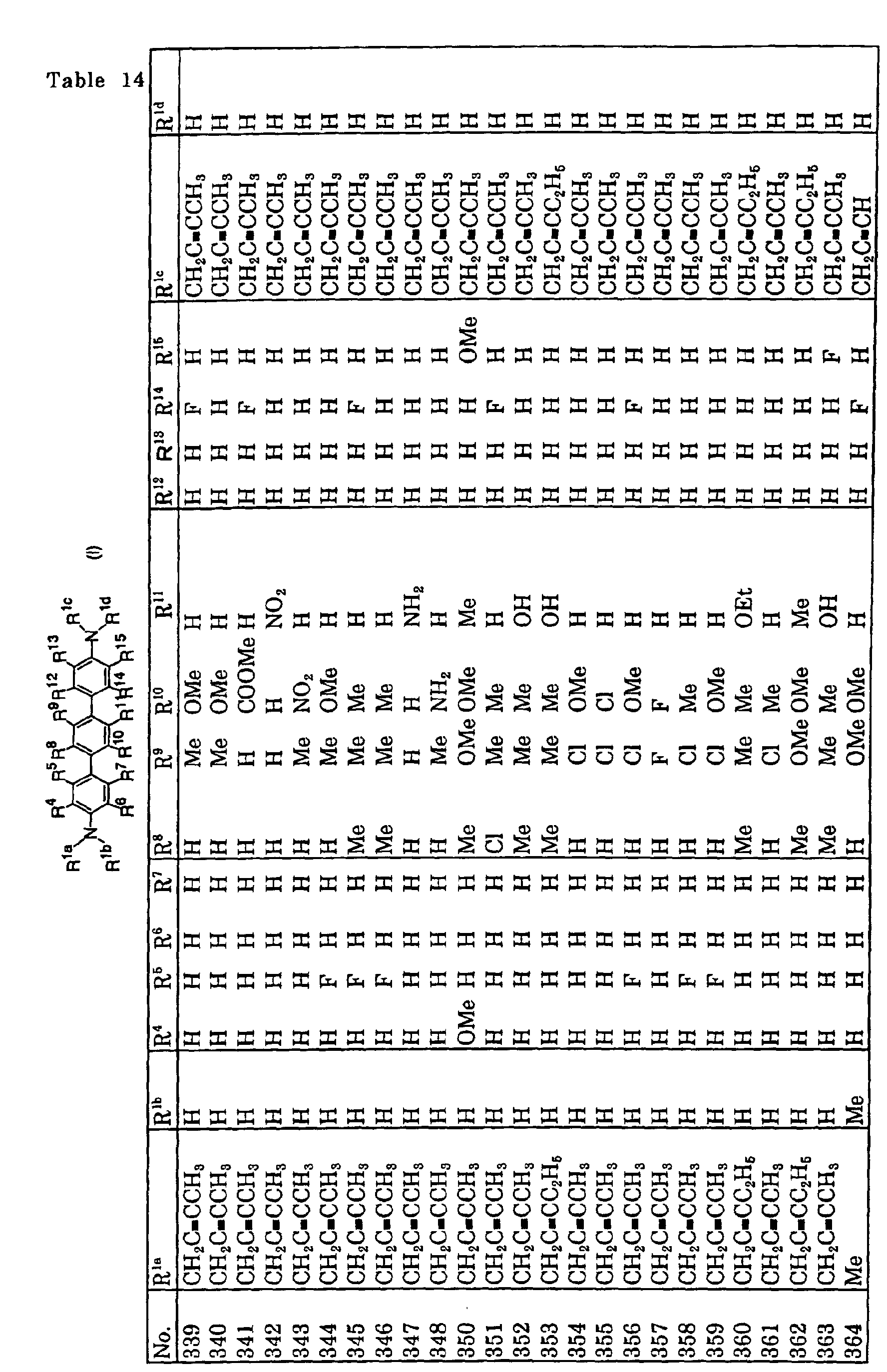

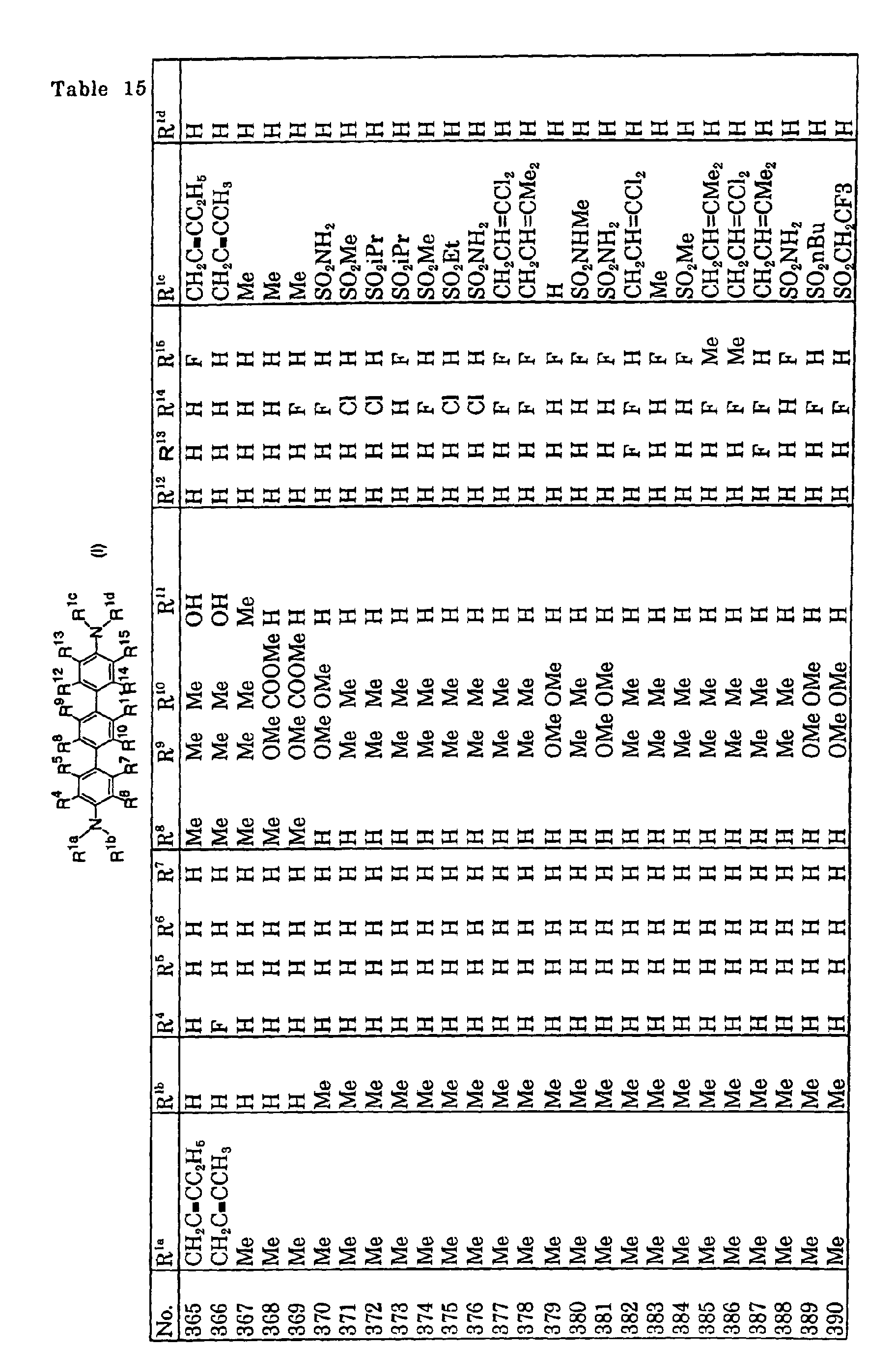

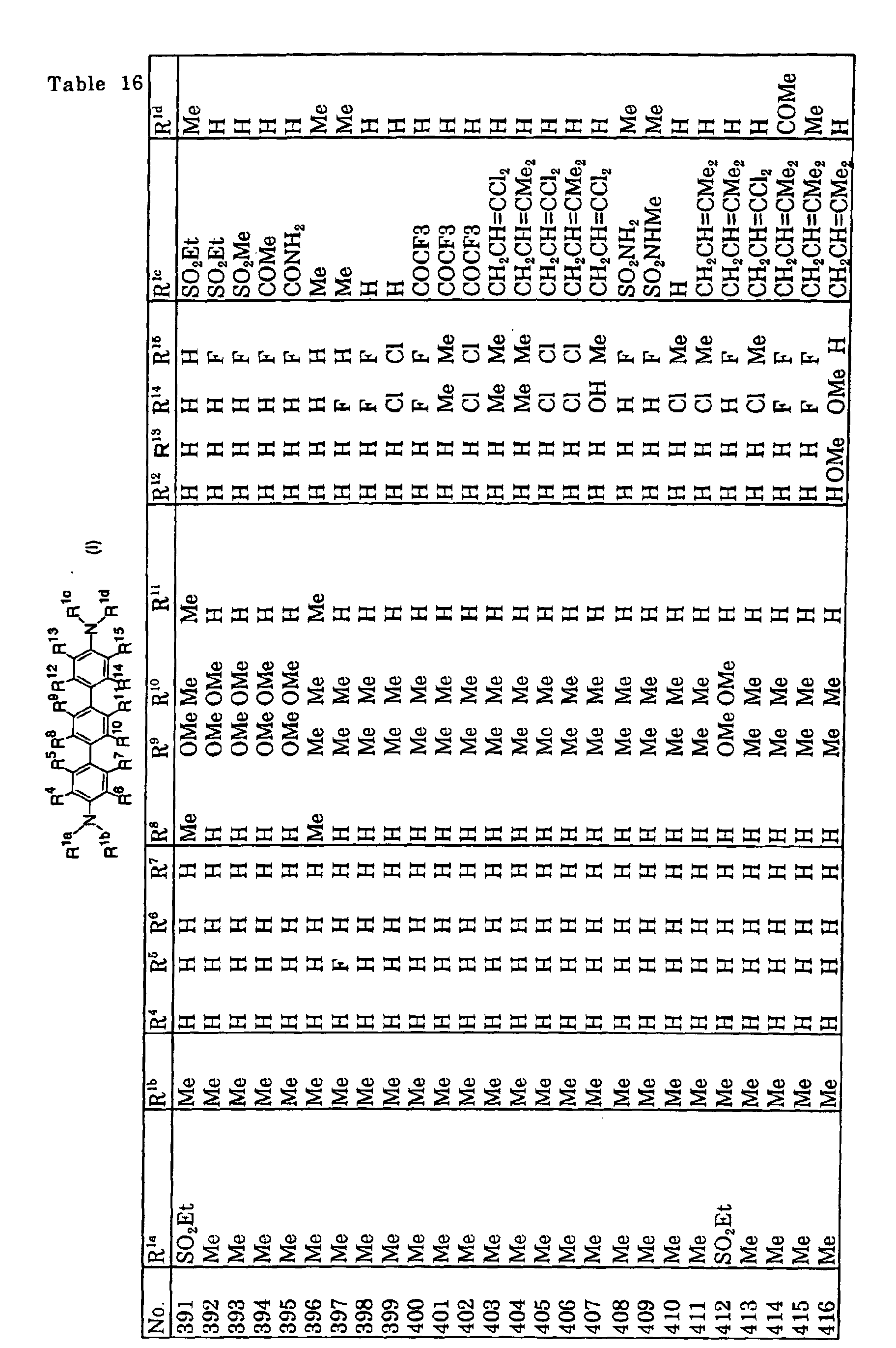

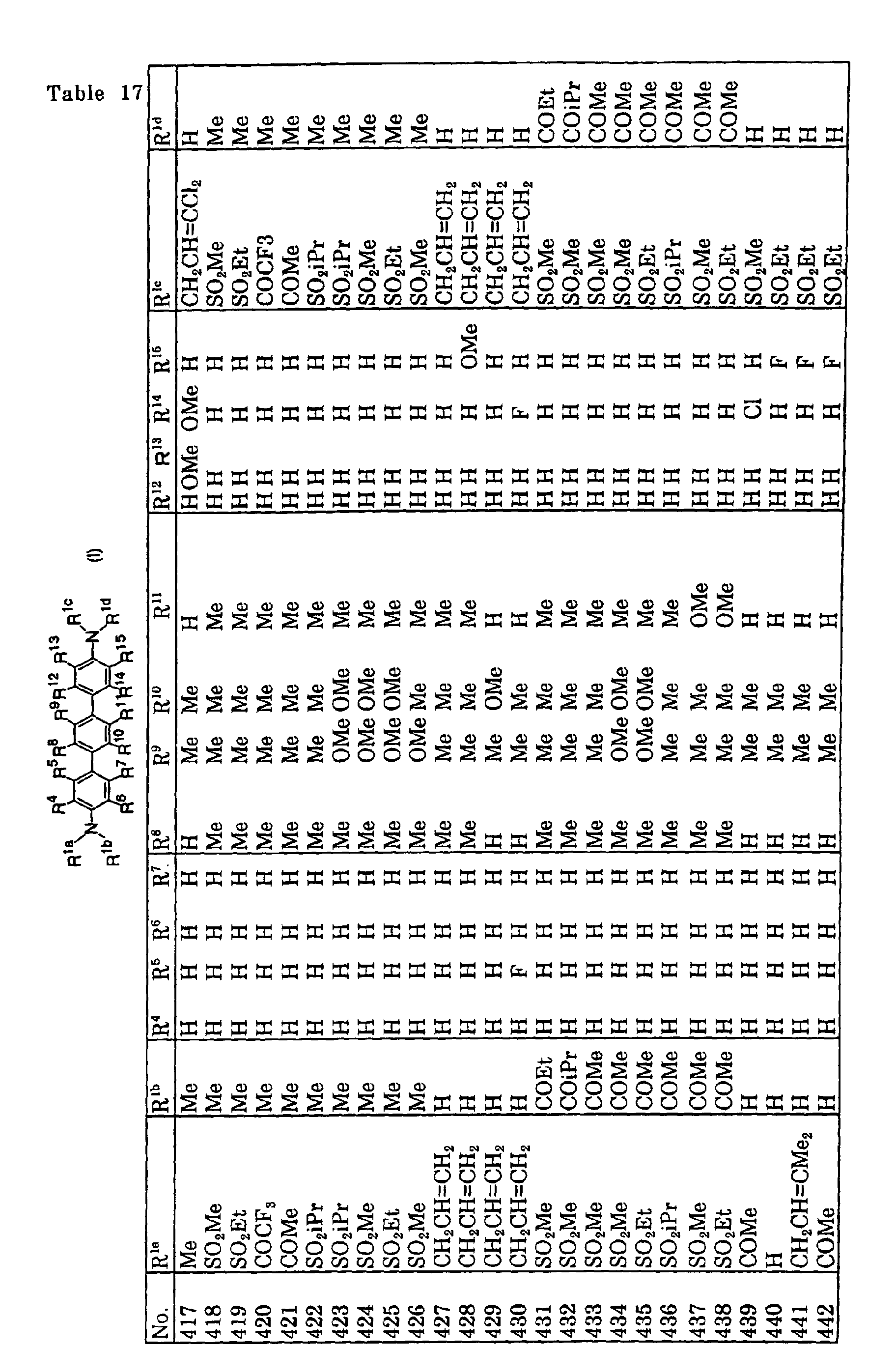

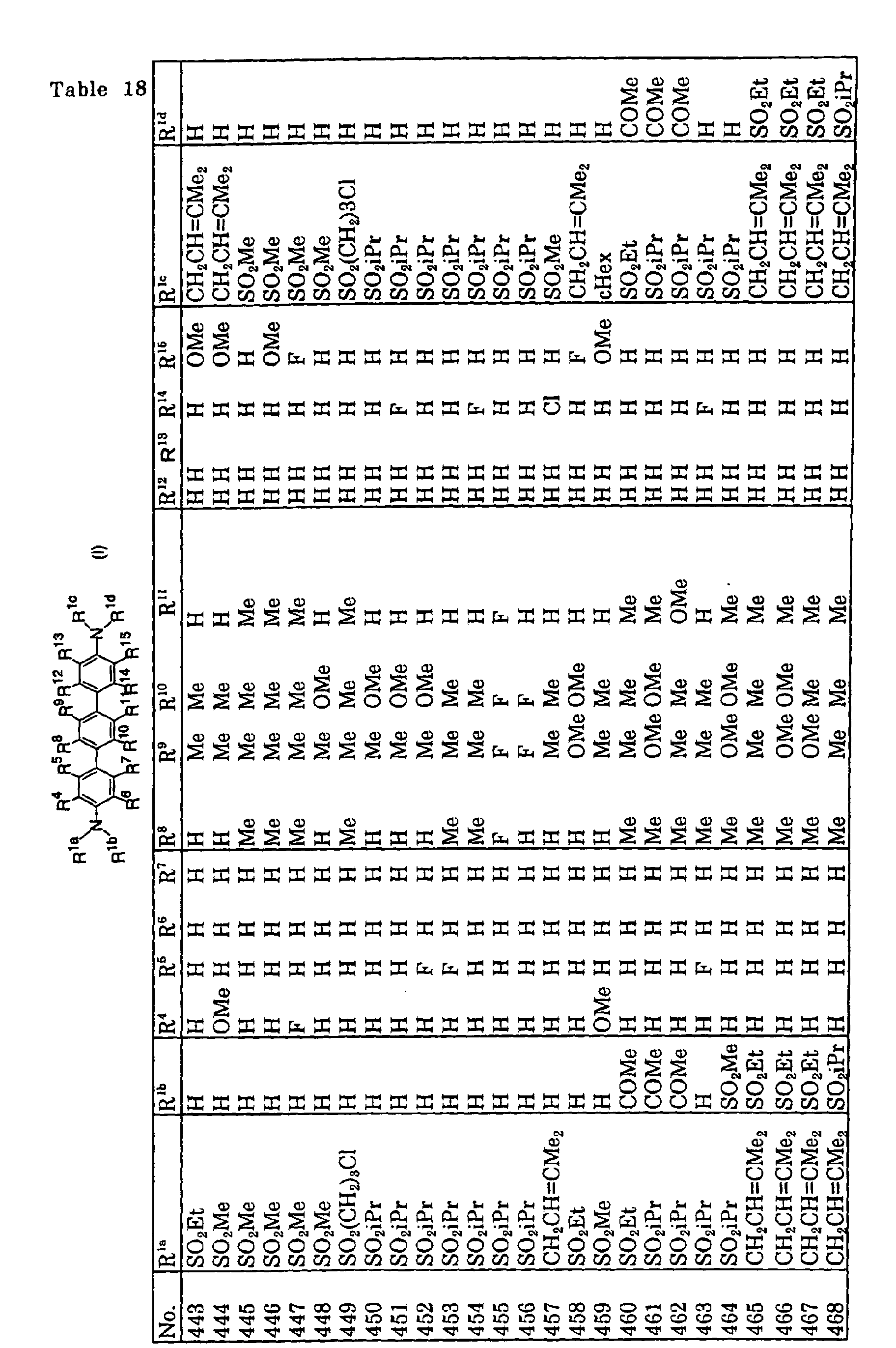

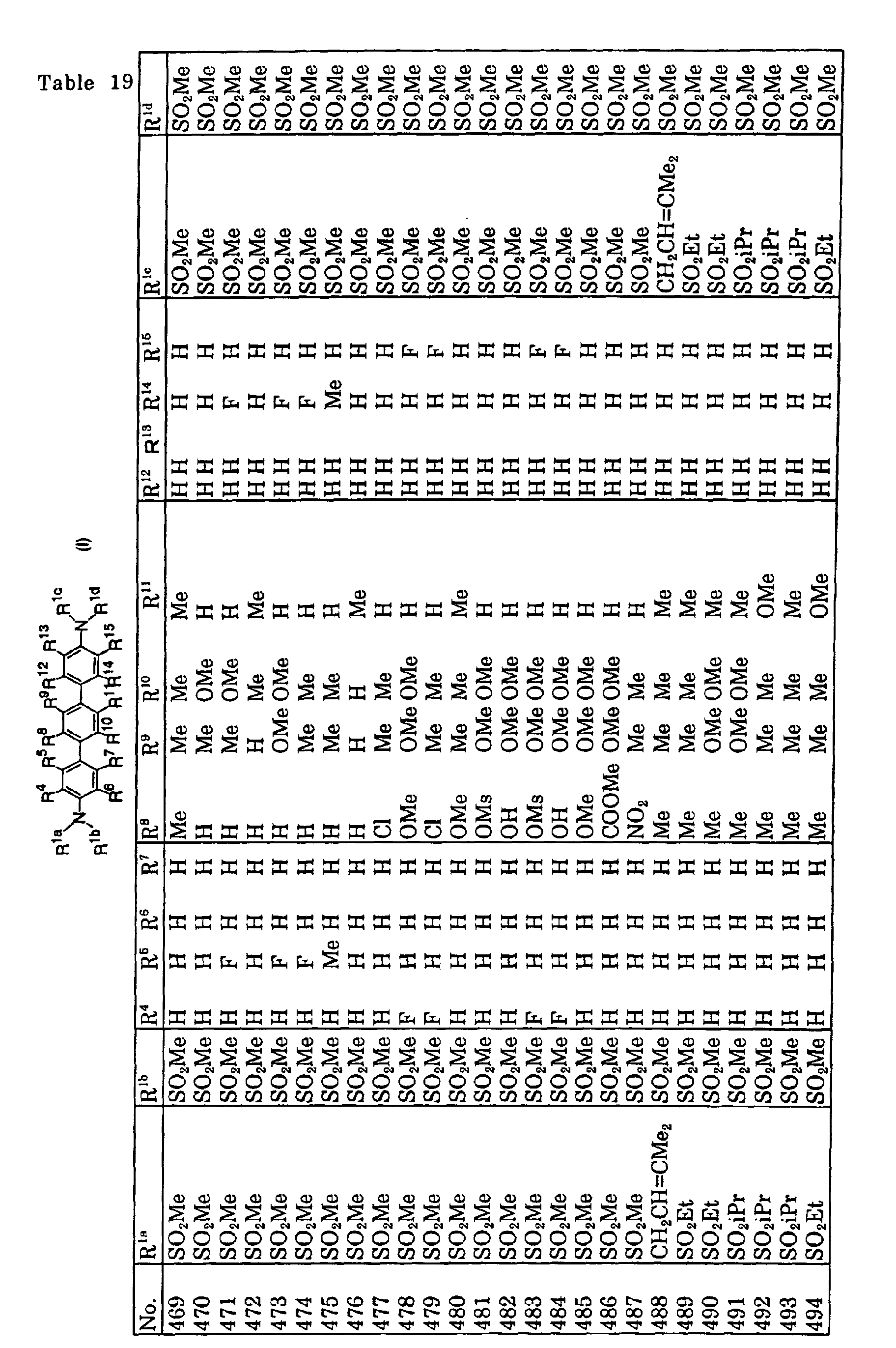

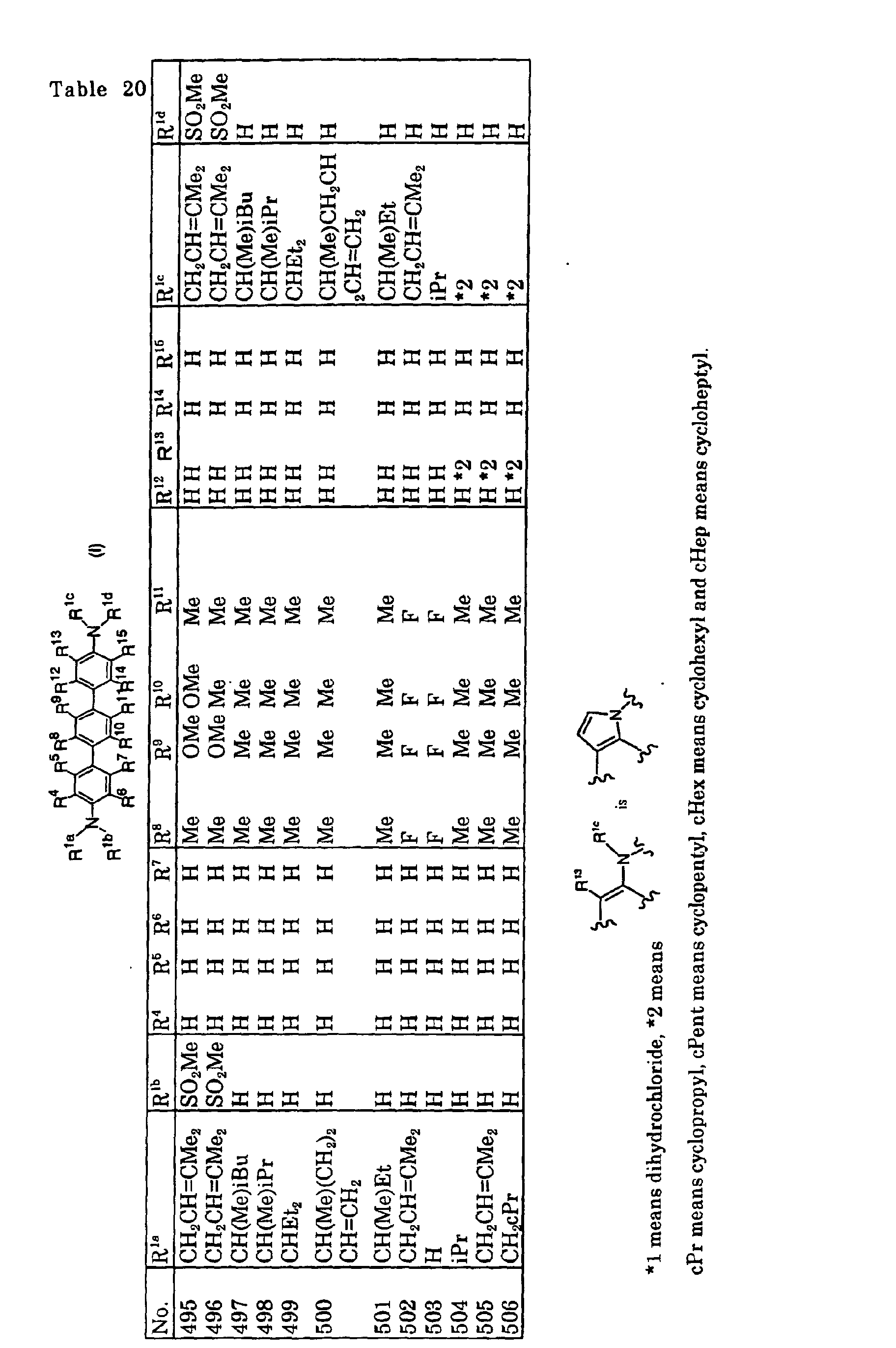

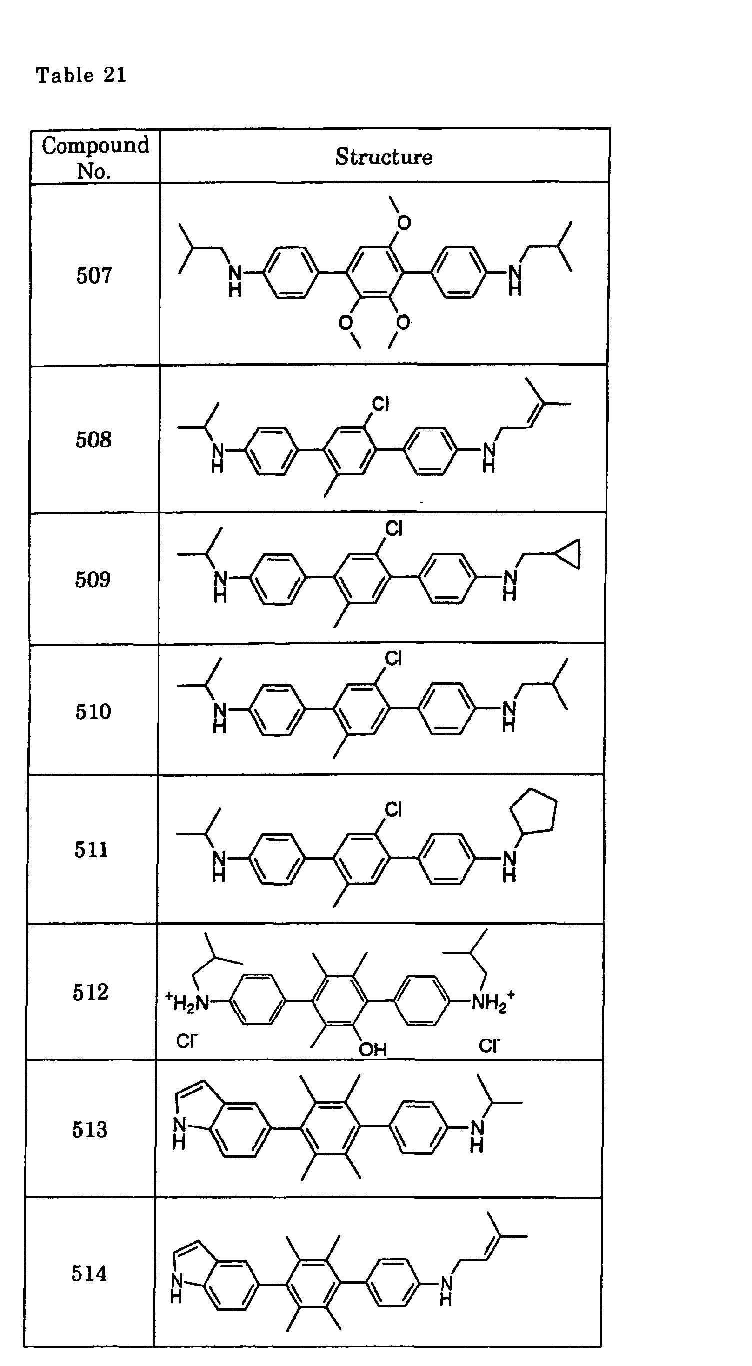

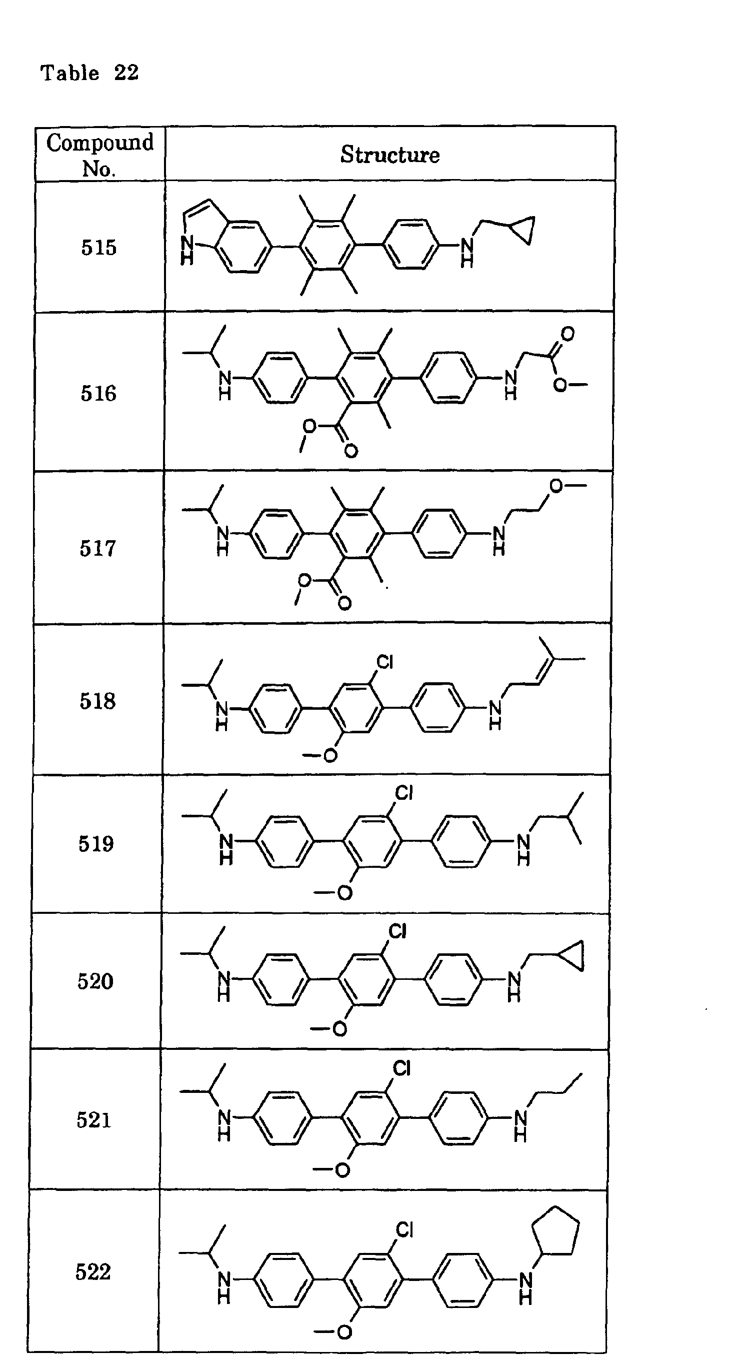

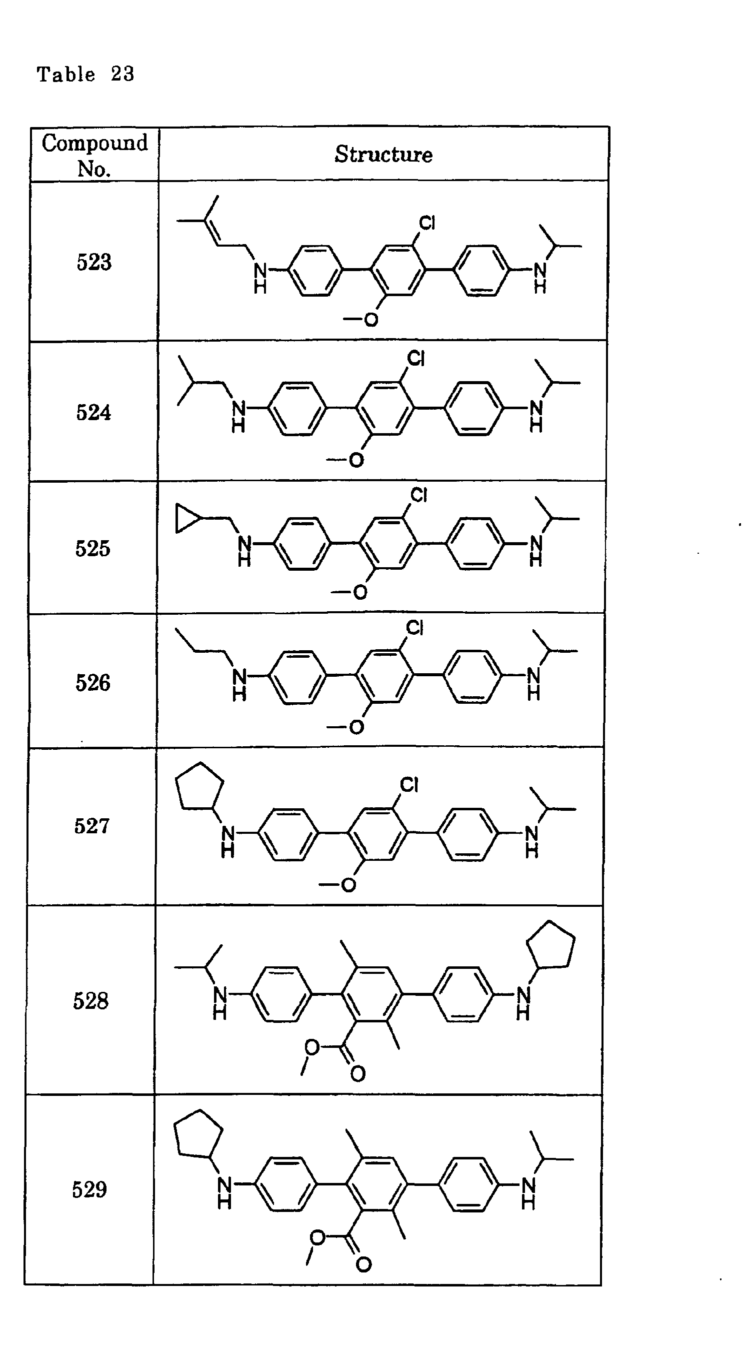

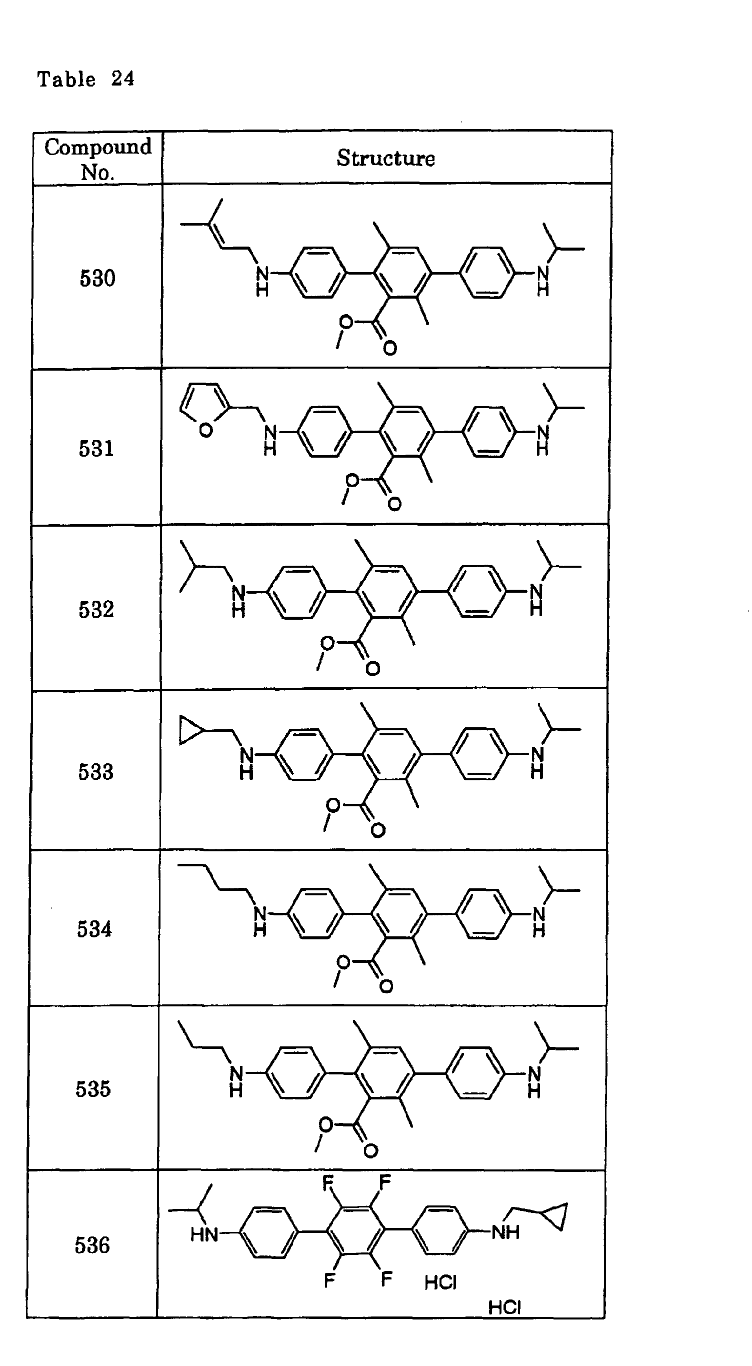

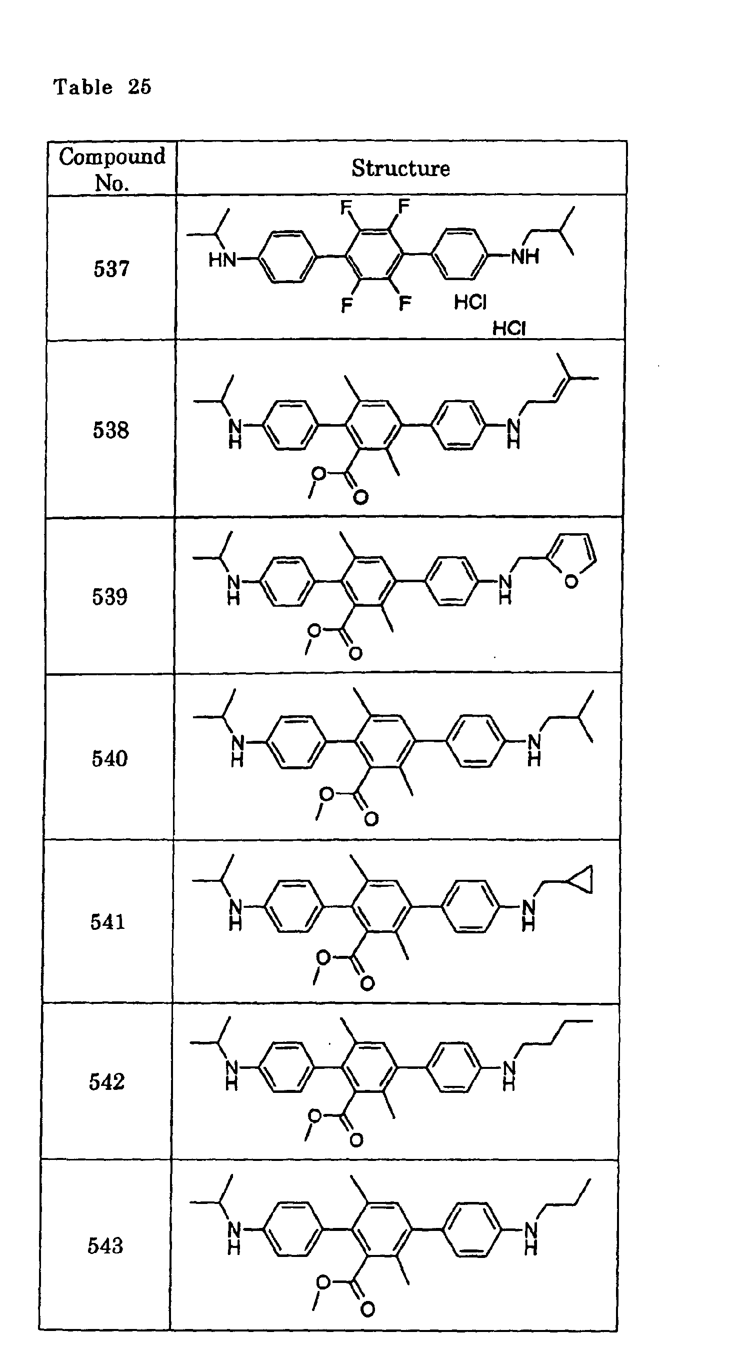

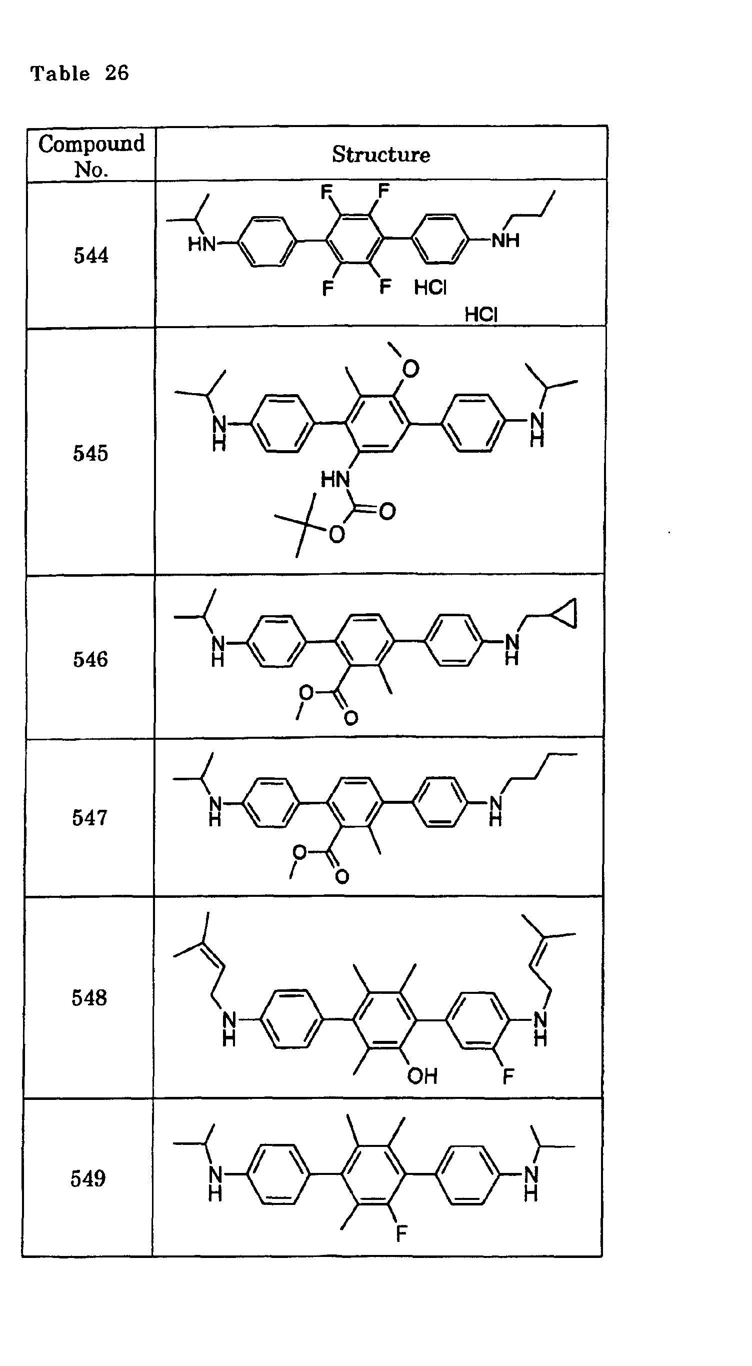

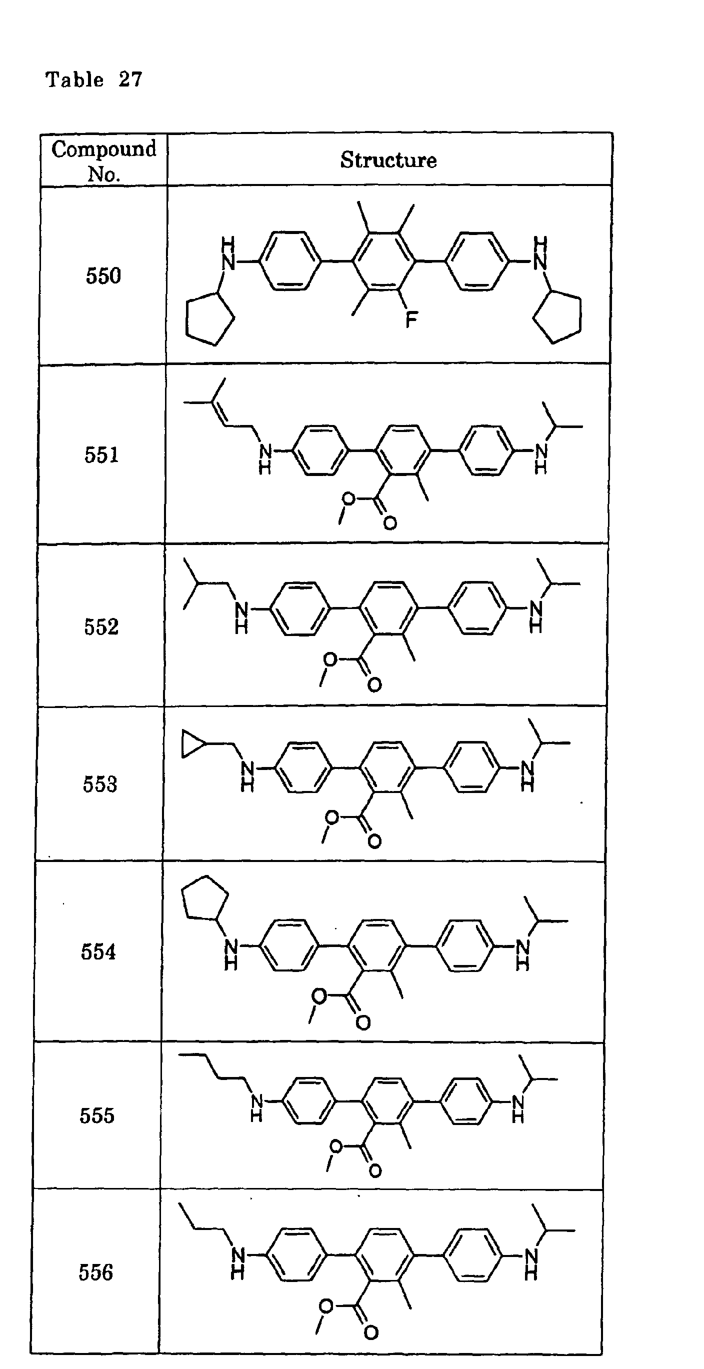

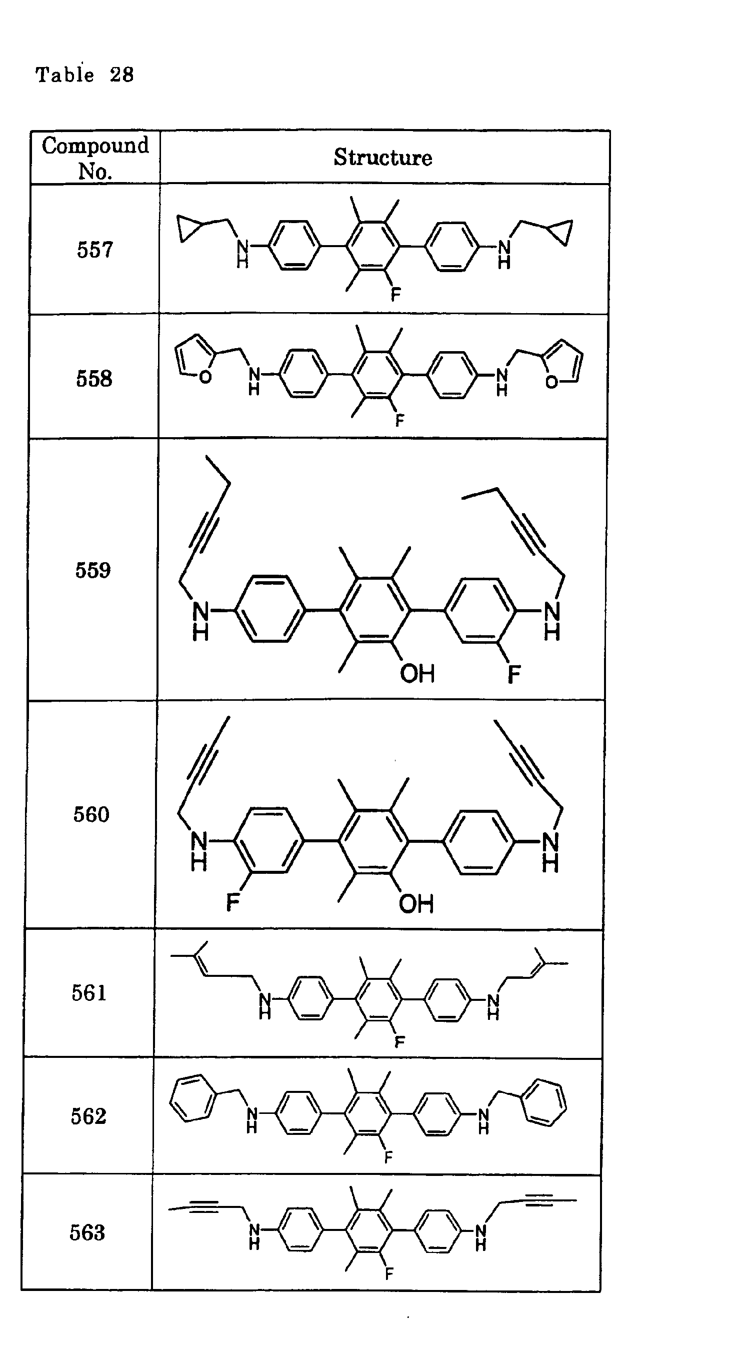

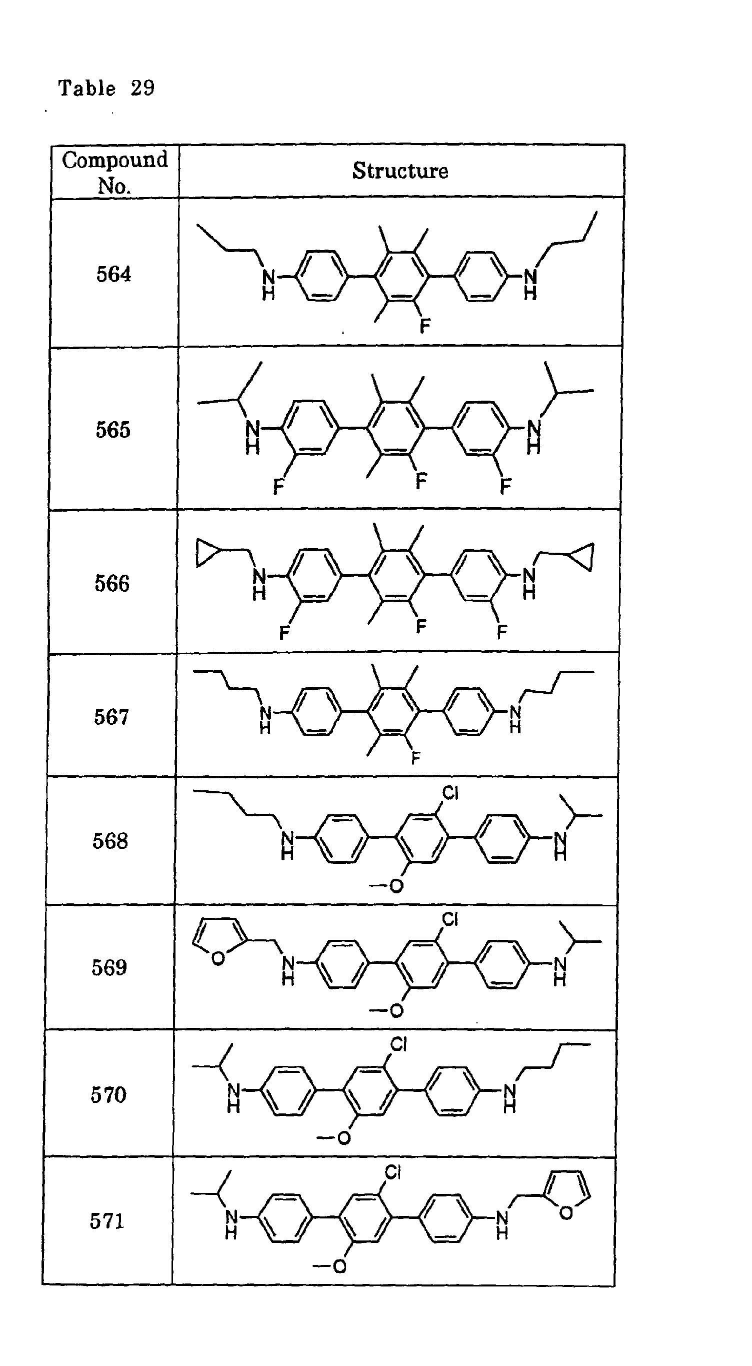

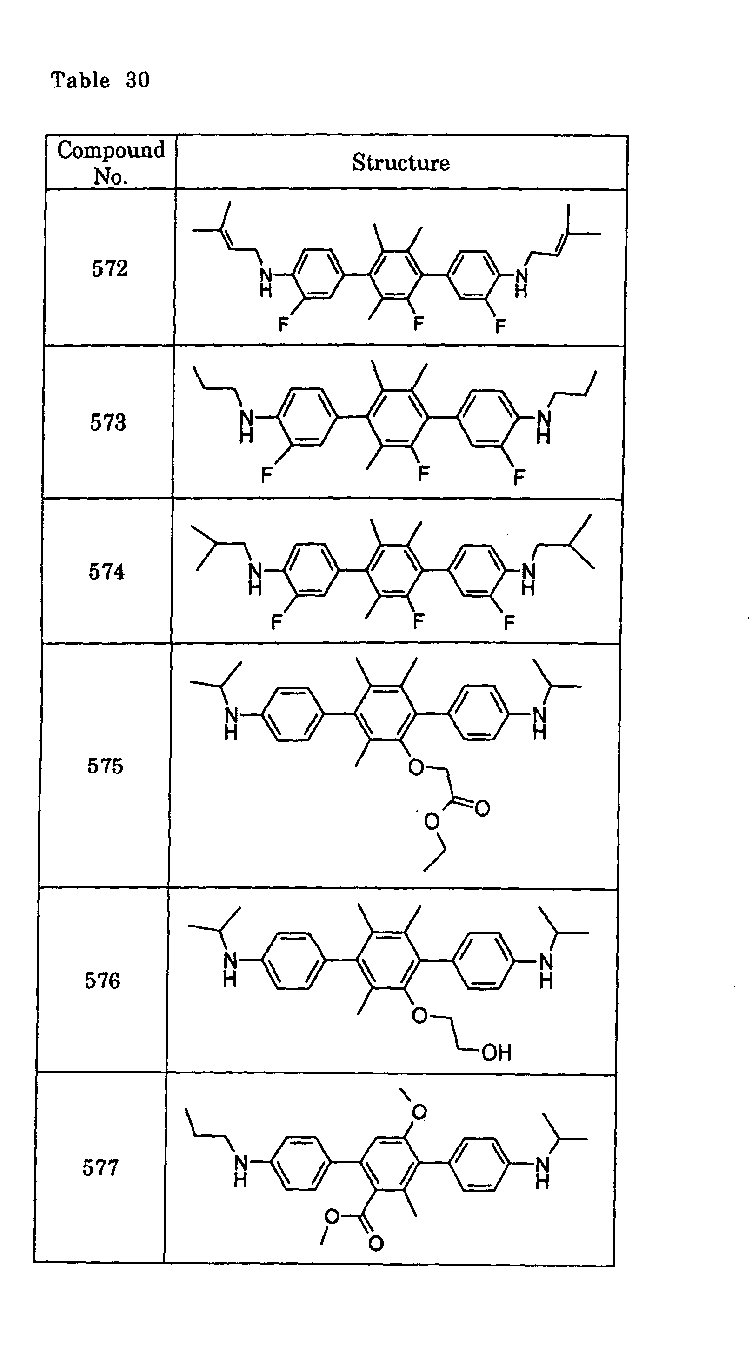

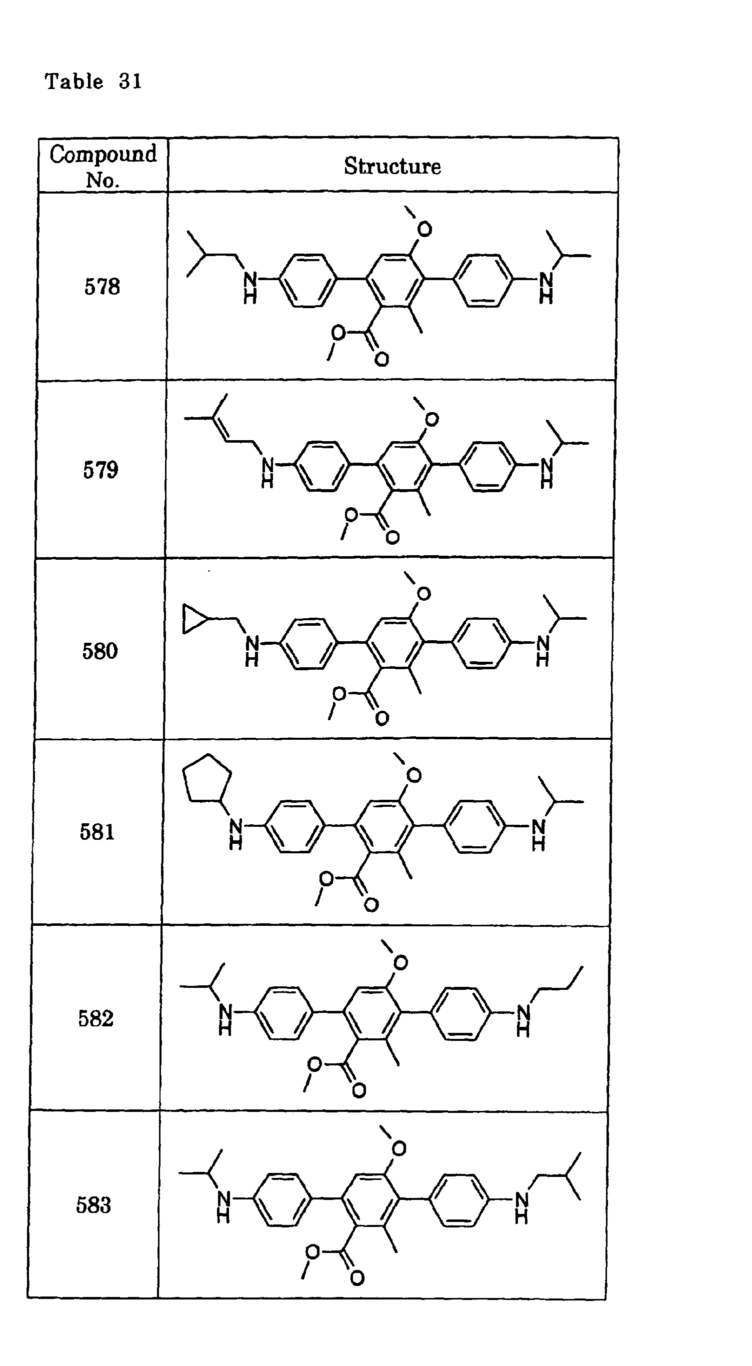

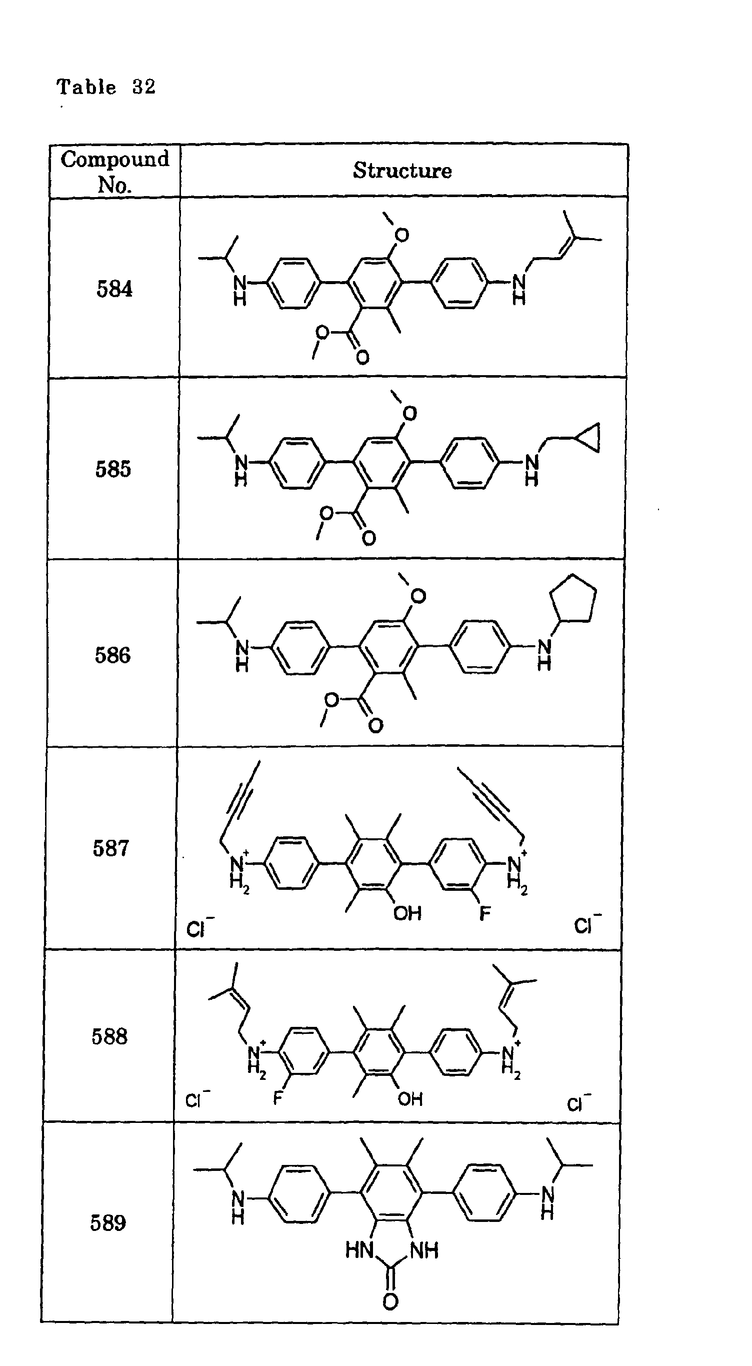

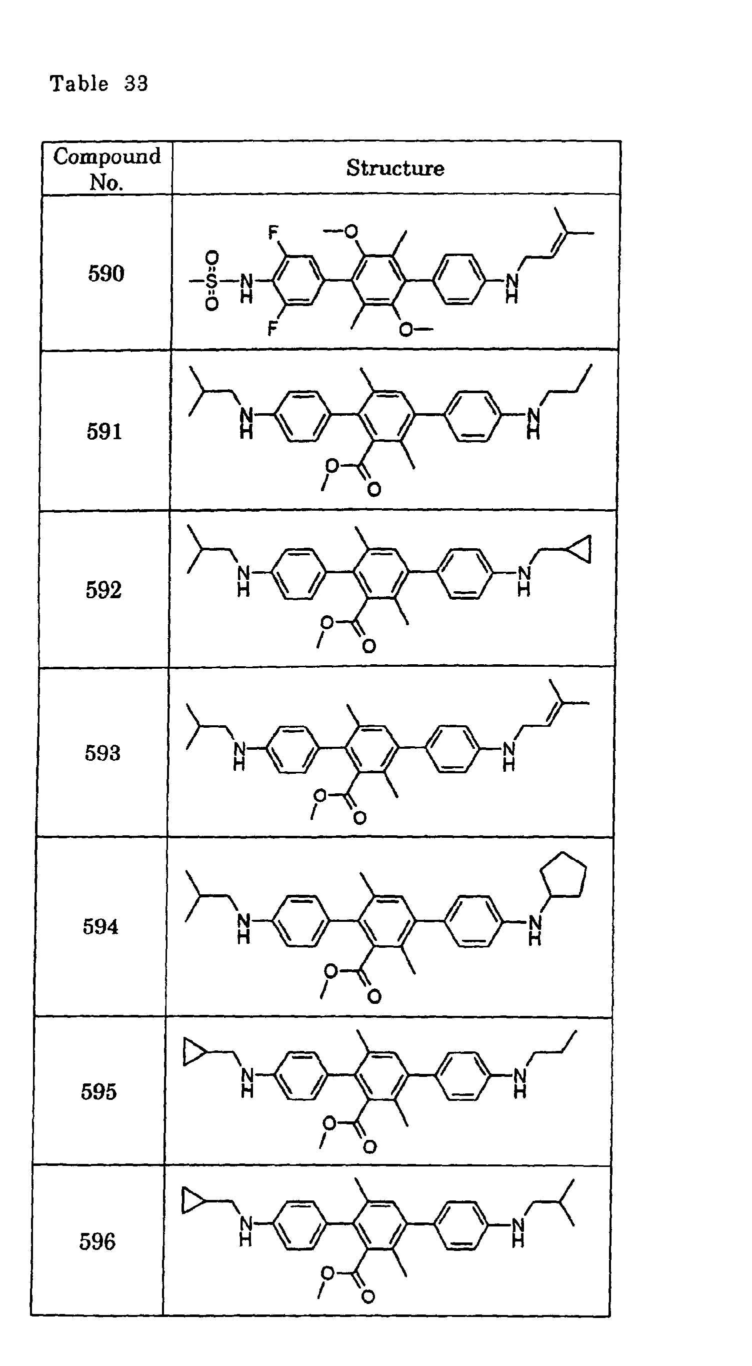

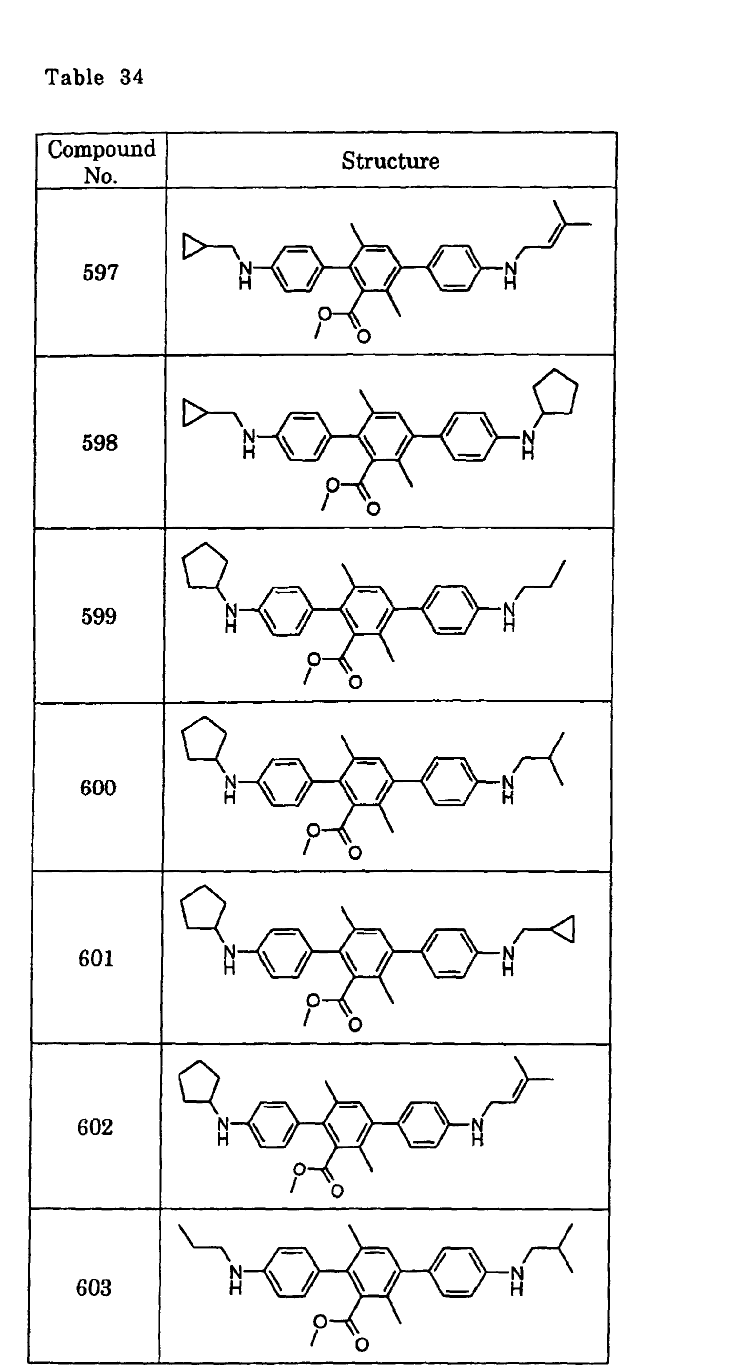

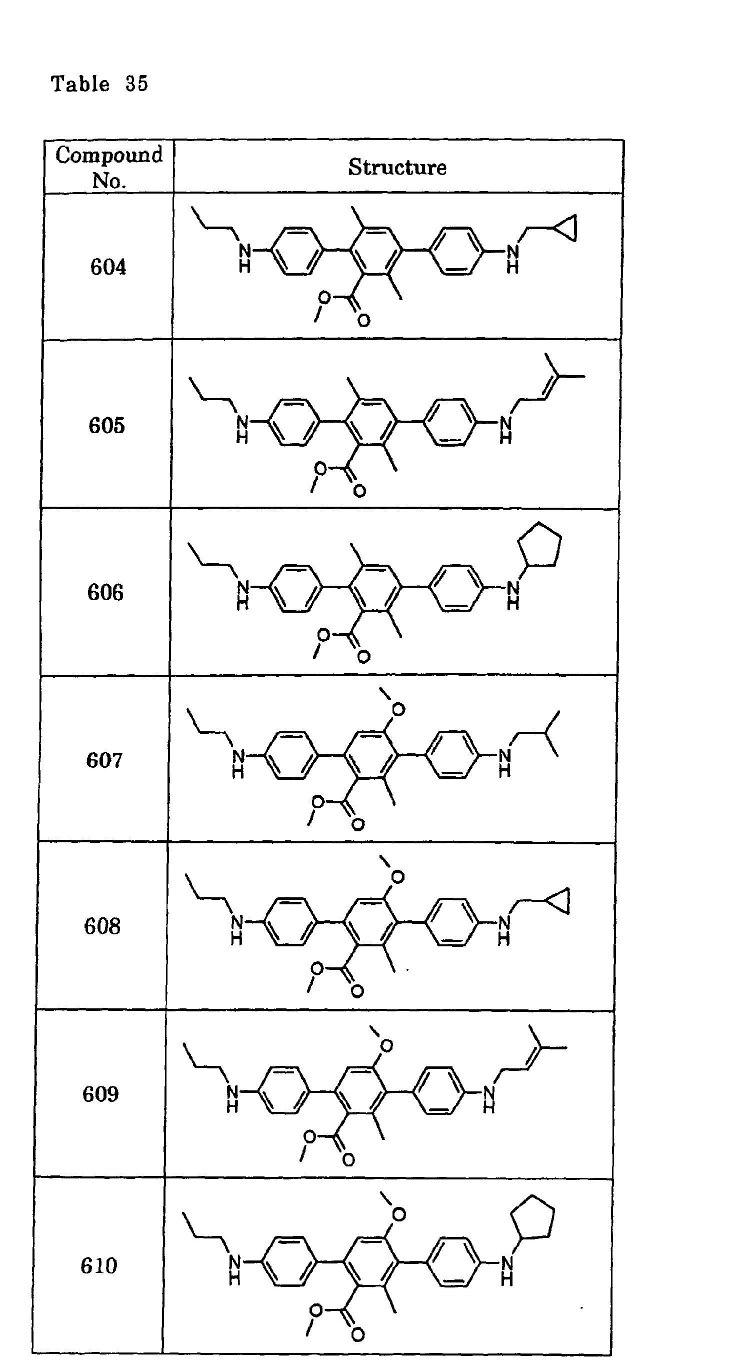

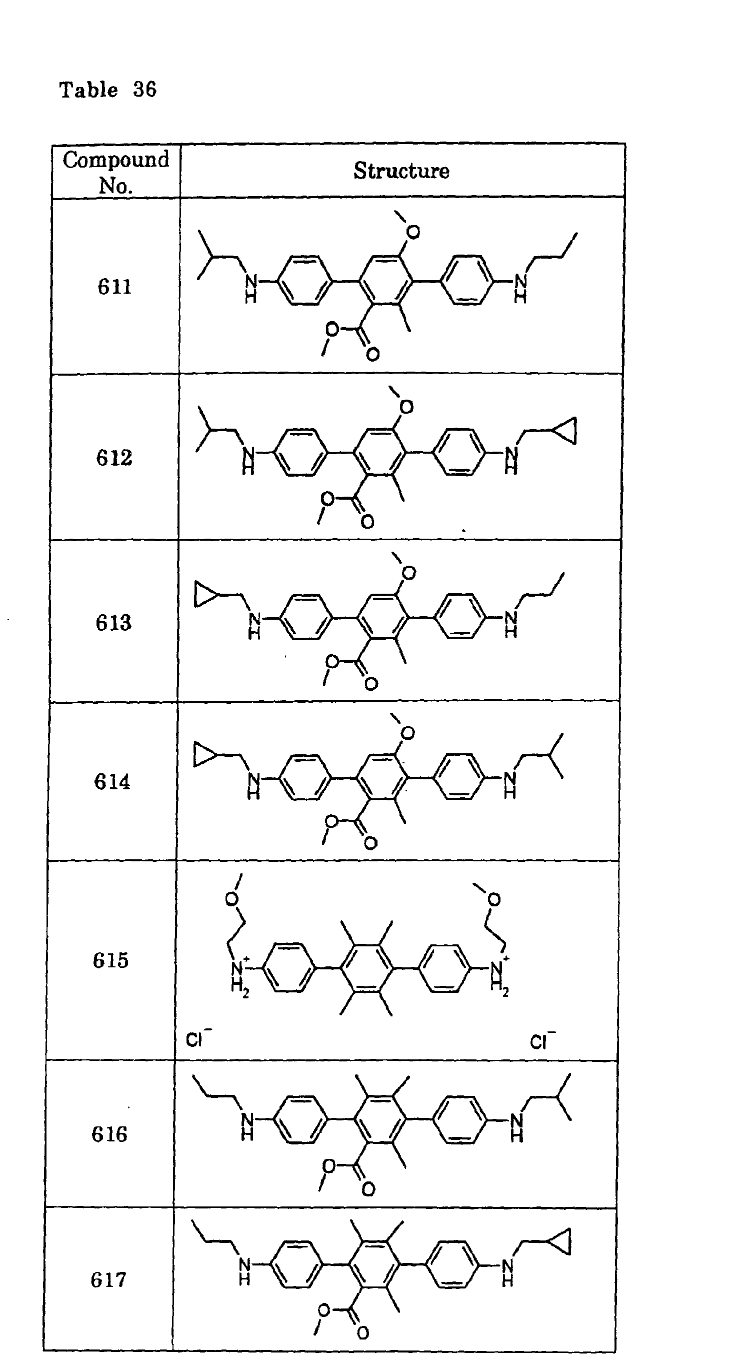

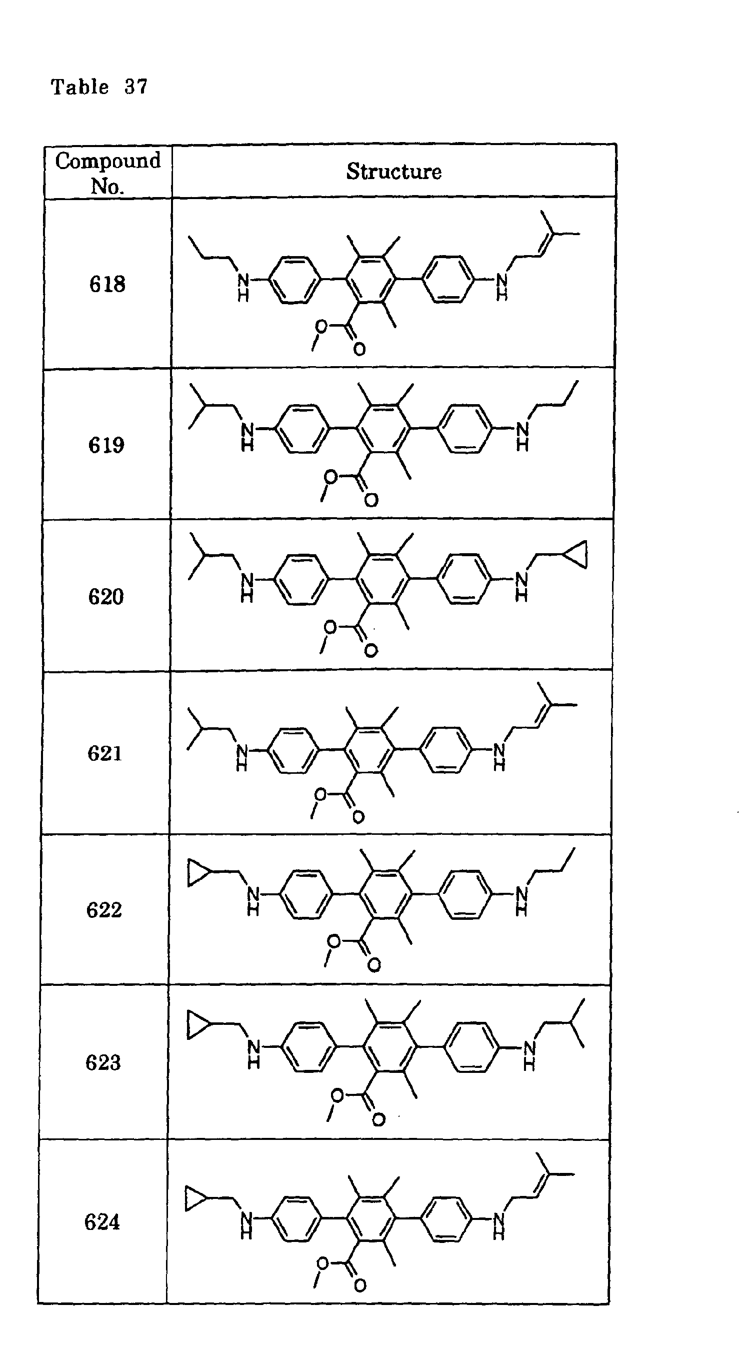

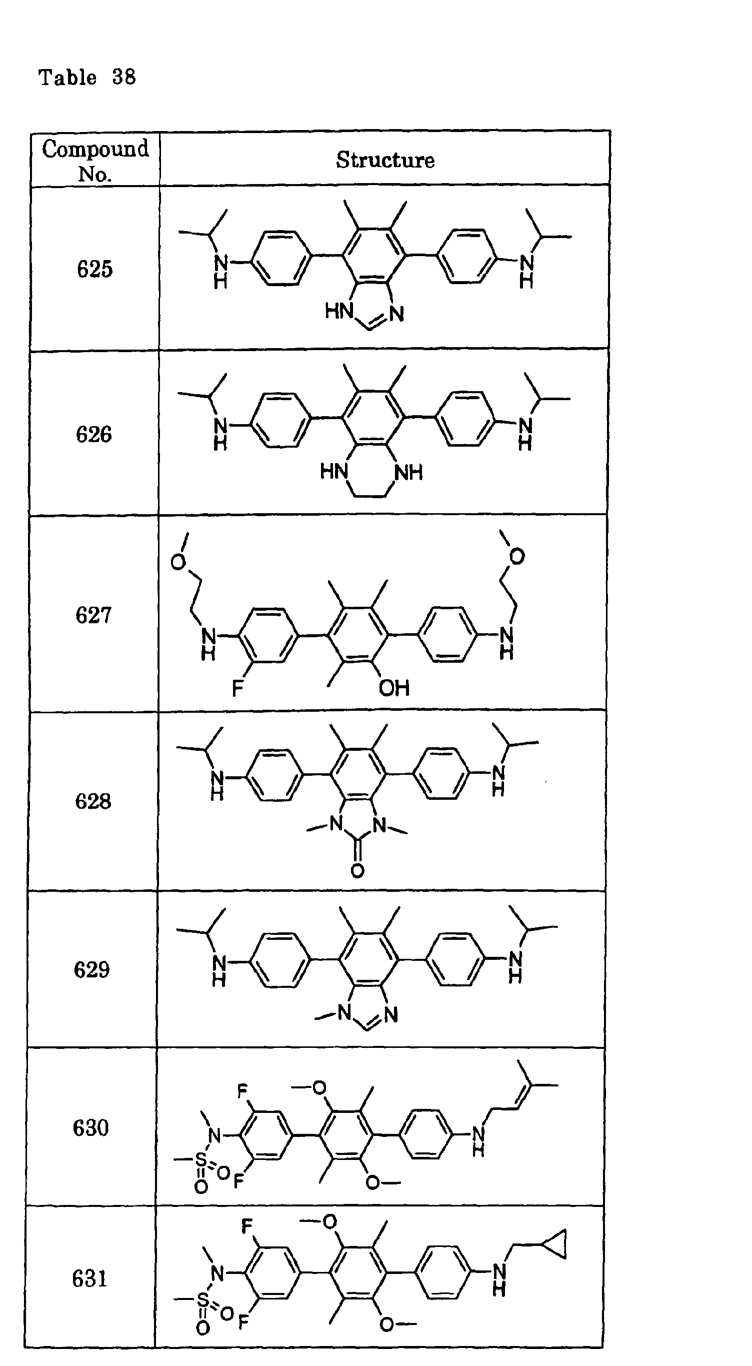

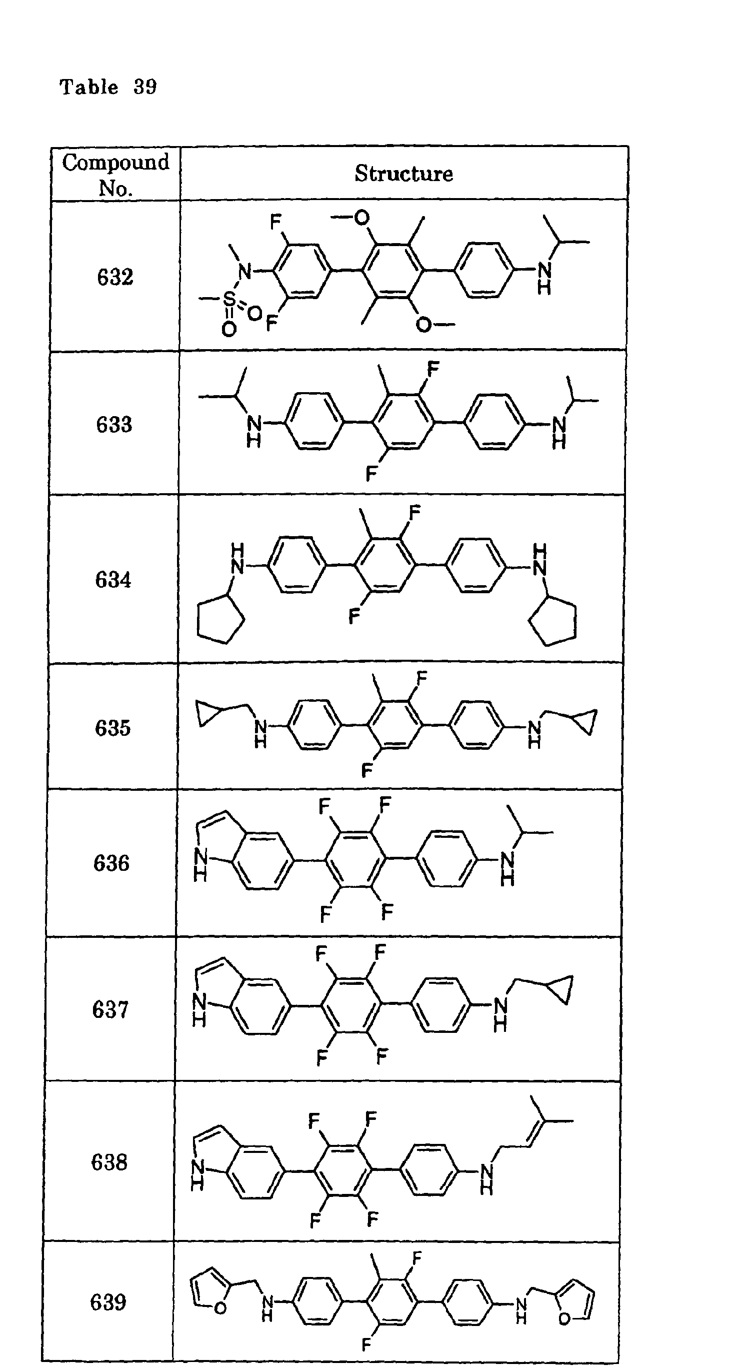

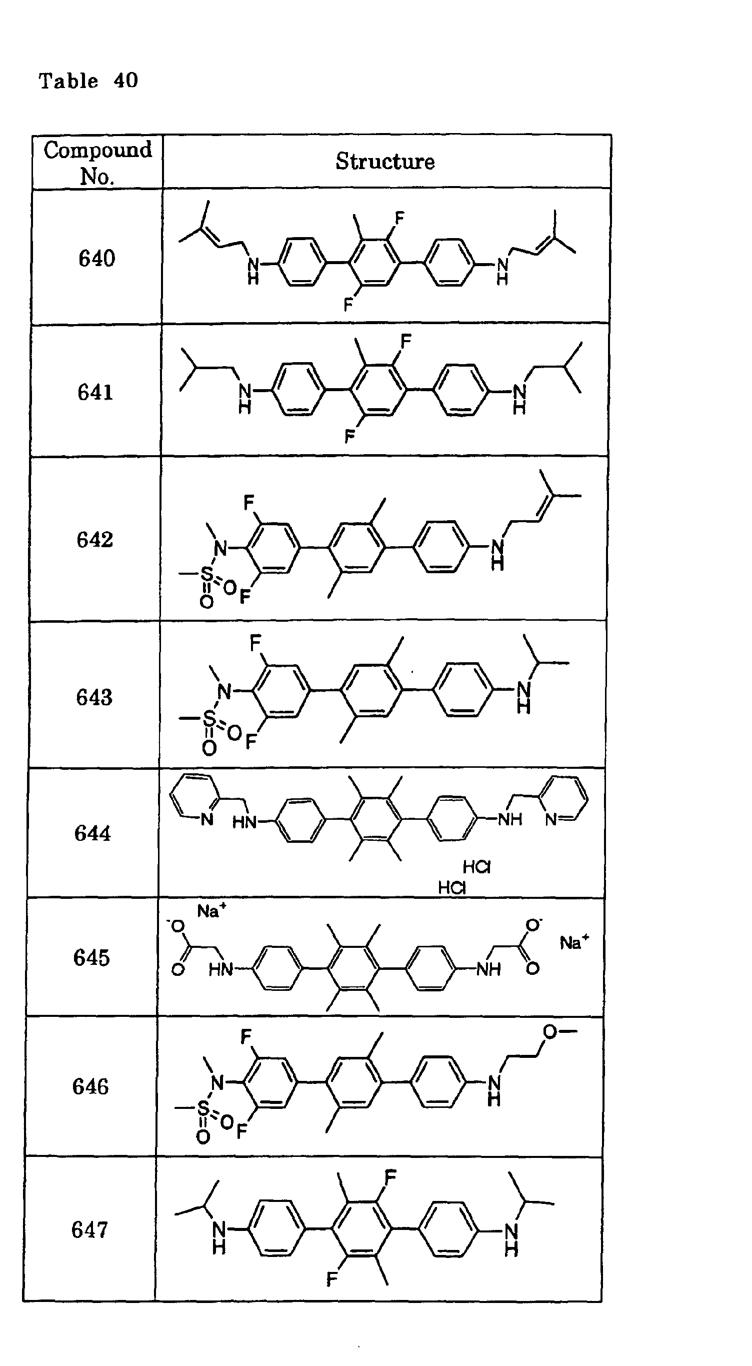

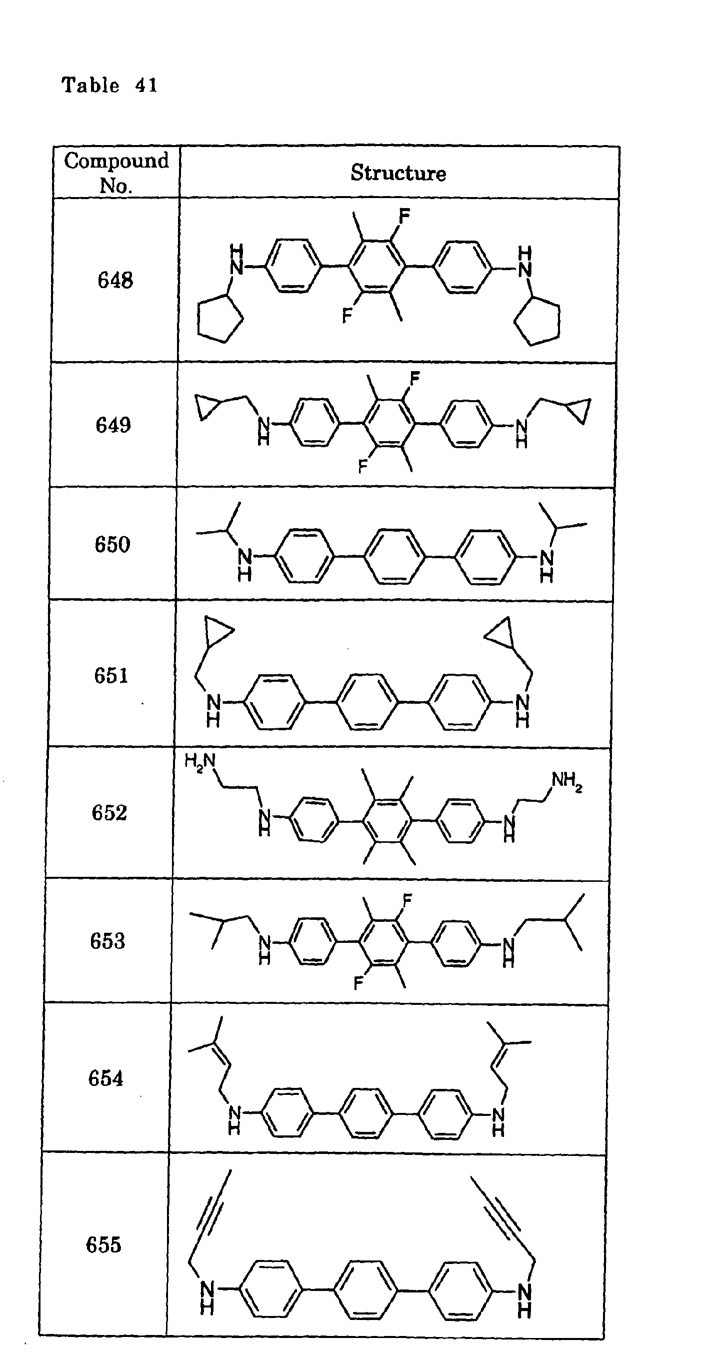

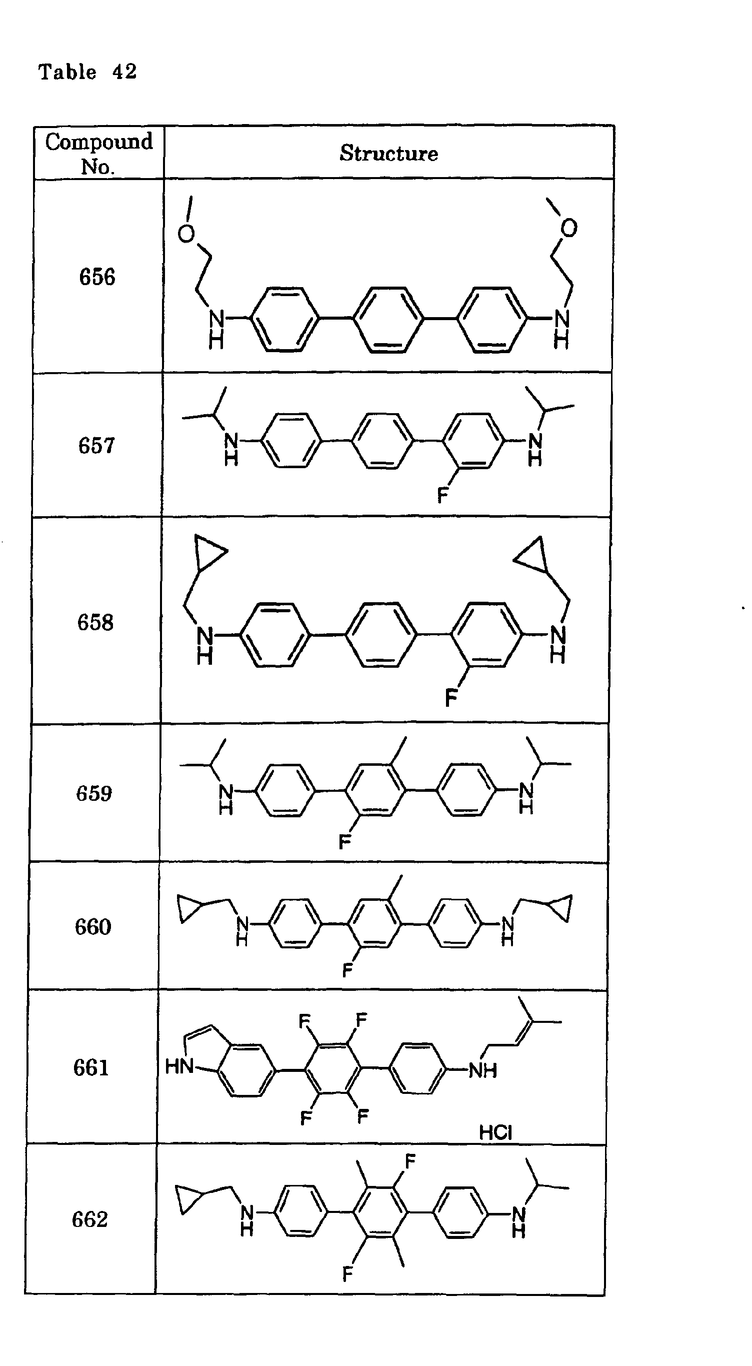

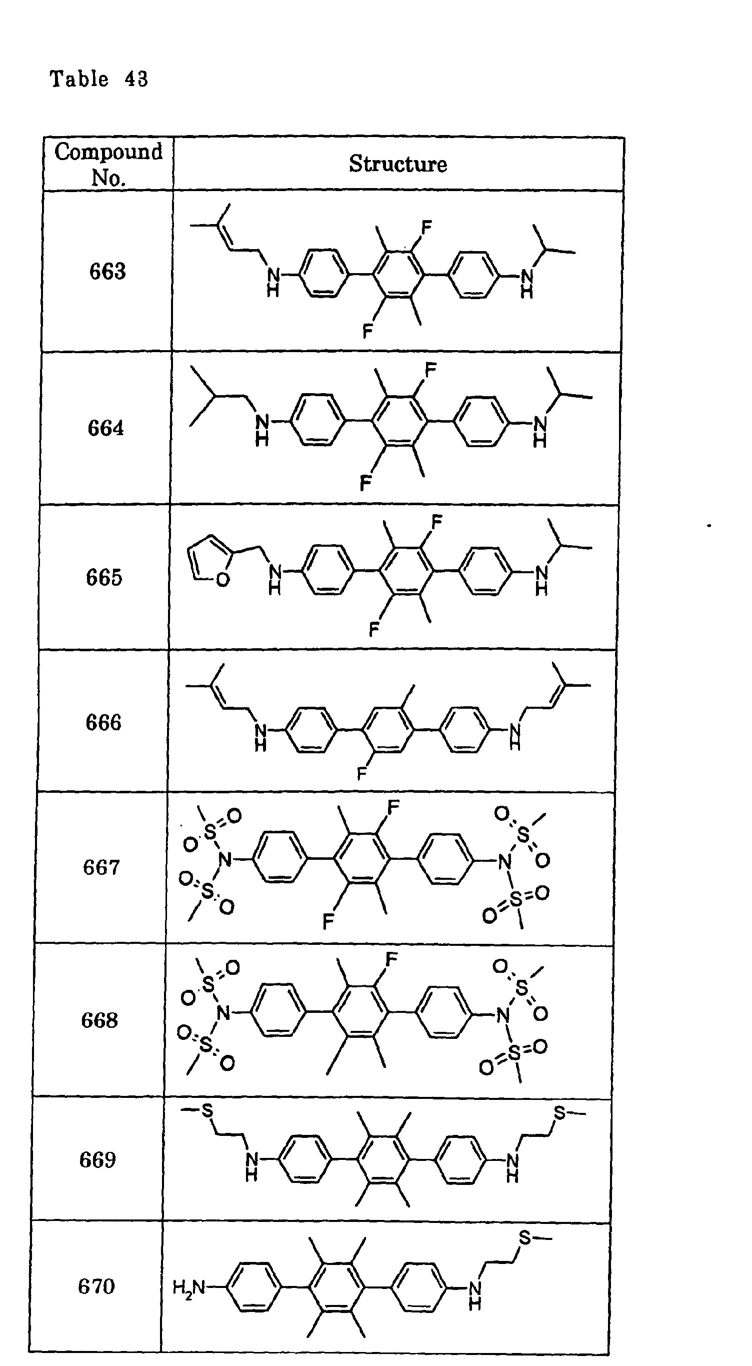

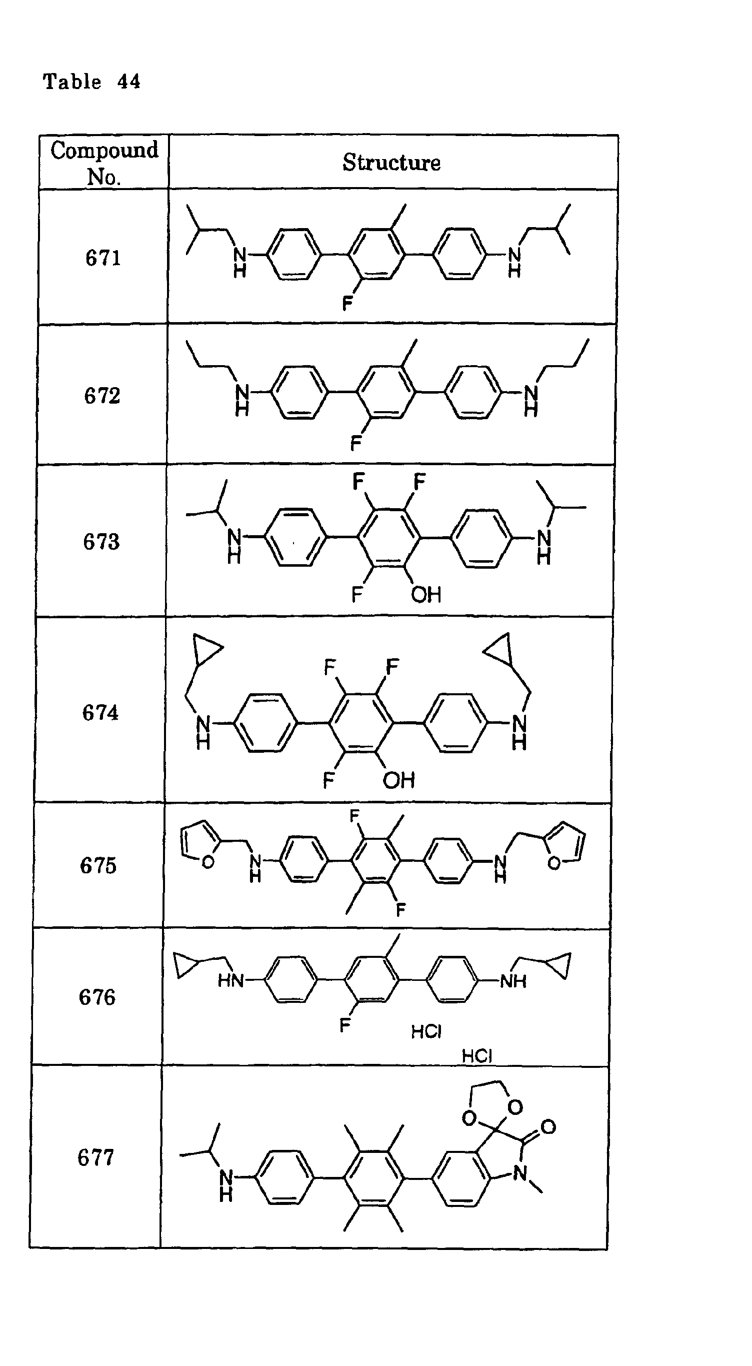

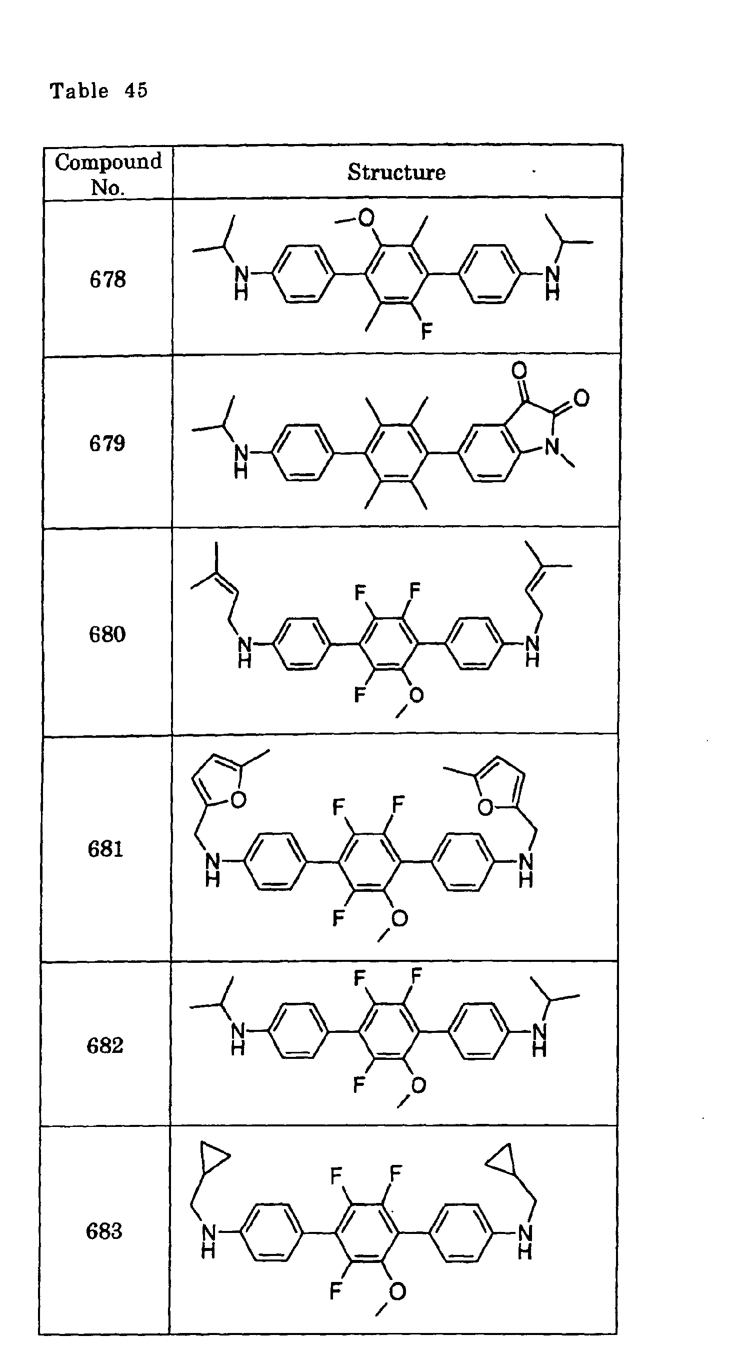

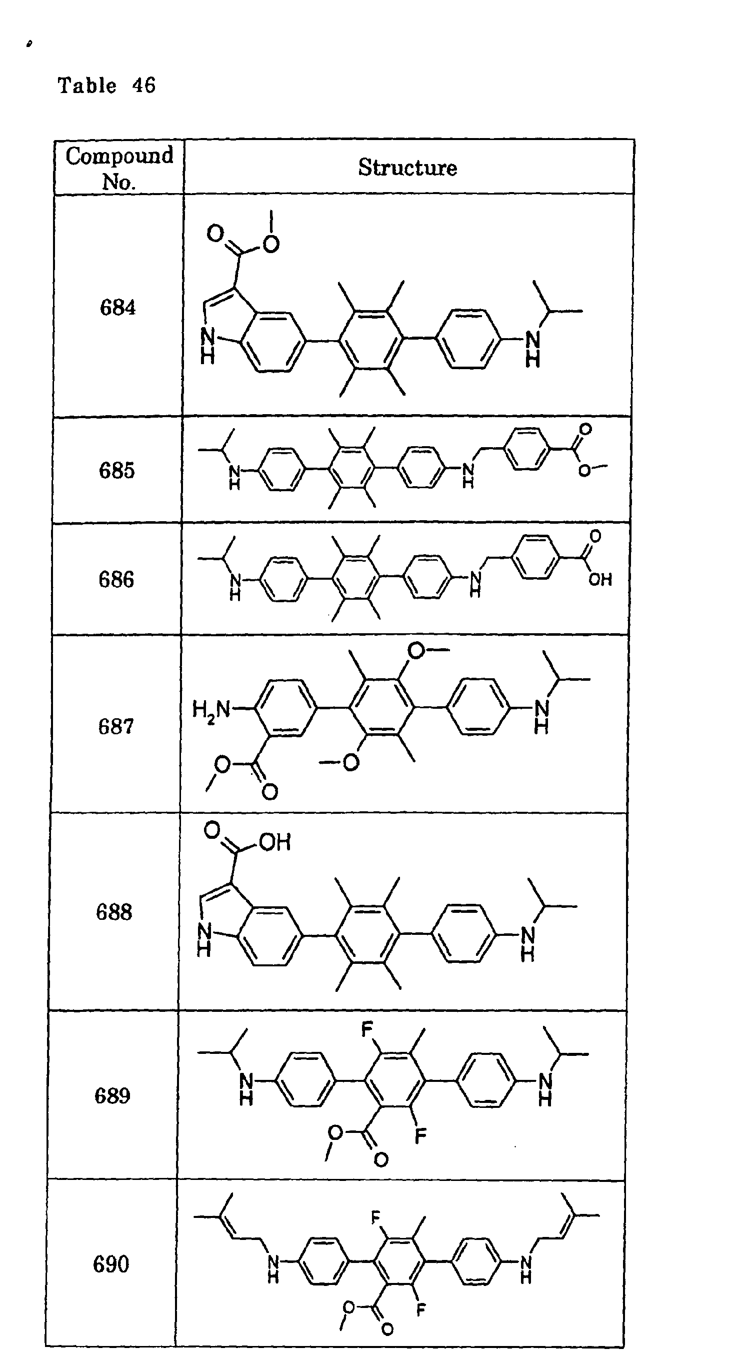

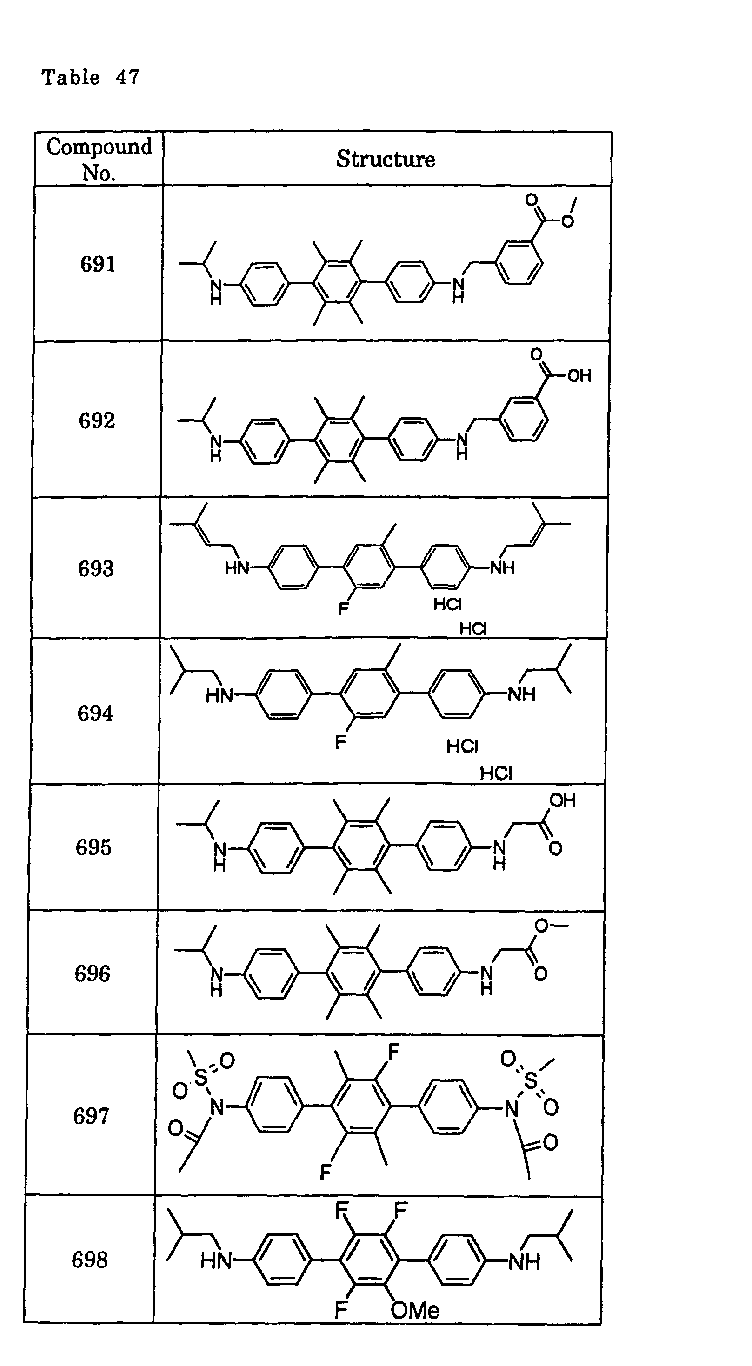

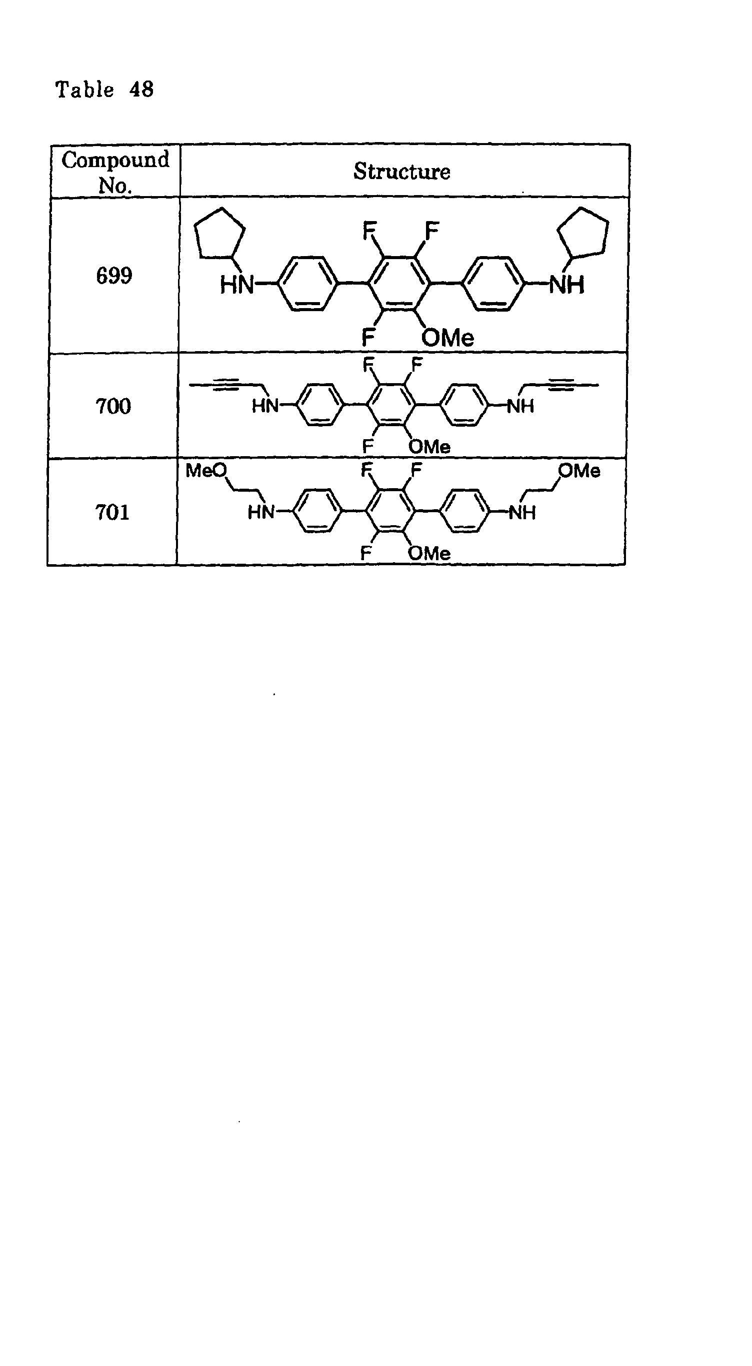

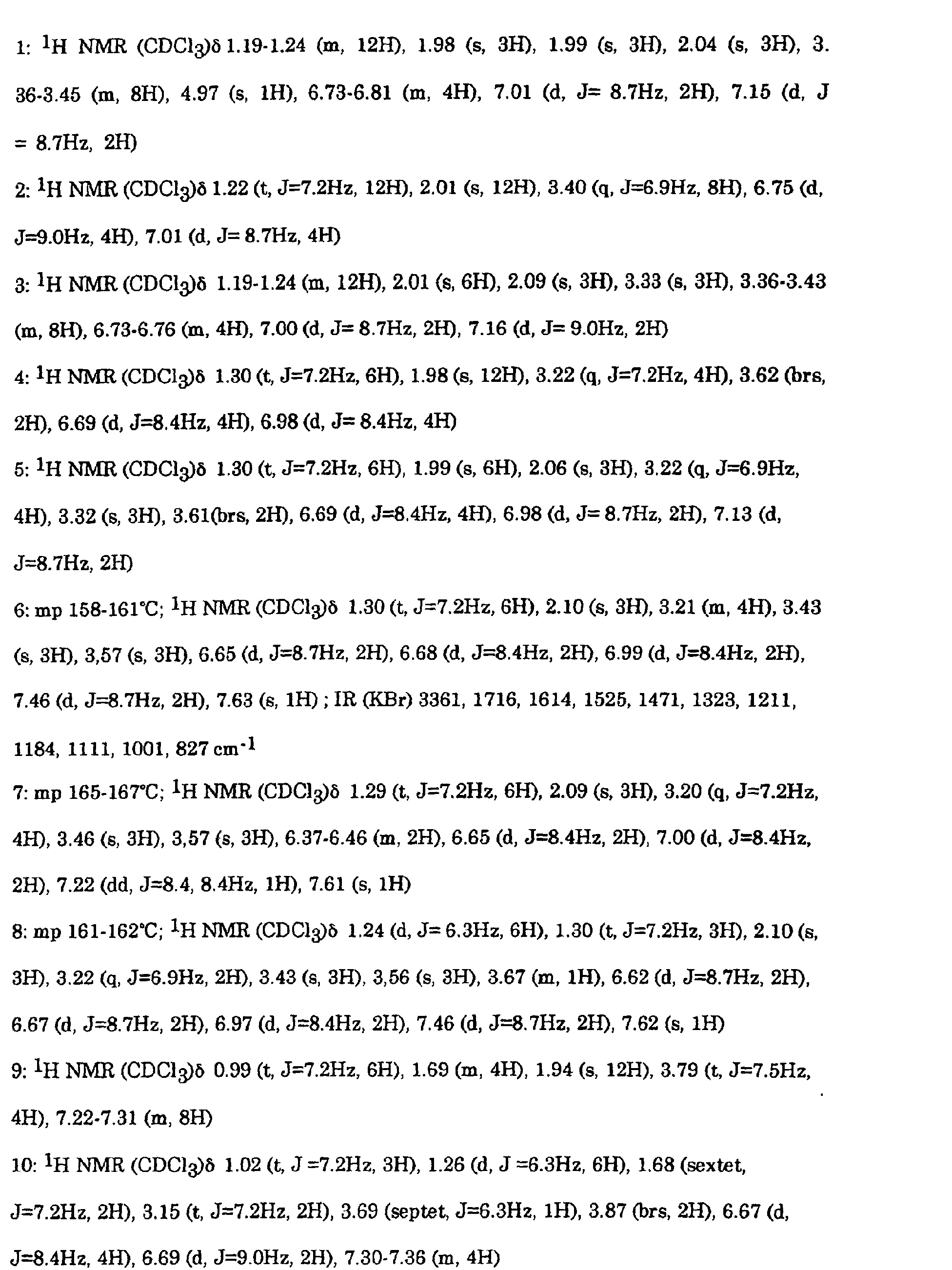

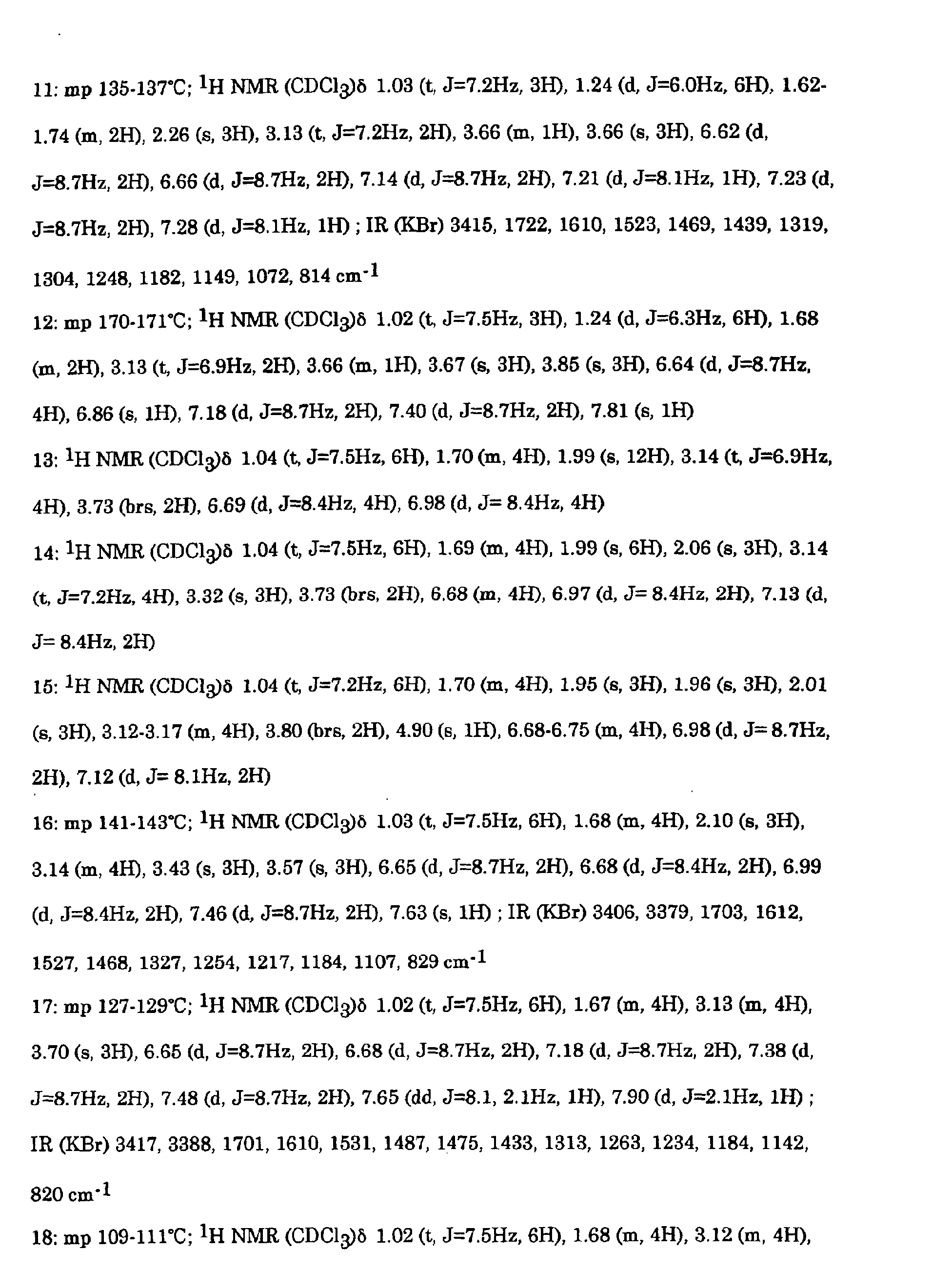

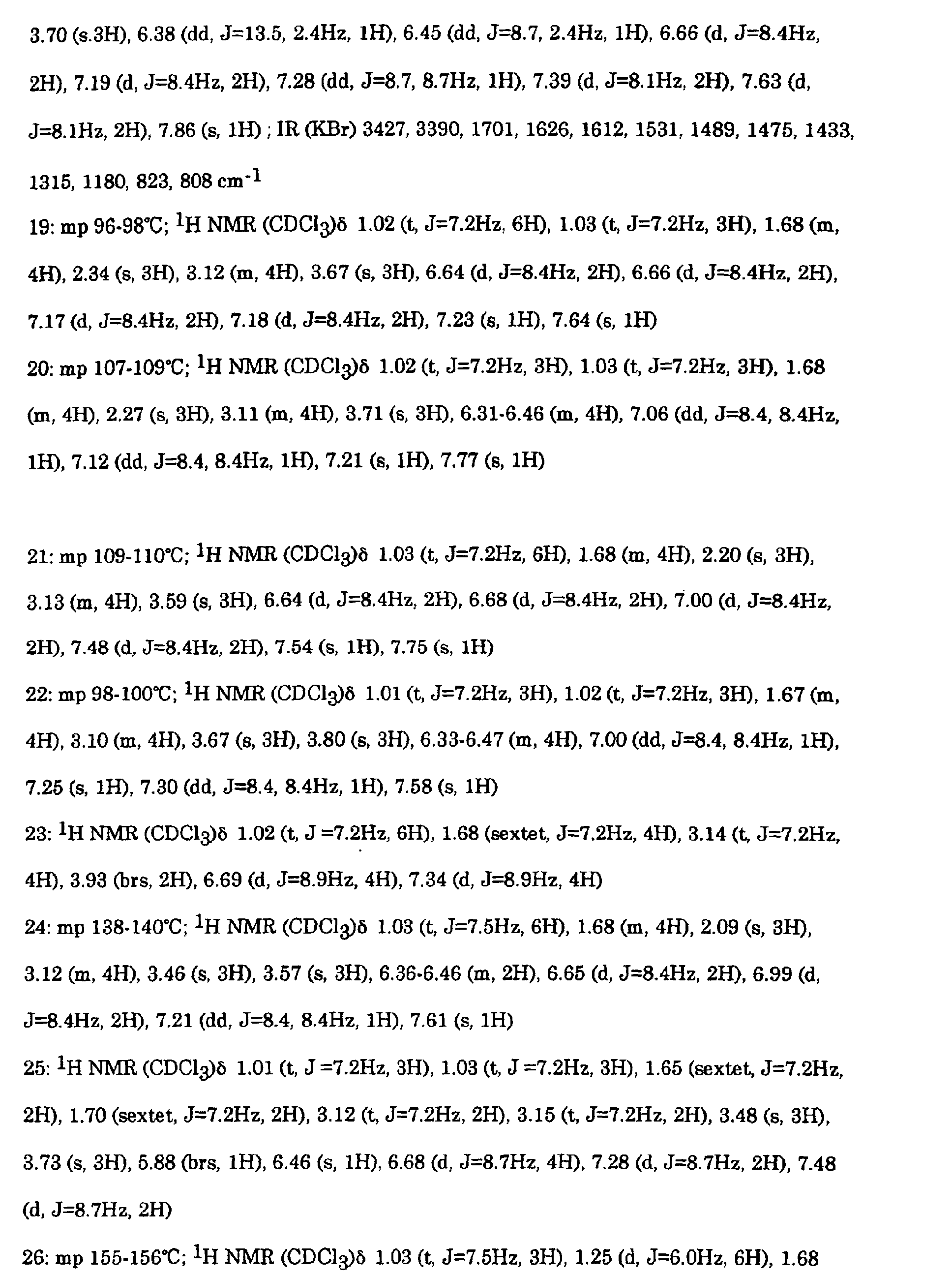

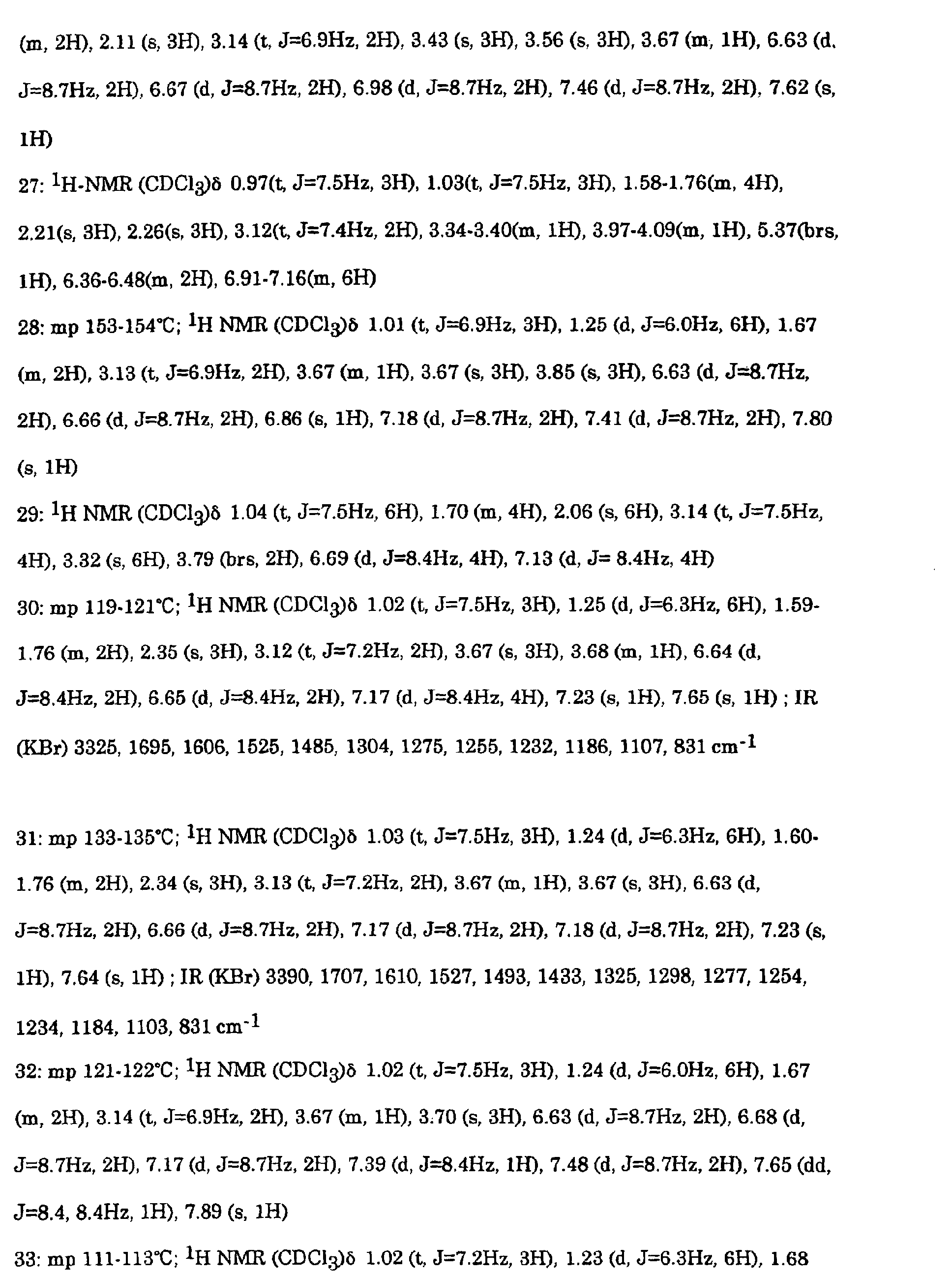

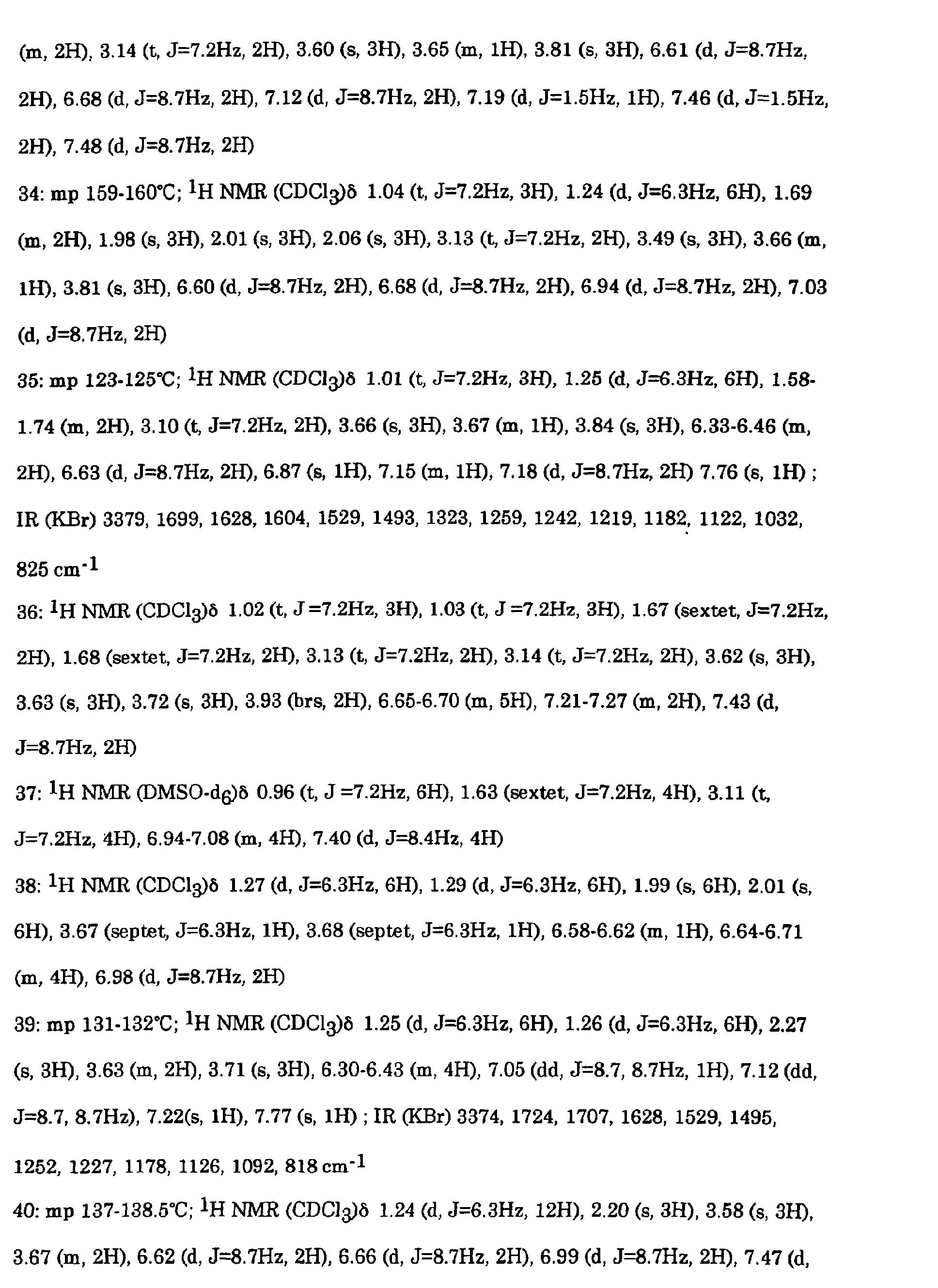

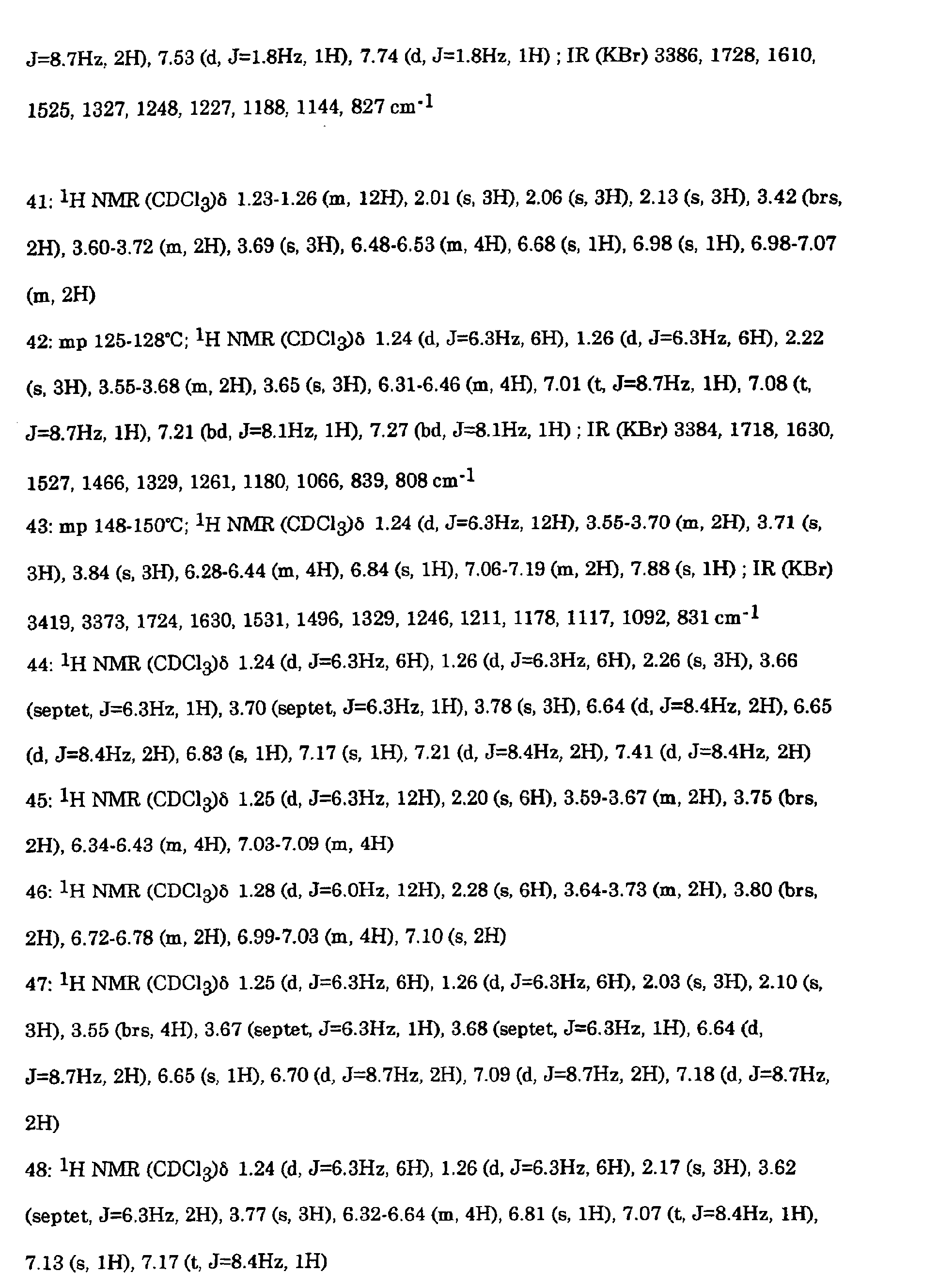

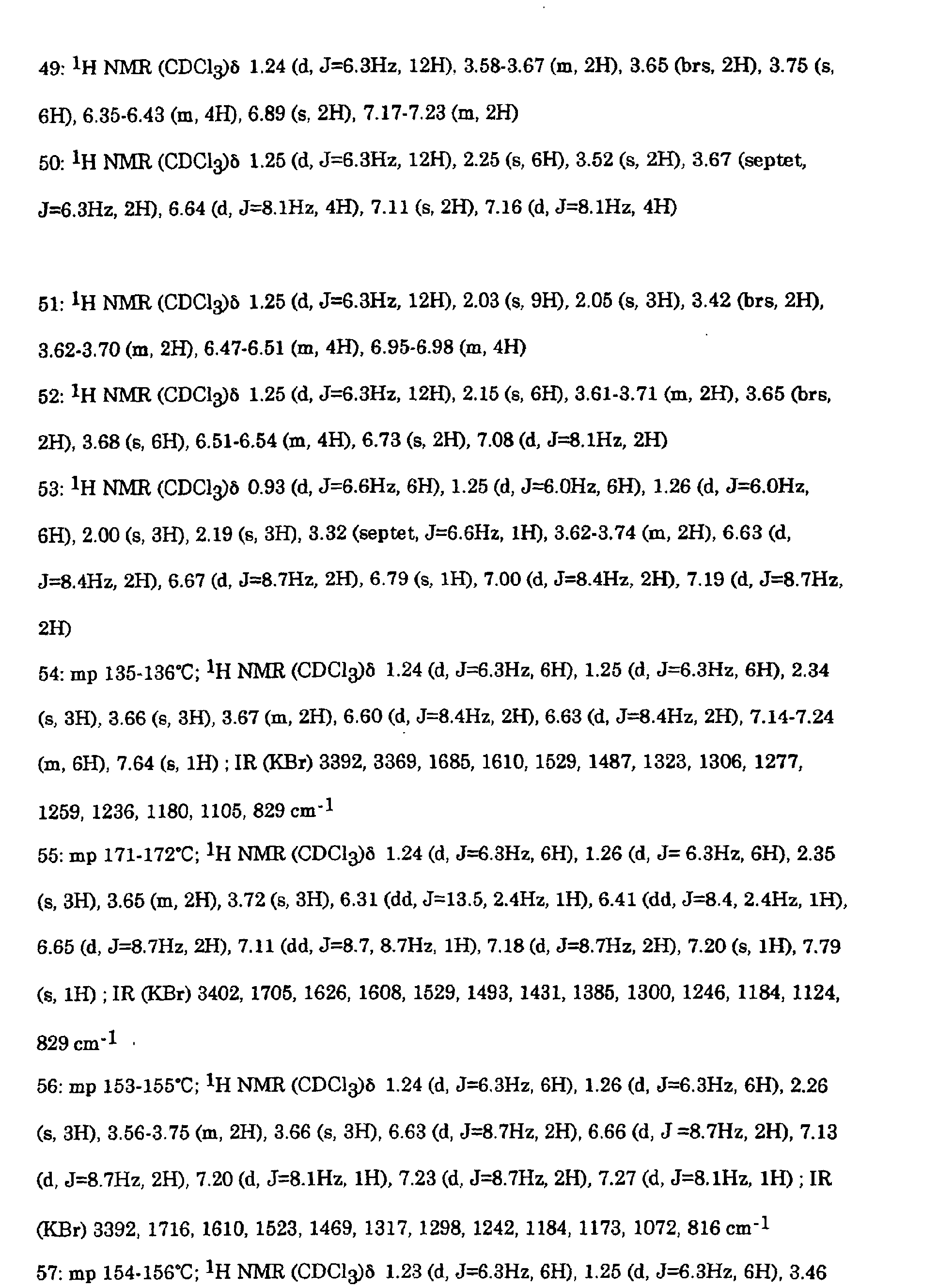

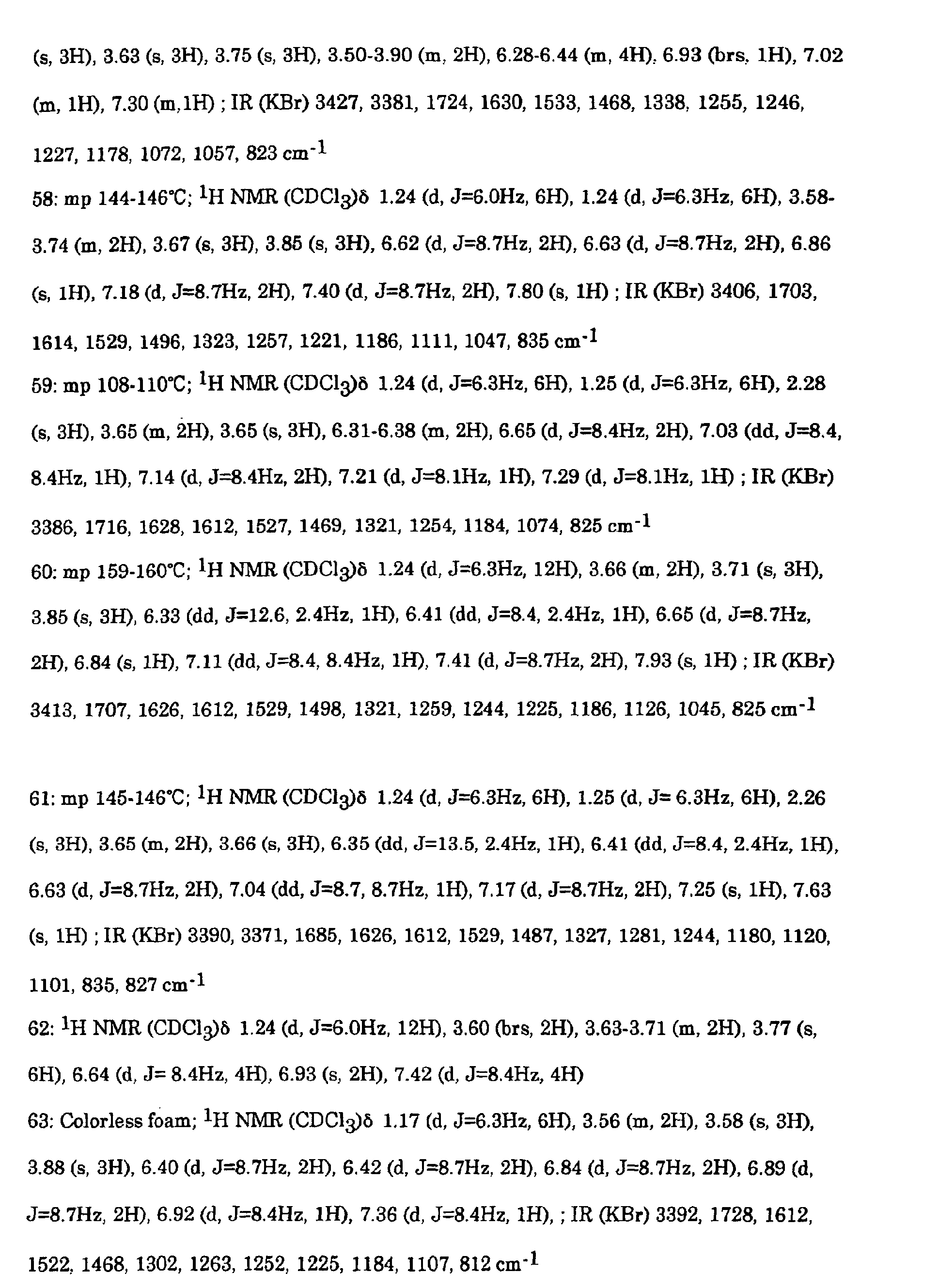

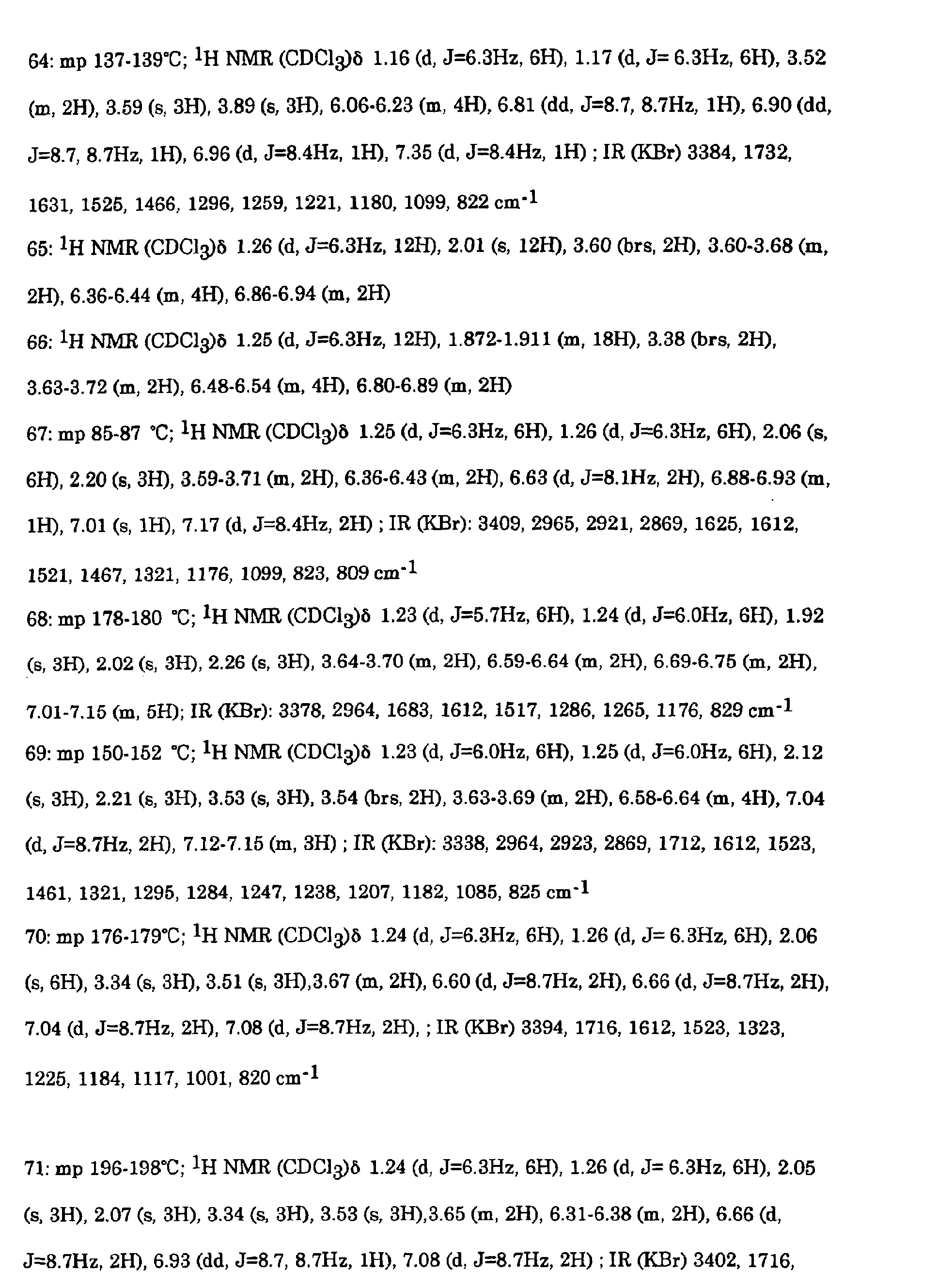

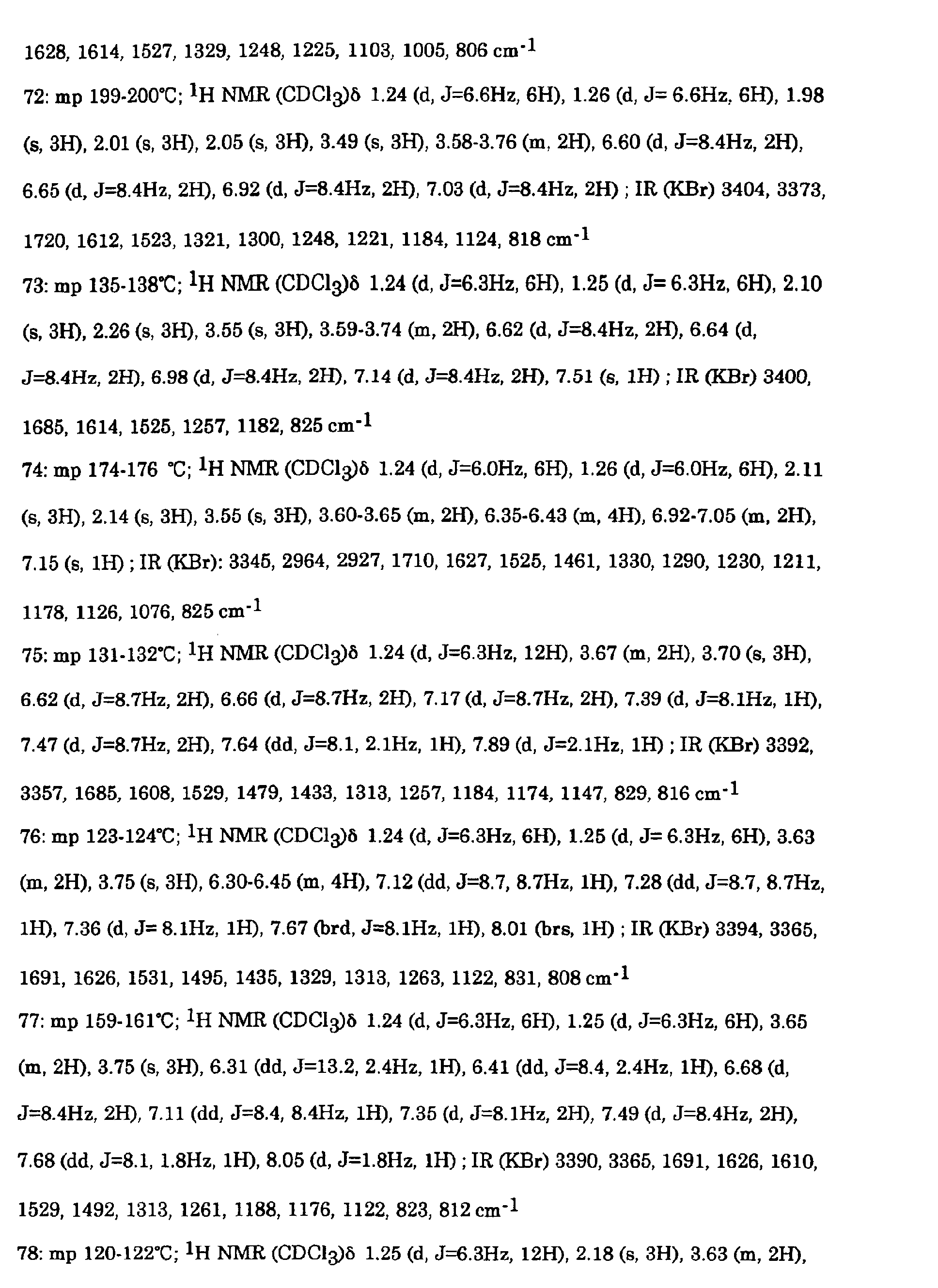

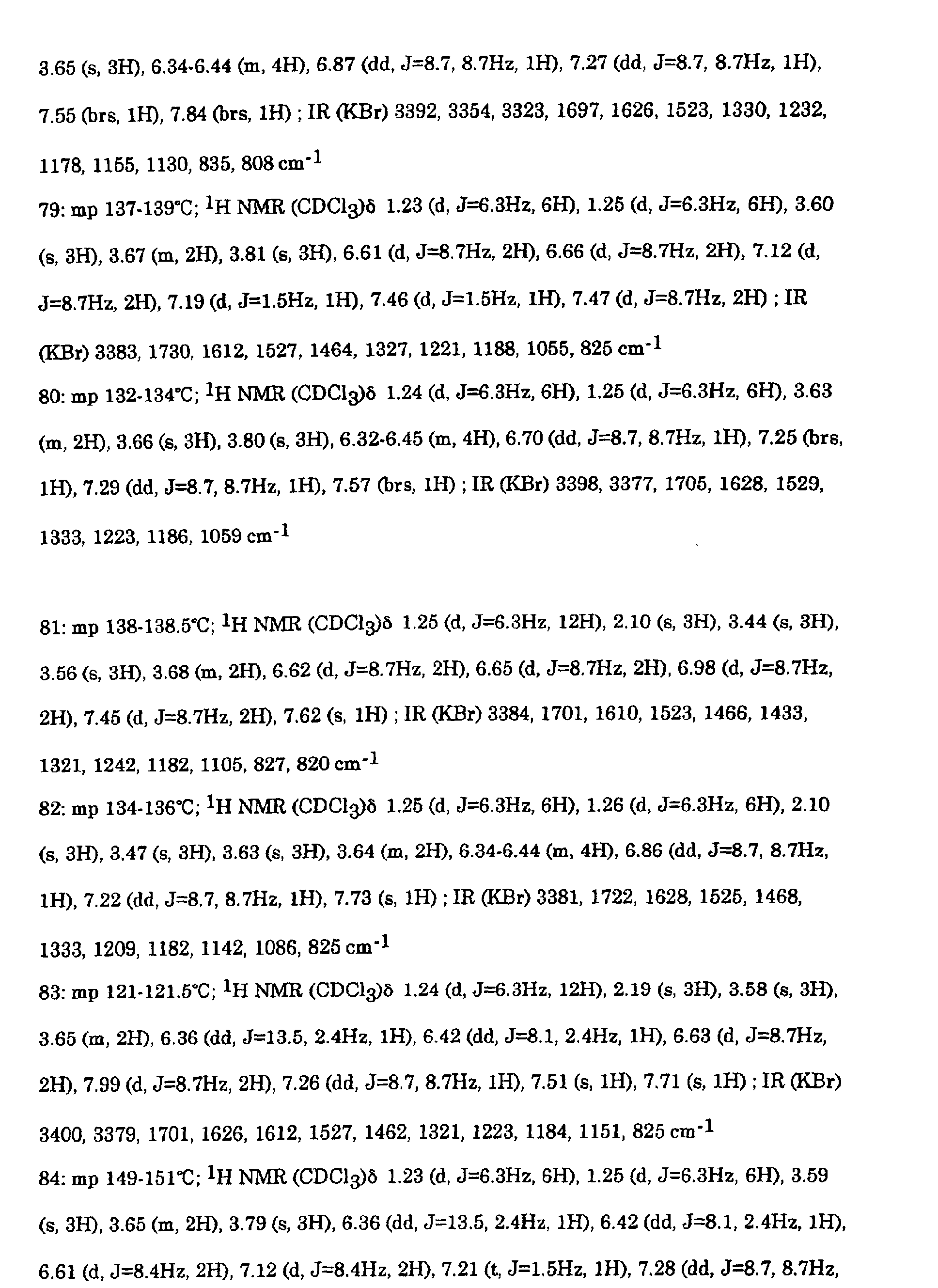

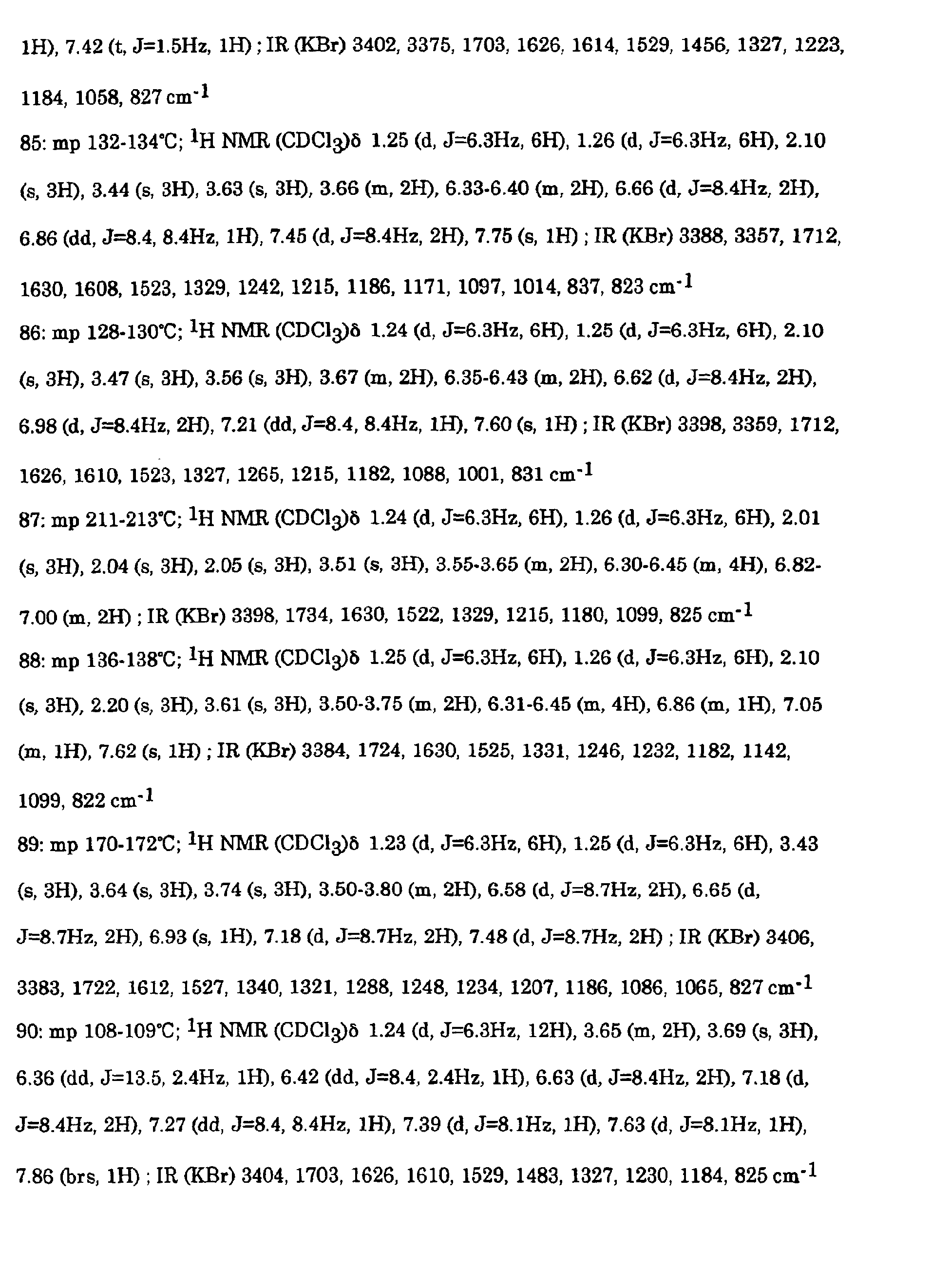

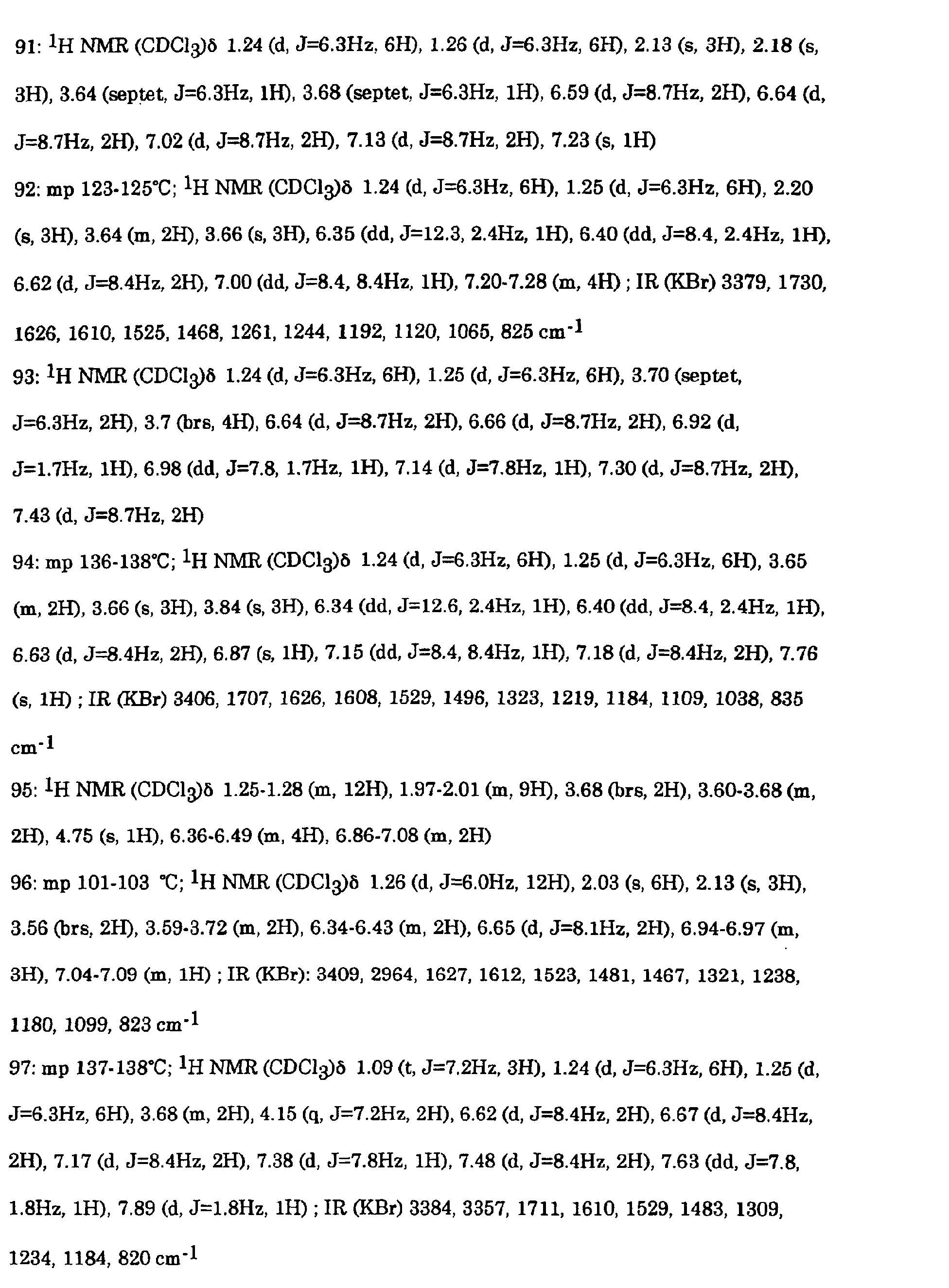

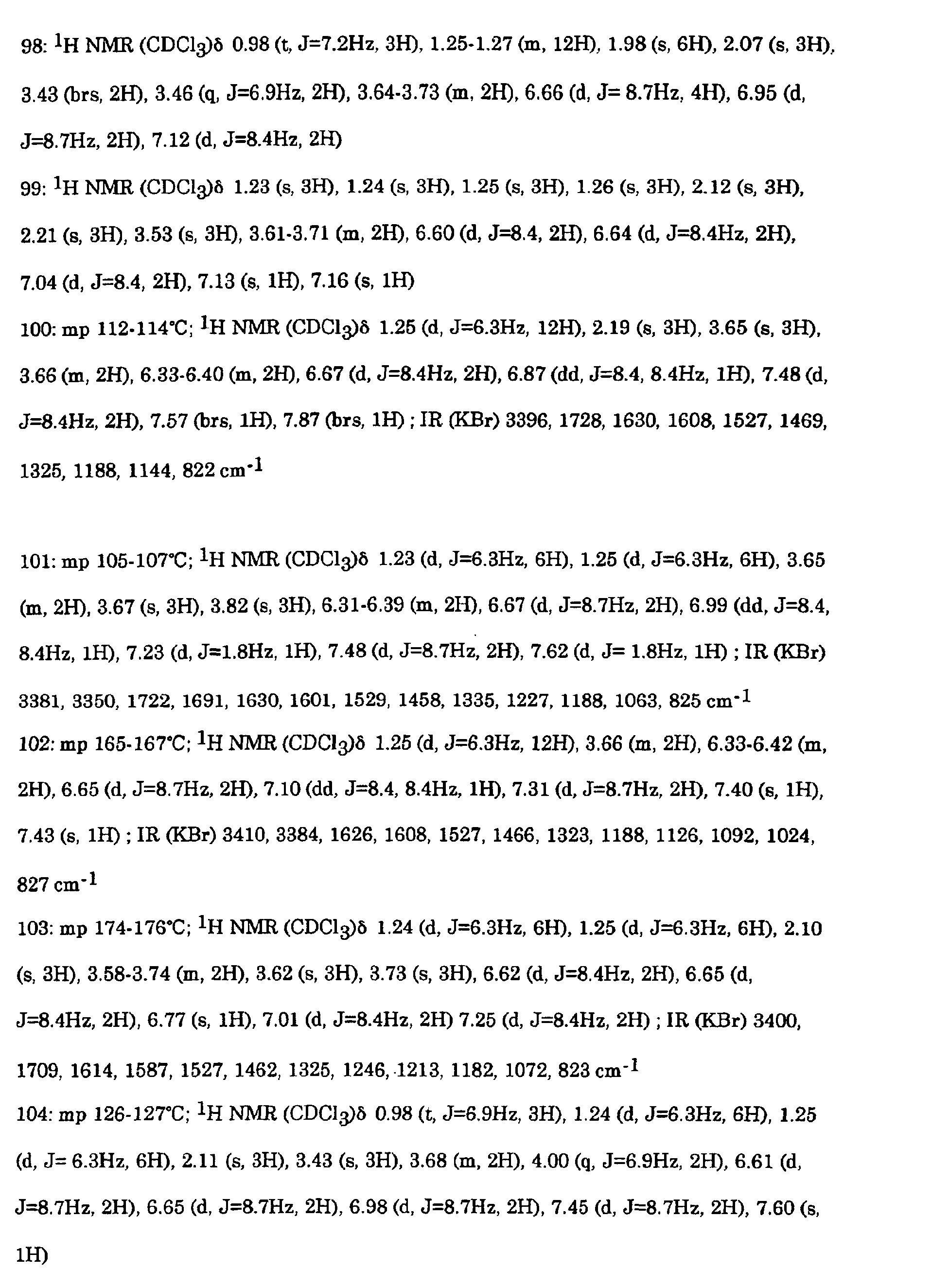

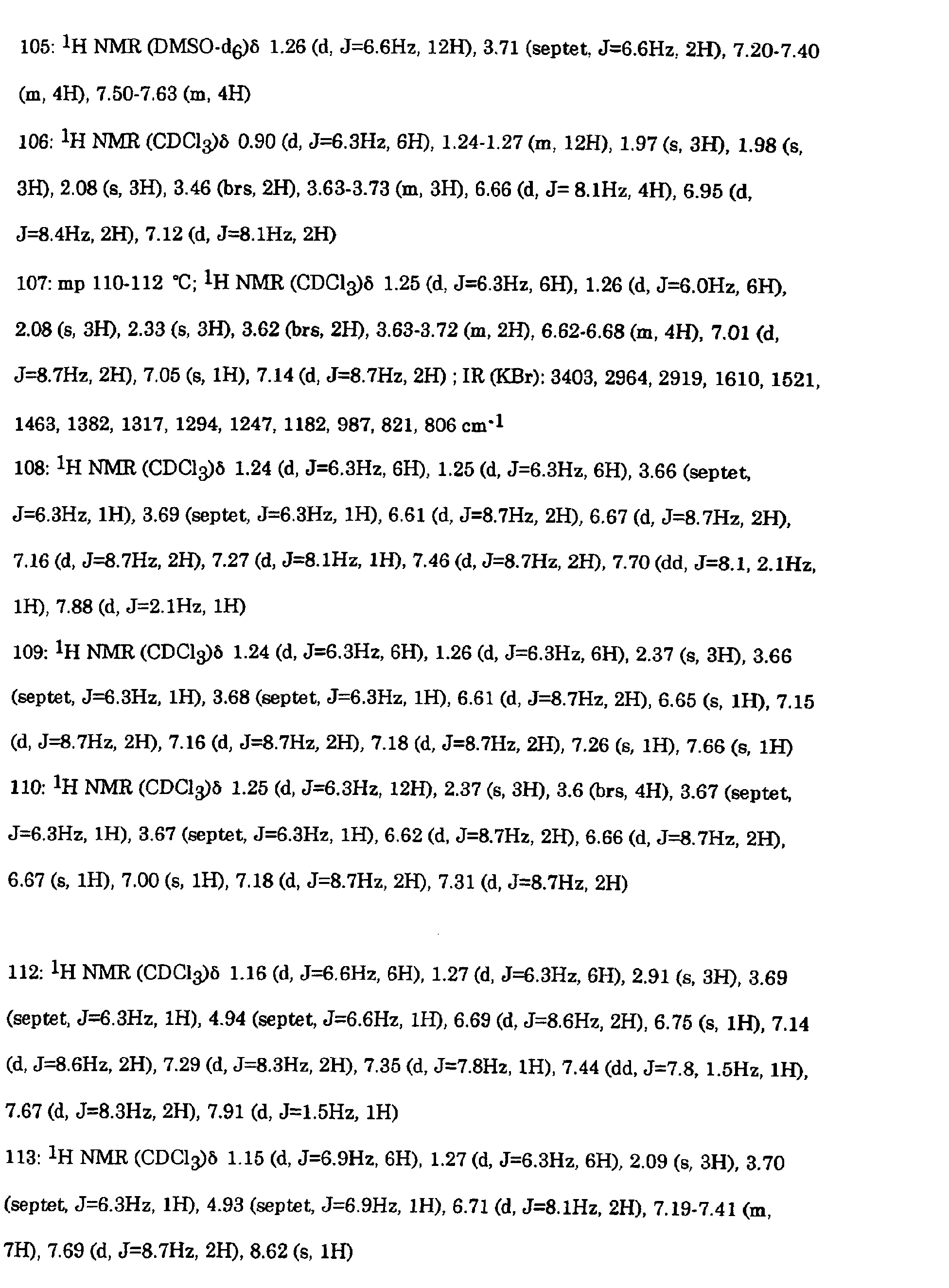

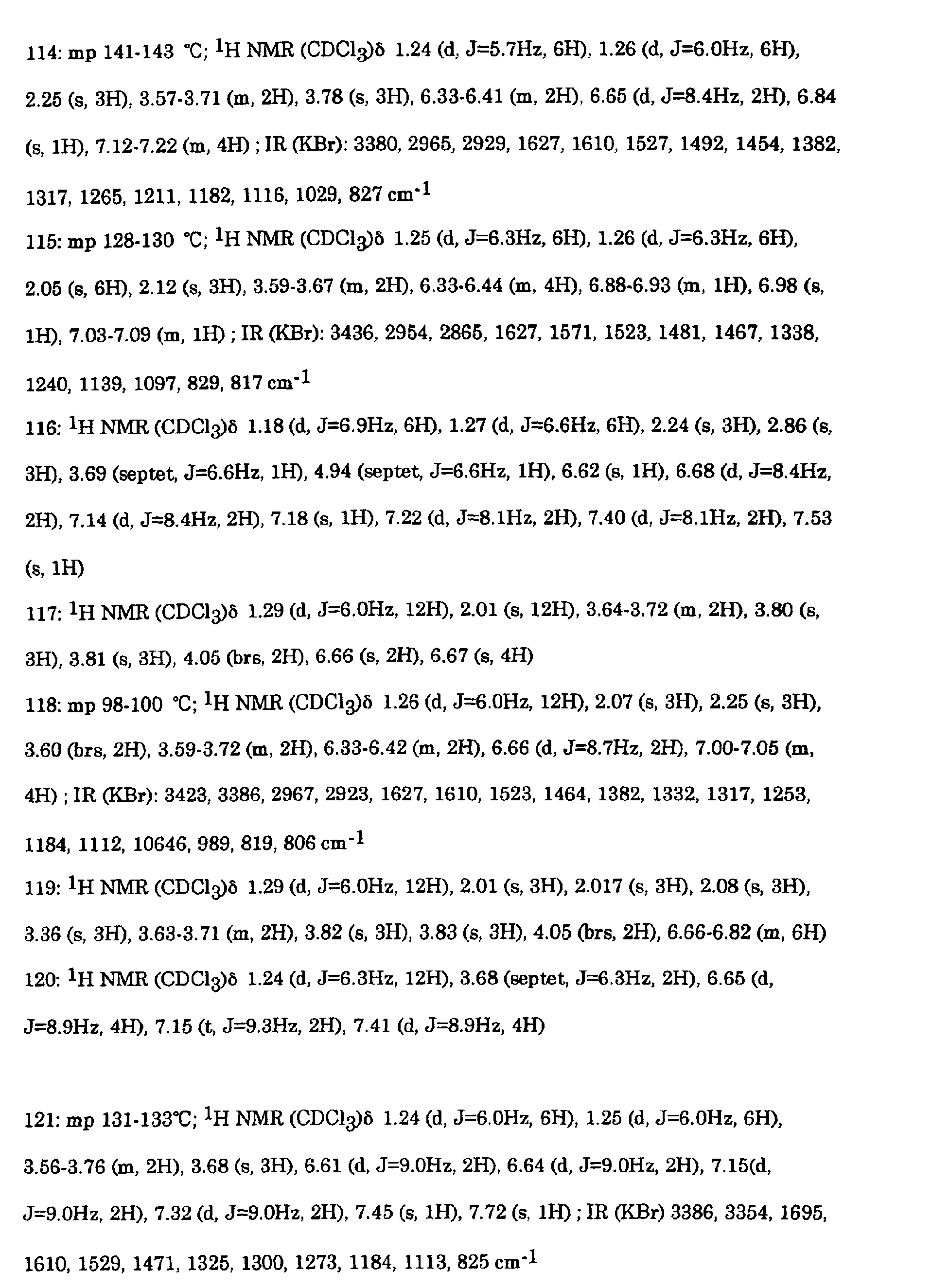

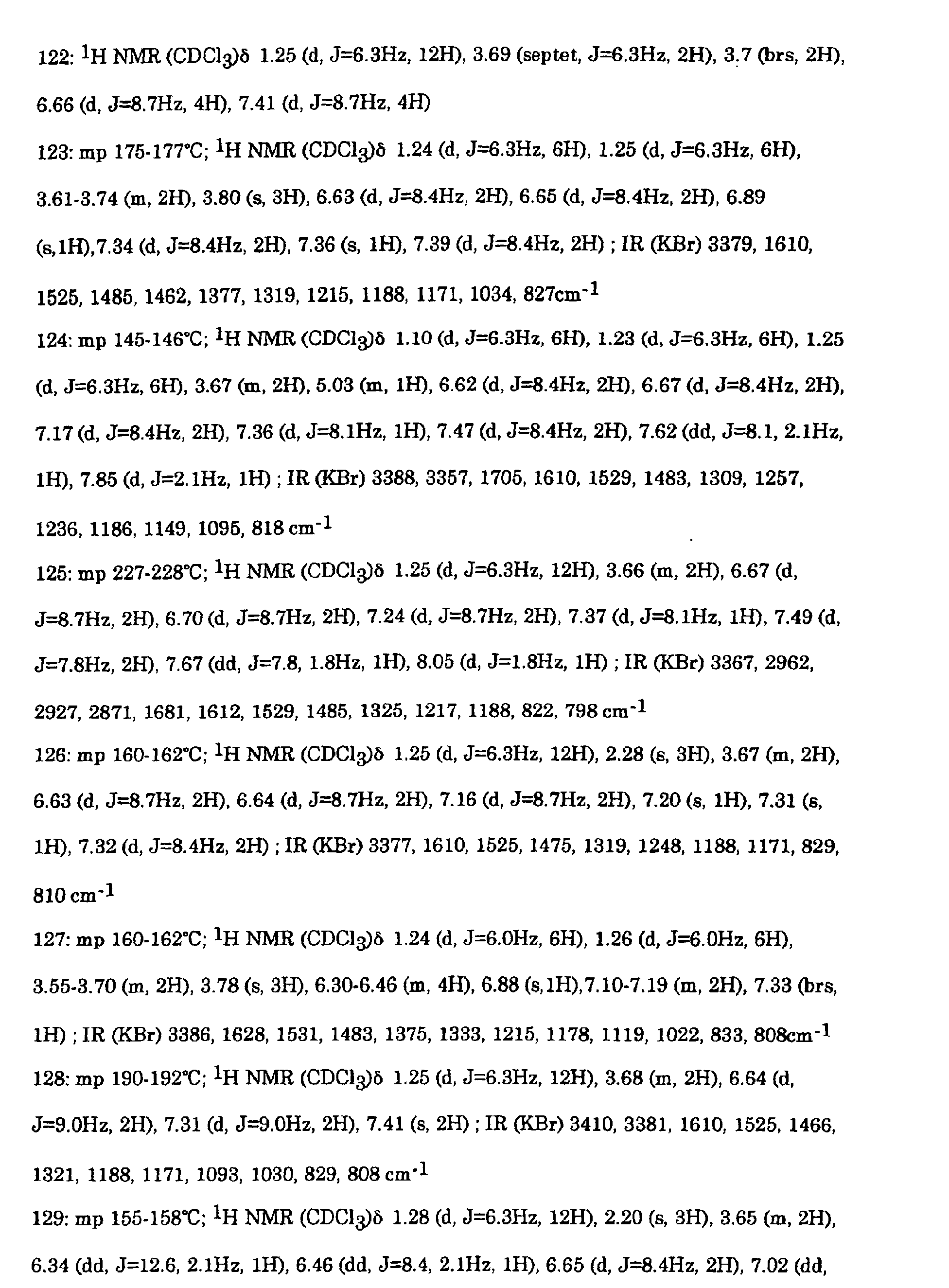

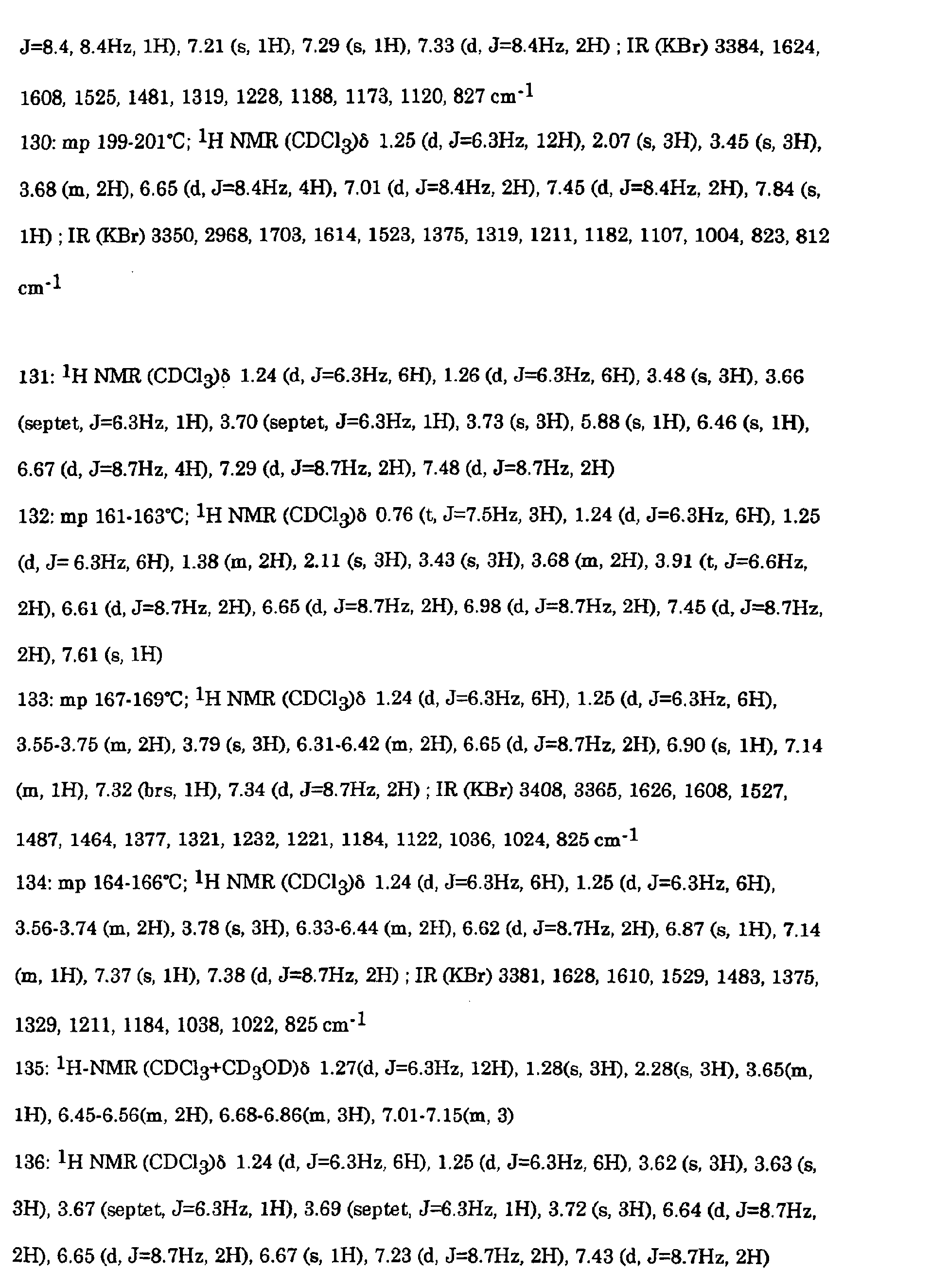

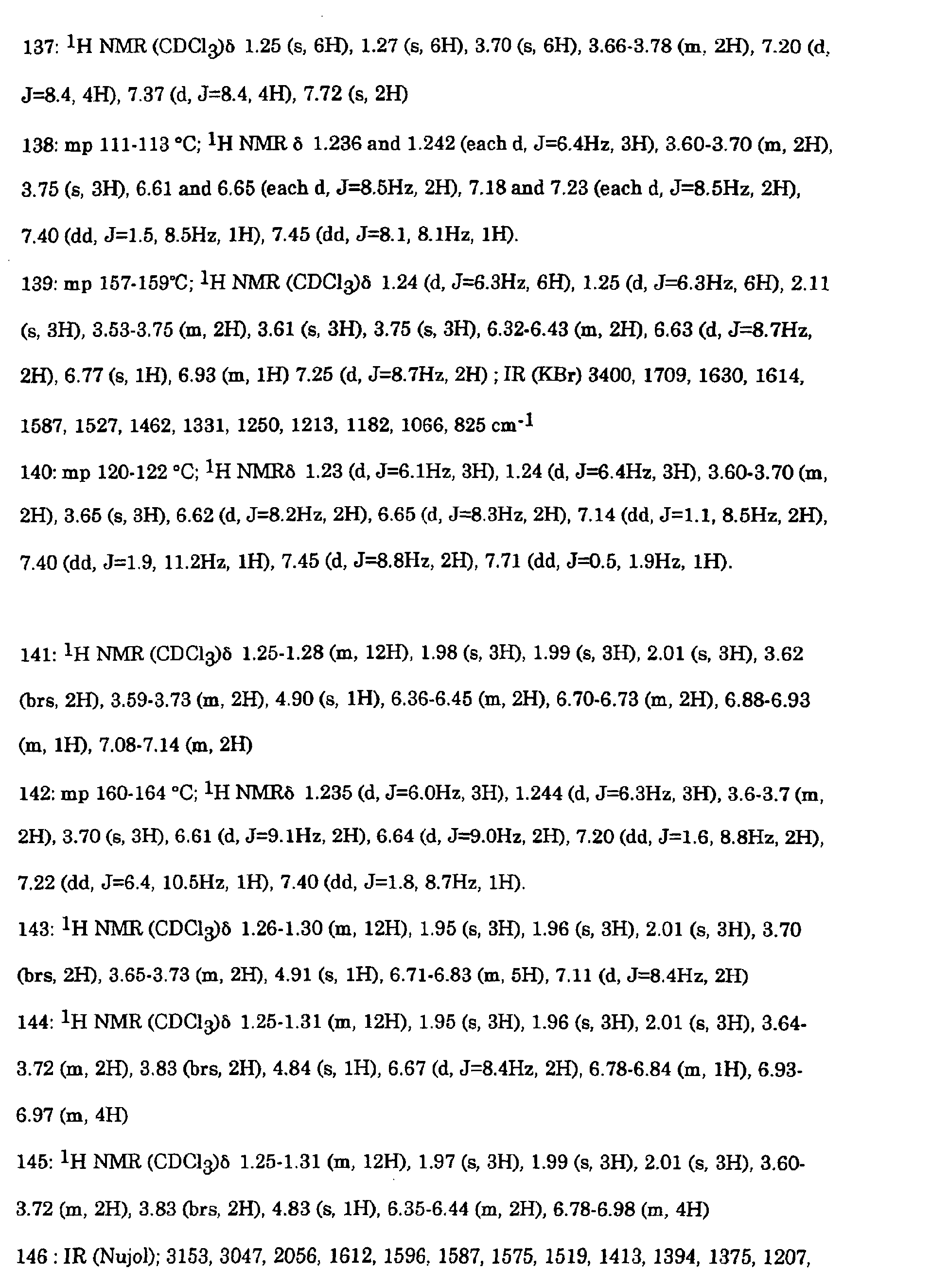

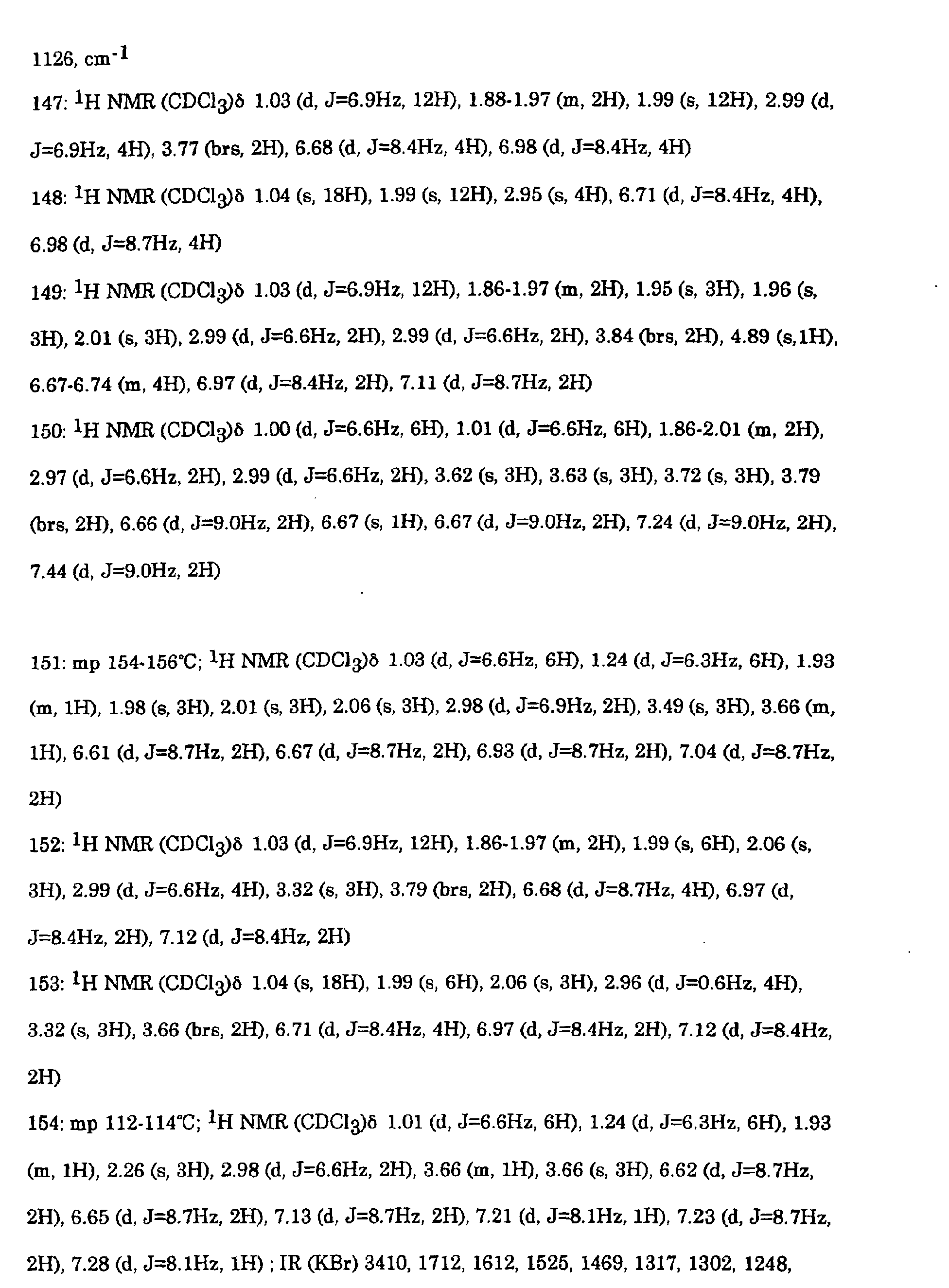

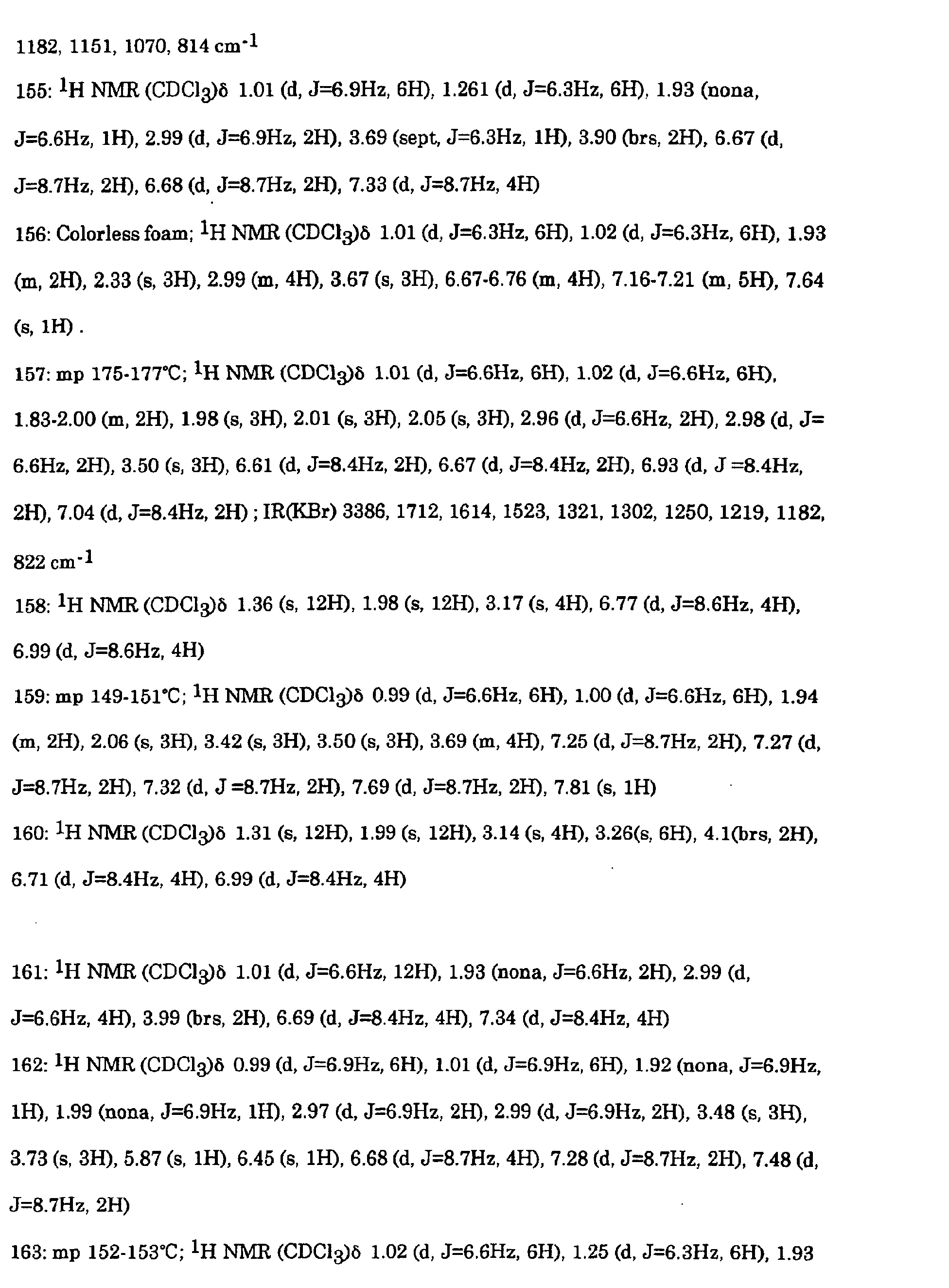

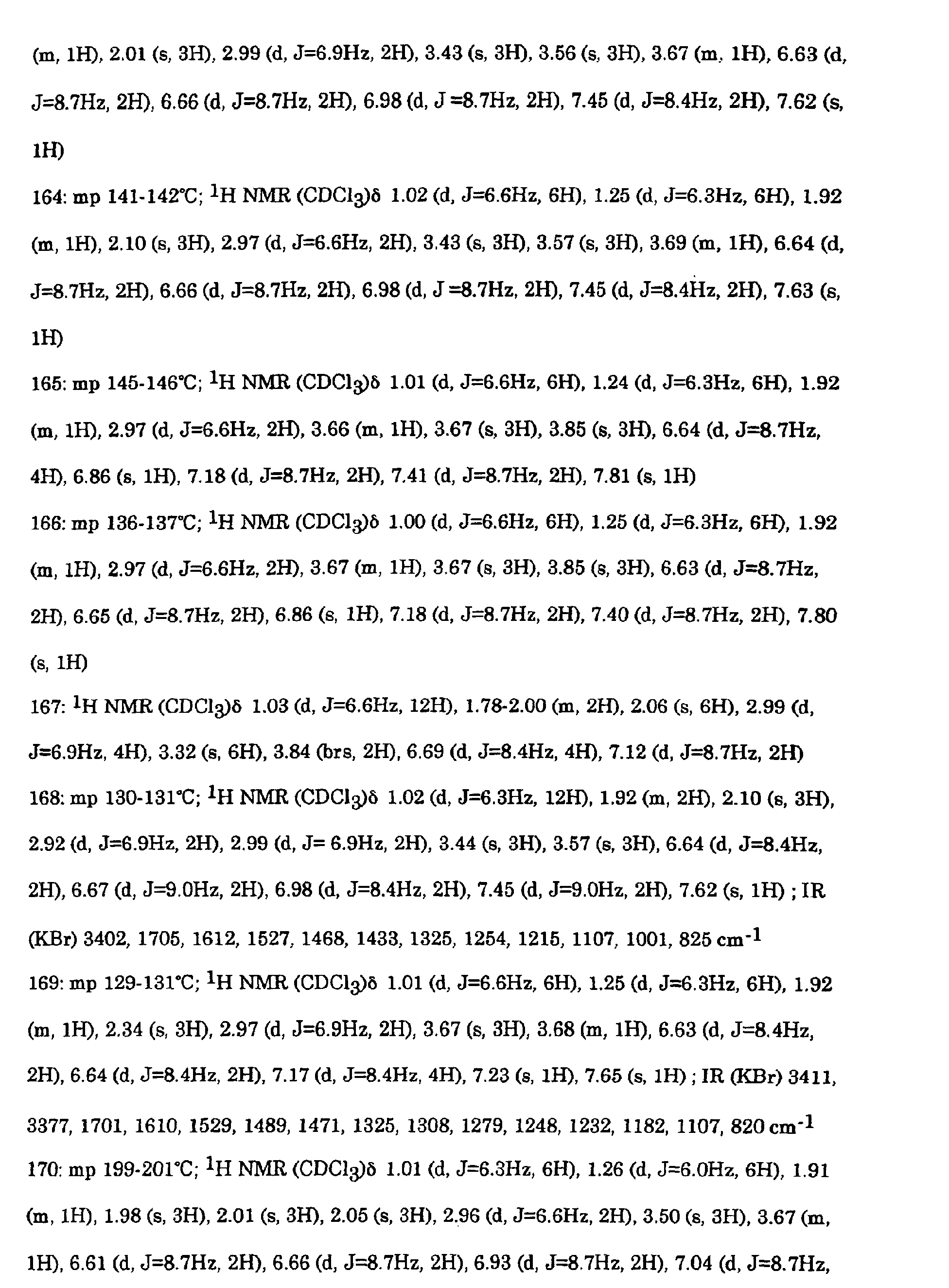

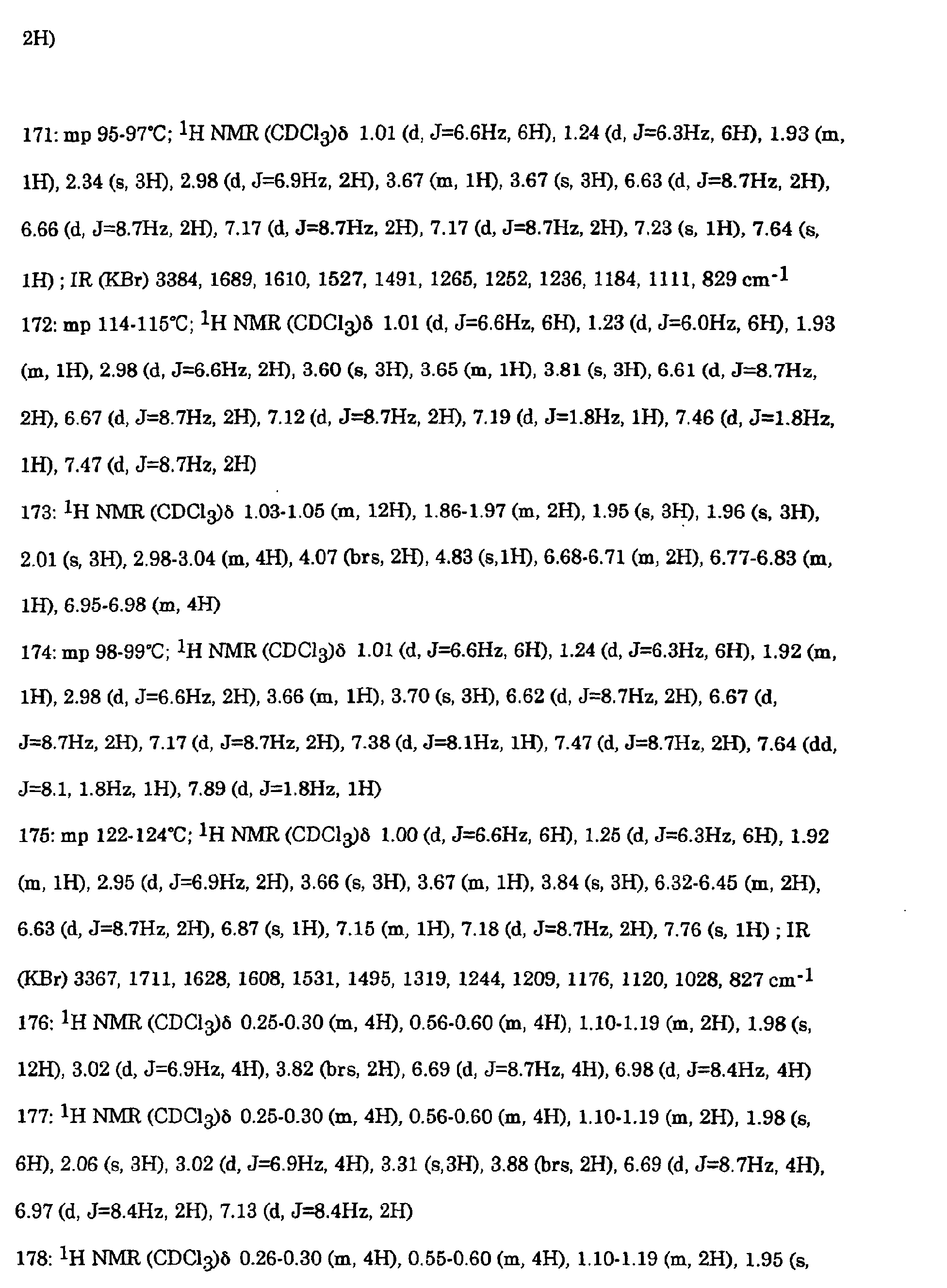

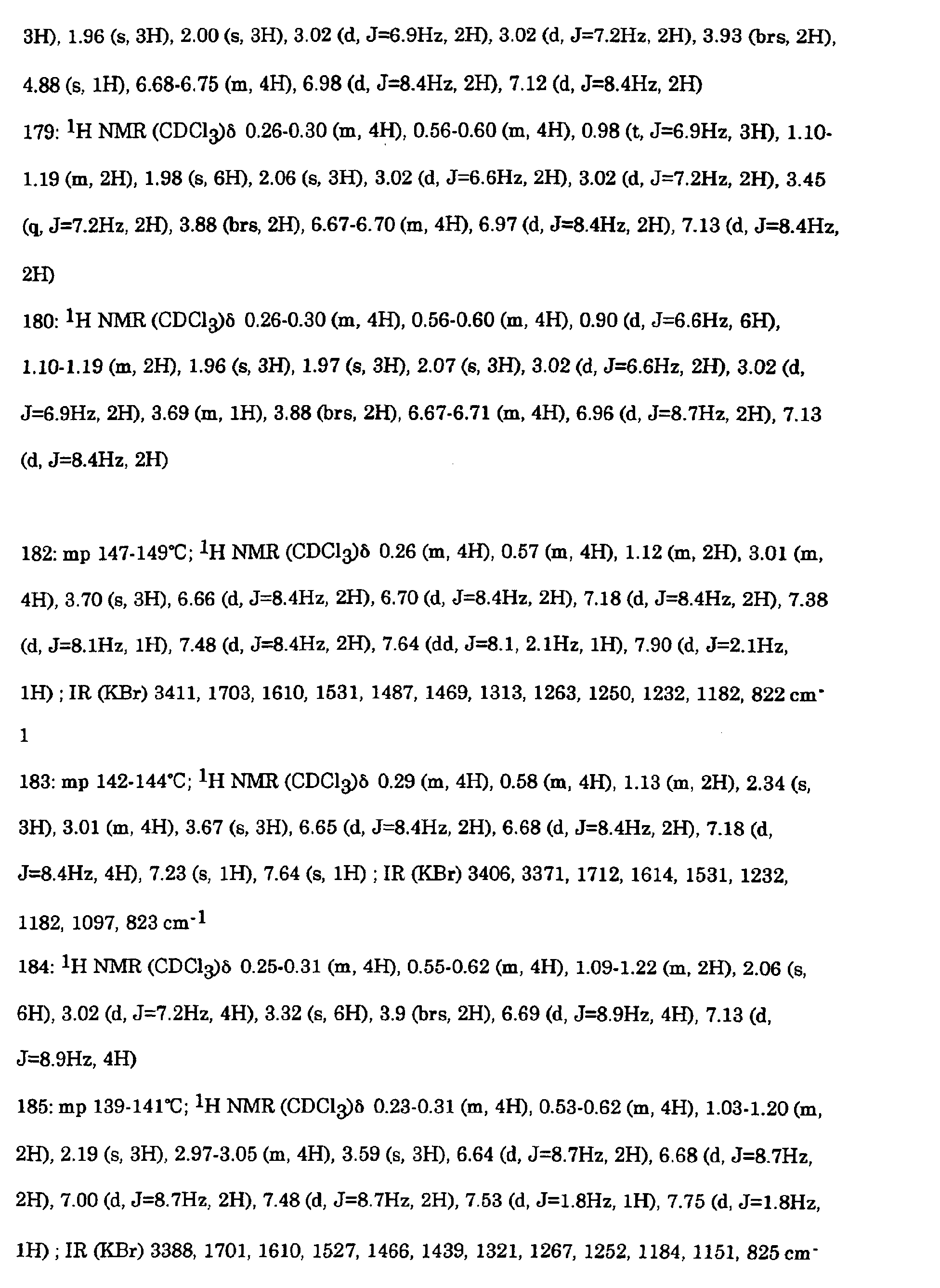

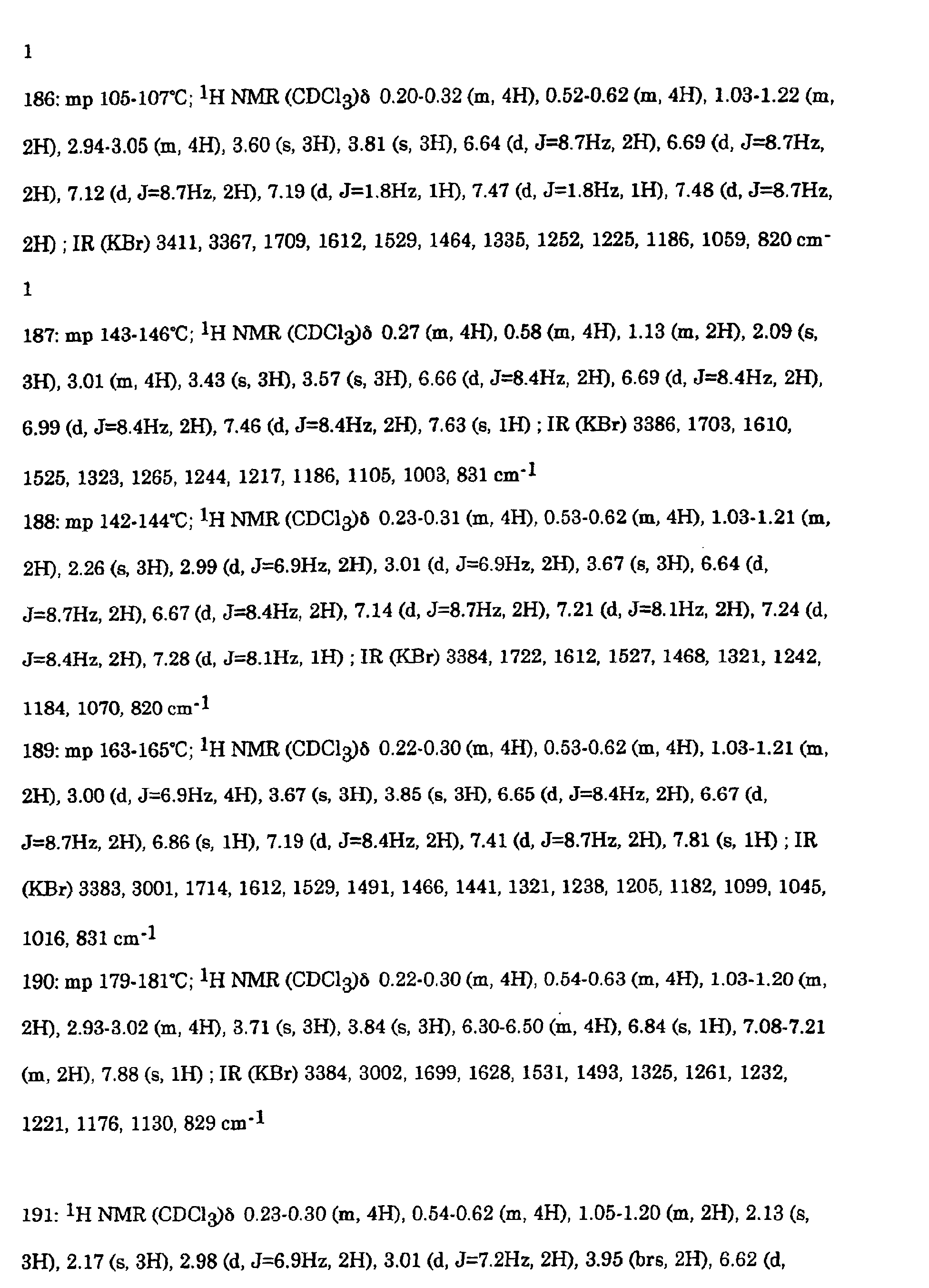

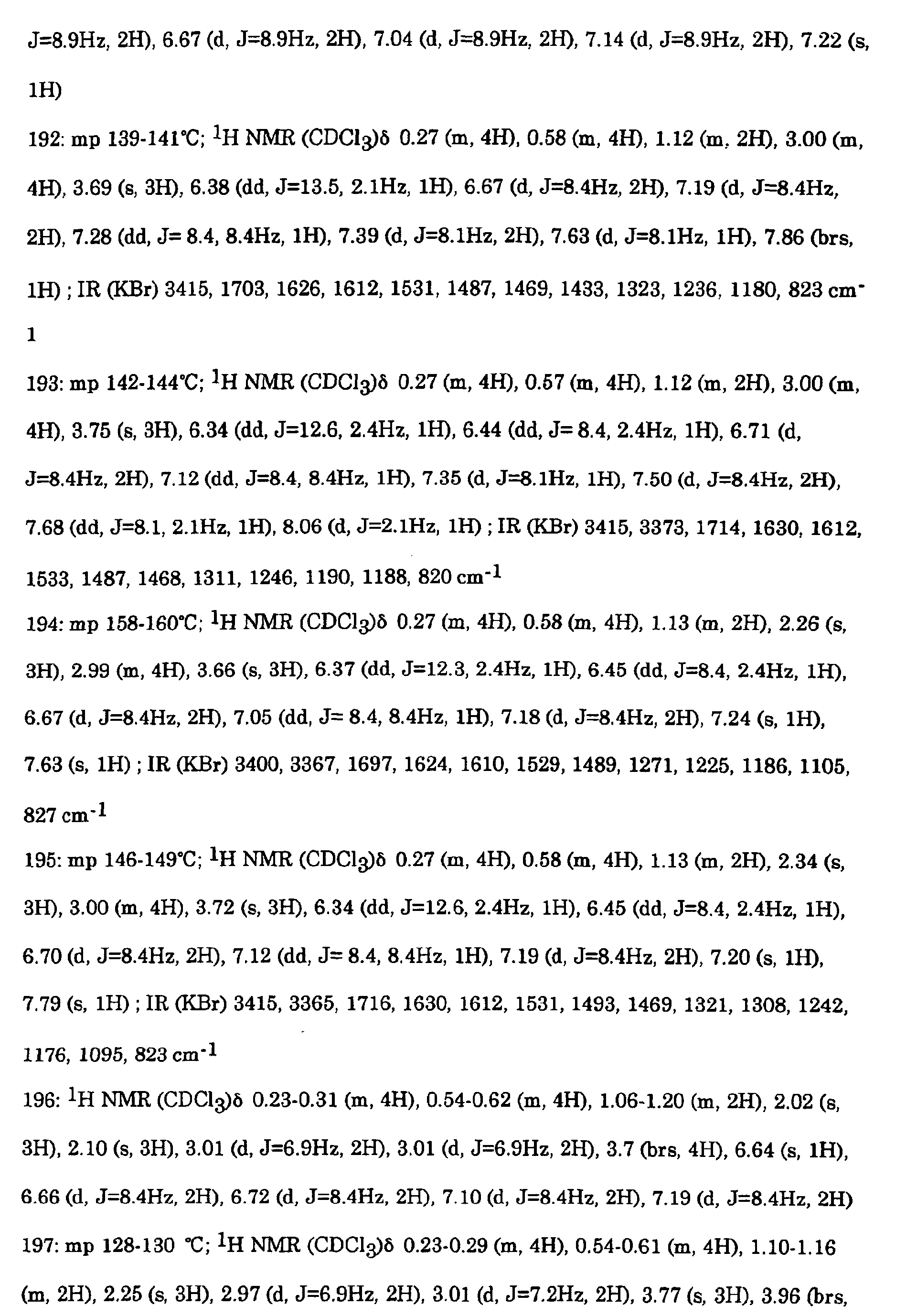

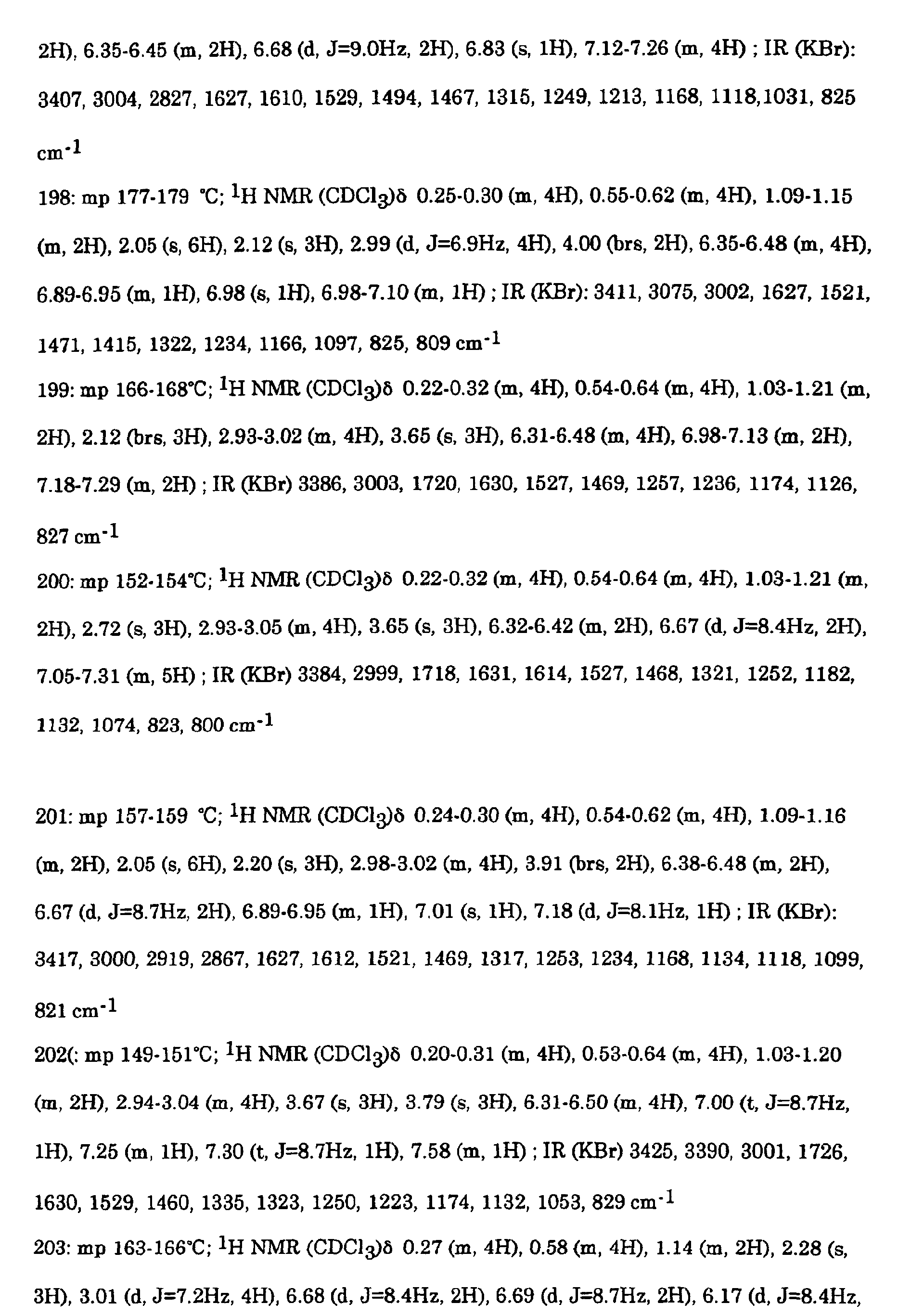

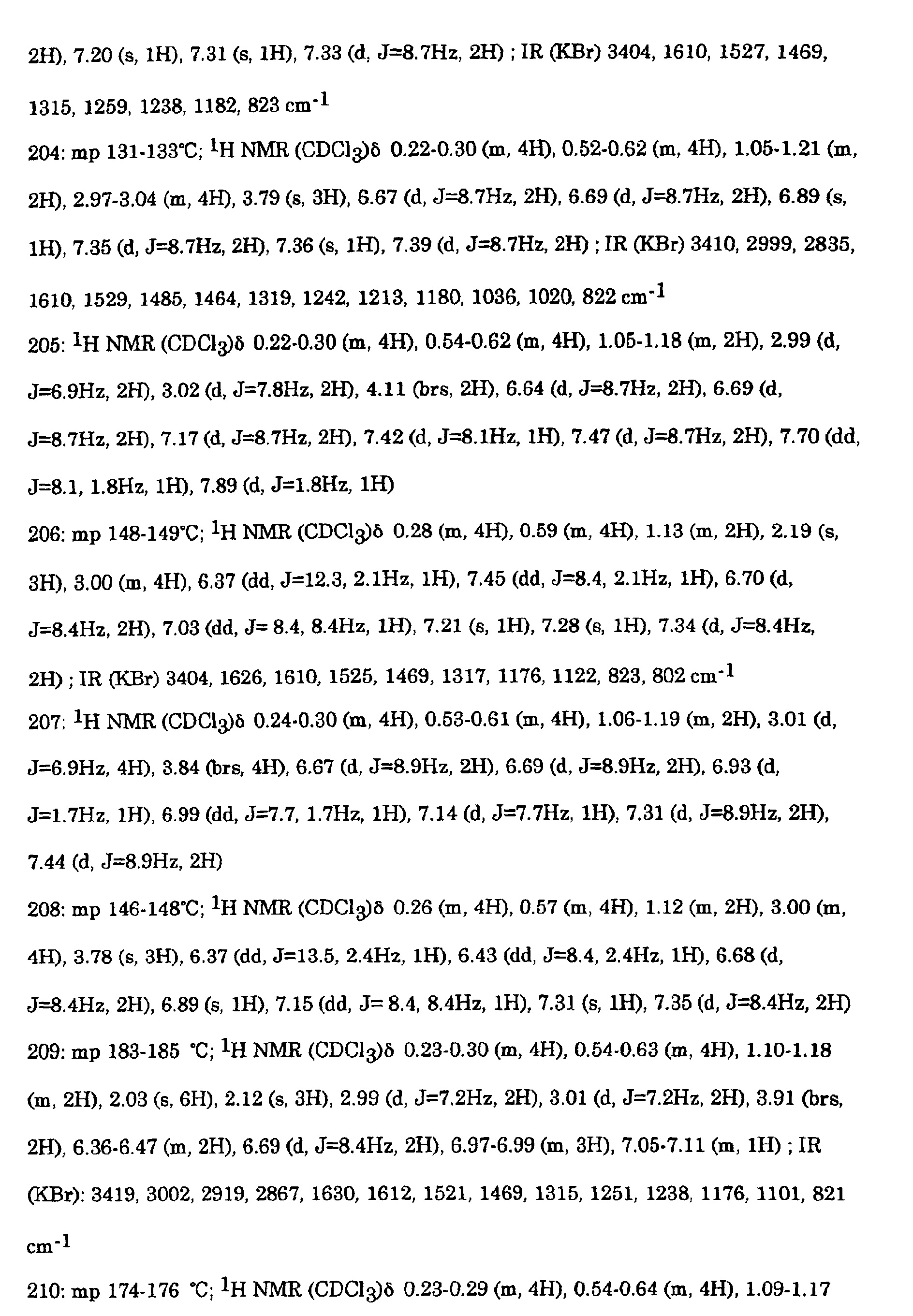

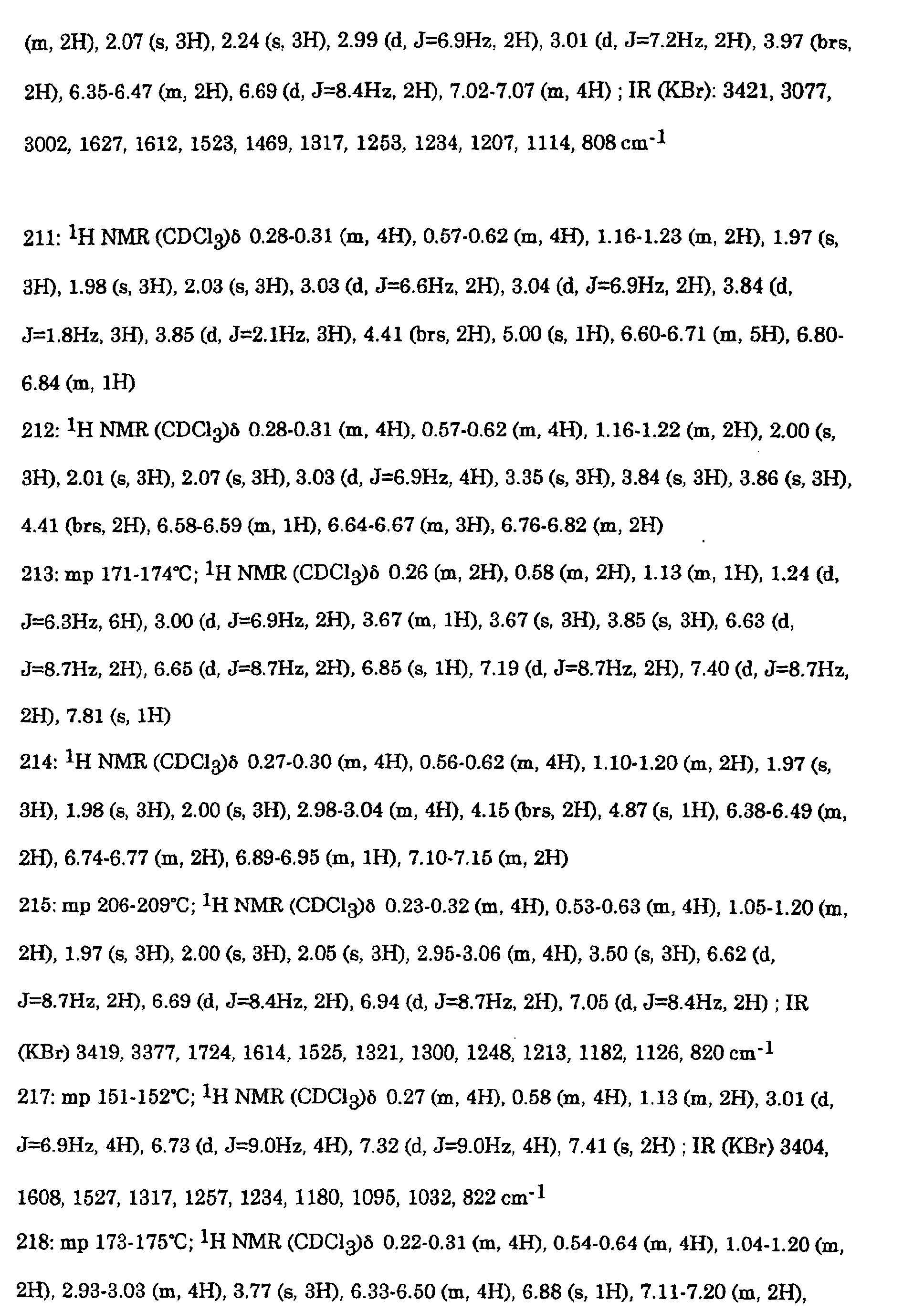

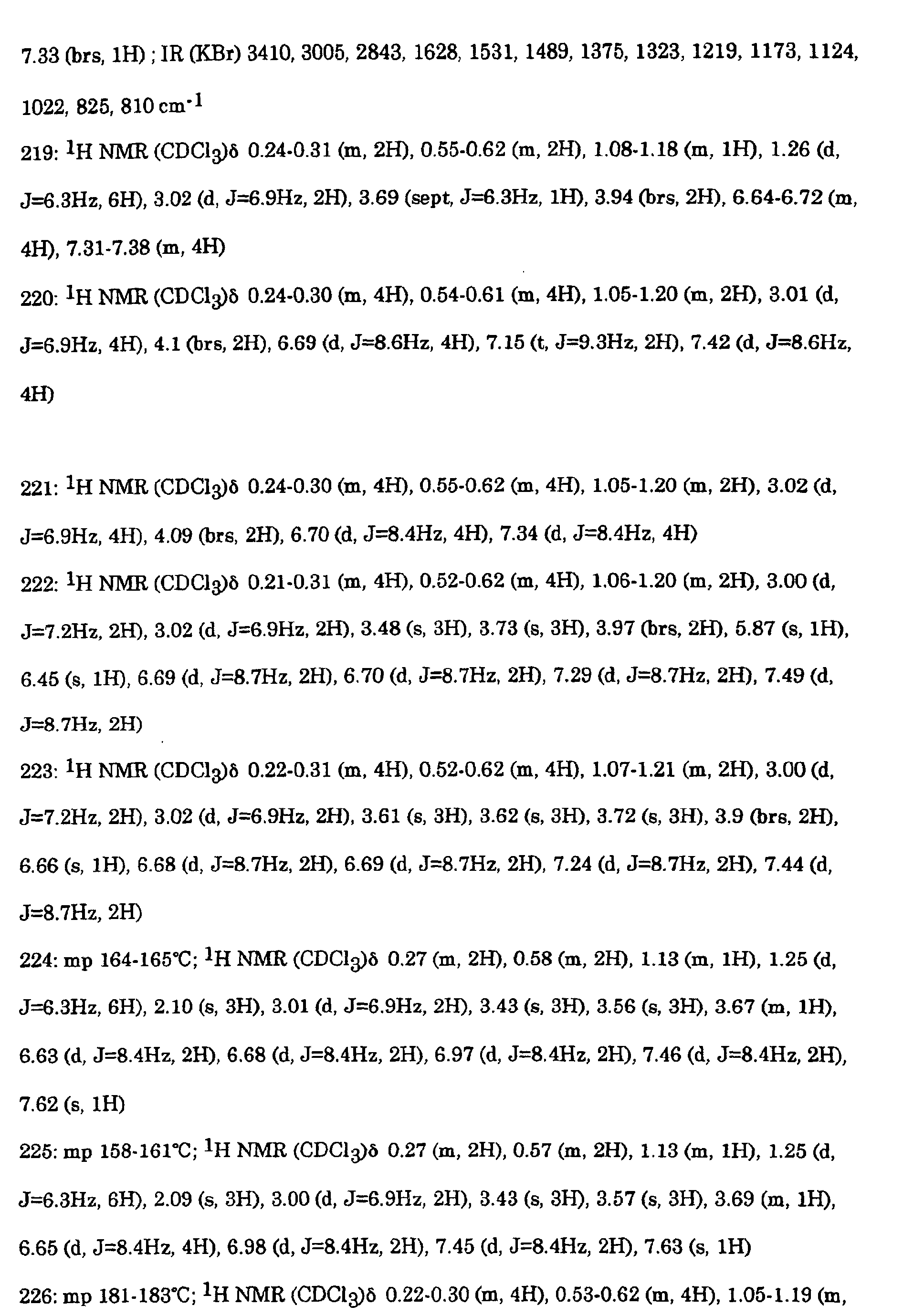

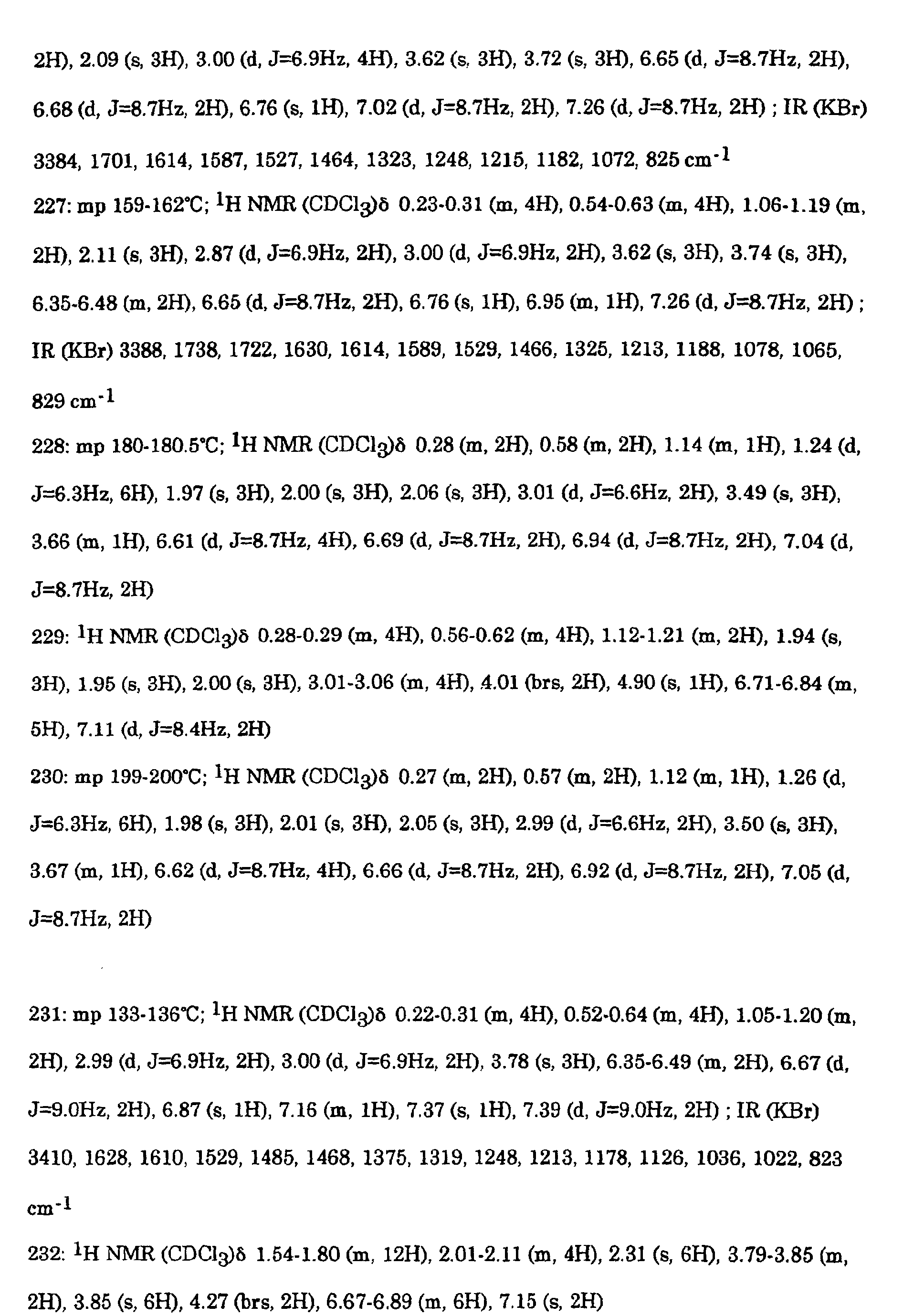

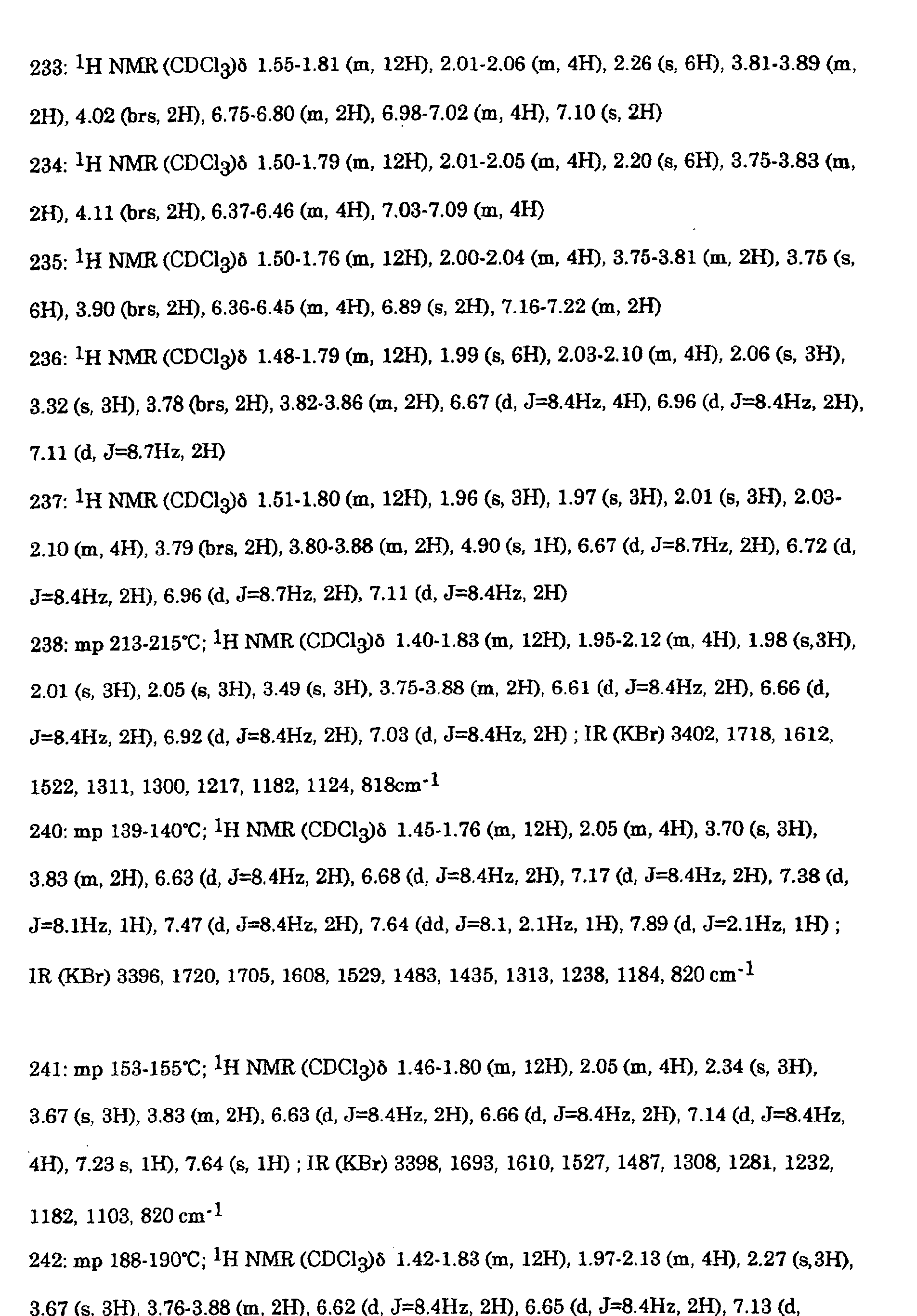

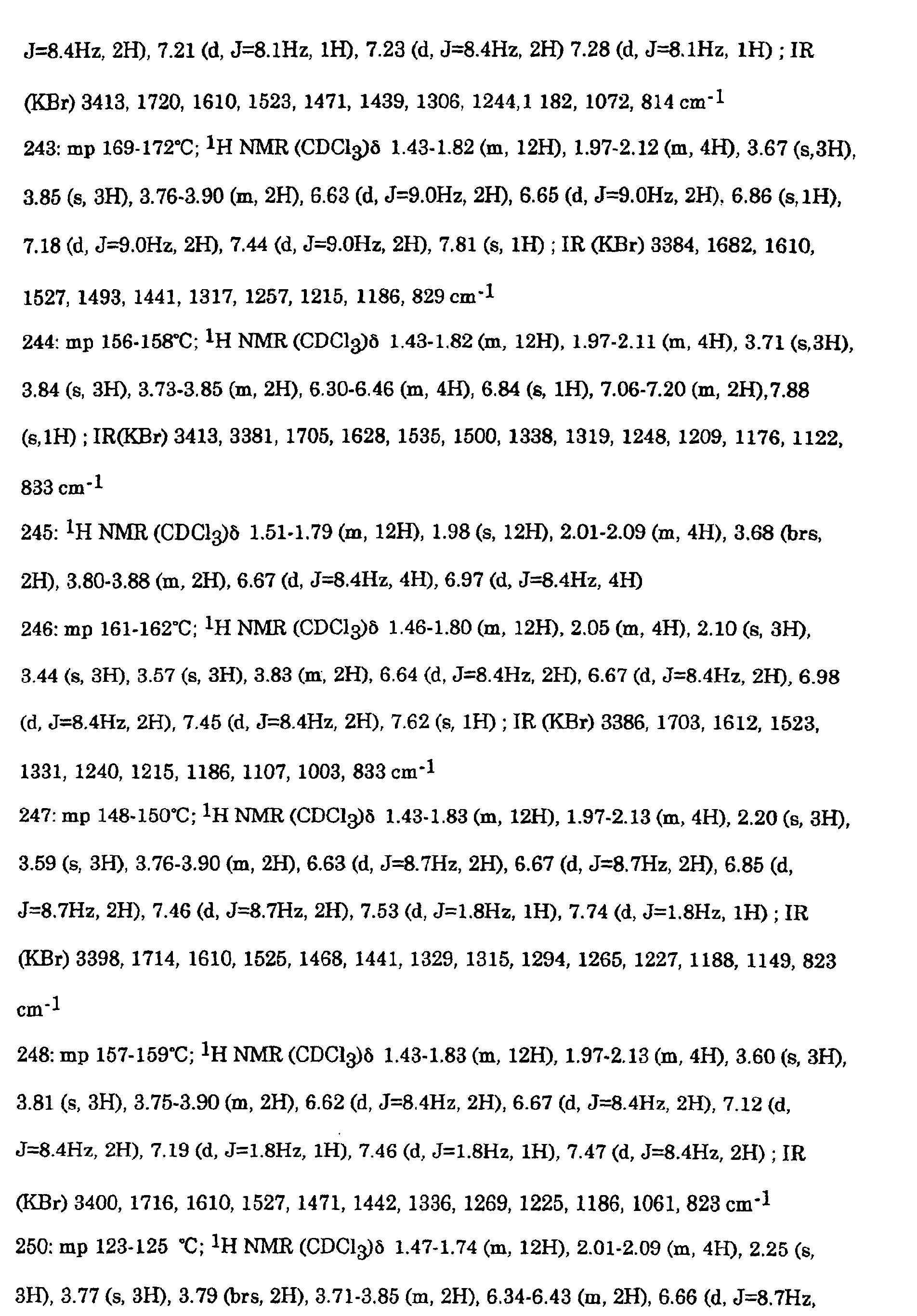

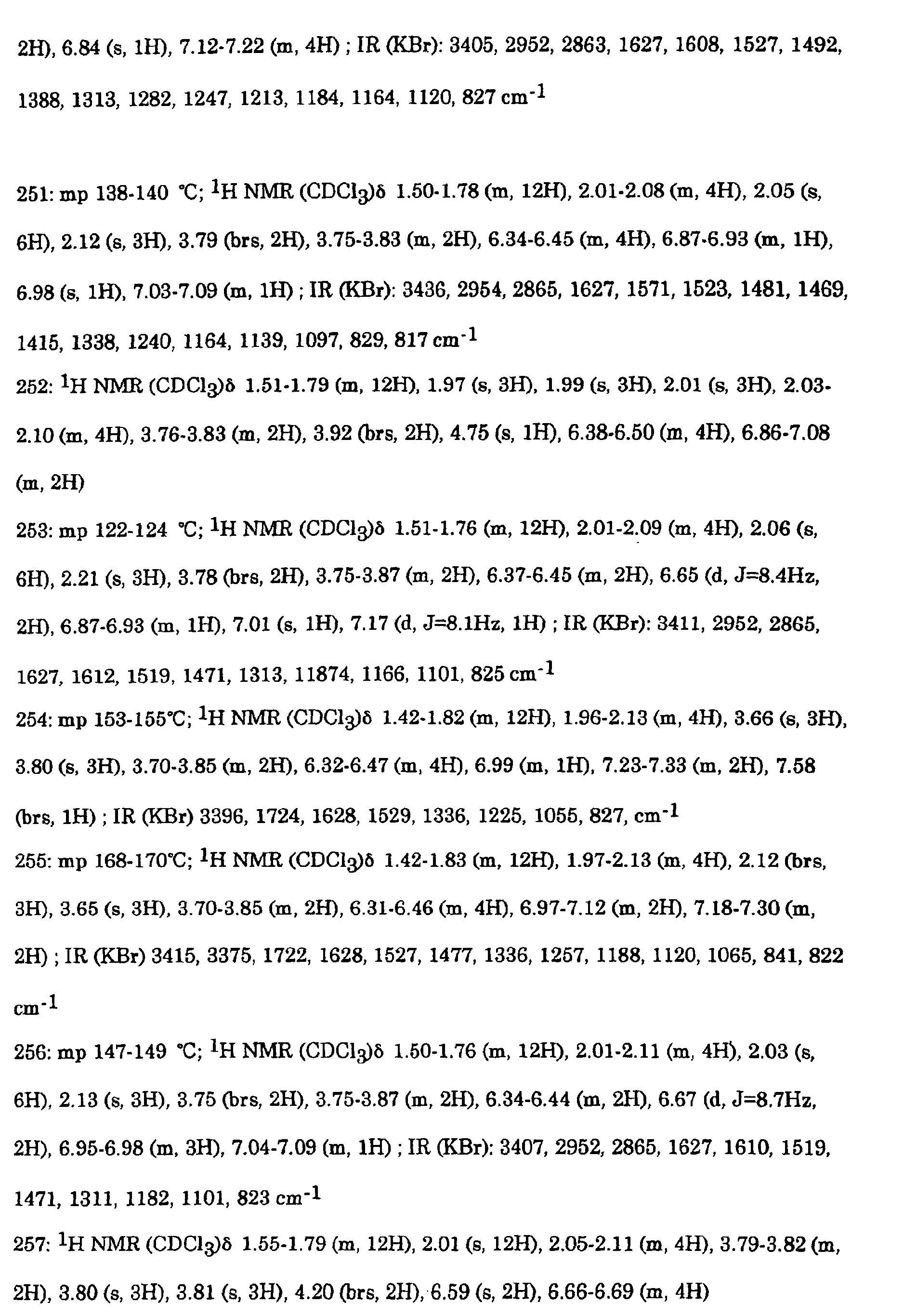

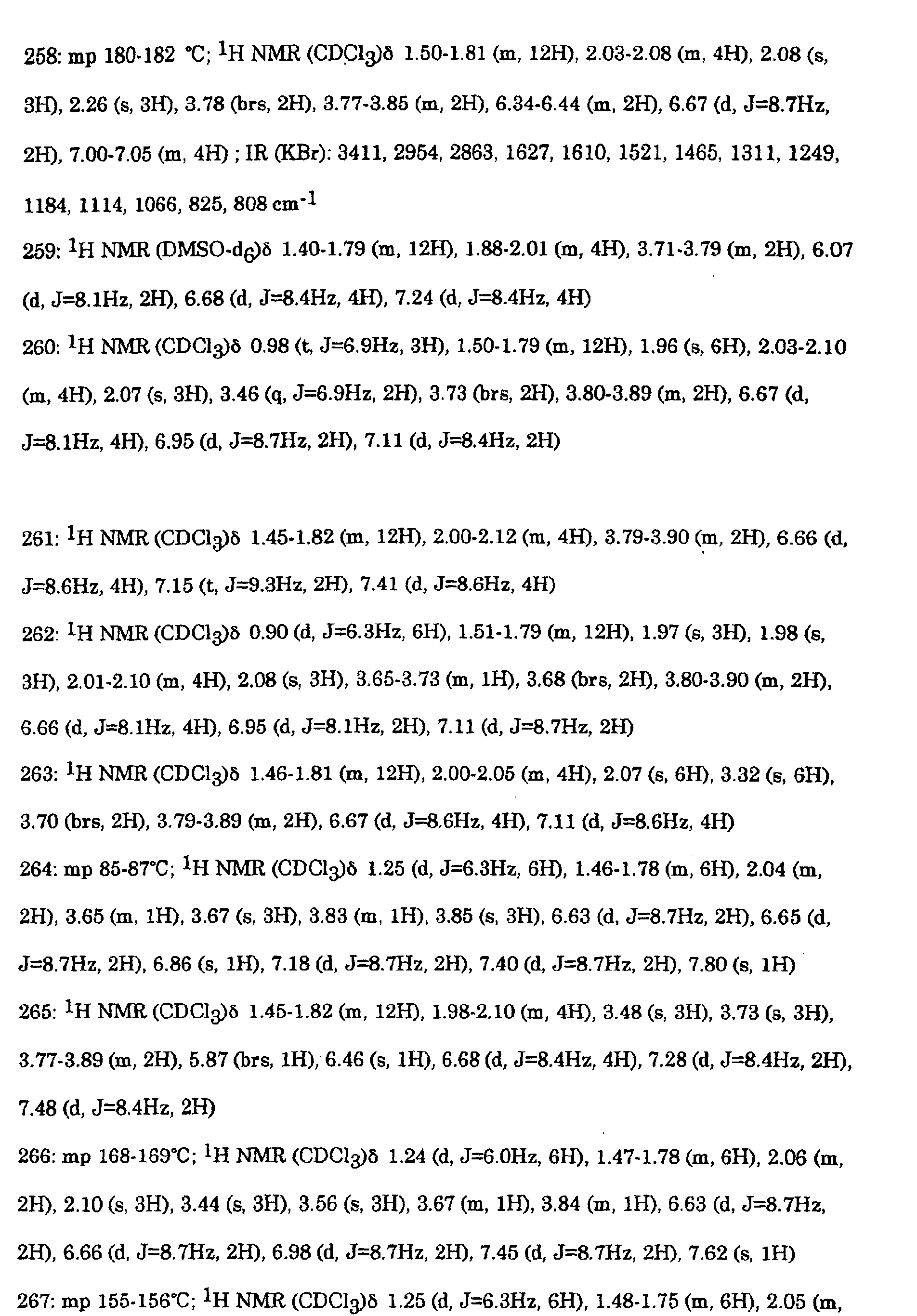

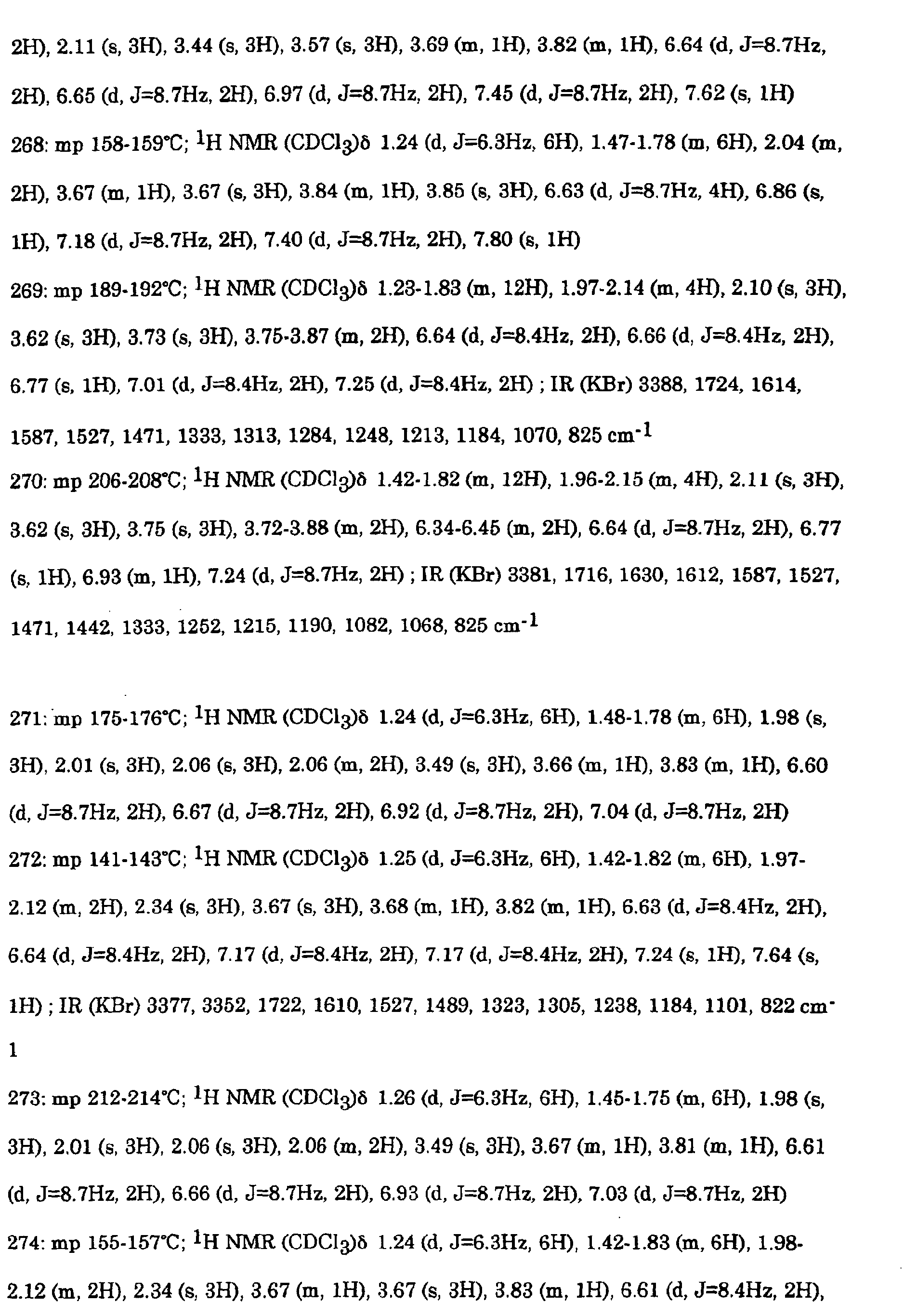

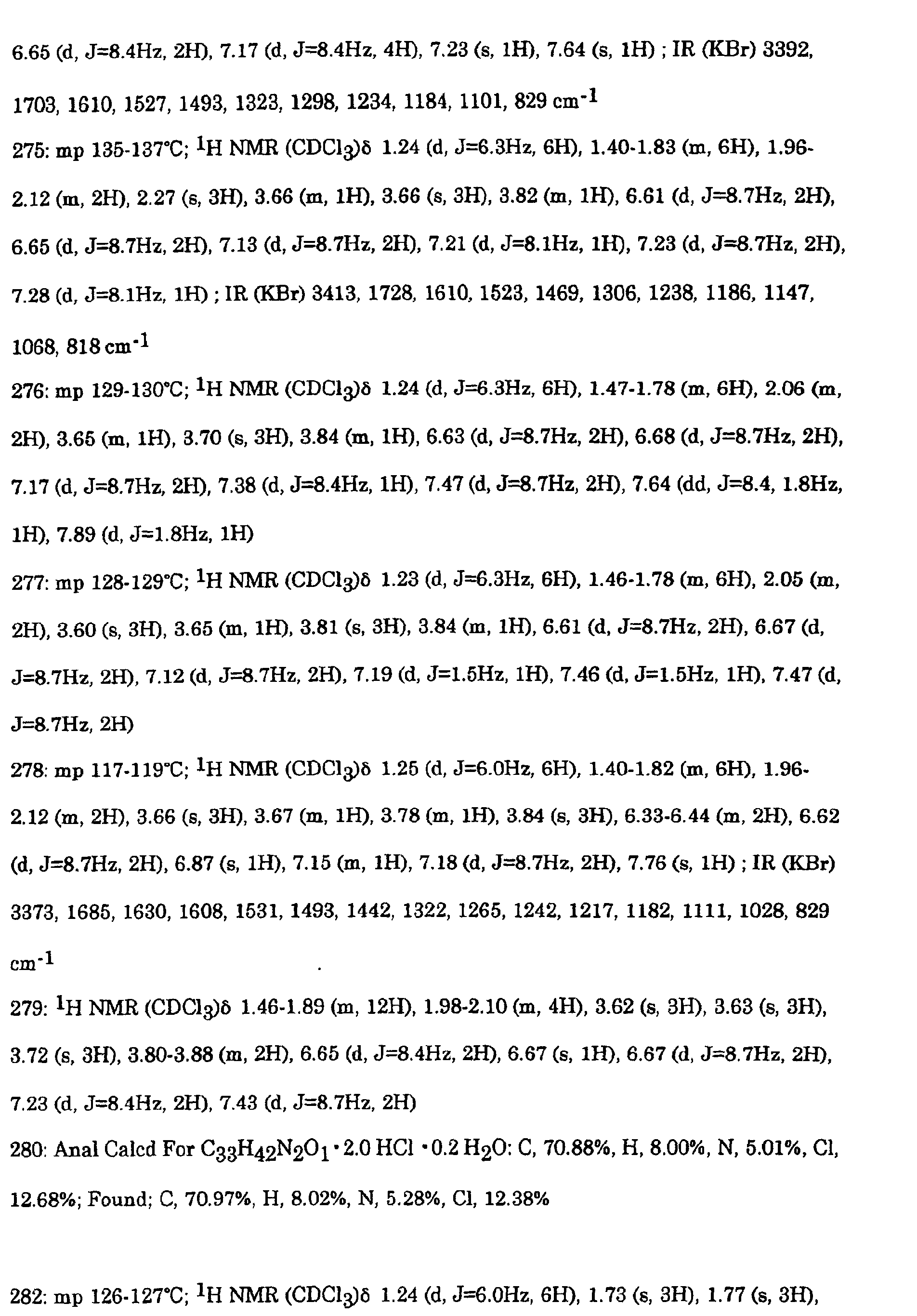

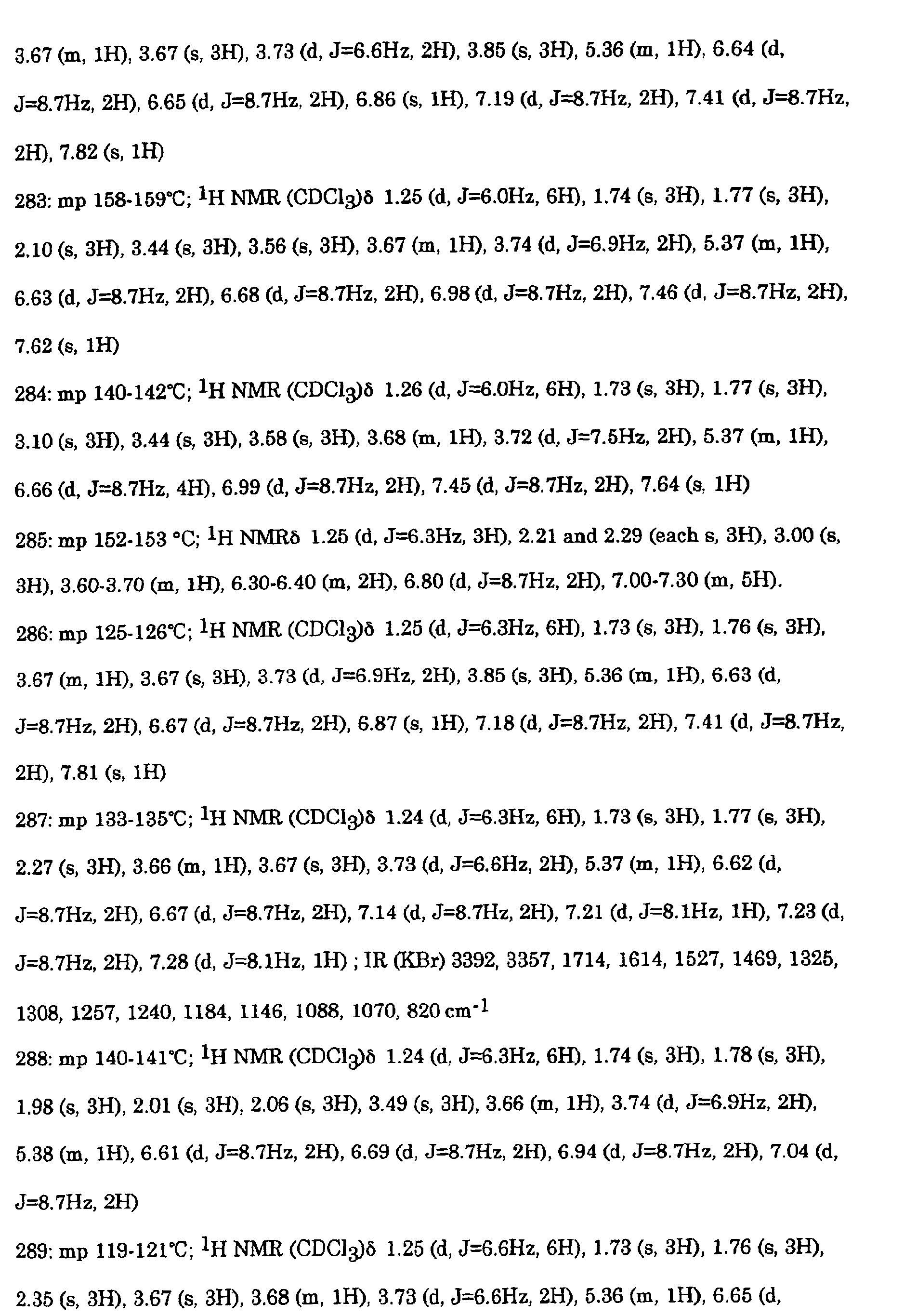

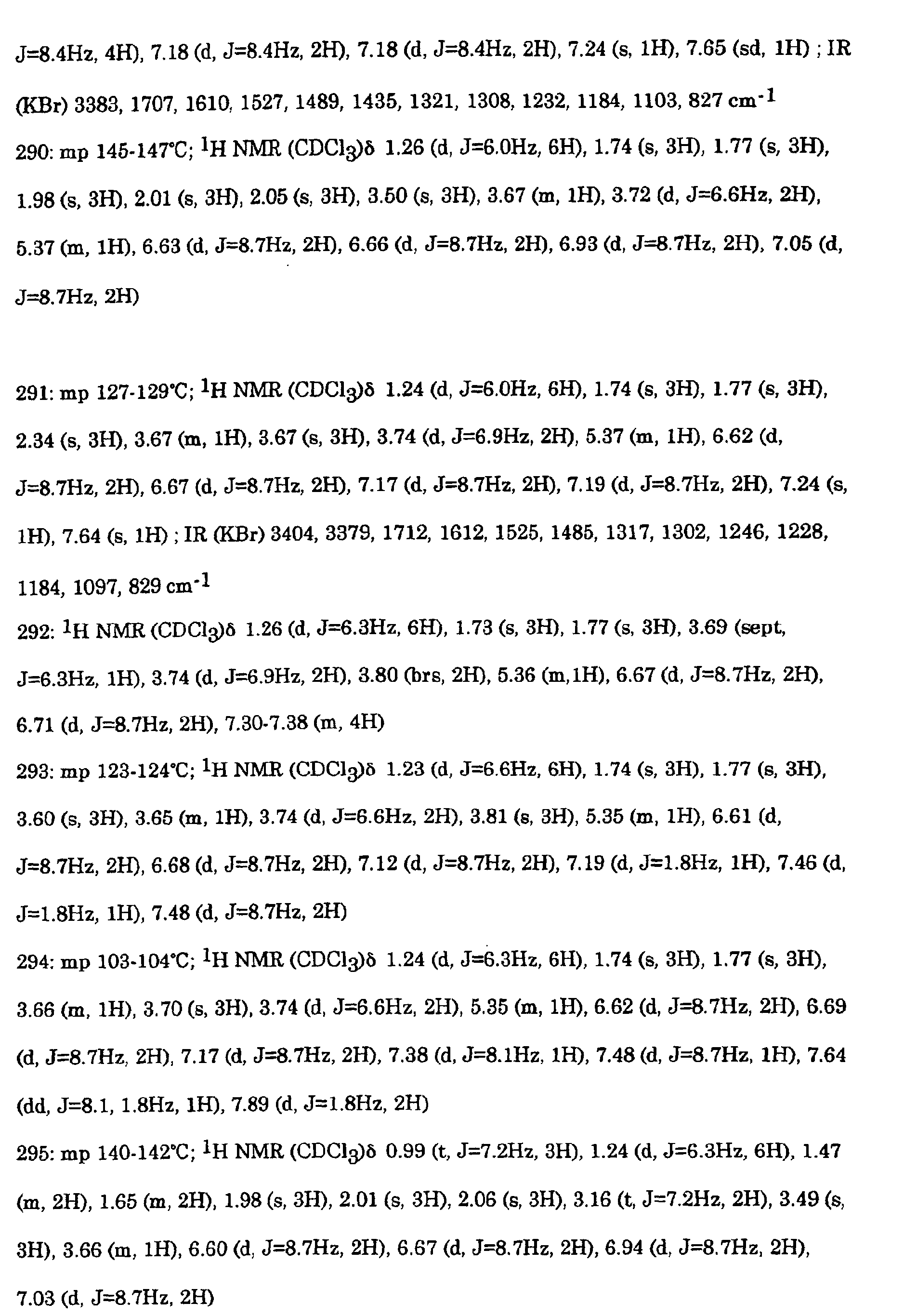

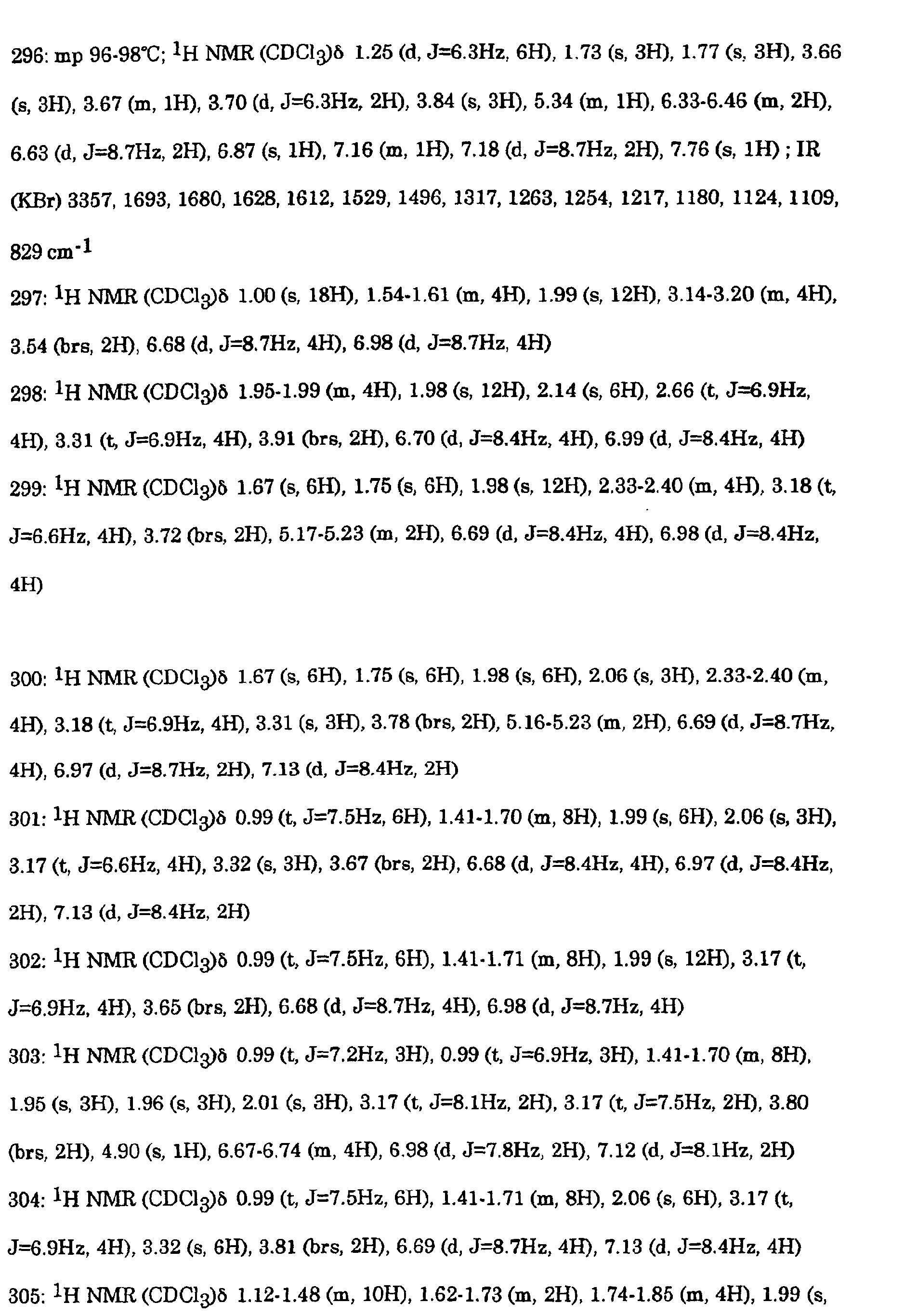

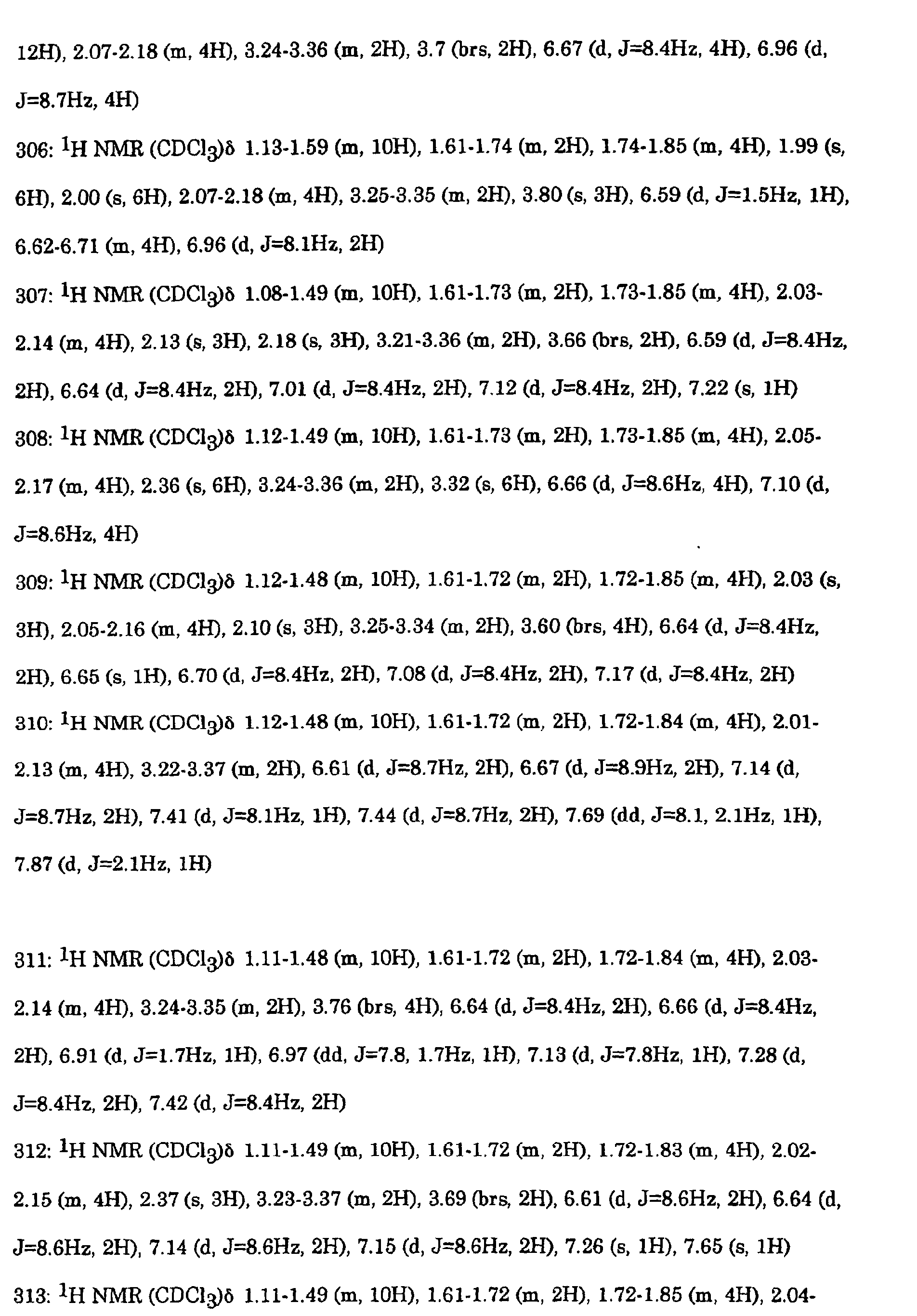

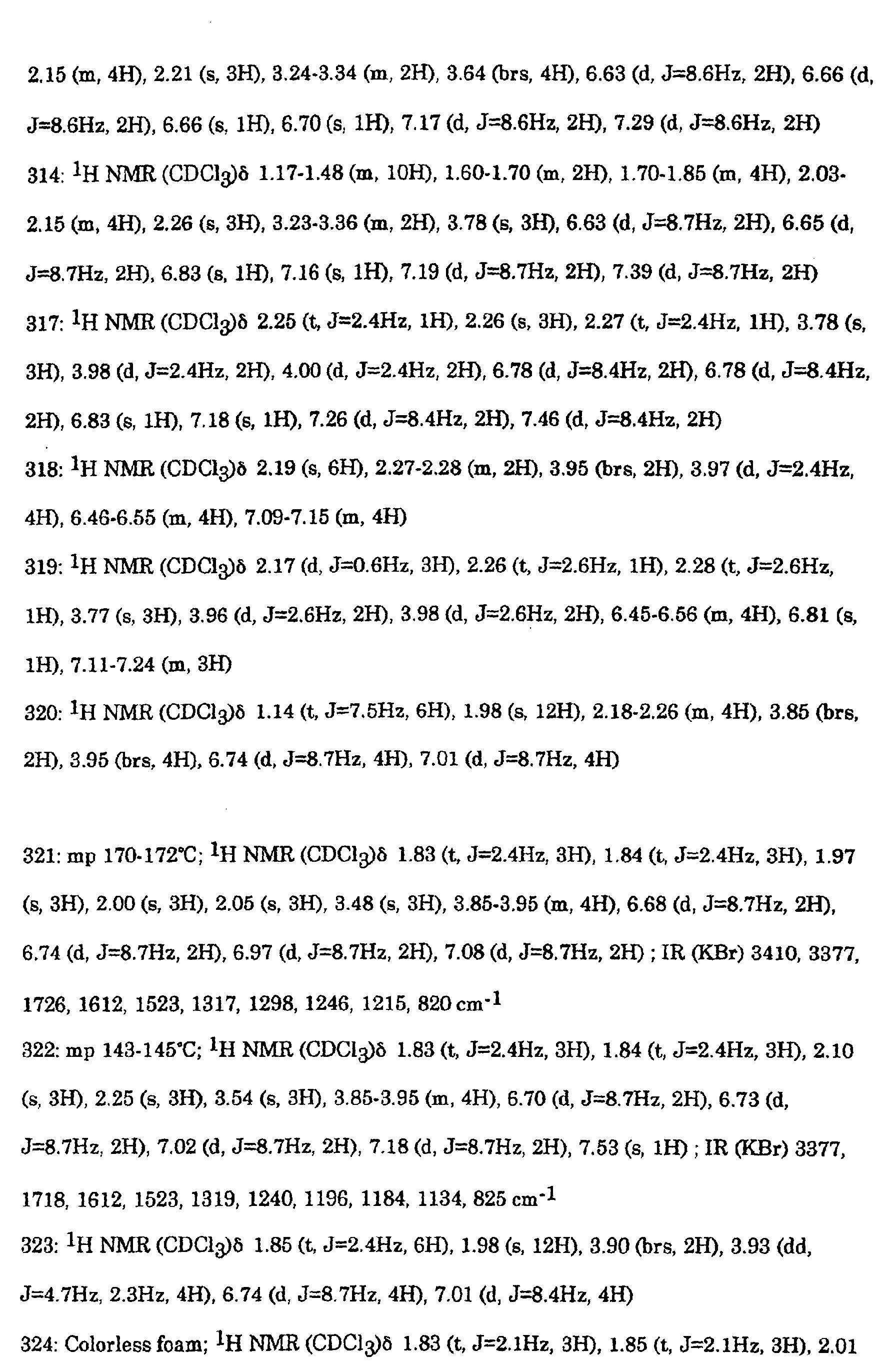

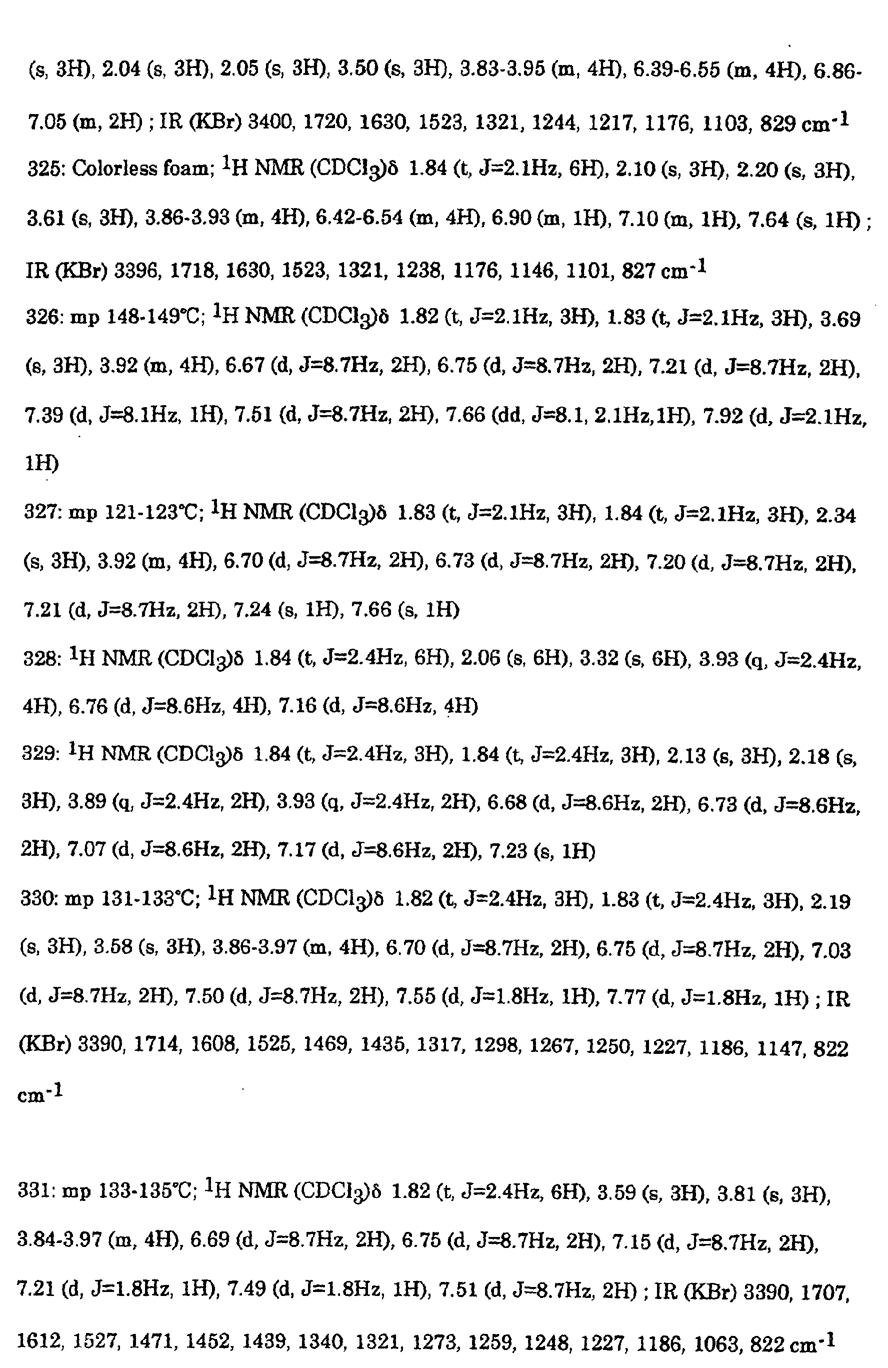

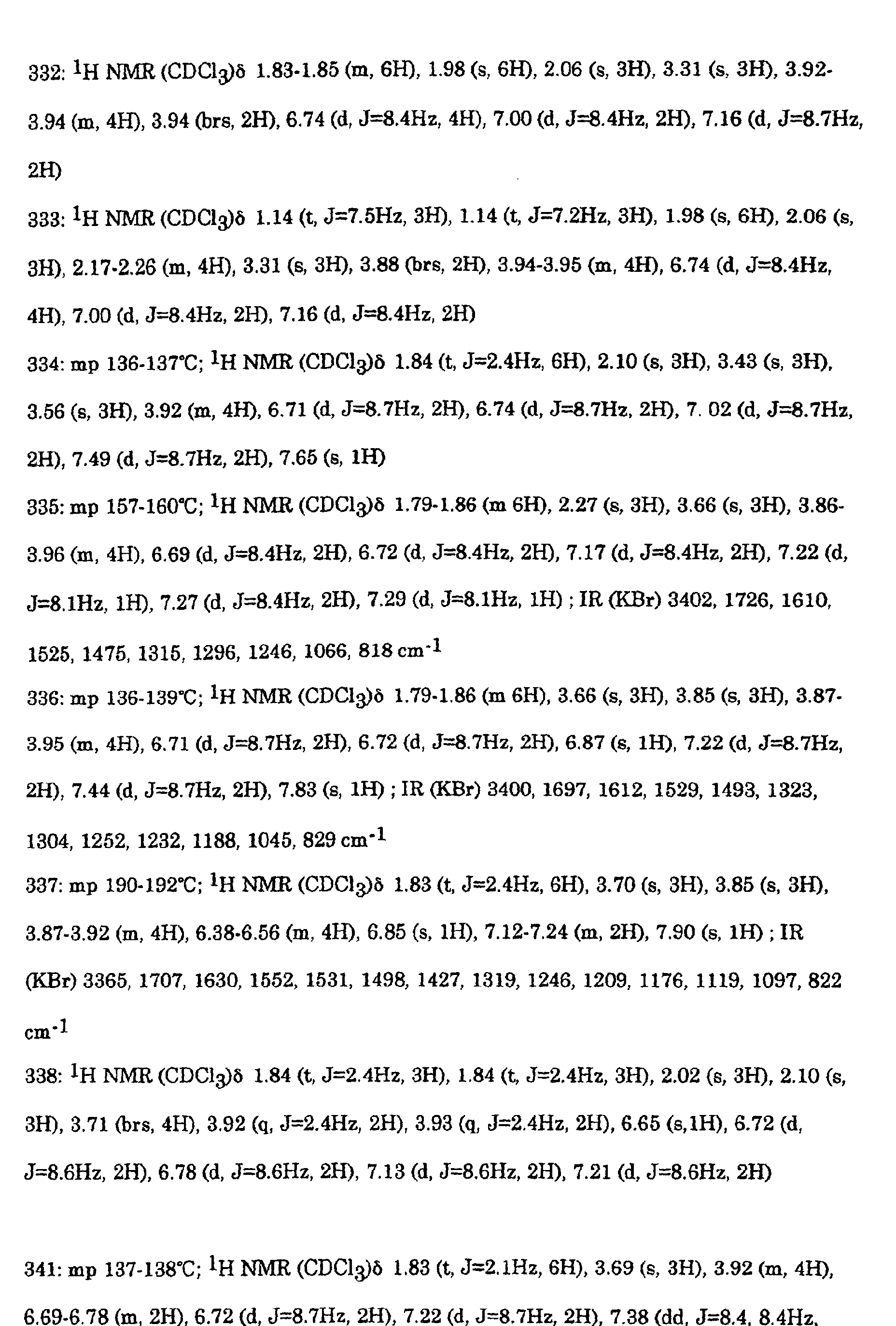

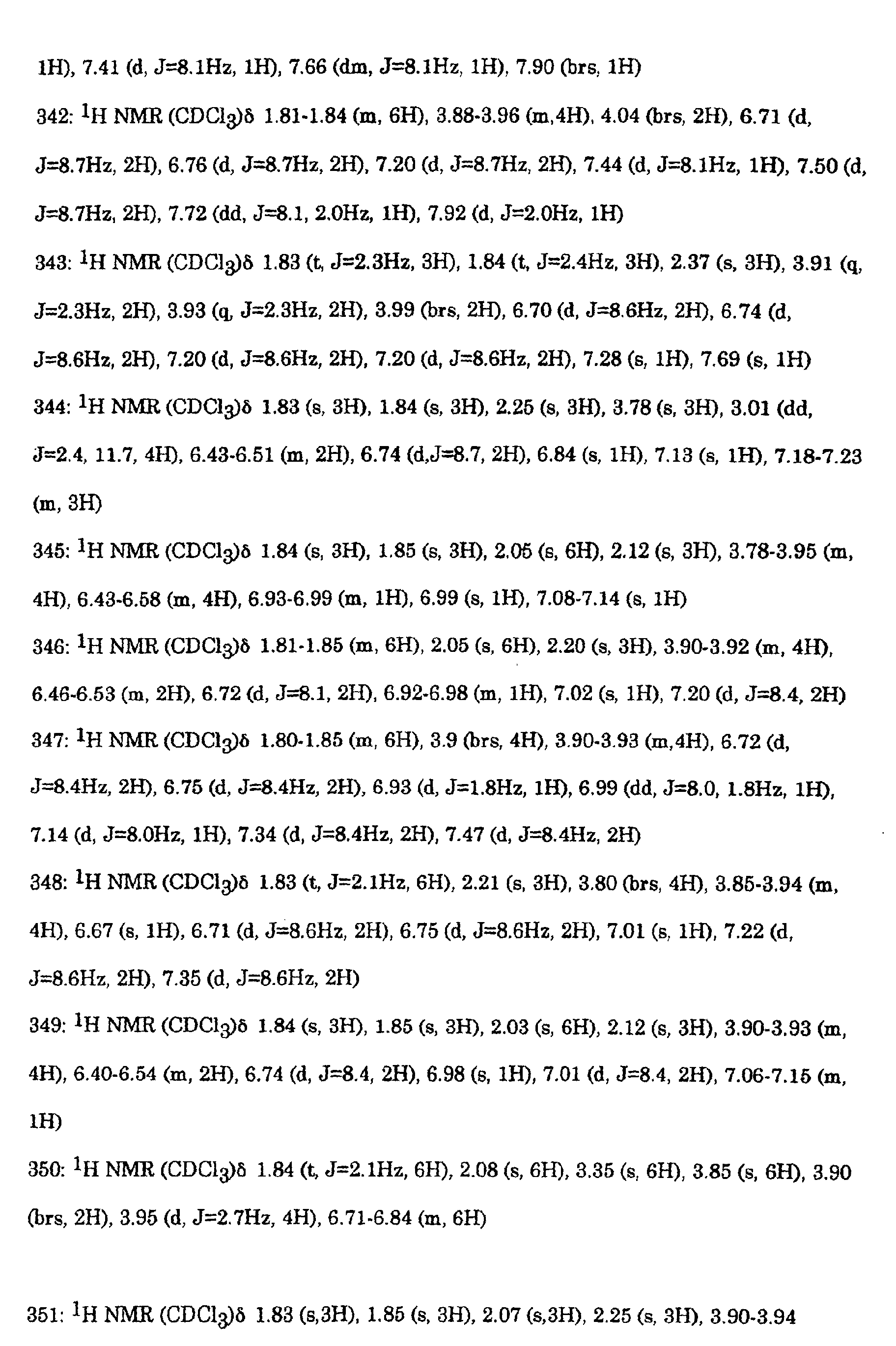

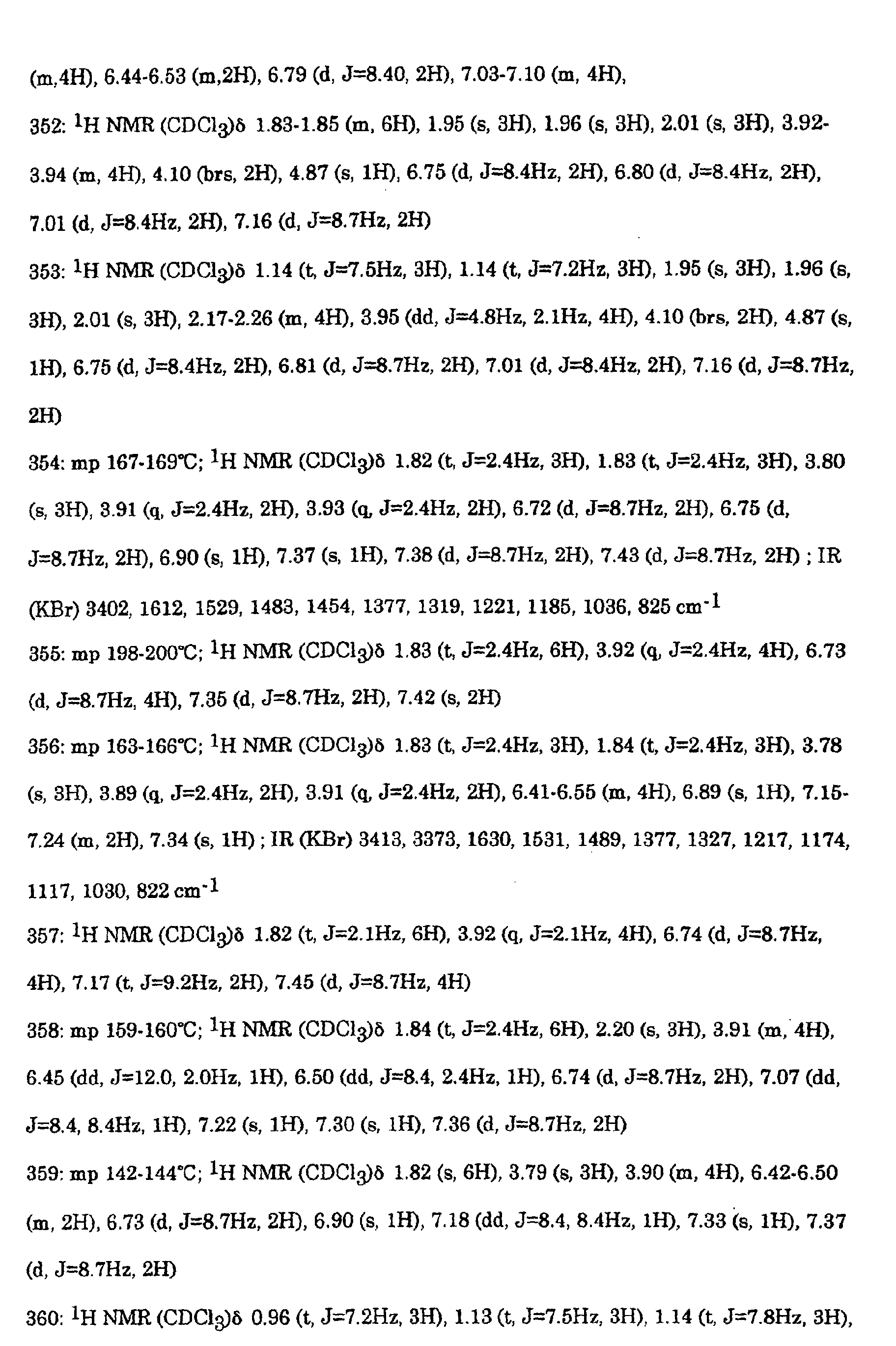

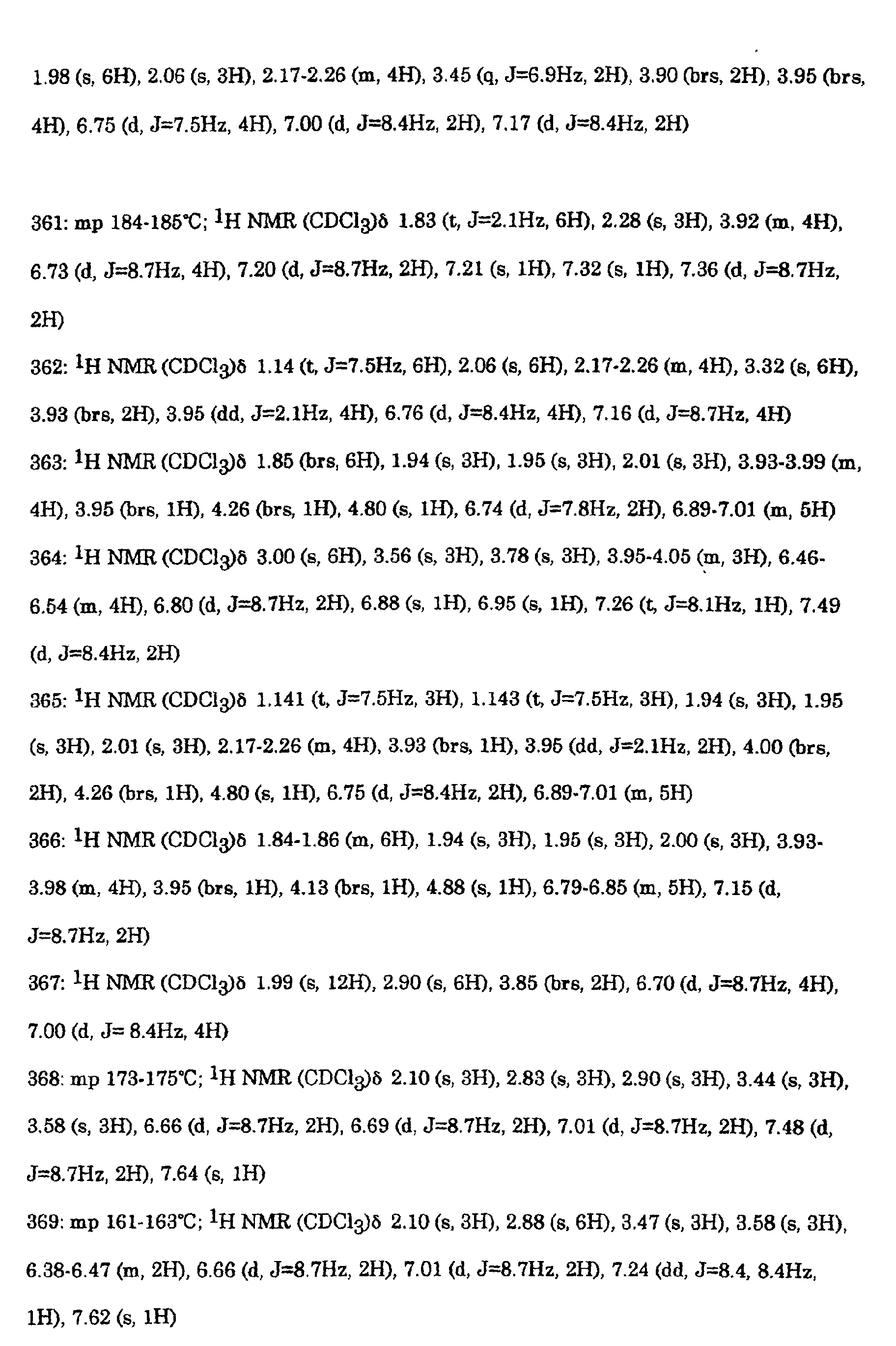

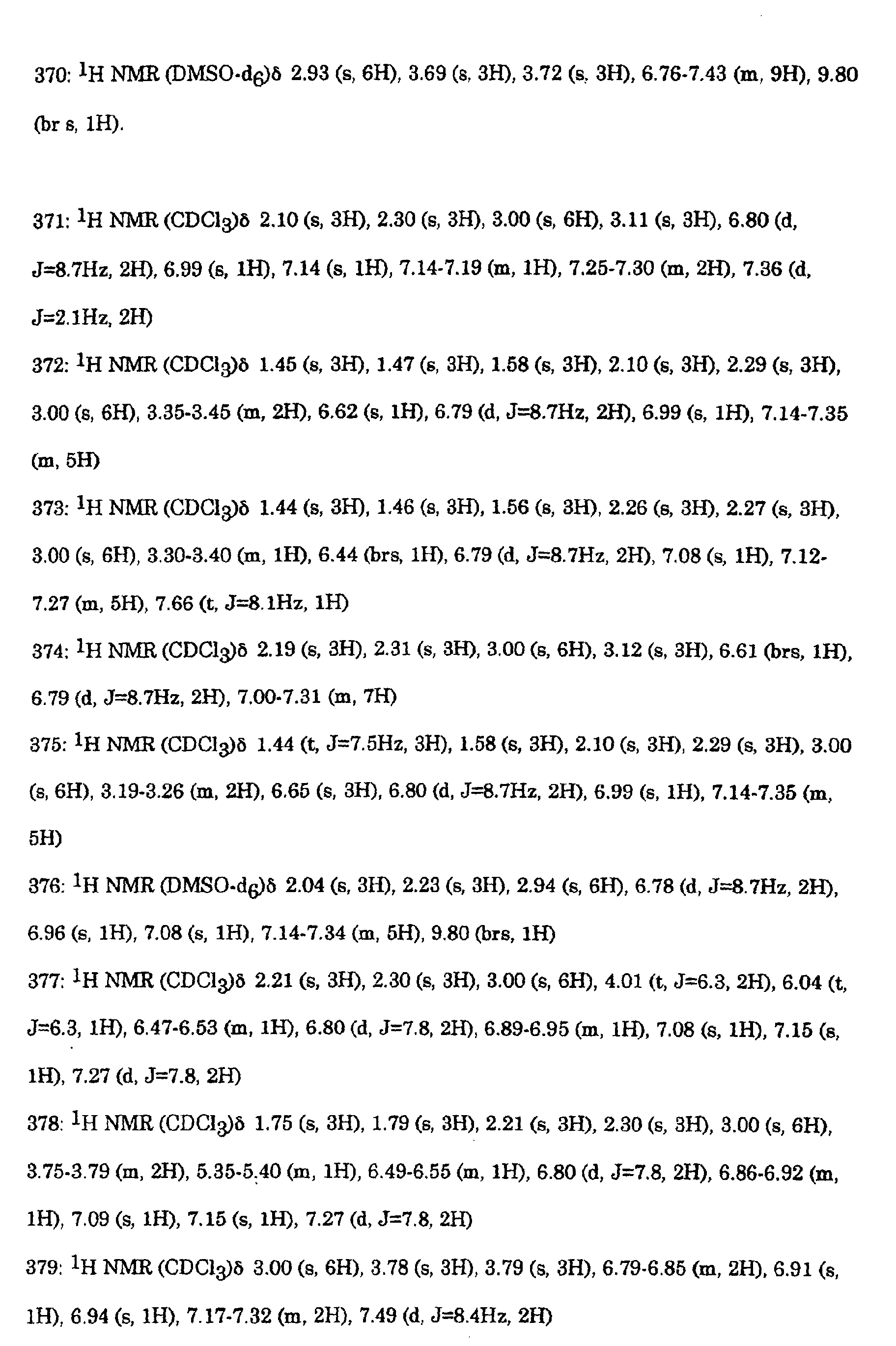

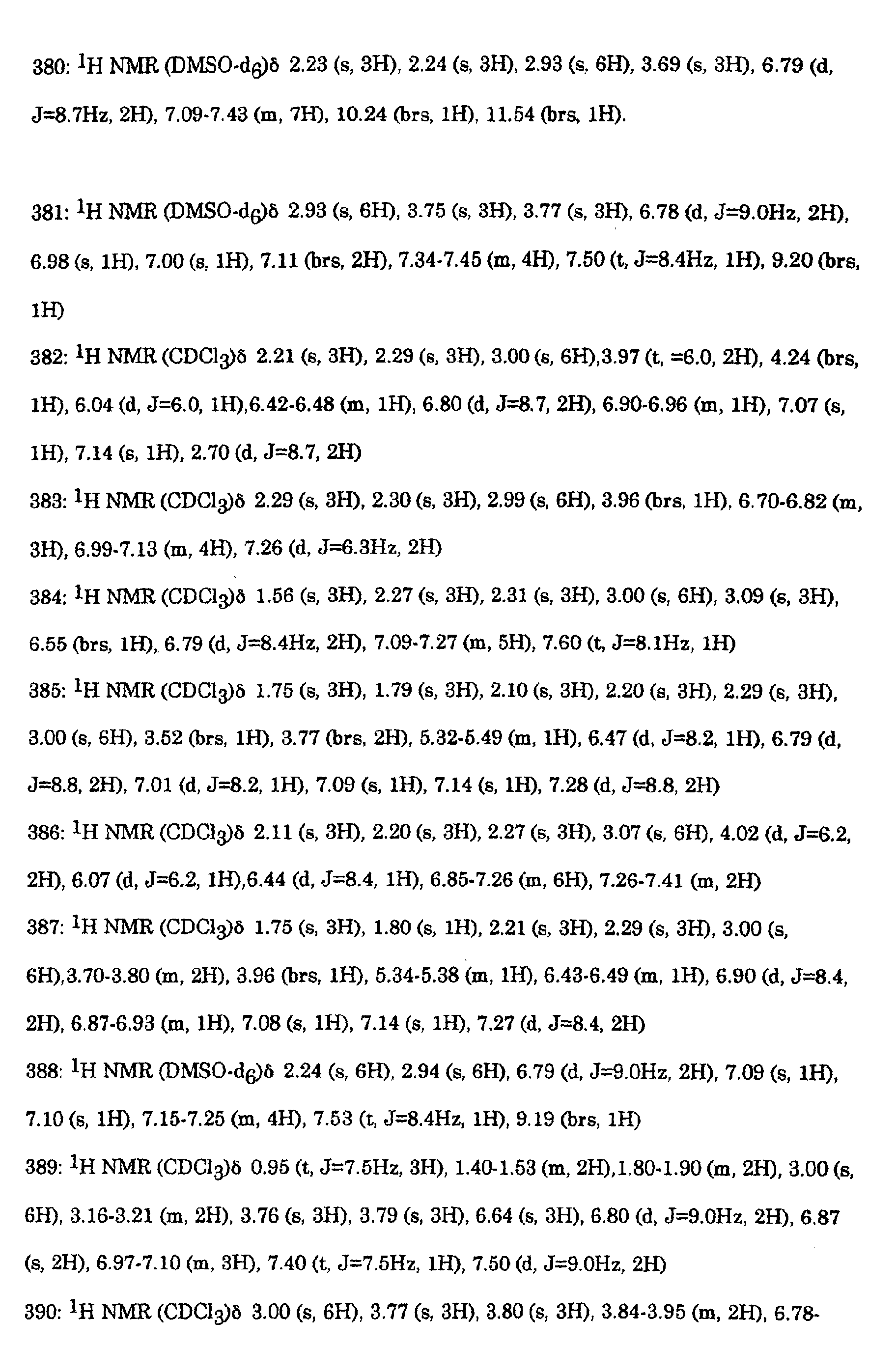

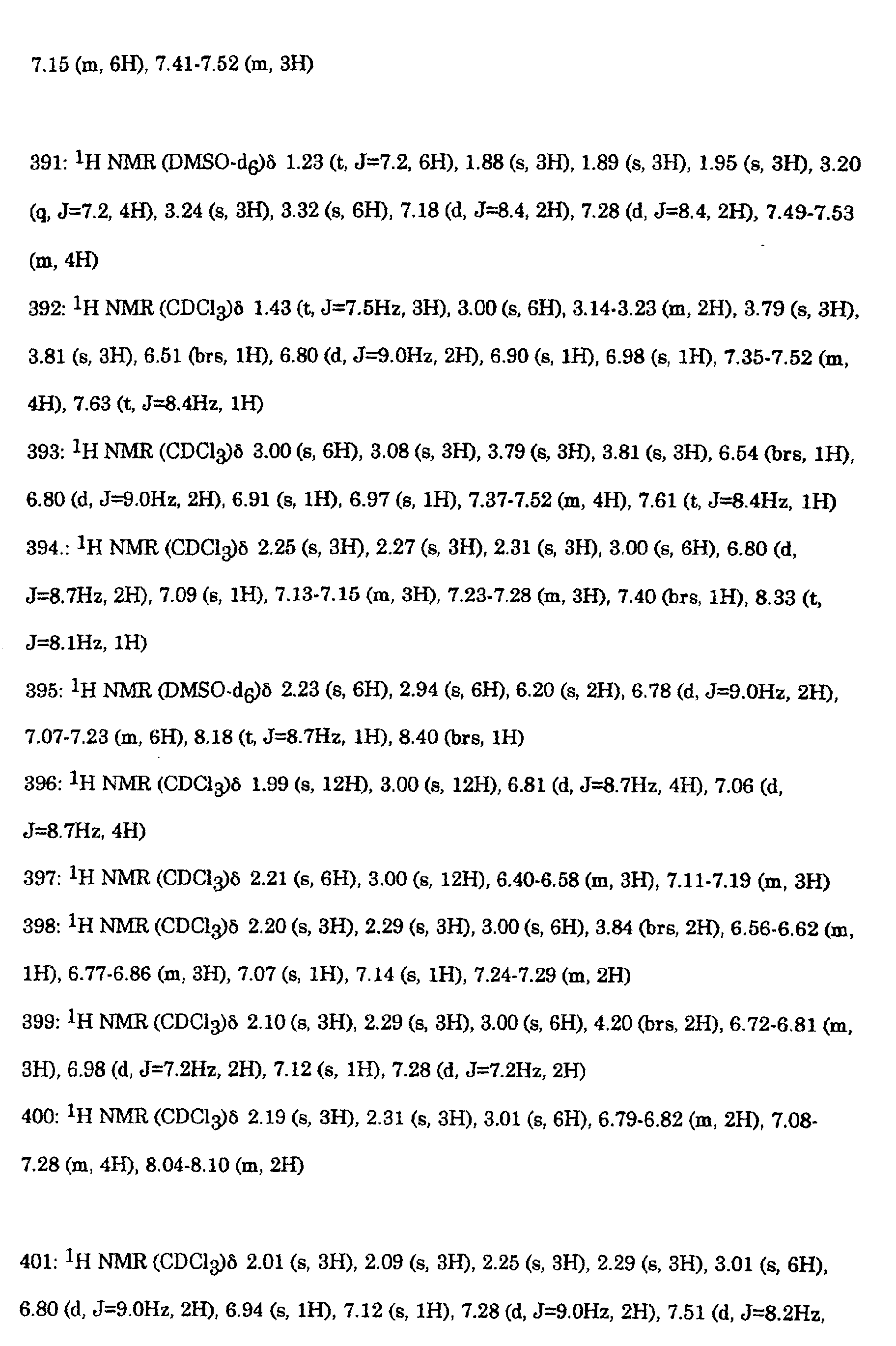

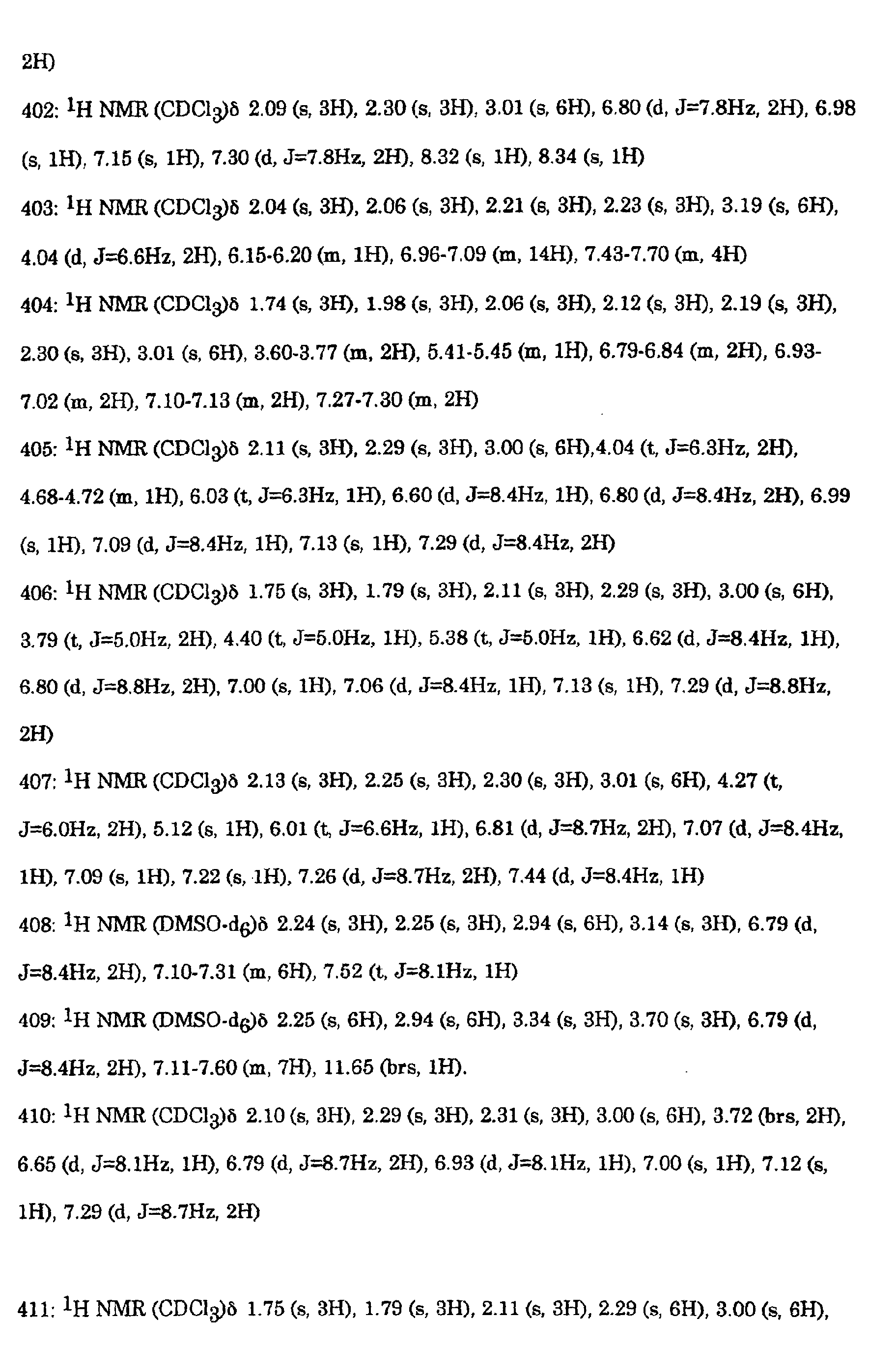

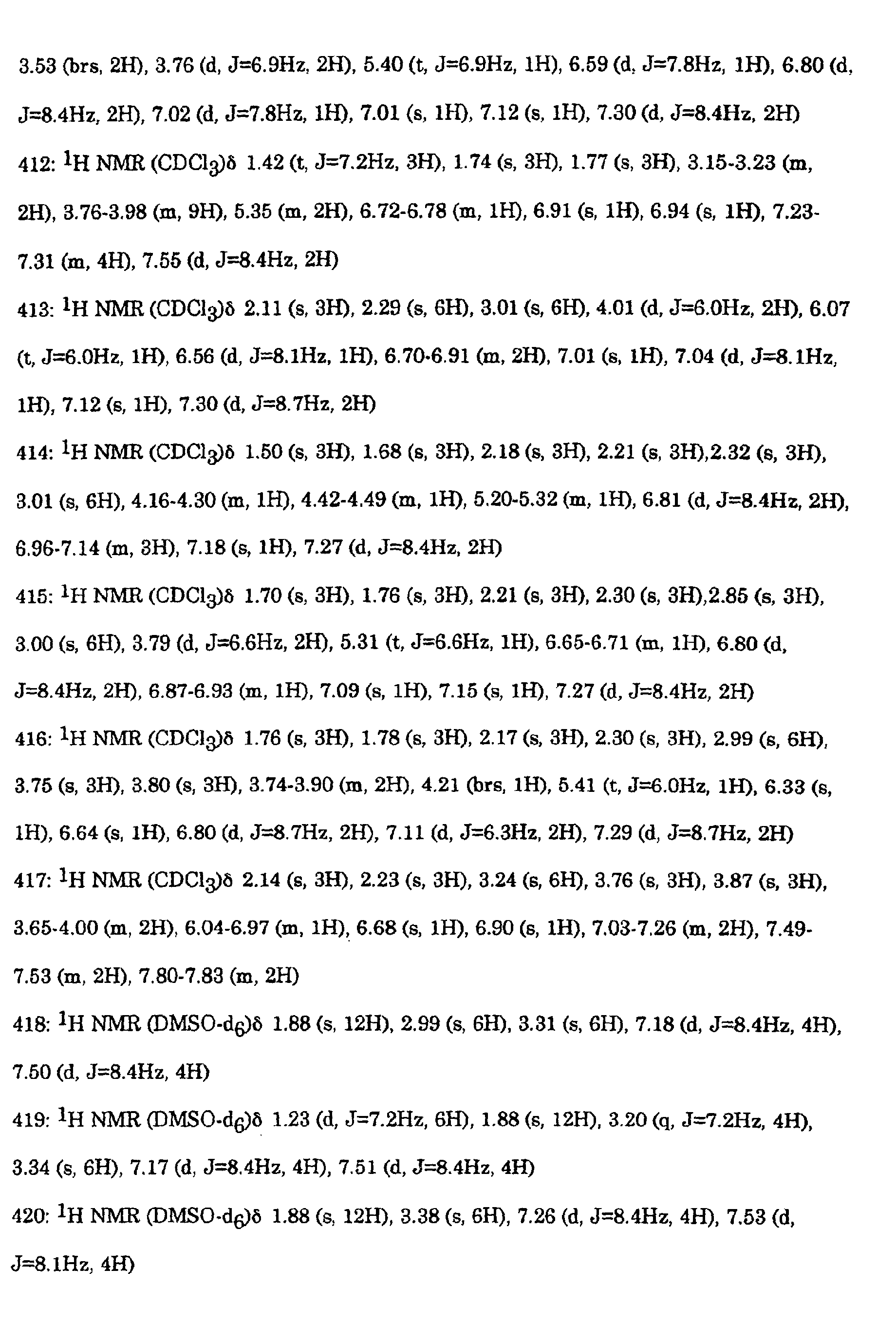

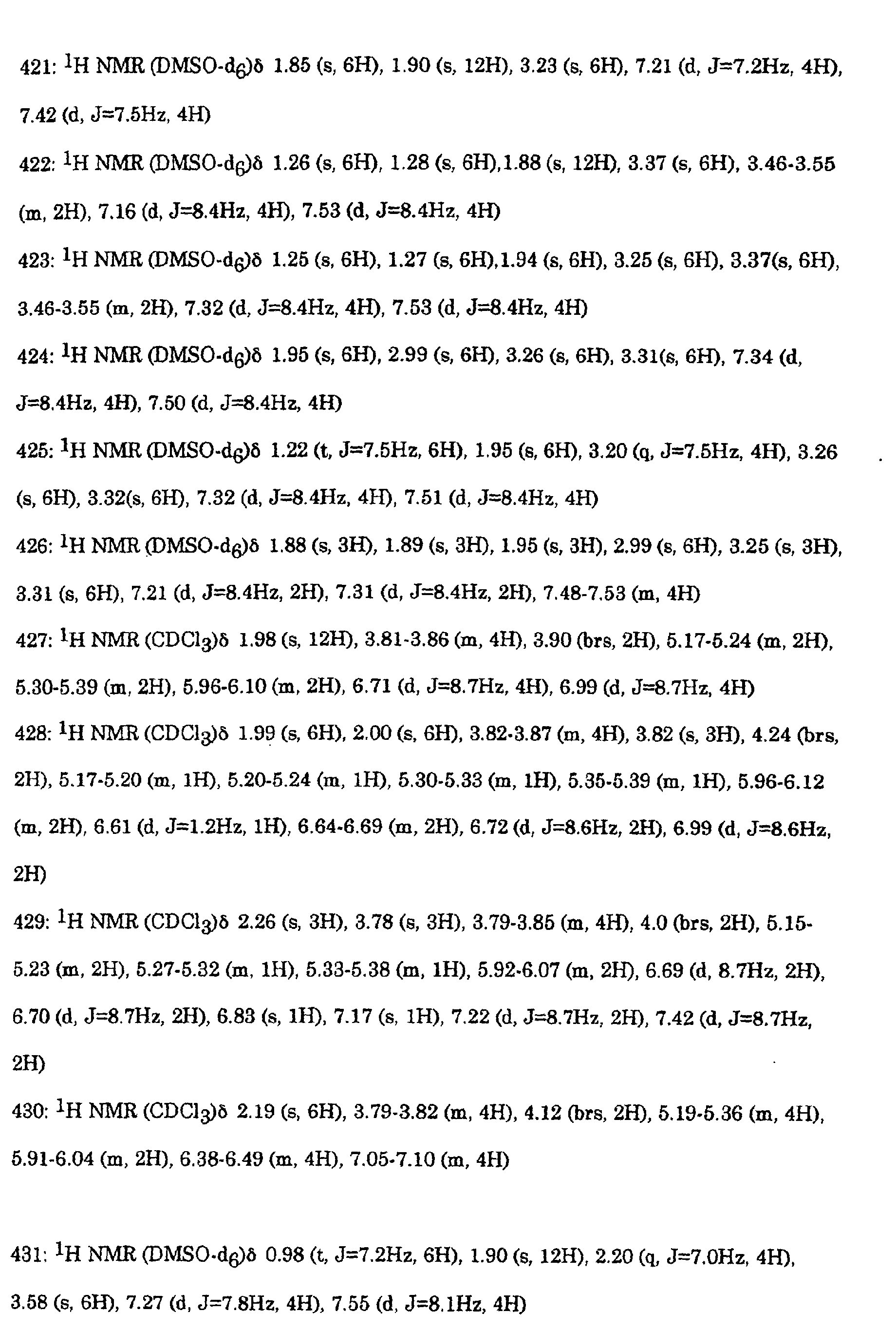

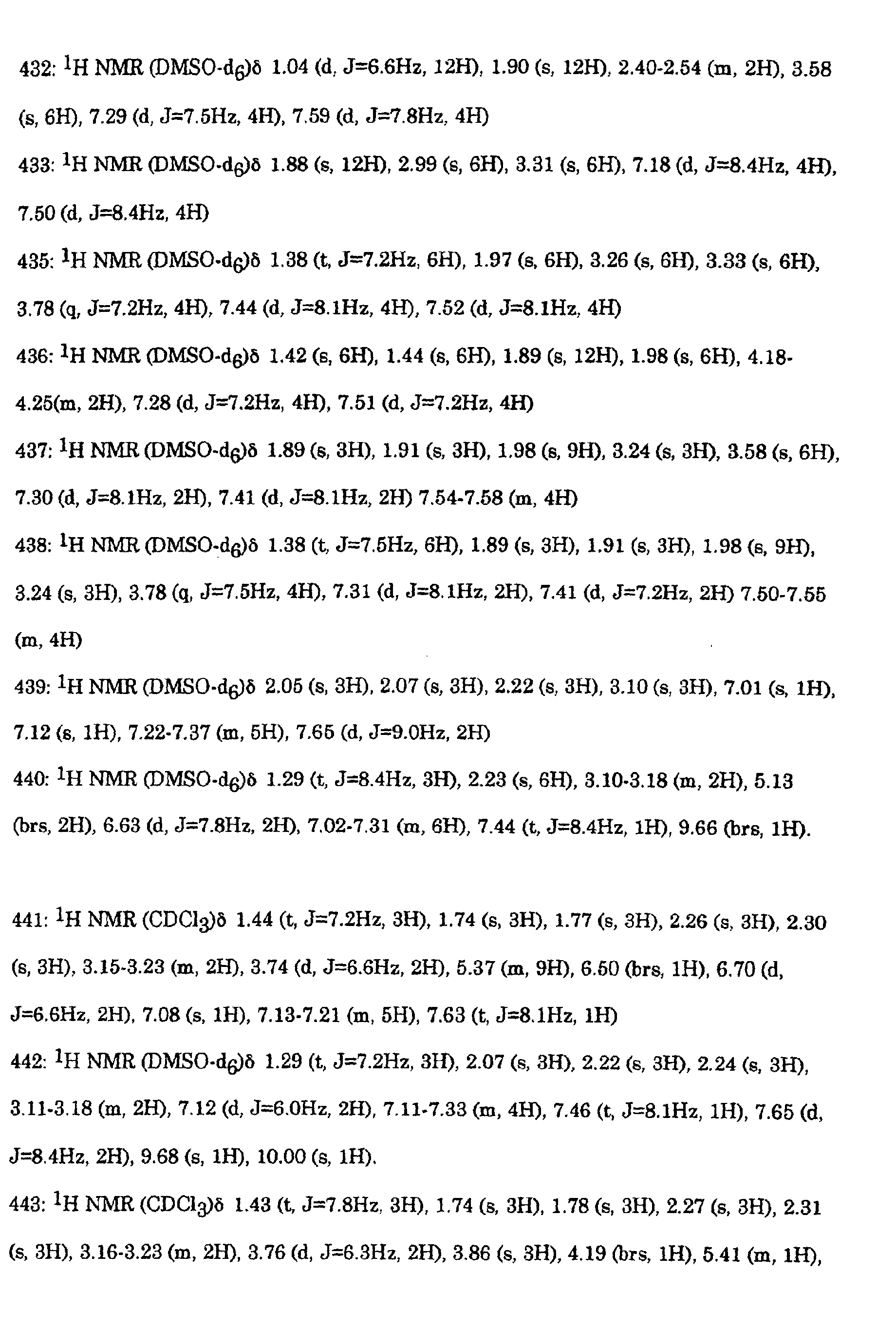

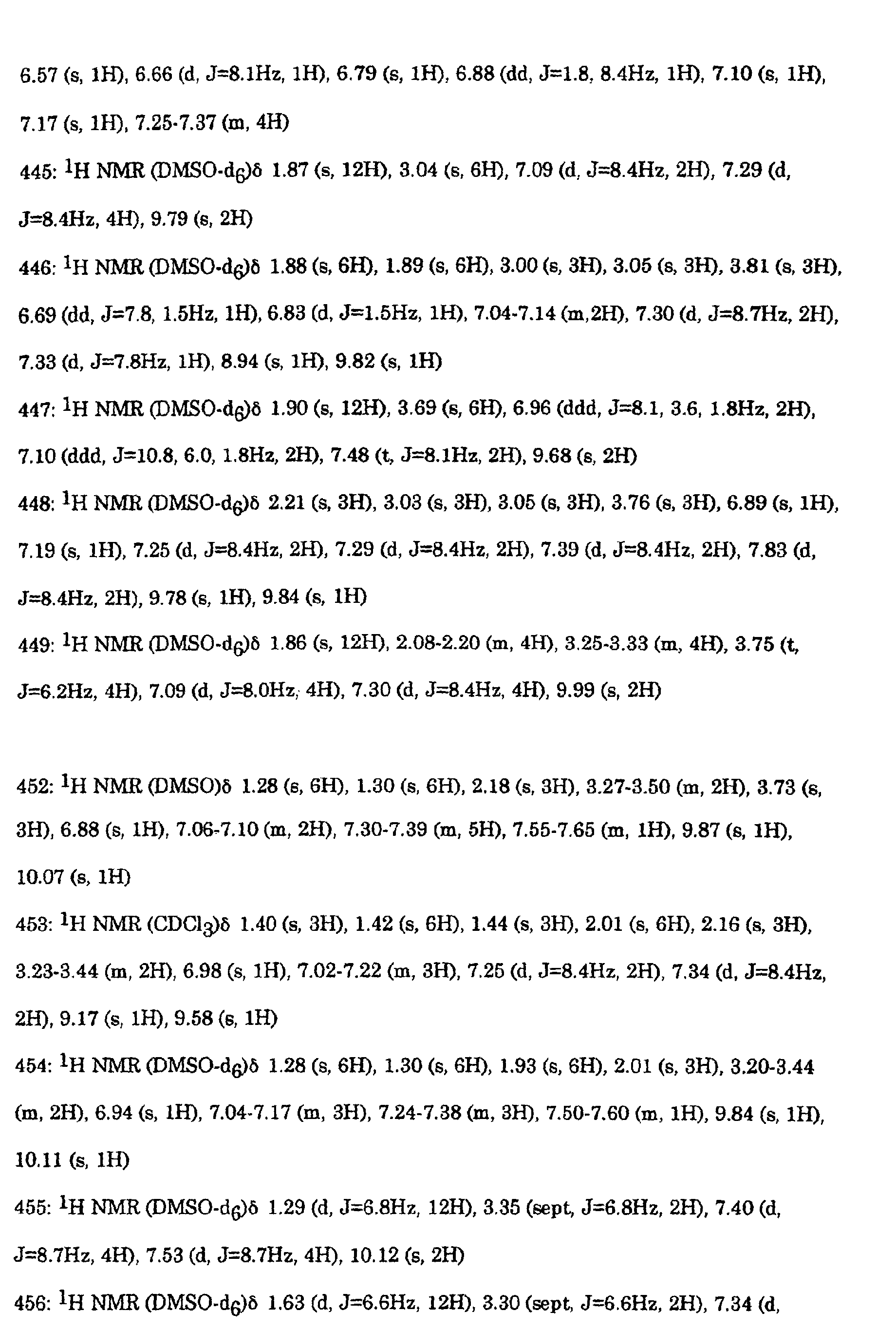

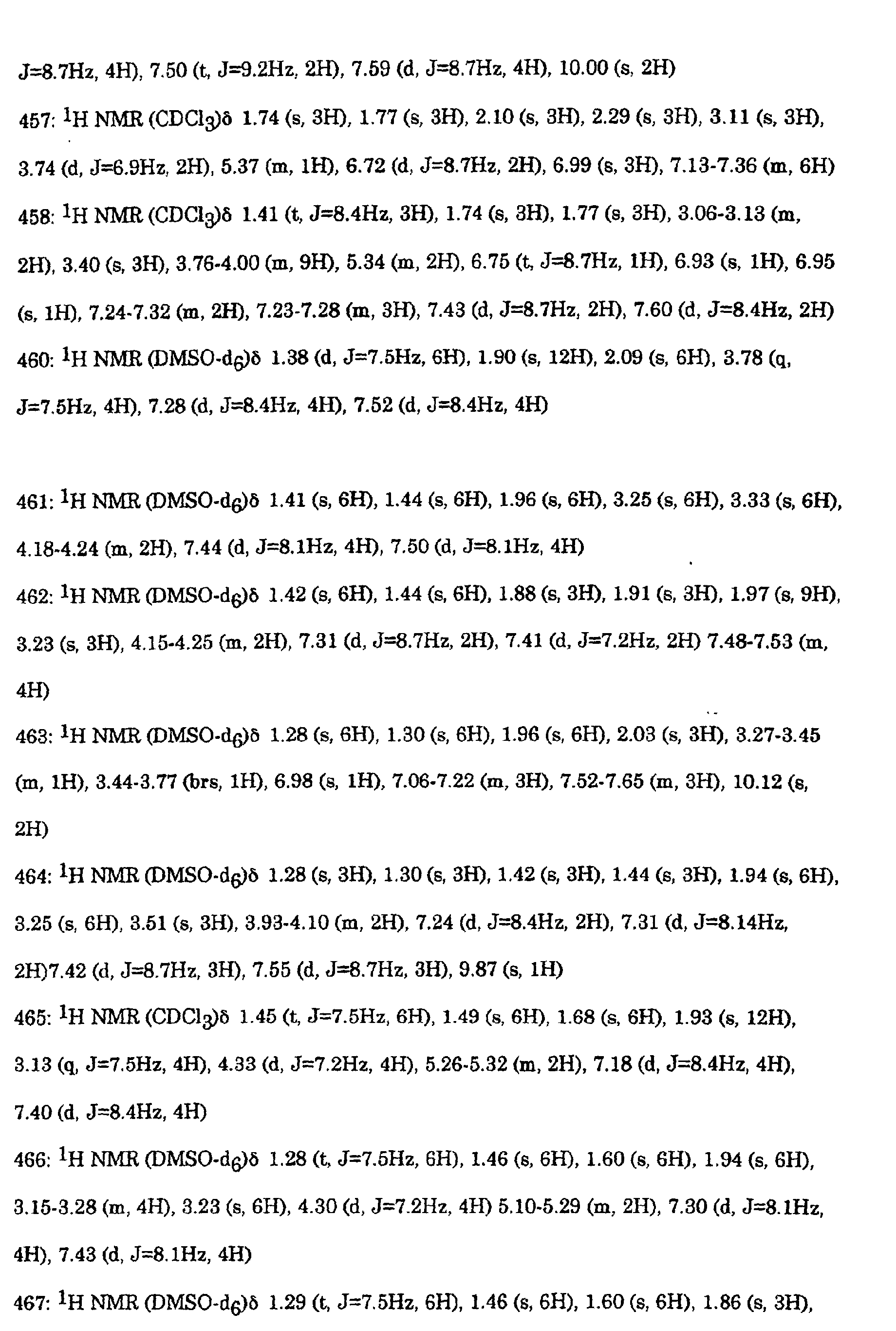

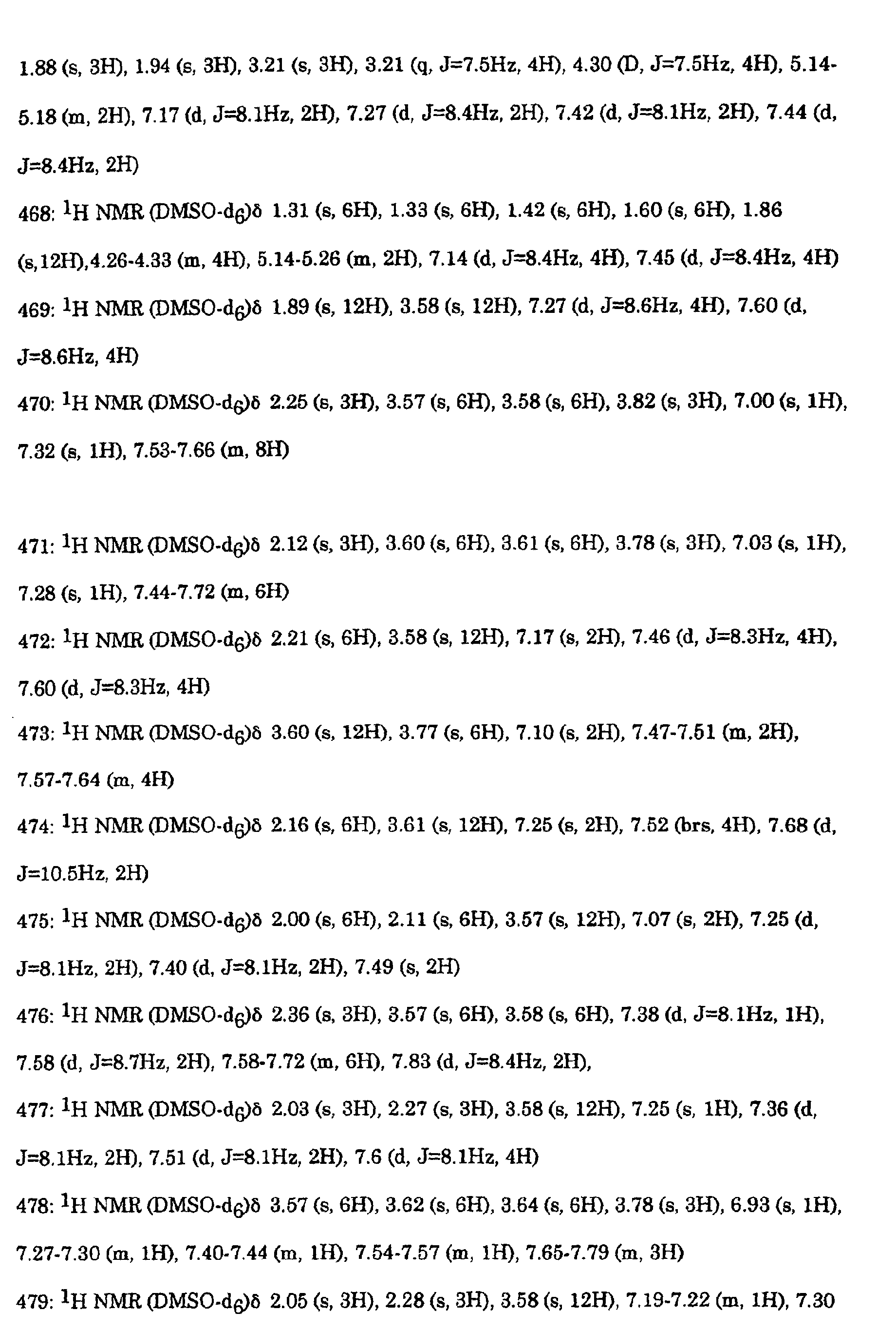

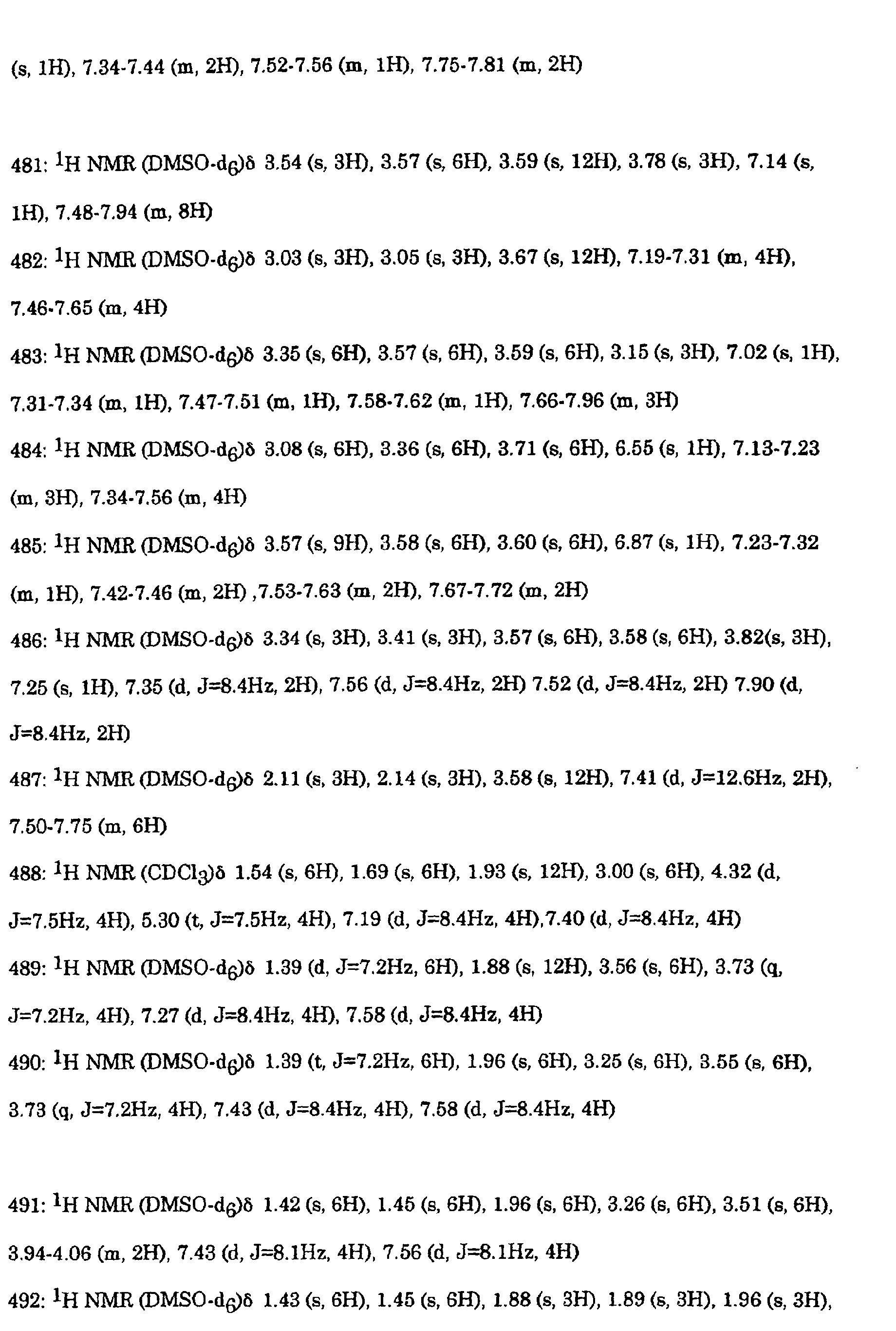

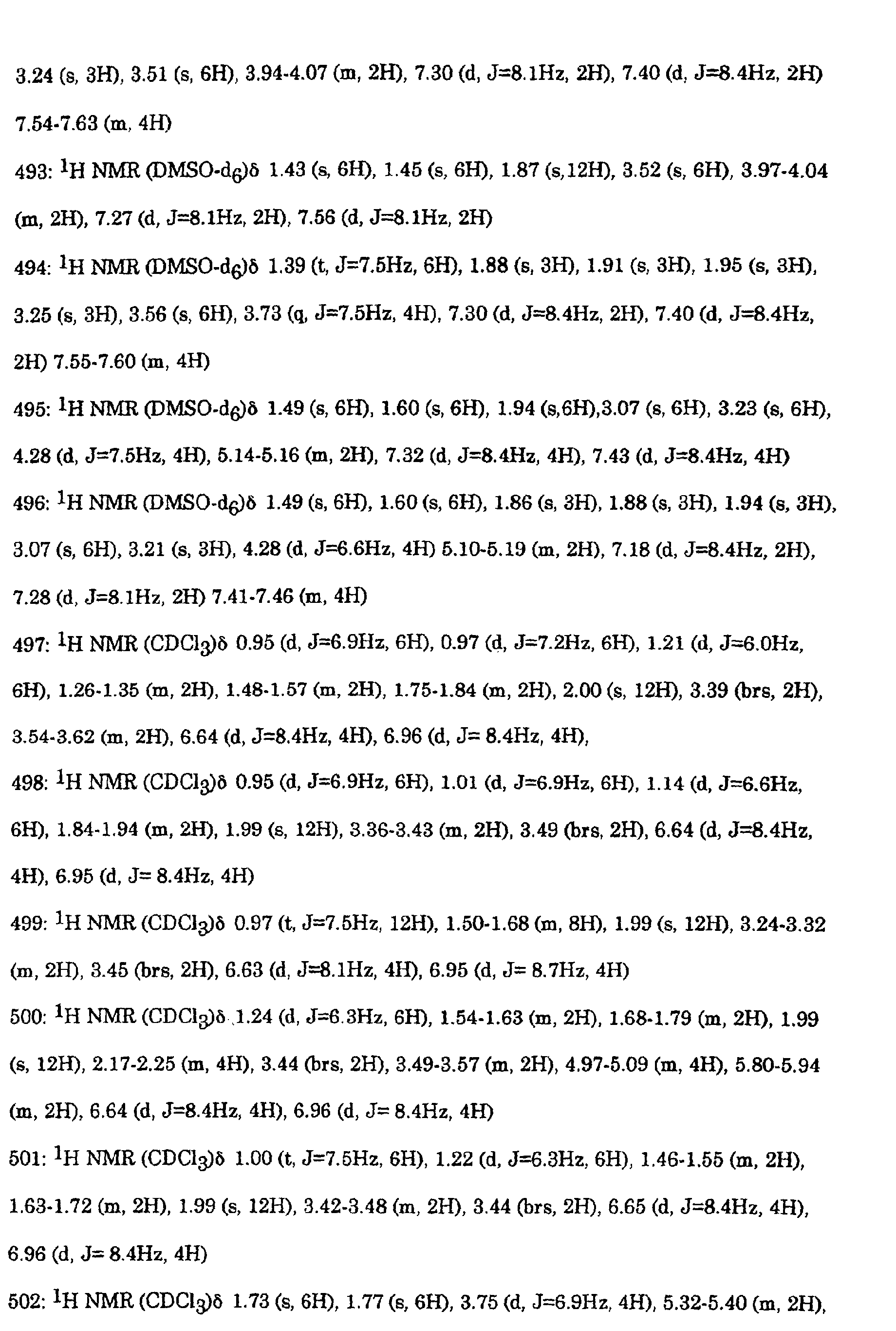

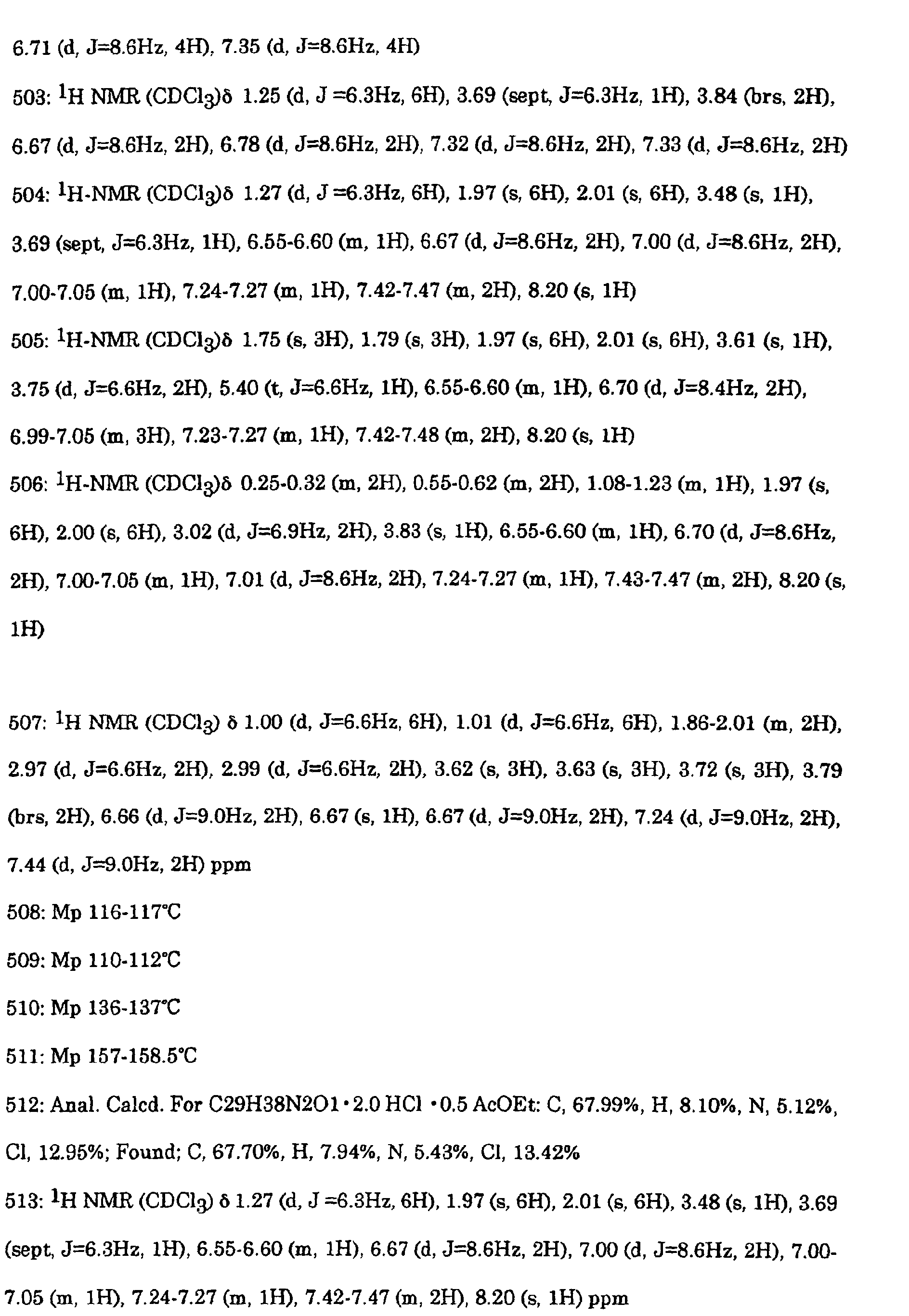

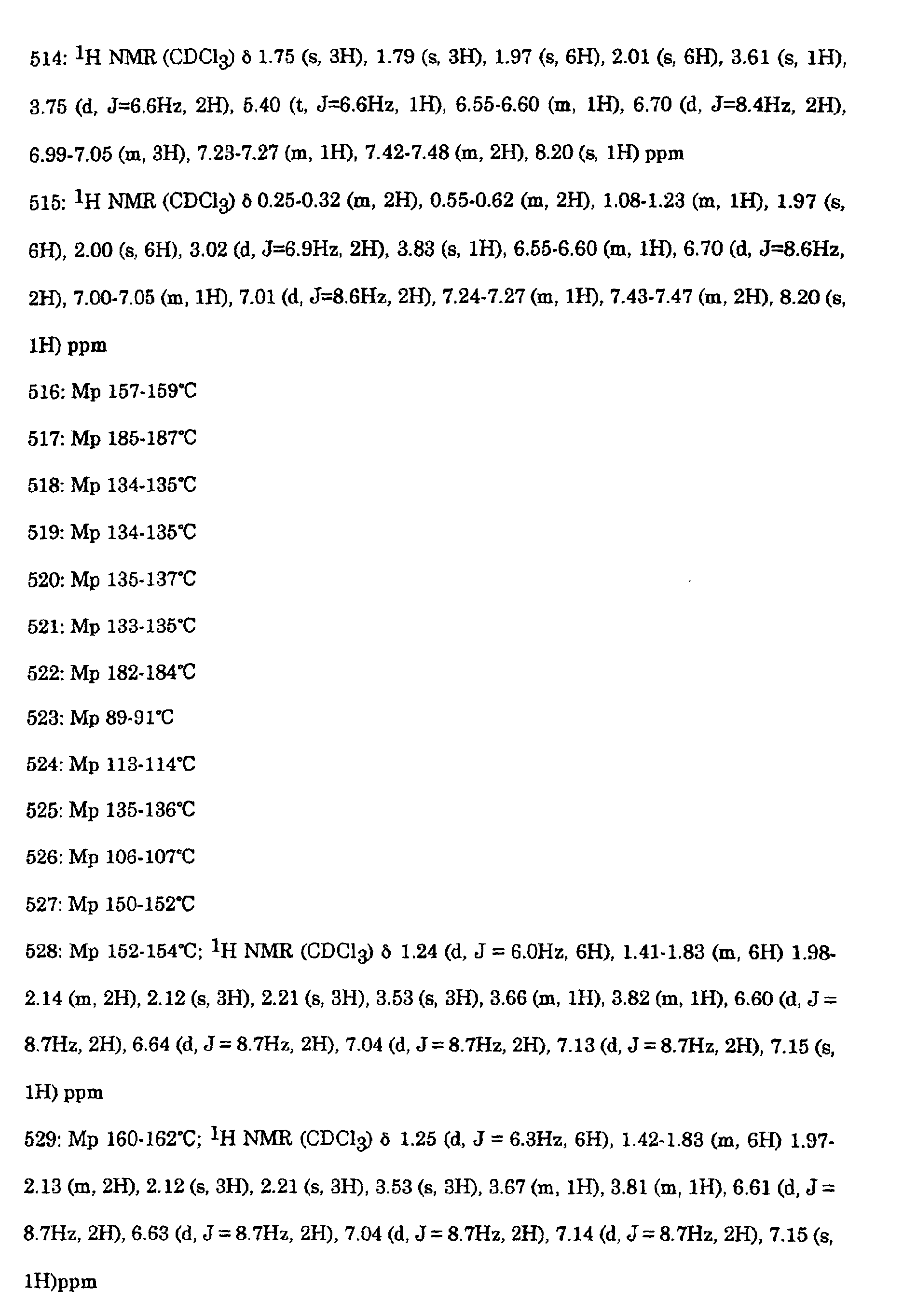

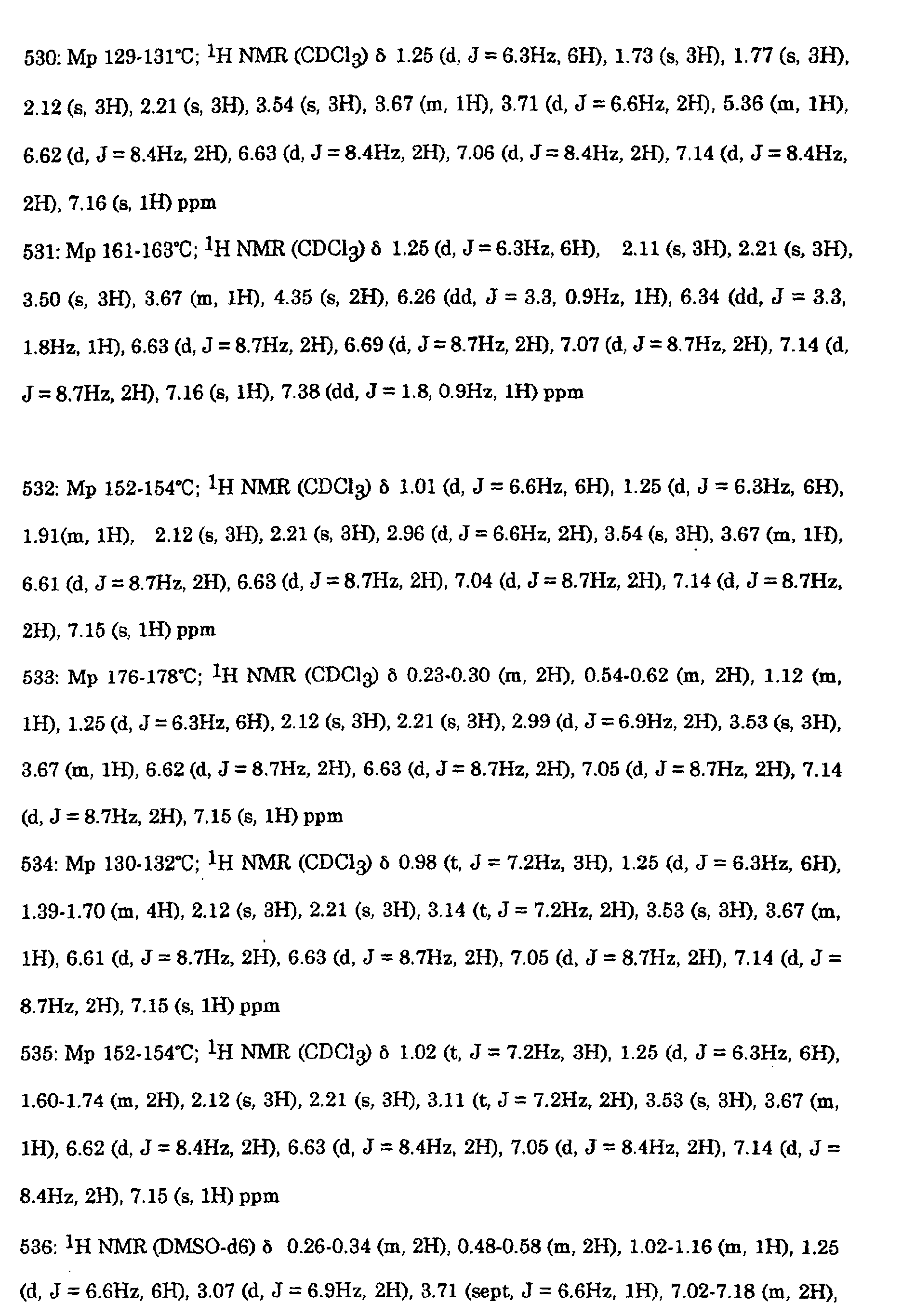

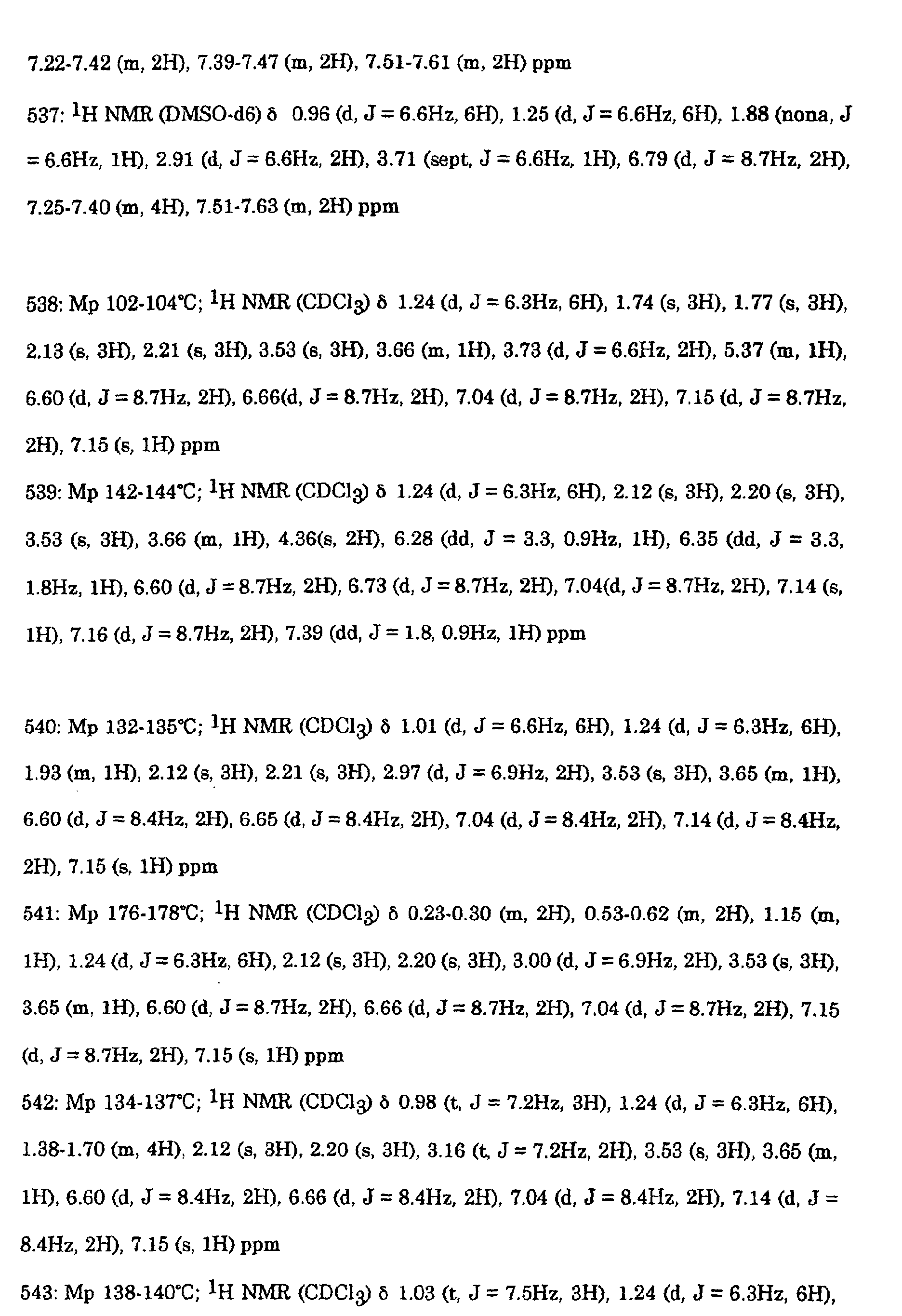

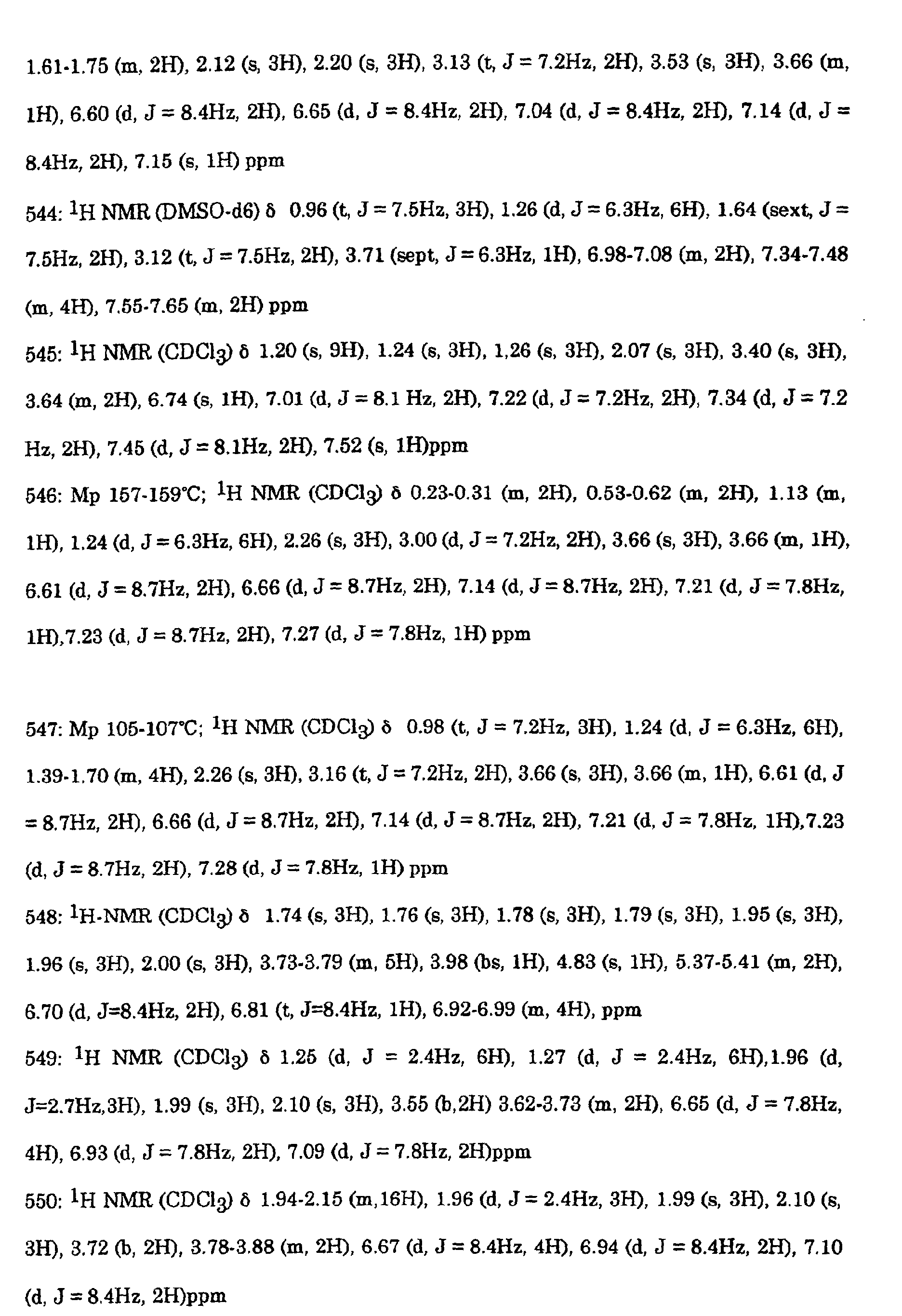

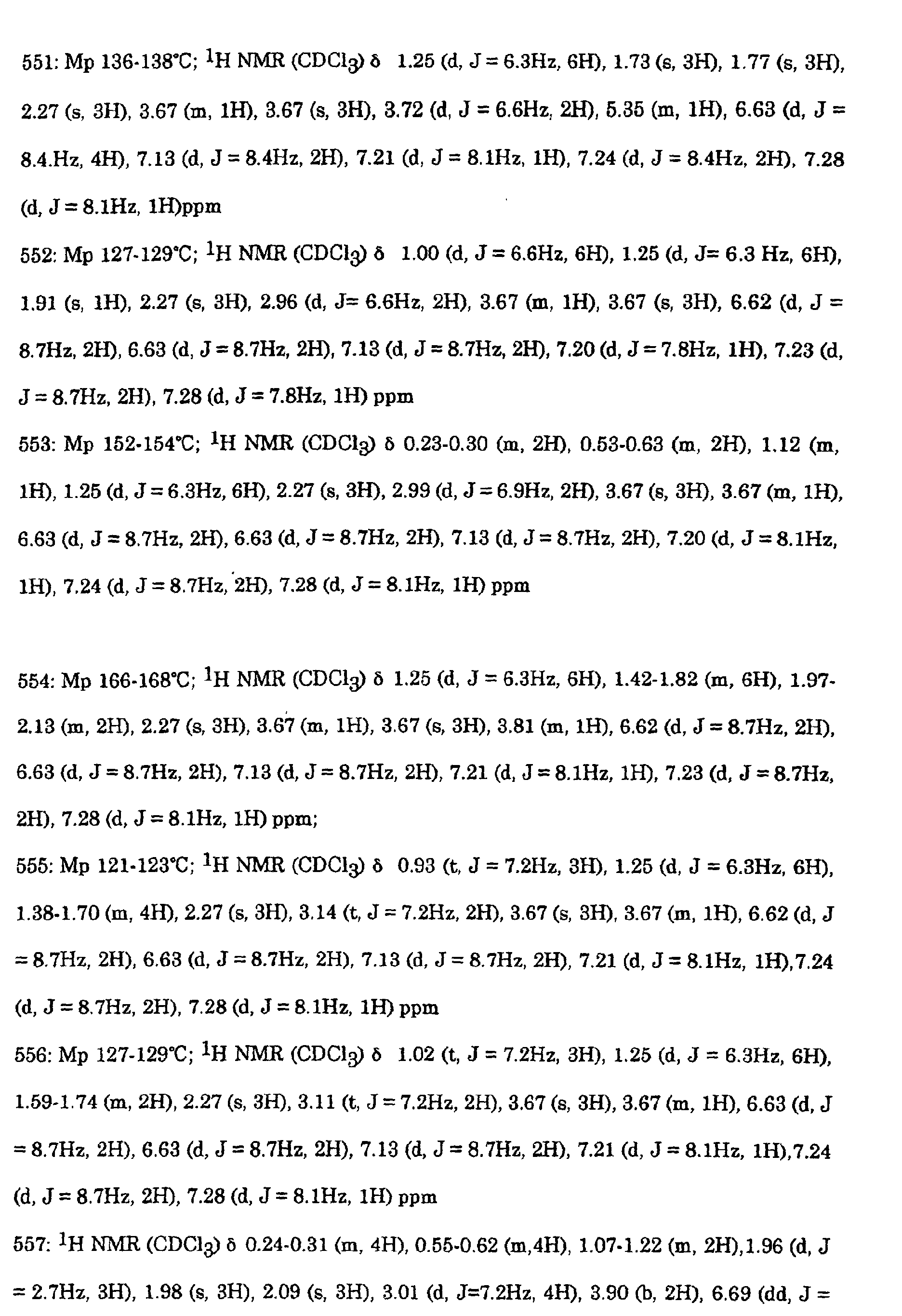

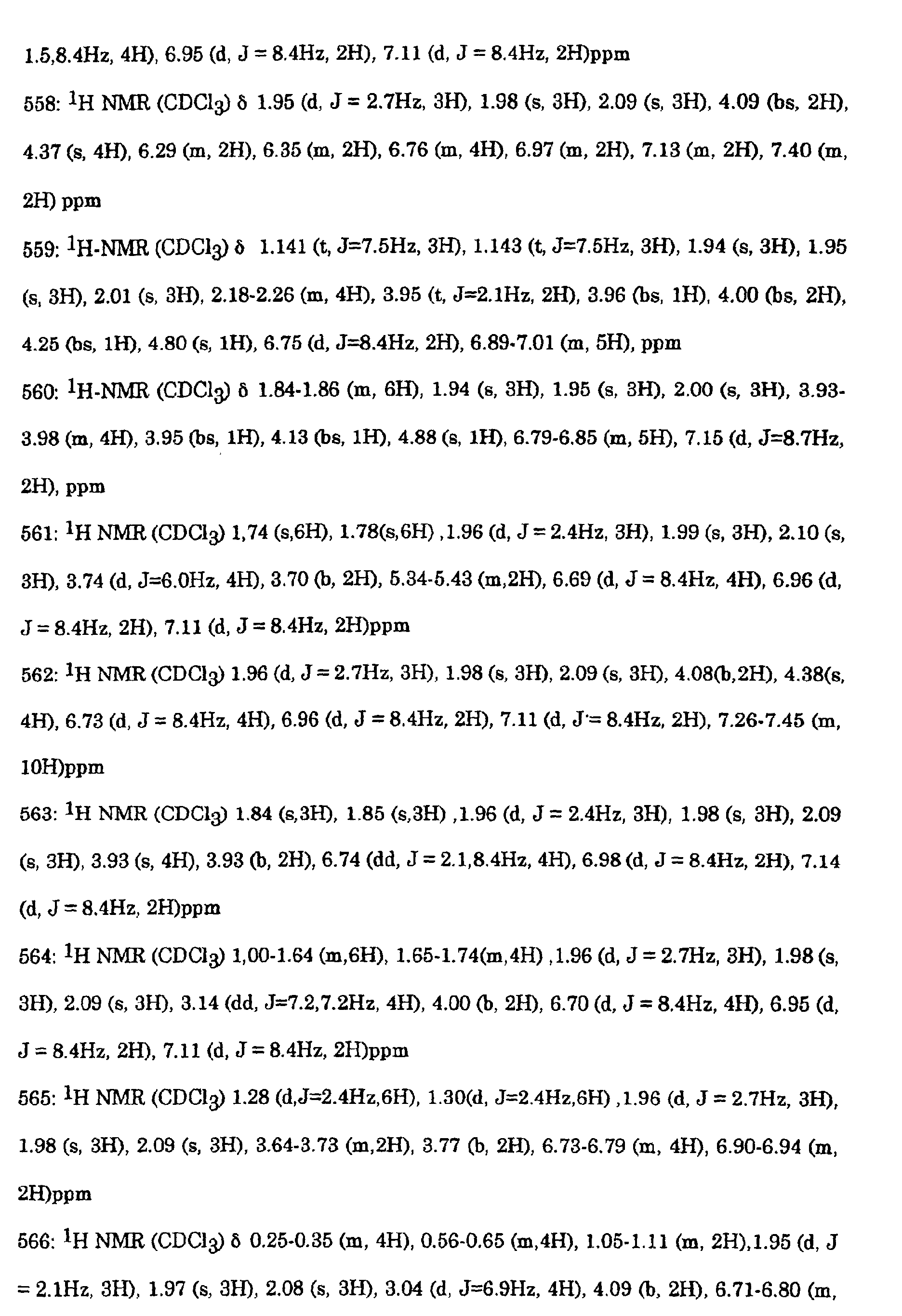

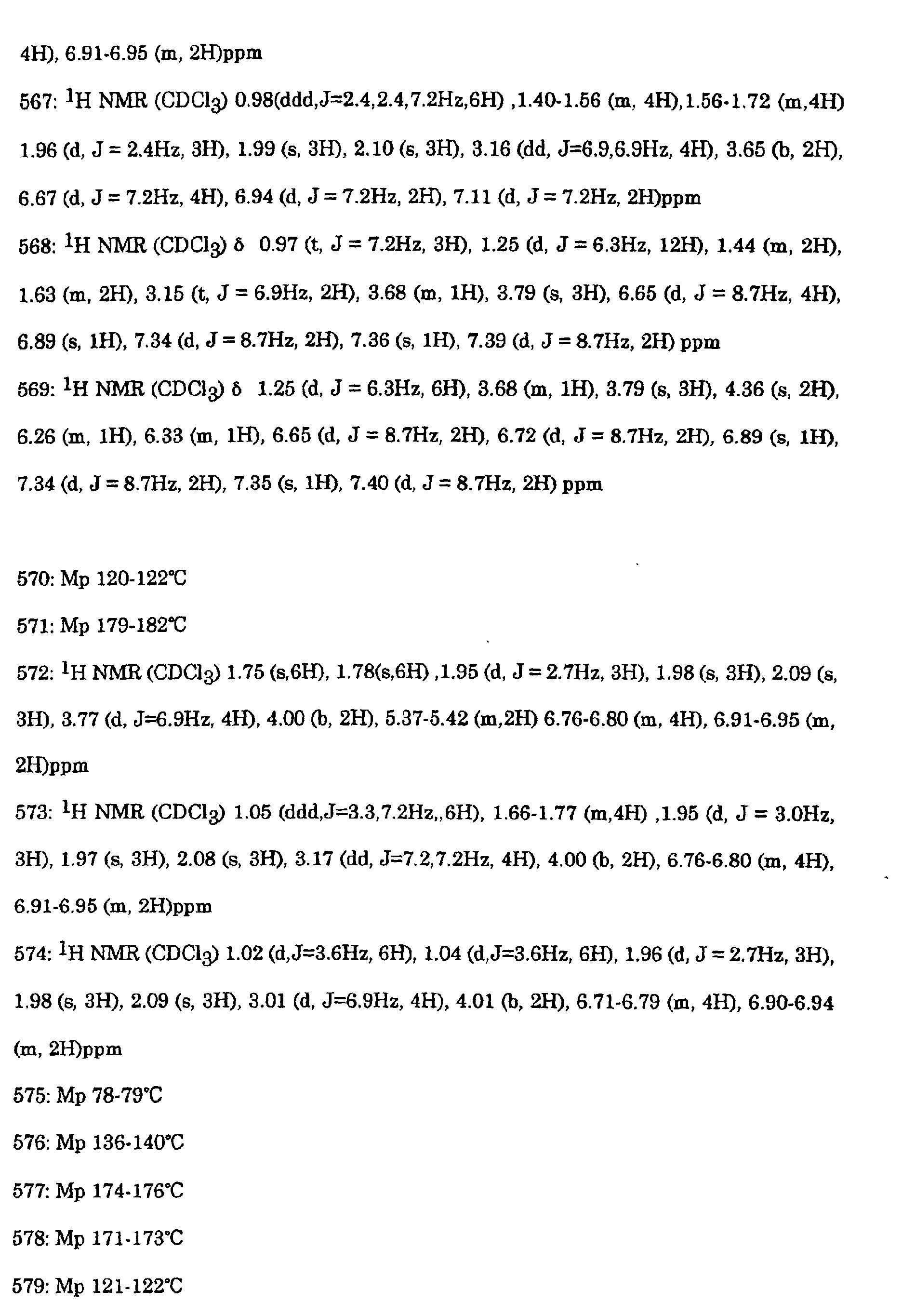

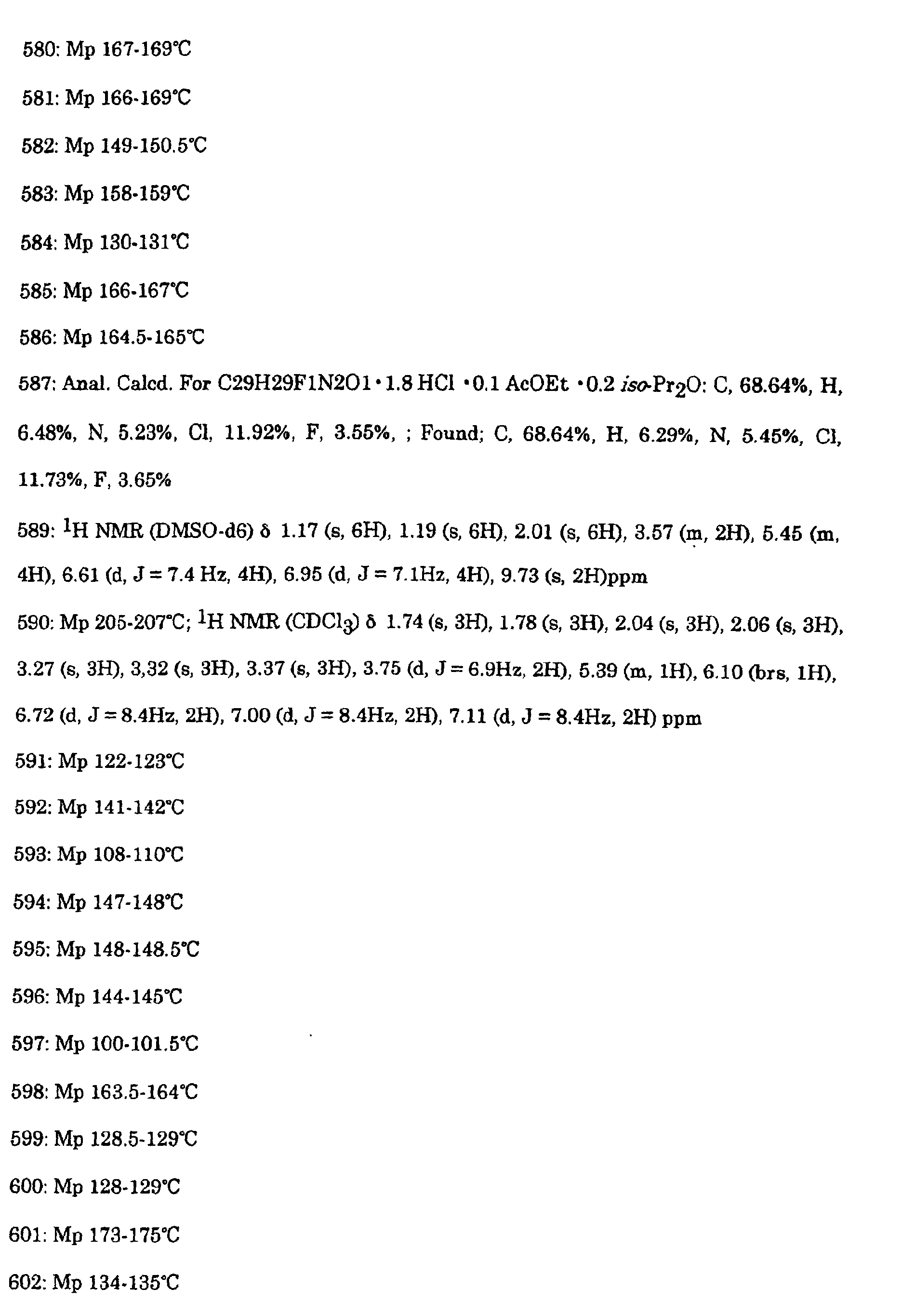

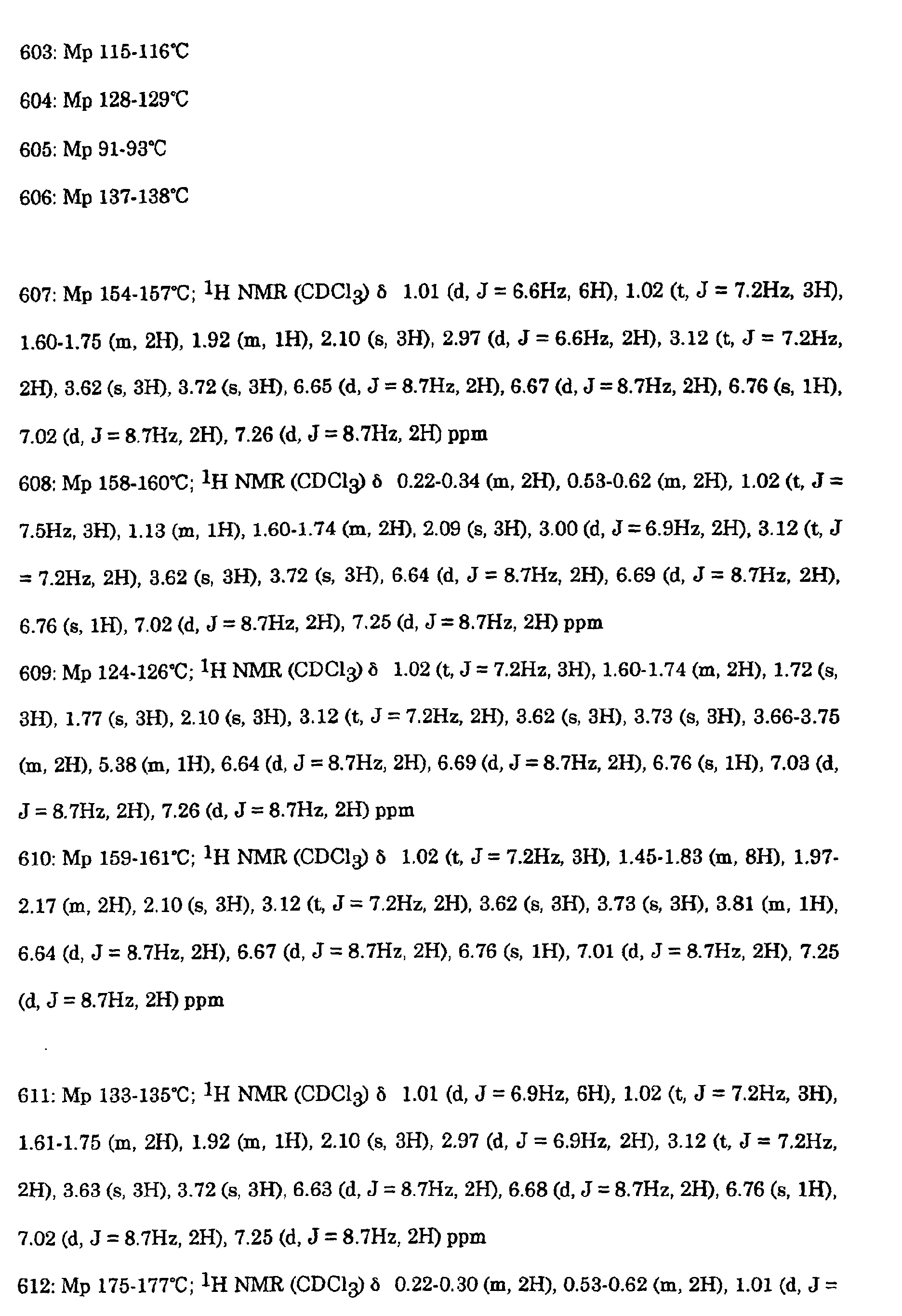

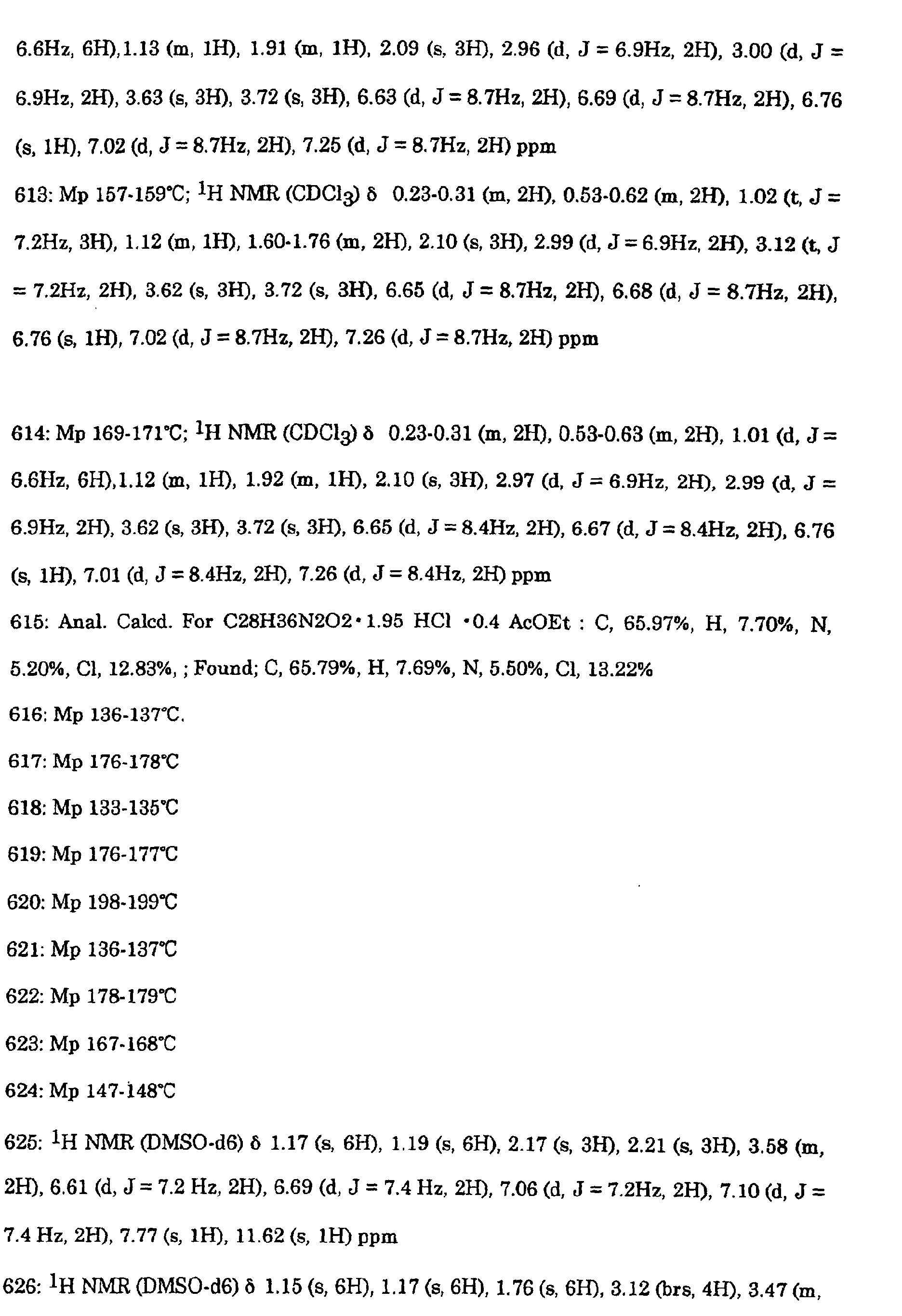

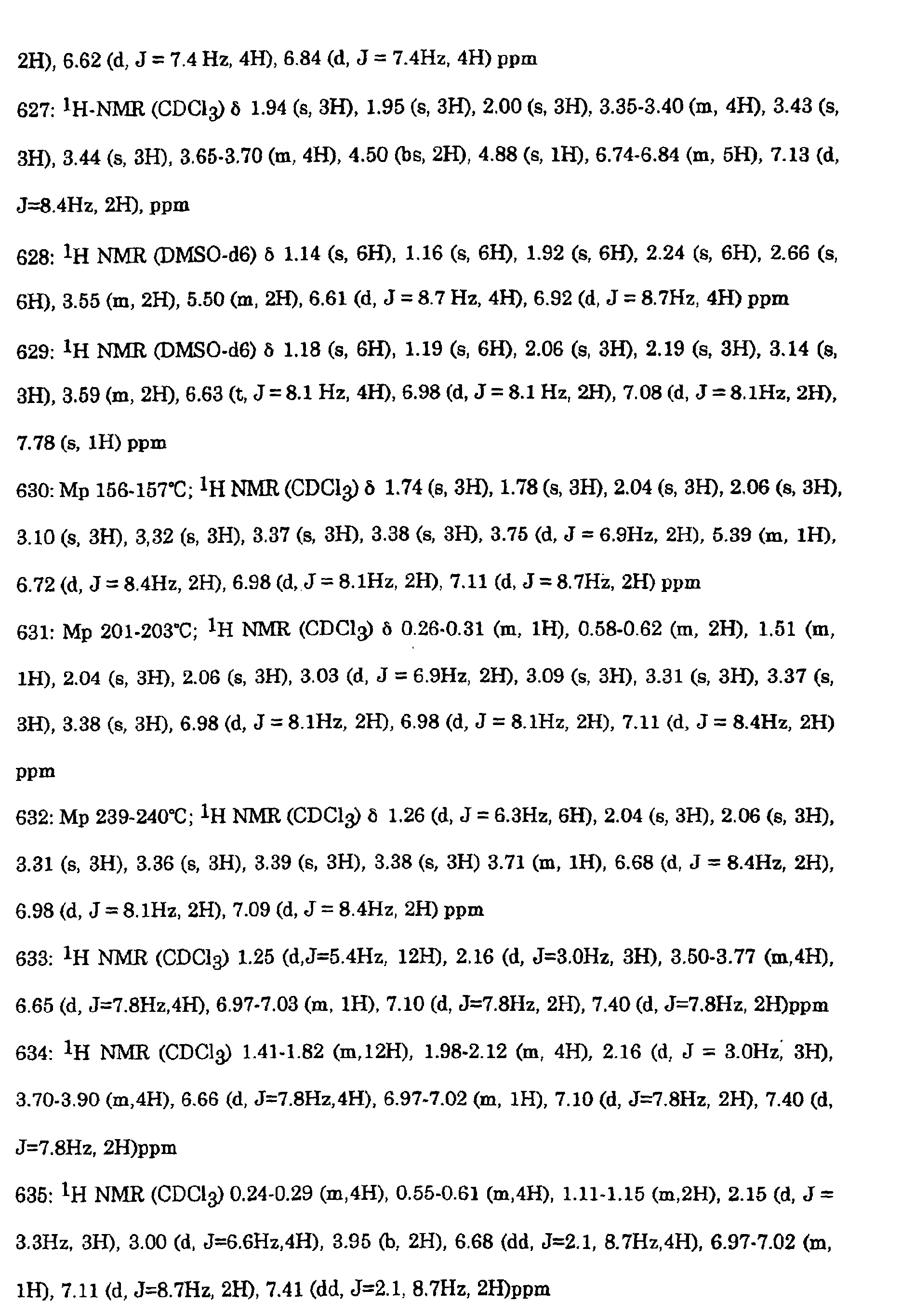

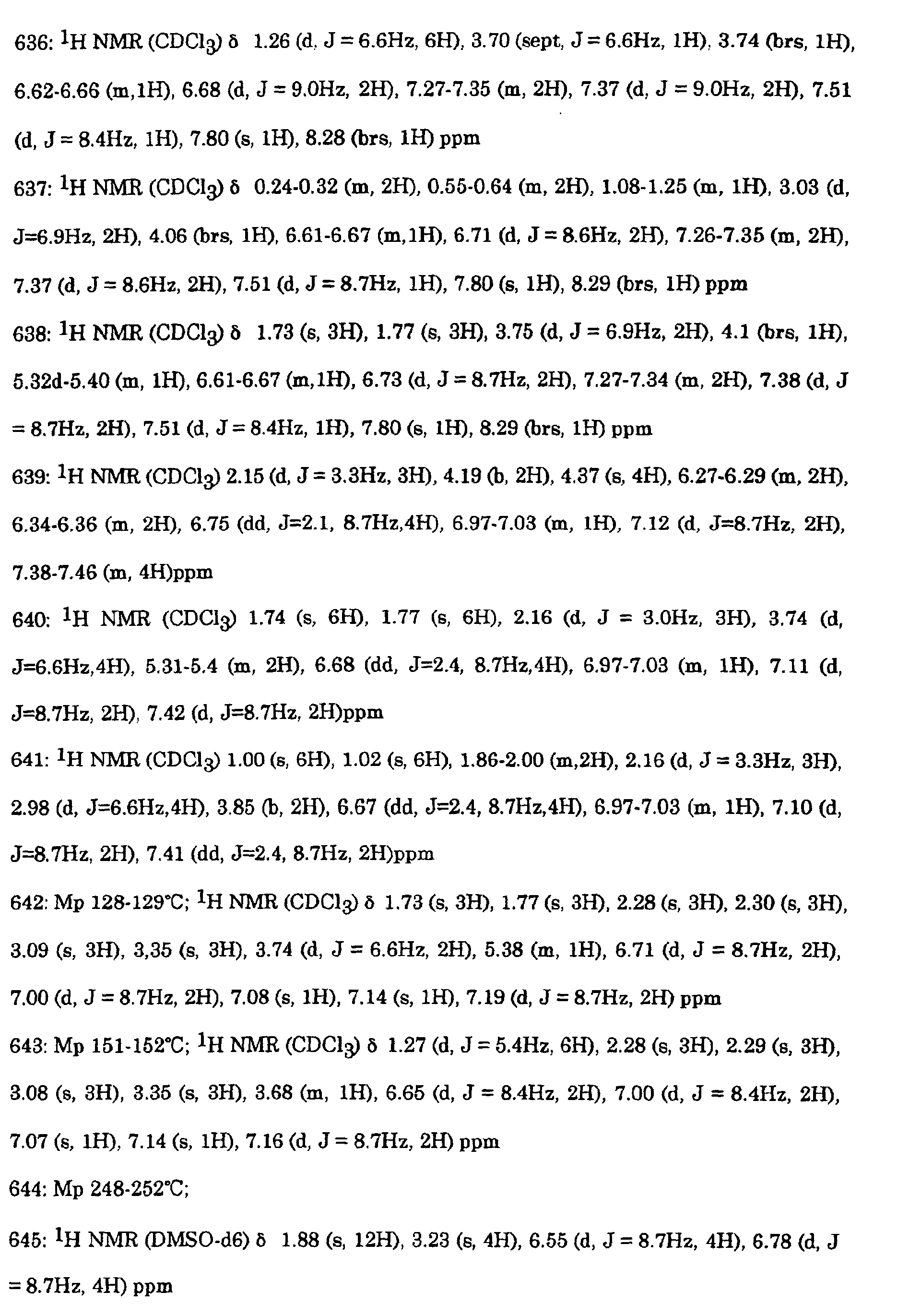

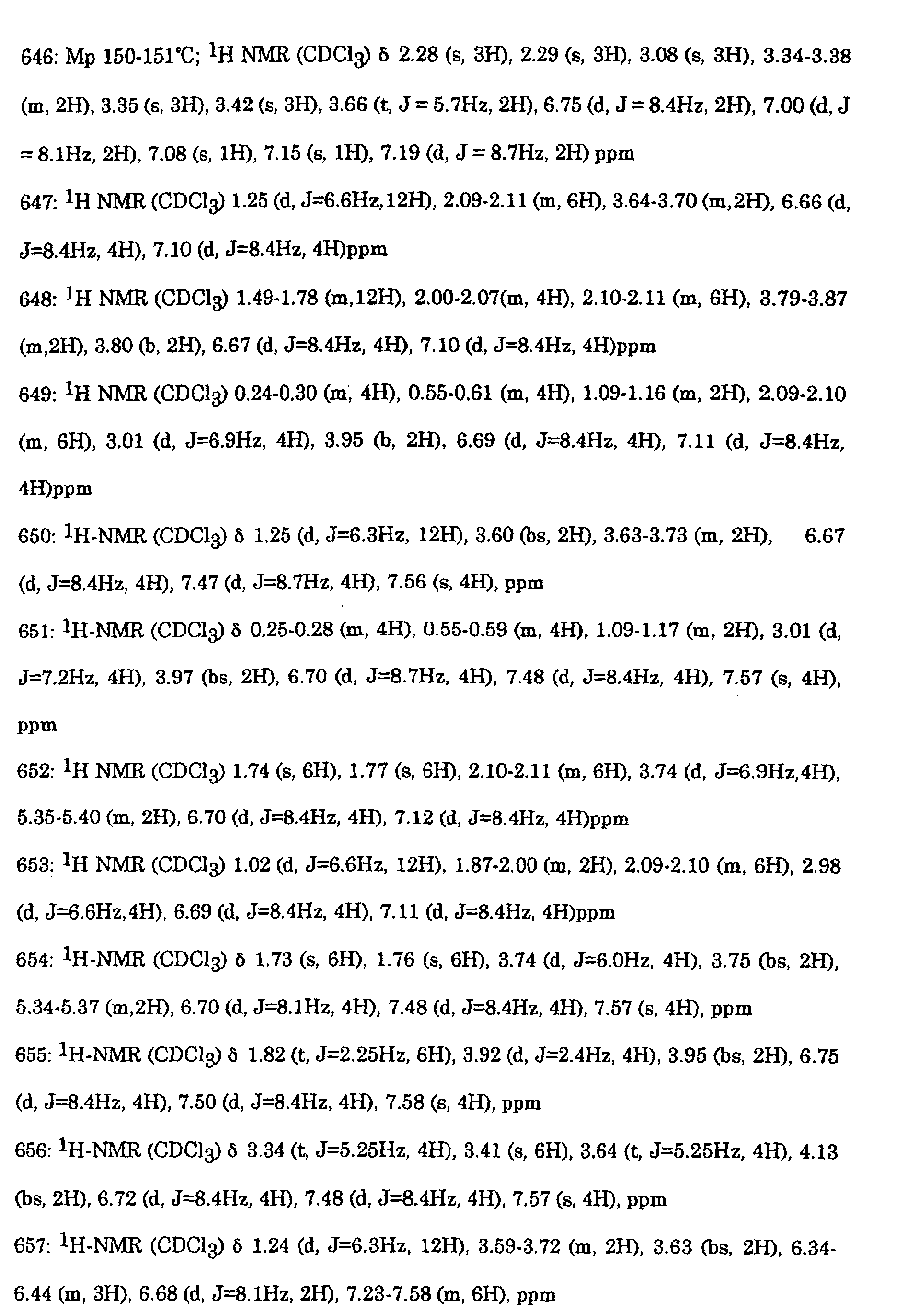

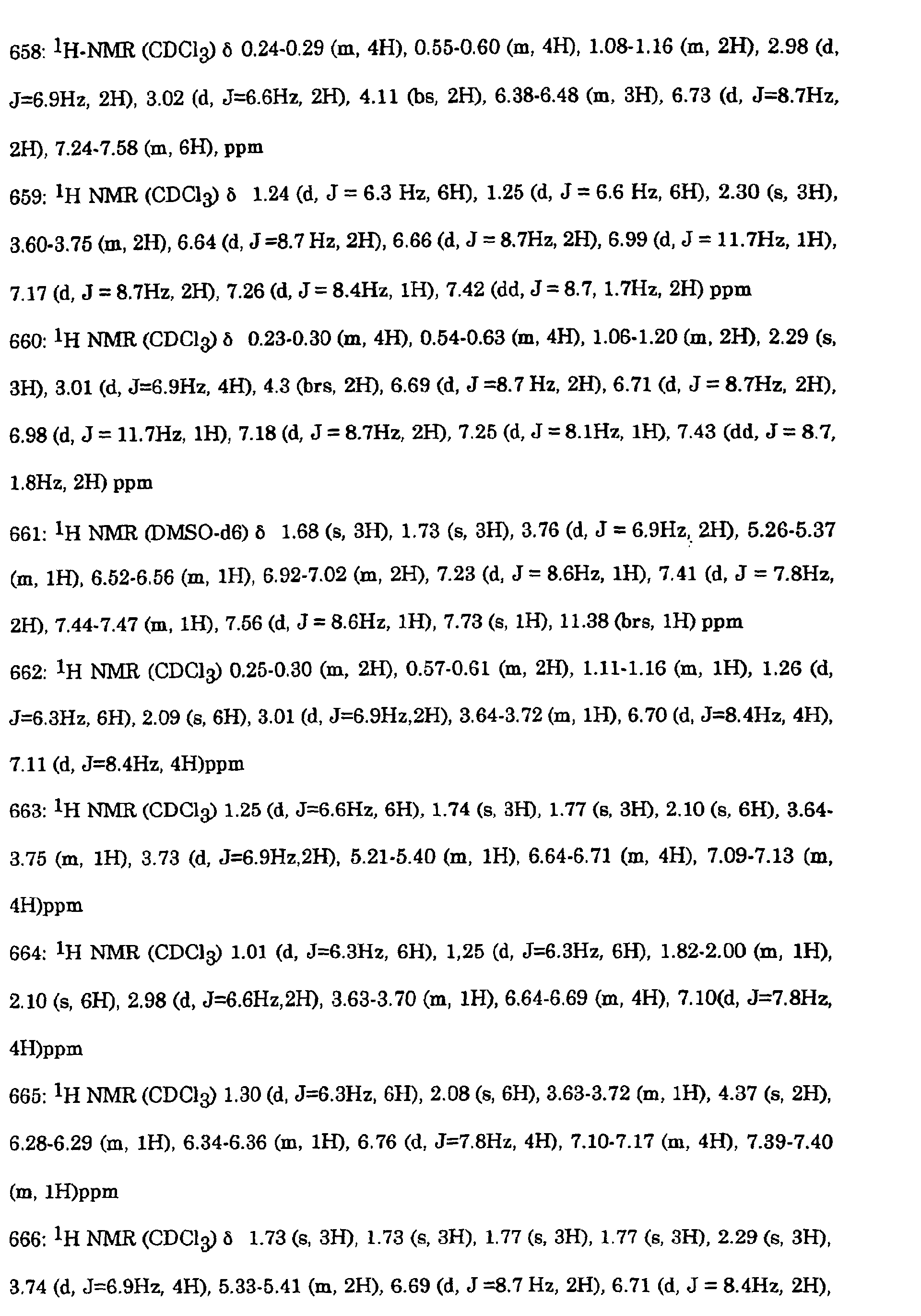

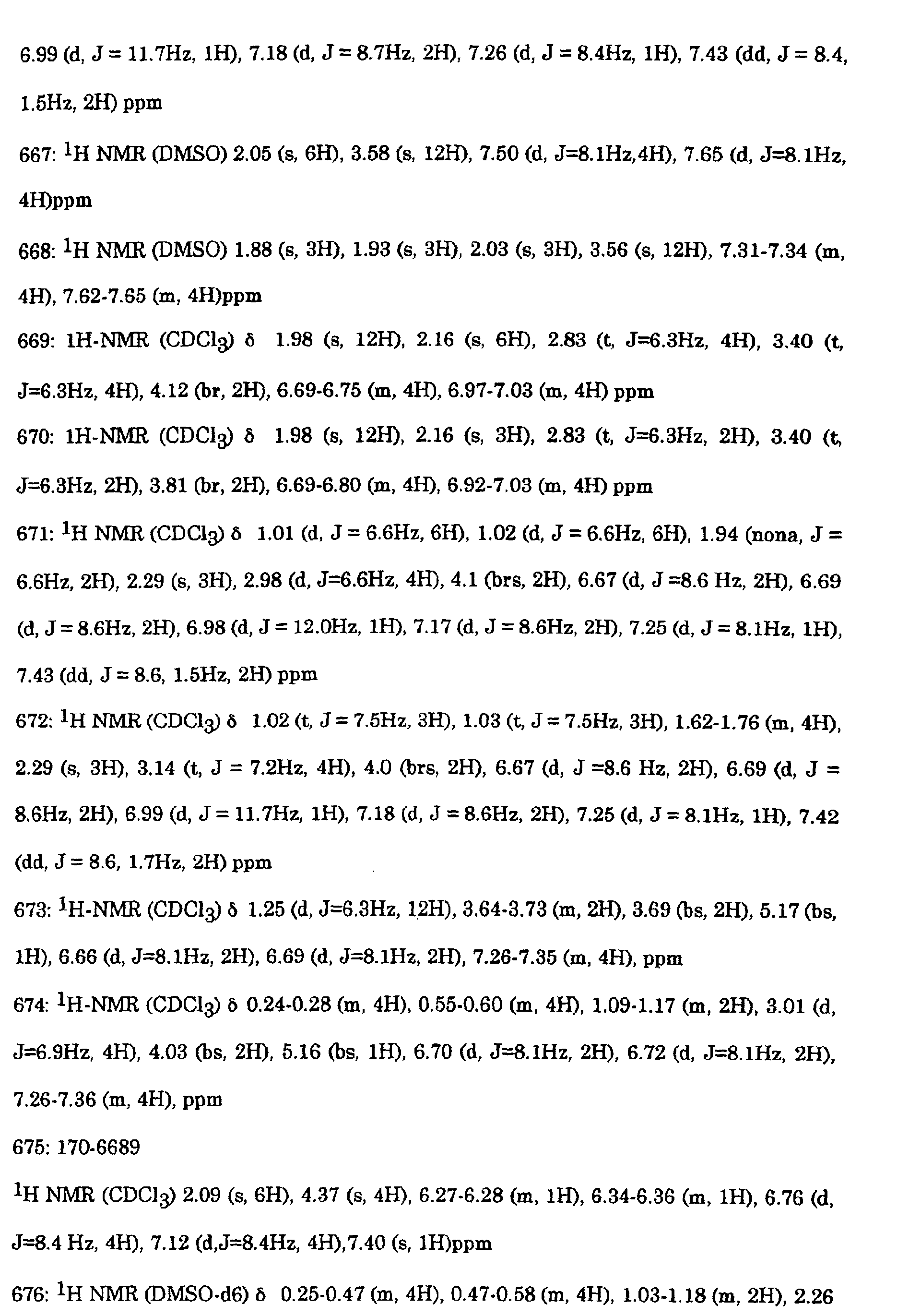

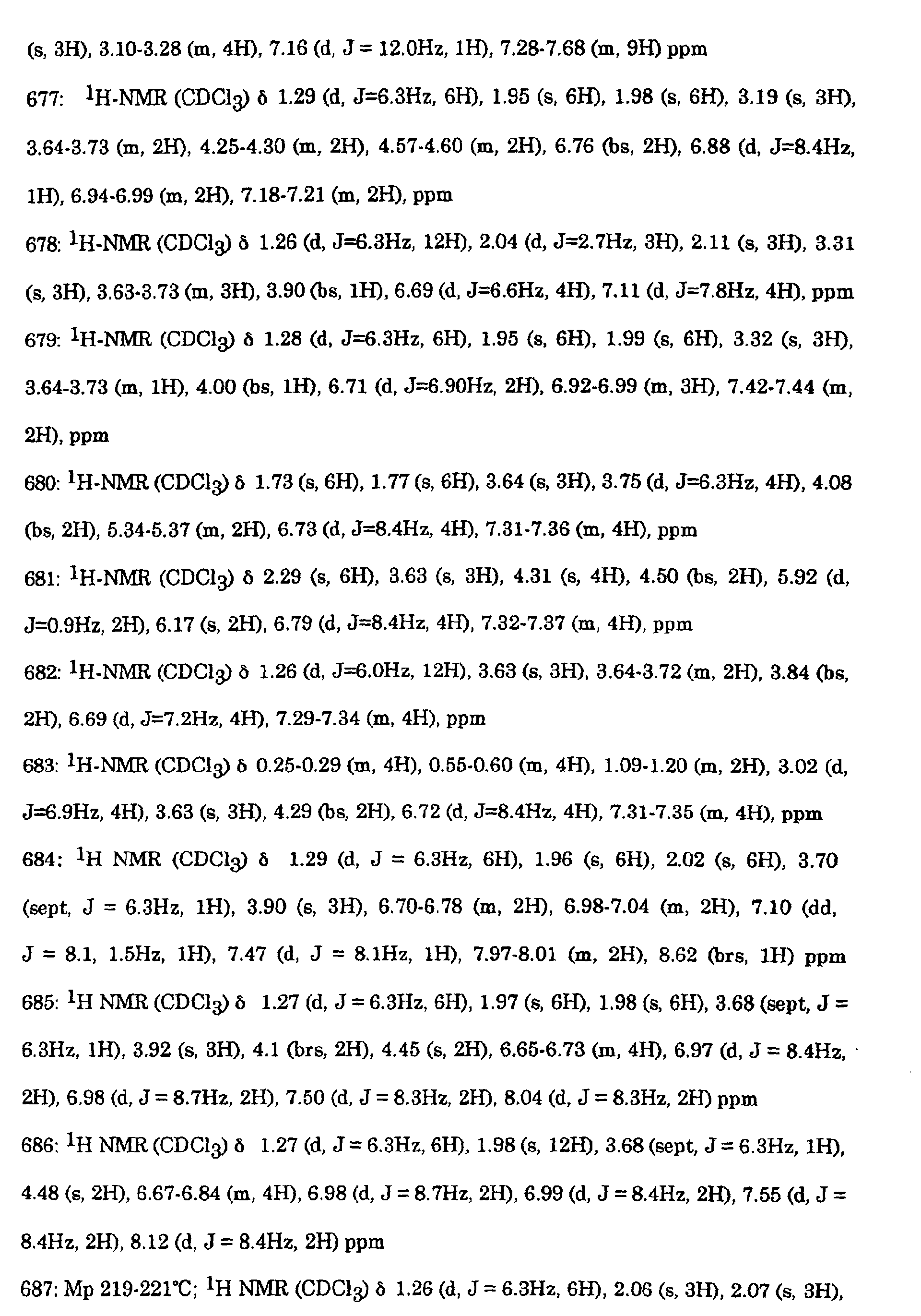

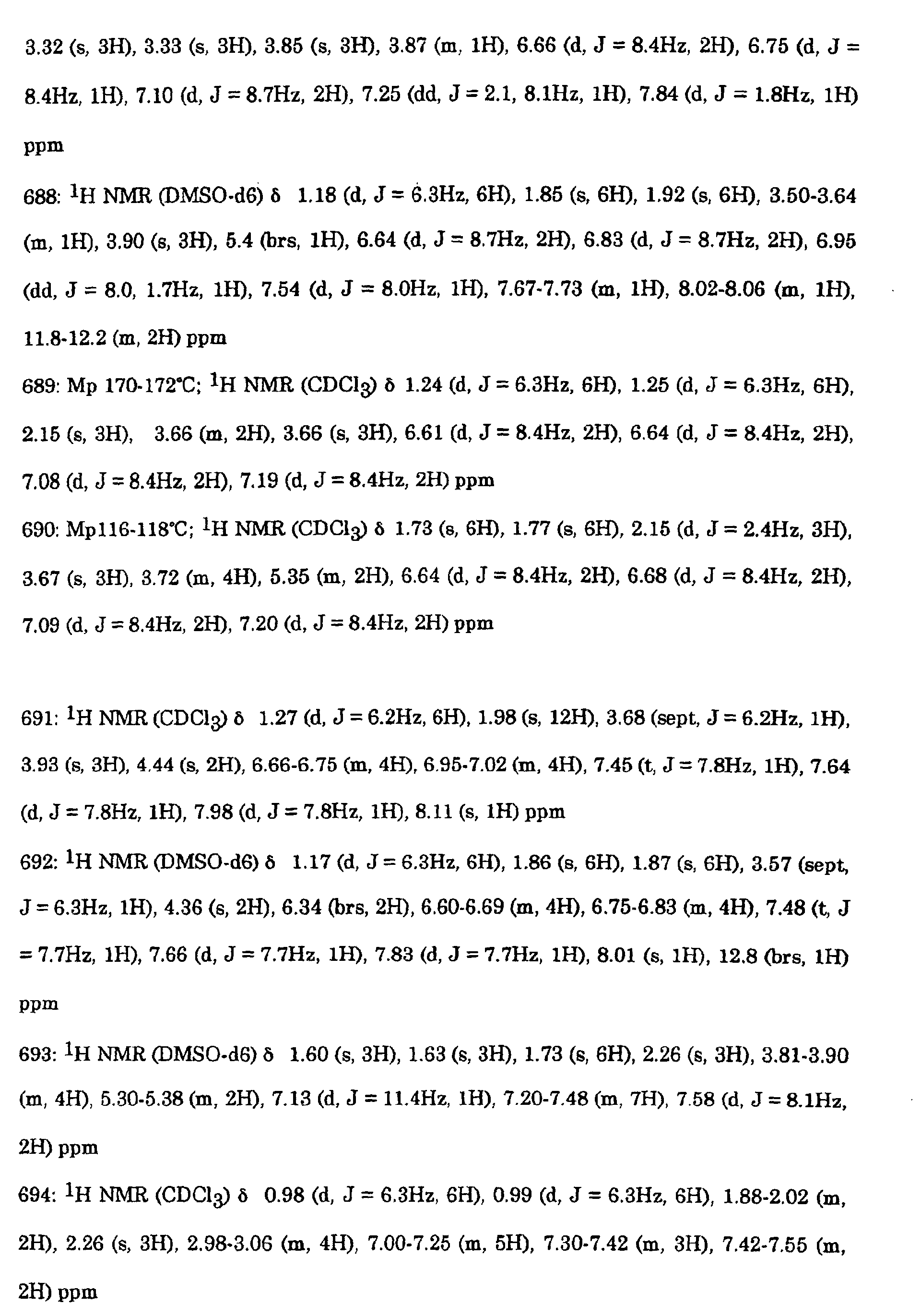

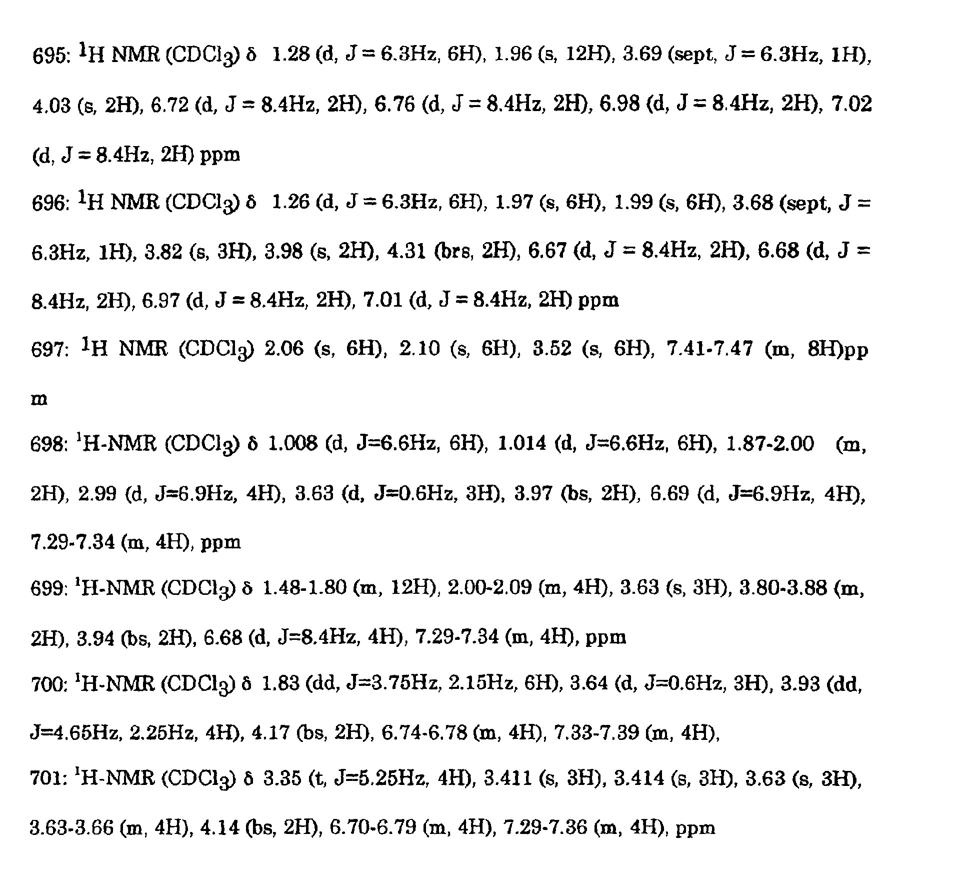

### Experiment 1 Suppressive effect on the IgE production against ovalbumin(OVA)

### 1) Animals

BALB/c mice (female, 8-10 weeks old) and Wistar rats (female, 8-10 weeks old) which were bought from Japan SLC, Inc. (Shizuoka) were used.

### 2) Immunizing method

BALB/c mice were immunized by an intraperitoneal administration of 0.2 ml suspension of 2 µg of ovalbumin (OVA) and 2 mg of aluminium hydroxide gel in physiological saline. After 10 days, blood was collected from hearts, then sera were separated and stocked at -40 °C till the measurement of IgE antibody titer.

### 3) Compounds

After the compound of the present invention was dissolved or suspended in N, N-dimethylacetoamide, the mixture was diluted 20 times with miglyol 812 neutral oil. The obtained solution was orally administered to mice at 0.1 ml per mouse (dose 40 mg/kg). The administration was continued for 10 days from the immunizing day to the day before the blood collection.

### 4) Measurement of anti-OVA IgE antibody titer (PCA titer)

The obtained mouse serum was 2-fold diluted with physiological saline, then each 50 µl of the solution was intradermally injected at dorsal skin of Wistar rats which previously hair cut. After 24 hours, a passive cutaneous anaphylaxis reaction (PCA) was induced by an intravenous injection of 0.5 ml of physiological saline containing 1 mg of OVA and 5 mg of Evans' blue dye. The rats were sacrified 30 minutes later and the highest dilution giving bluing with a diameter of 5 mm or more was recorded as the PCA titer. For example, when a serum is positive for the PCA reaction till 2⁷ times dilution, the anti-OVA IgE antibody titer of the mouse is defined as 7. The results are shown in Table 49.

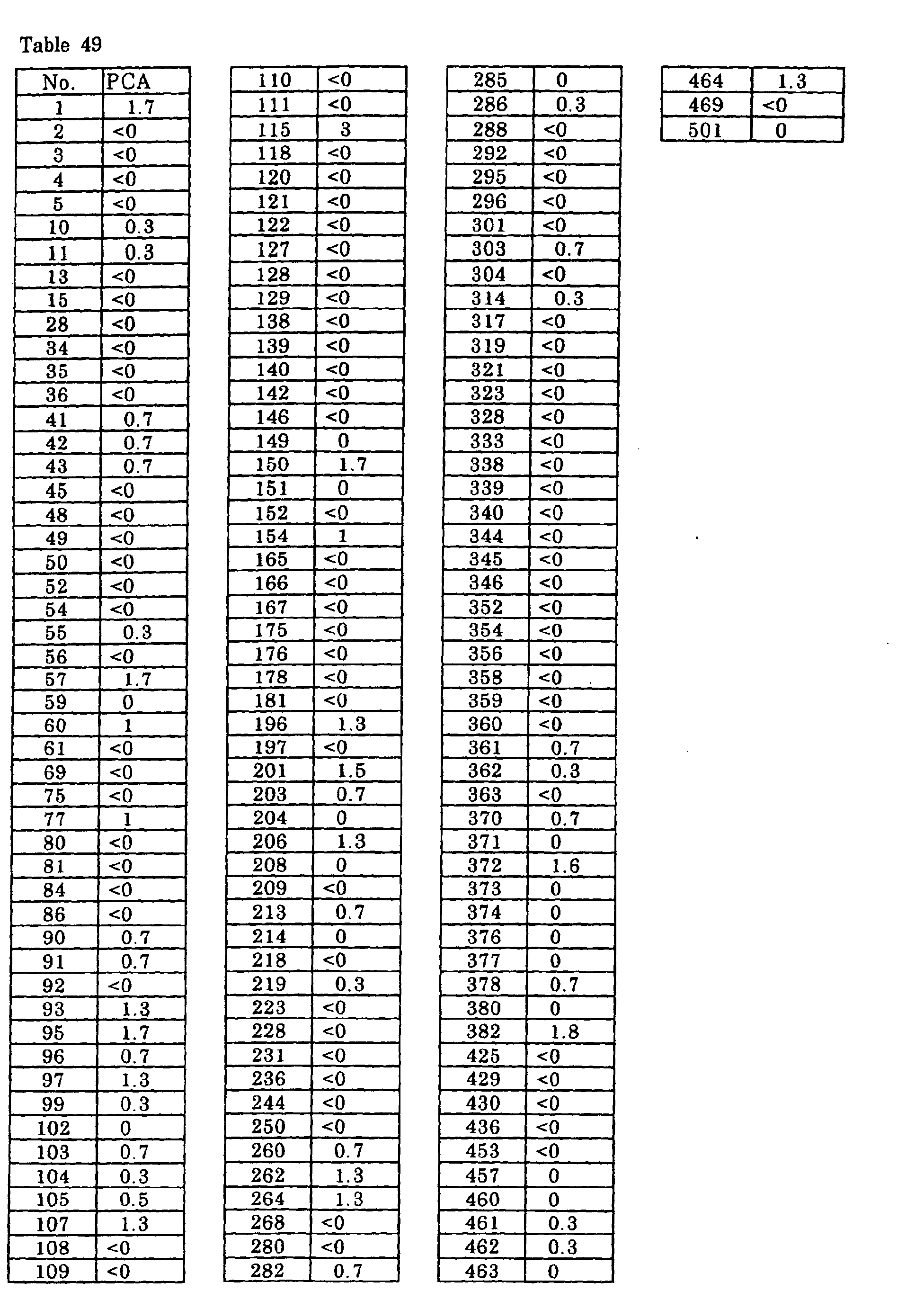

As shown in the above, the compound of the present invention has a suppressive effect on the IgE production.

### Experiment 2 Inhibitory effect on induction of differentiation from Th0 cells to Th2 cells

### 1. Animals

In an experiment to induce the differentiation from Th0 cells to Th2 cells, 9 to 11 week-old female mice of BALB/cCrSlc purchased from Japan SLC, Inc. or of BALB/cAnNCrj purchased from Charles River Japan, Inc. were used.

In an experiment to induce the differentiation from Th0 cells to Th1 cells, 8 week-old female mice of C57BL/6NCrj purchased from Charles River Japan, Inc. were used.

### 2. Immunization and administration of inventive compounds

In an experiment to induce the differentiation from Th0 cells to Th2 cells, a DNP-As (dinitrophenylated Ascaris protein (porcine ascarid extract protein)) was used as an antigen, 10 µg of which and 250 µg of Alum (aluminum hydroxide adjuvant) in physiological saline in the final volume of 50 µl was injected into the soles of the both hind legs of each mouse, whereby effecting immunization. In a negative control group, each animal was treated similarly by an injection of 50 µl of physiological saline. 6 Days after injection, two popliteal lymph nodes were removed from right and left knees, and passed through a metal mesh (200 mesh size) in Hanks' balanced salt solution (HBSS) to prepare a cell suspension. In the group treated with physiological saline, preparations from two animals were combined and subjected to the experiment because of a small cell number. A compound according to the invention was suspended in 0.5% methylcellulose (MC) and 0.1 ml per 20 g mouse was given orally every day over a period from the day of immunization through the 5th day. The immunized vehicle control group and the negative control group were treated with the same volume of 0.5% MC.

On the other hand, in an experiment to induce the differentiation from Th0 cells to Th1 cells, a non-viable Mycobacterium tuberculosis H37RA (DIFCO) was suspended in physiological saline, and 125 µg/50 µl was used for immunization as described above. In the group treated with physiological saline, the preparations from 4 animals were combined and subjected to the experiment.

### 3. Intracellular cytokines detection by FACS method

Cells prepared from popliteal lymph nodes of these mice were suspended at 1 - 2 x 10⁶ cells/ml (1-2 ml) in an RPMI 1640 medium (containing 10% fetal bovine serum - FBS and 50 µM 2-mercaptoethanol) and supplemented with PMA (Phorbol 12- Myristate 13-Acetate) at the final concentration of 50 ng/ml and 250 ng/ml A23187 (Ca ionophore) and then incubated at 37°C in the presence of 5% CO₂. After incubating for 4 hours, Brefeldin A was added at the final concentration of 10 µg/ml and the mixture was incubated further for 2 hours. The cells were recovered and washed twice with a staining buffer (PBS containing 1% FBS and 0.1% sodium azide) and suspended in 100 µl of the staining buffer containing 5 µg/ml of Fc Block (rat anti-mouse CD16/CD32 purified monoclonal antibody, Pharmingen) and incubated on ice for 5 minutes to block the non-specific adsorption of the labeled antibody, and then combined with an equal volume of a Cy-Chrome-labelled rat anti-mouse CD4 monoclonal antibody (Pharmingen) which had been 200-fold diluted with the staining buffer and incubated on ice for 30 minutes. After washing three times with the staining buffer, the cells were suspended in PBS and combined with an equal volume of a fixation solution (4% p-formaldehyde) and incubated at 4°C overnight, whereby effecting the fixation. The fixed cells thus obtained were washed twice with the staining buffer and suspended in a permeabilazation buffer (PBS containing 1% FBS, 0.5% saponin and 0.1% sodium azide) and incubated on ice for 10 minutes, and then each cell sample was recovered as being divided into two equal portions. Each portion was suspended in 100 µl of the permeabilazation buffer containing 5 µg/ml Fc Block and incubated on ice for 5 minutes to block the non-specific adsorption of the labeled antibody into the cells. One portion was combined with each 100 µl of an FITC-labeled rat anti-mouse IFNγ monoclonal antibody (Pharmingen) and a PE-labeled rat anti-mouse IL-4 monoclonal antibody (Pharmingen) each had been 50-fold diluted with the permeabilazation buffer and incubated on ice for 30 minutes. The other portion, serving as a control representing the non-specific adsorption of the labeled antibody, was combined with each 100 µl of each control antibody at the same concentration, i.e., an FITC-labeled rat IgG1κ purified antibody (Pharmingen) and a PE-labeled rat IgG1κ purified antibody (Pharmingen) to effect the similar staining. After washing three times with the cell membrane permeation buffer and twice with the staining buffer, the cells were suspended in 500 µl of the staining buffer and transferred through a nylon mesh into a FACS analysis tube.

FACScan (Nippon Becton Dickinson Company Ltd.) was used to determine the percentage of IFNγ positive cells (Th1) and the percentage of IL-4 positive cells (Th2) in CD4 positive T cells, from each of which the percentage of non-specific positive cells stained by a control antibody was subtracted to obtain % Th1 and % Th2, while in the Th2 differentiation experiment a Th2/Th1 cell ratio was determined, and then based on these data the effect of a compound according to the invention of the differentiation from Th0 to Th1 or Th2 was investigated. The significant difference was analyzed by Dunnett multiple comparison test and Student t test. The results are shown in Table 50.

**Table 50**

| Effect of compounds on change in %Th1, %Th2 and Th2/Th1 in popliteal lymph node of BALB/c mice 6 days after immunization with DNP-As | | | | | |
|---|---|---|---|---|---|
| Immunization method | Compound | Dose (mg/kg) | %Th2 | %Th1 | Th2/Th1 |
| Physiological saline | | | 0.09±0.01## | 0.14±0.03 | 0.74±0.20## |
| DNP-As/Alum | Control | | 2.24±0.31 | 0.21±0.02 | 11.16±2.14 |
| | 81 | 30 | 0.42±0.17** | 0.18±0.04 | 2.08±0.65* |

| | | | | | |
|---|---|---|---|---|---|
| % Th1 and % Th2 are the values after subtracting the percentage positive for the negative control antibody (n=4). *p<0.05, **p<0.01 vs control group (Dunnett's test), #p<0.05, ##P<0.01 vs control group (Student's t-test). | | | | | |

### Results

As shown in Table 50, the CD4 positive T cells in the popliteal lymph node of BALB/c mice immunized with DNP-As exhibited an increase in the % Th2 and in the Th2/Th1 ratio when compared with the non-immunized group received the injection only of physiological saline, thus validating the induction of the Th2-dominant differentiation. Against such induction, each of Compound 81 at 30 mg/kg significantly inhibited the increase in the %Th2 and in the Th2/Th1 ratio when given orally for consecutive 6 days after immunization, resulting in the correction from the Th2-dominant condition.

Accordingly, the compounds of the invention were revealed to have the inhibitory effect on the differentiation from Th0 to Th2.

### Formulation Example 1

| | |
|---|---|
| The compound of the present invention (Ia-1) | 15 mg |
| Starch | 15 mg |
| Lactose | 15 mg |
| Crystalline cellulose | 19 mg |
| Polyvinyl alcohol | 3 mg |
| Distilled water | 30 ml |
| Calcium stearate | 3 mg |

After all of the above ingredients except for calcium stearate were uniformly mixed, the mixture was crushed and granulated, and dried to obtain a suitable size of granules. After calcium stearate was added to the granules, tablets were formed by compression molding.

### Industrial Applicability

As explained in the above experiments, the compounds of the present invention have potent IgE production suppressive and Th2 differentiation inhibitory activities. The compounds of the present invention are useful as an immunosuppressant and/or an anti-allergic agent.

## Claims

1. A compound of the formula (I): 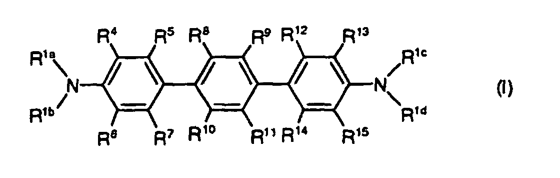 wherein R^{1a}, R^{1b}, R^{1c} and R^{1d} are each independently
hydrogen;
lower alkyl optionally substituted with at least one group selected from the group of (i)hydroxy; (ii)lower alkylthio; (iii)lower alkoxy; (iv)cycloalkyl; (v)lower alkoxycarbonyl; (vi)optionally substituted heterocyclyl wherein the substituents are lower alkyl; (vii)optionally substituted aryl wherein the substituents are lower alkoxycarbonyl and/or carboxy;
(viii)carboxy; and
(ix)amino;
lower alkenyl;
lower alkynyl;
acyl optionally substituted with halogen;
lower alkylsulfonyl optionally substituted with halogen;
or cycloalkyl,
R⁴ and R^{1a} taken together may form optionally substituted C2 or C3 alkenylene or optionally substituted C2 or C3 alkylene,
R¹³ and R^{1c} taken together may form optionally substituted C2 or C3 alkenylene or optionally substituted C2 or C3 alkylene,
R⁸ and R⁹ taken together may form optionally substituted C2 or C3 alkenylene diamino or optionally substituted C2 or C3 alkylene diamino,
R¹⁰ and R¹¹ taken together may form optionally substituted C2 or C3 alkenylene diamino or optionally substituted C2 or C3 alkylene diamino, and
R⁴, R⁵, R⁶, R⁷, R⁸, R⁹, R¹⁰, R¹¹, R¹², R¹³, R¹⁴ and R¹⁵ are each independently hydrogen, halogen, hydroxy, optionally substituted lower alkyl, optionally substituted lower alkoxy, optionally substituted lower alkenyl, optionally substituted lower alkenyloxy, optionally substituted cycloalkyloxy, optionally substituted acyl, optionally substituted acyloxy, carboxy, optionally substituted lower alkoxycarbonyl, optionally substituted lower alkenyloxycarbonyl, optionally substituted lower alkylthio, optionally substituted lower alkenylthio, optionally substituted amino, optionally substituted carbamoyl, optionally substituted sulfamoyl, guanidino, nitro, cyano, optionally substituted lower alkylsulfonyl, optionally substituted lower alkylsulfonyloxy, optionally substituted arylsulfonyl, optionally substituted lower alkylsulfinyl or optionally substituted arylsulfonyloxy,
a prodrug, a pharmaceutically acceptable salt or solvate thereof.

2. The compound as claimed in claim 1 wherein R^{1a} is lower alkylsulfonyl or 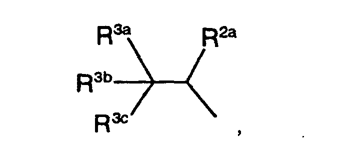 R^{1c} is lower alkylsulfonyl or 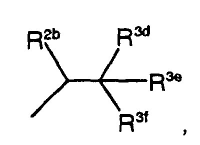 R^{1b} and R^{1d} are each independently hydrogen, lower alkyl or acyl,
R^{2a} and R^{2b} are each independently hydrogen or lower alkyl,
R^{3a} and R^{3d} are each independently hydrogen, lower alkyl or lower alkenyl,
R^{3b}, R^{3e}, R^{3c} and R^{3f} are each independently hydrogen or lower alkyl,
R^{3a} and R^{2a} or R^{3b} taken together may form optionally substituted alkylene, and
R^{3d} and R^{2b} or R^{3e} taken together may form optionally substituted alkylene excluding N⁴,N⁴"-diisopropyl -2',3',5',6'-tetramethyl -[1,1';4',1"]-terphenyl -4,4"-diamine, N⁴,N⁴"-diisopropyl -2',6'-dimethoxy-3',5'-dimethyl-[1,1';4',1"]-terphenyl -4,4"-diamine, N⁴,N⁴"-diisopropyl -2'-methoxy-3',5',6'-trimethyl-[1,1;4',1"]-terphenyl -4,4"-diamine, N⁴,N^{4"}-diisopropyl -2',5'-dimethyl-[1,1';4',1"]-terphenyl-4,4"-diamine, N⁴,N⁴"-diisopropyl -2'-hydroxy -3',5',6'-trimethyl-[1,1';4',1"]-terphenyl-4,4"-diamine and N⁴,N⁴"-diisopropyl -2',3',5'-trimethyl -[1,1';4',1"]-terphenyl -4,4"-diamine,
a prodrug, a pharmaceutically acceptable salt or solvate thereof.

3. The compound as claimed in claim 1 or 2 wherein at least one of R⁸, R⁹, R¹⁰ and R¹¹ is hydroxy, optionally substituted lower alkyl, optionally substituted lower alkoxy, optionally substituted lower alkenyl, optionally substituted lower alkenyloxy, optionally substituted cycloalkyloxy, optionally substituted acyl, optionally substituted acyloxy, carboxy, optionally substituted lower alkoxycarbonyl, optionally substituted lower alkenyloxycarbonyl, optionally substituted lower alkylthio, optionally substituted lower alkenylthio, optionally substituted amino, optionally substituted carbamoyl, optionally substituted sulfamoyl, guanidino, nitro, cyano, optionally substituted lower alkylsulfonyl, optionally substituted lower alkylsulfonyloxy, optionally substituted arylsulfonyl, optionally substituted lower alkylsulfinyl or optionally substituted arylsulfonyloxy, a prodrug, a pharmaceutically acceptable salt or solvate thereof.

4. The compound as claimed in any one of claims 1 to 3 wherein at least one of R^{1b} and R^{1d} is hydrogen, a prodrug, a pharmaceutically acceptable salt or solvate thereof.

5. The compound as claimed in any one of claims 1 to 3 wherein both of R^{1b} and R^{1d} are hydrogen, a prodrug or a pharmaceutically acceptable salt or solvate thereof.

6. The compound as claimed in any one of 2 to 5 wherein R^{2a} and R^{2b} are each independently hydrogen or C1 to C3 alkyl, a prodrug, a pharmaceutically acceptable salt or solvate thereof.

7. The compound as claimed in any one of claims 2 to 6 wherein R^{3a} and R^{3d} are each independently hydrogen or C1 to C3 alkyl, a prodrug, a pharmaceutically acceptable salt or solvate thereof.

8. The compound as claimed in any one of claims 2 to 6 wherein R^{3a} and R^{2a} or R^{3b} taken together may form unsubstituted alkylene and/or R^{3d} and R^{2b} or R^{3e} taken together may form unsubstituted alkylene, a prodrug, a pharmaceutically acceptable salt or solvate thereof.

9. The compound as claimed in any one of claims 2 to 8 wherein R^{3b} and R^{3e} are each independently hydrogen or C1 to C3 alkyl, a prodrug, a pharmaceutically acceptable salt or solvate thereof.

10. The compound as claimed in any one of claims 2 to 9 wherein R^{3c} and R^{3f} are each independently hydrogen or C1 to C3 alkyl, a prodrug, a pharmaceutically acceptable salt or solvate thereof.

11. The compound as claimed in claim 2 or 3 wherein both of R^{1b} and R^{1d} are hydrogen,
R^{2a}, R^{2b}, R^{3a}, R^{3d}, R^{3b}, R^{3e}, R^{3c} and R^{3f} are each independently hydrogen or simultaneously C1 to C3 alkyl, and
R^{3a} and R^{2a} or R^{3b} taken together may form unsubstituted alkylene and/or R^{3a} and R^{2b} or R^{3e} taken together may form unsubstituted alkylene, a prodrug, a pharmaceutically acceptable salt or solvate thereof.

12. The compound as claimed in claim 2 or 3 wherein both of R^{1b} and R^{1d} are hydrogen, and R^{2a}, R^{2b}, R^{3a}, R^{3d}, R^{3b}, R^{3e}, R^{3c} and R^{3f} are each independently hydrogen or methyl, a prodrug, a pharmaceutically acceptable salt or solvate thereof.

13. The compound as claimed in any one of claims 1 to 3 wherein both of R^{1a} and R^{1c} are isopropyl, and both R^{1b} and R^{1d} are hydrogen, a prodrug, a pharmaceutically acceptable salt or solvate thereof.

14. The compound as claimed in any one of claims 1 to 12 wherein R⁴ and R⁵ are each independently hydrogen, halogen, lower alkyl or lower alkoxy, and both of R⁶ and R⁷ are hydrogen, a prodrug, a pharmaceutically acceptable salt or solvate thereof.

15. The compound as claimed in any one of claims 1 and 3 to 14 wherein 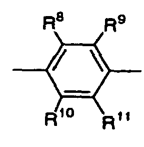 is 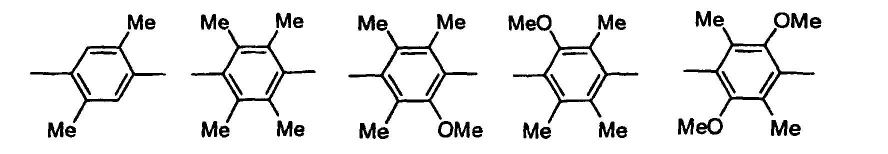 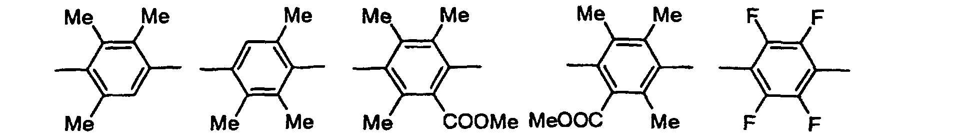 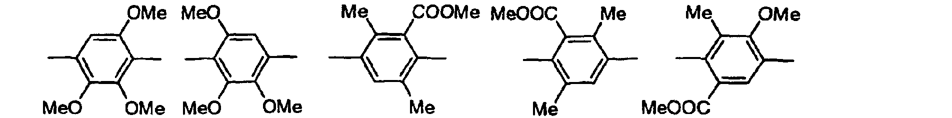 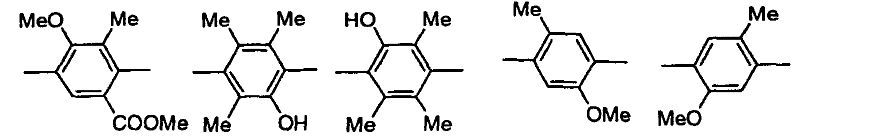 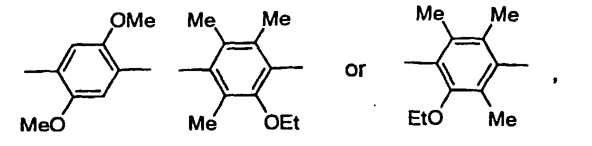 a prodrug, a pharmaceutically acceptable salt or solvate thereof.

16. The compound as claimed in any one of claims 1 and 3 to 14 wherein 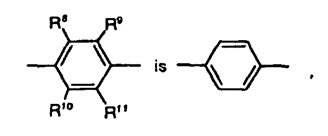 a prodrug, a pharmaceutically acceptable salt or solvate thereof.

17. The compound as claimed in any one of claims 1 and 3 to 14 wherein 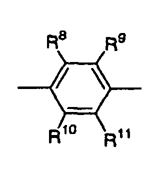 is 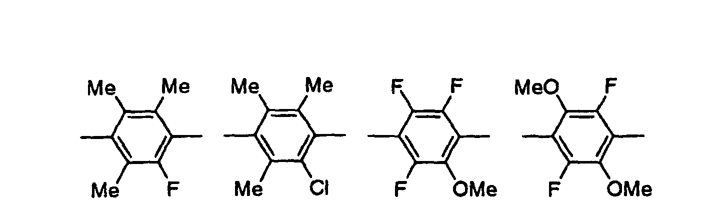 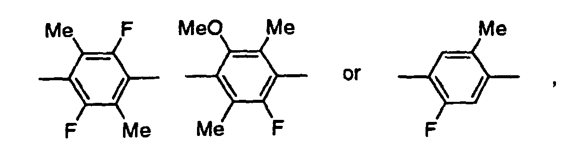 a prodrug, a pharmaceutically acceptable salt or solvate thereof.

18. The compound as claimed in any one of claims 1 to 17 wherein both of R¹² and R¹³ are hydrogen, and R¹⁴ and R¹⁵ are each independently hydrogen, halogen, lower alkyl or lower alkoxy, a prodrug, a pharmaceutically acceptable salt or solvate thereof.

19. The compound as claimed in claim 1 wherein R^{1a} and R^{1c} are each independently C1 to C3 alkyl optionally substituted with C3 to C6 cycloalkyl, R^{1b}, R^{1d}, R⁴ to R⁷ and R¹² to R¹⁵ are hydrogen, and 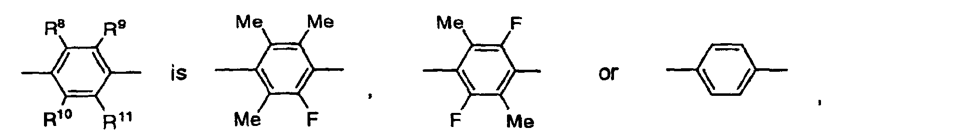 a prodrug, a pharmaceutically acceptable salt or solvate thereof.

20. A pharmaceutical composition comprising a compound, a prodrug, a pharmaceutically acceptable salt or solvate thereof of any one of claims 1 to 19.

21. An IgE production suppressive composition comprising a compound, a prodrug, a pharmaceutically acceptable salt or solvate thereof of any one of claims 1 to 19.

22. A Th2 differentiation inhibitory composition comprising a compound, a prodrug, a pharmaceutically acceptable salt or solvate thereof of any one of claims 1 to 19.

23. An anti-allergic composition comprising a compound, a prodrug, a pharmaceutically acceptable salt or solvate thereof of any one of claims 1 to 19.

24. An immunosuppressive composition comprising a compound, a prodrug, a pharmaceutically acceptable salt or solvate thereof of any one of claims 1 to 19.

25. A method for suppressing IgE production comprising administering a compound, a prodrug, a pharmaceutically acceptable salt or solvate thereof of any one of claims 1 to 19.

26. A method for inhibiting Th2 differentiation comprising administering a compound, a prodrug, a pharmaceutically acceptable salt or solvate thereof of any one of claims 1 to 19.

27. A method for treating and/or preventing an allergic disease comprising administering a compound, a prodrug, a pharmaceutically acceptable salt or solvate thereof of any one of claims 1 to 19.

28. A method for suppressing an immune reaction comprising administering a compound, a prodrug, a pharmaceutically acceptable salt or solvate thereof of any one of claims 1 to 19.

29. Use of a compound, a prodrug, a pharmaceutically acceptable salt or solvate thereof of any one of claims 1 to 19 for preparing a medicine for suppressing IgE production.

30. Use of a compound, a prodrug, a pharmaceutically acceptable salt or solvate thereof of any one of claims 1 to 19 for preparing a medicine for inhibiting Th2 differentiation.

31. Use of a compound, a prodrug, a pharmaceutically acceptable salt or solvate thereof of any one of claims 1 to 19 for preparing an anti-allergic agent.

32. Use of a compound, a prodrug, a pharmaceutically acceptable salt or solvate thereof of any one of claims 1 to 19 for preparing an immunosuppressive agent.
